(19) Europäisches Patentamt
European Patent Office
Office européen des brevets

(11) EP 3 296 298 A1

(12) EUROPÄISCHE PATENTANMELDUNG

(43) Veröffentlichungstag:
**21.03.2018 Patentblatt 2018/12**

(21) Anmeldenummer: **16188728.6**

(22) Anmeldetag: **14.09.2016**

(51) Int Cl.:
***C07D 471/04*** *(2006.01)* ***C07D 491/107*** *(2006.01)*
***A61K 31/4375*** *(2006.01)* ***A61P 9/00*** *(2006.01)*
***A61P 13/00*** *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Bayer Pharma Aktiengesellschaft**
**13353 Berlin (DE)**

(72) Erfinder: **Die Erfindernennung liegt noch nicht vor**

(74) Vertreter: **BIP Patents**
**c/o Bayer Intellectual Property GmbH**
**Alfred-Nobel-Straße 10**
**40789 Monheim am Rhein (DE)**

# (54) 7-SUBSTITUIERTE 1-ARYL-NAPHTHYRIDIN-3-CARBONSÄUREAMIDE UND IHRE VERWENDUNG

(57) Die vorliegende Anmeldung betrifft neue 7-substituierte 1-Aryl-naphthyridin-3-carbonsäureamide, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von kardiovaskulären Erkrankungen und/oder Nierenerkrankungen.



Printed by Jouve, 75001 PARIS (FR)

**Beschreibung**

Die vorliegende Anmeldung betrifft neue 7-substituierte 1-Aryl-naphthyridin-3-carbonsäureamide, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von kardiovaskulären Erkrankungen und/oder Nierenerkrankungen.

Muskarinerge Rezeptoren, sind membranständige Rezeptoren, die als endogenen Liganden den Neurotransmitter Acetylcholin (ACh) binden (Acetylcholinrezeptoren), aber auch von Muskarin aktiviert werden können. Diese G-Protein-gekoppelte Rezeptoren gibt es als fünf Subtypen (M1-M5), die in fast allen Geweben im menschlichen Organismus exprimiert werden. Man findet sie sowohl im zentralen als auch im peripheren Nervensystem sowie in vielen Organen des vegetativen Nervensystems.

Der M2-Typ (M2R) wird überwiegend im Herzen exprimiert. Auf der zellulären Ebene bewirkt eine M2R Stimulation durch den Agonisten Acetylcholin eine Hemmung der Adenylcyclase und eine Aktivierung des einwärtsgleichrichtenden Kaliumkanals (IKACh-Kanal, GIRK engl.: G protein activated inwardly rectifying K+ channel; auch Kir3.x). Hierdurch vergrößert sich die Kaliumleitfähigkeit, was zu einer Hyperpolarisation der Muskelzellen führt. Dementsprechend werden die Zellen schwerer depolarisierbar, was zu einer negativ chronotropen und dromotropen Wirkung führt, so dass die Herzfrequenz sinkt. Der M2R ist der Hauptmediator der parasympathischen Kontrolle der Herzfunktion, die durch den Nervus vagus gesteuert wird. Dabei vermindert der rechte Nervus vagus über den Sinusknoten die Herzfrequenz, der linke erhöht über den Atrioventrikularknoten (AV-Knoten) überwiegend die atrioventrikuläre Überleitungszeit. Insgesamt überwiegt im Vergleich zum Sympathikus der Einfluss des Vagus auf die Ruhefrequenz des Herzens. Die Auswirkungen einer Stimulation von M2R sind somit entgegengesetzt zu denen der beta-adrenergen Stimulation..

Die Aktivierung des M2-Rezeptors durch den endogenen Agonisten Acetylcholin, aber auch durch synthetische Analoga wie Carbachol, Oxotremorin-M oder Iperoxo (Schrage et al., Biochem. Pharmacol. 2014, 90(3), 307-319) erfolgt durch die Bindung des Agonisten an die sog. orthosterische Bindestelle des Rezeptors und einer hierdurch ausgelösten Konformationsänderung des Rezeptors bzw. Stabilisierung der aktiven Rezeptorkonformation. Zu den klassischen natürlich vorkommenden Muskarin-Rezeptor-Agonisten gehören neben dem endogenen Agonisten Acetylcholin (ACh) verschiedene pflanzliche Alkaloide, wie Arecolin, Muscarin, sowie auch Pilocarpin (Neubig et al., Pharmacol Rev., 2003, 55, 597-606). Die orthosterische Bindungstelle aller muskarinergen Acetylcholin-Rezeptoren ist evolutionär stark konserviert und weist eine hohe Sequenz- und Strukturhomologie zwischen den verschiedenen Subtypen auf. Daher sind viele der bekannten Agonisten unselektiv gegenüber den verschiedenen Subtypen der muskarinergen Acetylcholin-Rezeptoren (Kruse et al., Mol Pharmacol., 2013, 84(4), 528-540). Der M2R weist neben einer orthosteri-schen auch eine allosterische Bindungstelle auf (Gregory et al., Current Neuropharmacol., 2007, 5(3), 157-167). Der älteste bekannte allosterische Modulator ist das Gallamin (Clark and Mitchelson, Br. J. Pharmac., 1976, 58, 323-331).

Allosterische Modulatoren weisen gegenüber klassischen, orthosterischen Liganden deutliche Unterschiede auf. Der allosterische Modulator selbst hat keinen direkten Einfluss auf die Rezeptoraktivierung. Durch die Bindung des Allosters kommt es vielmehr zu einer Modulation der Bindungsaffinität und/oder Effektivität des orthosterischen Agonisten. Die Wirkung eines allosterischen Modulators kann sich also nur in der Anwesenheit des endogenen Liganden entfalten. Daraus ergibt sich eine räumliche und zeitliche Spezifität der allosterischen Wirkung (Conn et al., Nat. Rev. Drug Disc., 2009, 8, 41-54; Conn et al, Nat. Rev. Drug. Disc., 2014, 13, 692-708). Darüber hinaus ist der Effekt eines allosterischen Modulators selbstlimitierend, wenn er in hohen Konzentrationen die Bindung des Agonisten stabilisiert. Hieraus wiederum resultiert grundsätzlich ein günstigeres sicherheitspharmakologisches Profil im Vergleich zu Agonisten, da toxische Effekte bedingt durch eine Rezeptorüberaktivierung begrenzt sind (Christopoulos, Mol. Pharmacol., 2014, 86, 463-478).

Die als Kooperativität bezeichnete gegenseitige Beeinflussung von allosterischen und orthosterischen Liganden hinsichtlich Affinität und intrinsischer Aktivität wird von beiden Liganden bestimmt. Im Falle eines positiven allosterischen Modulators des M2R werden die Effekte des ACh (orthosterischer Ligand) verstärkt (positive Kooperativität). Aufgrund ihrer Fähigkeit, Rezeptorkonformationen in Gegenwart eines orthosterischen Liganden zu modulieren, können allosterische Liganden eine Feinabstimmung pharmakologischer Effekte hervorrufen (Wang et al., J. Pharmacol. Exp. Therap., 2009, 331, 340-348). Daraus ist im Falle des positiven allosterischen Modulators des M2R ein vorteilhaftes Wirkungsprofil, reduziertes Nebenwirkungsrisiko und ein Ansatzpunkt für die Entwicklung subtypselektiver Liganden gegenüber einem vollen Agonisten zu erwarten.

Von dem positiv allosterischen M4R und M2R-Liganden LY2119620 (3-Amino-5-chlor-*N*-cyclopropyl-4-methyl-6-[2-(4-methylpiperazin-1-yl)-2-oxoethoxy]thieno[2,3-b]pyridin-2-carboxamid) wurde die Kristallstruktur im Komplex mit dem M2R veröffentlicht. Die allosterische Bindungsstelle des M2R befindet sich räumlich benachbart, aber klar abgegrenzt zur orthosterischen Bindungsstelle und zeigt im Vergleich mit den anderen muskarinergen Rezeptor-Subtypen eine geringere Konservierung bzw. weist größere Sequenzunterschiede auf (Kruse et al., Nature, 2013, 504, 101-106). LY2119620 wurde als ein unselektiver M2R/M4R positiv allosterischer Modulator beschrieben (Croy et al., Molecular Pharmacology, July 2014 86, 1, 106-115; Schober et al., Molecular Pharmacology, July 2014 86, 1, 116-123).

Der M2R spielt als ein Bestandteil des autonomen Nervensystems eine wichtige Rolle in der Pathogenese und Progression von kardiovaskulären Erkrankungen. Autonomes Ungleichgewicht gekennzeichnet durch vagale (parasympathische) Abschwächung und eine Dominanz des sympathischen Nervensystems steht im engen Zusammenhang mit einer erhöhten Morbidität und Mortalität. Die klinische und prognostische Bedeutung des autonomen Ungleichgewichts ist gut dokumentiert in diversen Herz-Kreislauf-Erkrankungen, einschließlich Herzinsuffizienz (HF), Herzrhythmusstörungen, Ischämie /Reperfusion (I / R), Hypertension (He et al., Br. J. Pharmacol. 2014**,** Epub) und chronischer Nierenerkrankung (Ranpuria et al., Nephrol Dial Transplant. 2008, 23(2), 444-4499). Besonders bei Patienten mit Komorbiditäten wie Diabetes kann die autonome Imbalance zu gesteigerter Morbidität und Mortalität beitragen (Vinik et al., Diabet Med., 2011, 28(6), 643-651). Barorezeptor-Reflex-Funktionsstörungen, wie hypertensive Krisen oder Bluthochdruck-Variabilität, als Anzeichen einer Störung des autonomen Nervensystems, begleiten oft die akute Phase des ischämischen oder hämorrhagischen Schlaganfalls (Sykora et al., Stroke, 2009, 40(12), 678-682).

Die oft beobachtete Komorbidität zwischen kardiovaskulären und psychischen Erkrankungen, wie zwischen Herzinsuffizienz und Depression, basiert wahrscheinlich auf gemeinsamen Pathomechanismen, die mit der autonomen Imbalance einhergehen (Halaris et al., Mod Trends Pharmacopsychiatri., 2013, 28, 144-161). Chronischer Stress verschiebt das homöostatische Gleichgewicht des autonomen Nervensystems. Der verminderte Vagotonus trägt zu einem pro-inflammatorischen Status bei, wobei die Neurotransmitter-Regulierung, insbesondere die serotonerge Transmission, beeinträchtigt ist. Mit der autonomen Dysregulation wurden auch andere psychische Erkrankungen in Zusammenhang gebracht, wie z.B. die Aufmerksamkeitsdefizit-/Hyperaktivitätsstörung (ADHS), die sich durch Enthemmung, mangelhafte emotionale Selbstkontrolle, Unaufmerksamkeit und Hyperaktivität kennzeichnet (Rash and Aguirre-Camacho. Atten Defic Hyperact Disord., 2012, 4(4), 167-177).

Eine Stärkung der parasympathischen Aktivität durch einen positiven allosterischen Modulator einschließlich zu erwartender antiinflammatorischer Effekte, Erhöhung des Stickstoffmonoxids (NO), Regulierung des Redox-Zustands, Verbesserung der mitochondrialen Funktion und der Calcium-Regulation könnte daher ein neues Therapieprinizip insbesondere bei Herz-Kreislauferkrankungen darstellen. Es gibt zahlreiche Hinweise, dass die Modulation der parasympathischen Aktivität als potenzielles Therapieziel bei chronischer Herzinsuffizienz in Betracht gezogen werden kann. Vagusnervstimulation bei Hunden mit abgeheiltem Myokardinfarkt konnte die Inzidenz des plötzlichen Herztodes sowie bei chronisch herzinsuffizienten Ratten die Mortalität signifikant senken (De Ferrari, J. Cardiovasc. Transl. Res., 2014, 7(3), 310-320). In einem Hundemodell mit Herzinsuffizienz (LVEF 35%) und implantiertem Vagusstimulator konnte nachgewiesen werden, dass bei der Therapiegruppe im Vergleich zur Sham-Gruppe eine signifikante Verbesserung der linksventrikulären Ejektionsfraktion (LVEF) und Reduktion der endsystolischen und -diastolischen Volumina (LVESV; LVEDV) auftrat ebenso wie eine signifikante Reduktion der Herzfrequenz innerhalb von 3 Monaten. Der beschriebene Effekt der VNS war additiv zur Betablockergabe (De Ferrari, J. Cardiovasc. Transl. Res., 2014, 7(3), 310-320). Die Plasmalevel für TNF-$\alpha$ und IL-6 als auch deren myokardiale Proteinexpression konnte durch eine Vagusstimulation in diesem Tiennodel gesenkt werden, was dafür spricht, dass eine Stärkung des parasympatischen Nervensystems neben den Effekten auf LV-Remodeling auch positive Effekte auf proinflammatorische Zytokine hat.

Basierend auf den experimentellen präklinischen Daten erfolgten mittlerweile die ersten klinischen Studien zur vagalen Stimulation bei Patienten mit chronischer Herzinsuffizienz, wie bereits in der Behandlung der Epilepsie und Depression etabliert. Der Effekt der Stärkung des Parasympatikus über direkte Vagusnervstimulation (VNS) wurde in einer nicht randomisierten Beobachtungsstudie mit 32 Patienten mit einer linksventrikulären (LV) systolischen Dysfunktion bewertet, wobei die Ergebnisse darauf hindeuteten, daß eine Vagusreizung die Lebensqualität, die körperliche Belastbarkeit und das LV-Remodeling günstig beeinflusst (De Ferrari GM et al., Eur. Heart J., 2011, 32, 847-855) In der multizentrischen Open-Label-Machbarkeitsstudie ANTHEM-HF wurden die Sicherheit, Verträglichkeit und Wirksamkeit der Vagus-Stimulation bei Patienten mit chronischer stabiler, symptomatischer Herzinsuffizienz mit reduzierter Auswurffraktion (HFrEF) zusätzlich zur Standardtherapie geprüft (Premchand RK et al., J. Card. Fail., 2014, 20(11), 808-816). Die in dieser Studie angewandte kontinuierliche Vagusnervstimulation führte zu einer Verbesserung der Auswurffraktion, Herzfrequenzvariabilität, NYHA-Klasse und Lebensqualität. Die erste Placebo-kontrollierte klinische Studie NECTAR-HF konnte hingegen keine signifikante Wirkung der Vagusnervstimulation auf die Herzfunktion von HF Patienten nach 6 Monaten zeigen (Zannad et al., Eur. Heart J., 2015, 36(7), 425-433). Lediglich die Lebensqualität konnte verbessert werden. Die INOVATE-HF Studie mit 650 HF Patienten konnte keine Effekte dieser Therapie in Bezug auf die Mortalität und Hospitalisierung zeigen. (Gold et al., J Am Coll Cardiol., 2016, Mar 29. pii: S0735-1097(16)32404-4. doi: 10.1016/j.jacc.2016.03.525). Lebensqualität und Gehstrecke konnten signifikant verbessert werden.

Neben dem Infektionsrisiko und den potentiellen Risiken eines chirurgischen Eingriffs ist eine Therapie mittels elektrischer Stimulierung des Vagusnervs limitiert durch Nebenwirkungen wie: Dysphonie, Husten und Mund-/Rachenschmerzen (Premchand RK et al, J. Card. Fail., 2014, 20(11), 808-816). Eine medikamentöse Stärkung des parasympatischen Nervensystems durch eine direkte Wirkung auf den M2R könnte eine neue Therapieoption darstellen.

Vorhofflimmern ist die häufigste anhaltende Herzrhythmusstörung und ihre Prävalenz nimmt mit dem Lebensalter zu (Chen et al., Circ. Res., 2014, 114(9), 1500-1515). Oft sind Vorhofflimmern und Herzinsuffizienz miteinander vergesellschaftet und verstärken sich gegenseitig. So nimmt die Prävalenz von Vorhofflimmern mit dem klinischen

Schweregrad der Herzinsuffizienz zu (Maisel and Stevenson, Am. J. Cardiol., 2003, 91, (suppl) 2D-8D). Klinische Daten legen nahe, dass Patienten, bei denen eine Herzinsuffizienz von Vorhofflimmern begleitet wird, eine schlechte Prognose haben. Sowohl die Letalität (Gesamtletalität, plötzlicher Tod und Pumpversagen) als auch die Morbidität (Hospitalisierung) erwies sich bei dieser Patientengruppe als signifikant erhöht.

Bei der Therapie des Vorhofflimmerns werden zwei Behandlungsstrategien unterschieden: die sog. Frequenzkontrolle mit Einstellung und möglichst Normalisierung der Kammerfrequenz im Rahmen des Vorhofflimmerns und die sog. Rhythmuskontrolle, welche Maßnahmen umfasst, mit denen ein Sinusrhythmus hergestellt oder erhalten werden soll. Eine effektive Behandlung besteht aus einer Kombination von nichtmedikamentösen sowie medikamentösen oder interventionellen Maßnahmen (Levalter T, Fortbildungsprogramm Pharmazie, 2011, 5, 106-127).

Zur medikamentösen Rhythmuskontrolle nach Kardioversion werden Betablocker, Klasse-I- und Klasse-III-Antiarrhythmika nach Maßgabe der kardialen Grunderkrankung und dem Ausmaß der linksventrikulären Pumpfunktionseinschränkung eingesetzt. Bei Patienten mit permanentem Vorhofflimmern als auch bei oligosymptomatischen (oft älteren) Patienten mit persistierendem oder paroxysmalem Vorhofflimmern ist die reine Frequenzkontrolle unter Beibehaltung und Belassen des Vorhofflimmerns oft die Therapie der Wahl. Eingesetzt werden primär Medikamente, die einen Einfluss auf die Refraktärperiode bzw. die Leitungskapazität des AV-Knotens haben. Prinzipiell könnte diese Wirkung durch eine Stimulation des M2R, der physiologisch die Schlüsselrolle an dieser Stelle spielt, z.B. mit Hilfe eines positiven allosterischen Modulators erreicht werden. Bis jetzt stehen Betablocker, Digitalis, Calciumantagonisten sowie in Einzelfällen Amiodaron zur Verfügung, die unter Berücksichtigung des Lebensstils, der kardialen Grunderkrankung bzw. etwaiger Begleiterkrankungen individualisiert zum Einsatz kommen. Insbesondere bei Patienten mit reduzierter linksventrikulärer Pumpfunktion und schwerer Herzinsuffizienz sind allerdings die Möglichkeiten der medikamentösen Therapie unzureichend. Calciumantagonisten sind kontraindiziert in dieser Patientengruppe. Therapie mit Digoxin führt, wie die neuesten Studien gezeigt haben, zu einer erhöhten Mortalität von Patienten mit Vorhofflimmern (Leong-Sit and Tang, Curr. Opin. Cardiol., 2015, Epub). Für Betablocker wurde in einer Metaanalyse eine fehlende Wirksamkeit bei Patienten mit Vorhofflimmern und Herzinsuffizienz (Leong-Sit and Tang, Curr. Opin. Cardiol., 2015, Epub). Entsprechend hoch ist der medizinische Bedarf nach neuen effizienten und sicheren Therapien zur Frequenzkontrolle. Dies könnte durch eine medikamentöse Stimulation des M2R erreicht werden.

Die Aufgabe der vorliegenden Erfindung liegt in der Identifizierung und Bereitstellung neuer Substanzen, die potente, positiv allosterische Modulatoren des muskarinergen M2 Rezeptors darstellen und sich als solche zur Behandlung und/oder Prävention insbesondere von kardiovaskulären Erkrankungen und/oder Nierenerkrankungen eignen.

1-Benzyl-substituierte 4-Oxo-1,4-Dihydrochinoline-3-carbonsäuren sind als allosterische Modulatoren des M1-Muskarinrezeptors zur Behandlung von neurodegenerativen Erkrankungen wie Alzheimer und Schizophrenie beschrieben (Scammells et al., ACS Chem. Neurosci., 2013, 4(7), 1026-1048; Mistry et al., J. Med. Chem. 2013, 56, 5151-5172). Unter anderem aus EP 0945435 B1 sind Pyridoncarbonsäurederivate bekannt, welche eine antibakterielle Antivität aufweisen. In WO 2002/085886-A2, WO 2003/050107-A1 und WO 2005/026145-A2 werden 7-Piperidino-substituierte Chinolon-Carbonsäurederivate, sowie in WO 2005/026165-A1 und WO 2005/049602-A1 verschiedene 7-Pyrrolidino-substituierte Chinolon-Carbonsäurederivate sowie in EP 1650192-A1 spezielle 7-Azetidinyl-Chinolon-Carbonsäurederivate mit einer antimikrobiellen / antibakteriellen Aktivität beansprucht. Aus WO 2005/009971-A1 und JP 2005012561 sind Chinolonderivate bekannt, welche als Thrombozytenaggregationshemmer eingesetzt werden können. In WO 2015/189560-A1 werden 1,4-Dihydrochinolinderivate als NPRC-Agonisten zur Behandlung von kardiovaskulären Erkrankungen offenbart. Chinolon-Carbonsäurederivate als MCT-Modulatoren werden in WO 2016/081464-A1 zur Behandlung von insbesondere Tumor-Erkrankungen und inflammatorischen Prozessen beschrieben.

Die vorliegende Erfindung betrifft Verbindungen der allgemeinen Formel (I)

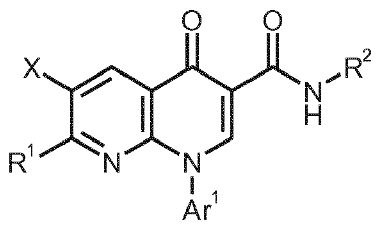

(I),

X für Halogen steht,

$R^1$ für Wasserstoff steht,
oder
für $-NR^4R^5$ steht,
worin

$R^4$ Wasserstoff, Methyl, $(C_2-C_4)$-Alkyl oder $(C_3-C_6)$-Cycloalkyl bedeutet, wobei $(C_2-C_4)$-Alkyl mit Hydroxy oder bis zu dreifach mit Fluor substituiert sein kann
und

$R^5$ $(C_1-C_6)$-Alkyl, $(C_3-C_6)$-Cycloalkyl, 3- bis 6-gliedriges gesättigtes Heterocyclyl oder $(C_1-C_4)$-Alkylsulfonyl bedeutet,
wobei $(C_1-C_6)$-Alkyl, $(C_3-C_6)$-Cycloalkyl und 3- bis 6-gliedriges gesättigtes Heterocyclyl, bis zu dreifach, gleich oder verschieden, mit Methyl, Difluormethyl, Trifluormethyl, Hydroxy, Hydroxycarbonyl, Oxo, Methoxy, Difluormethoxy, Trifluormethoxy, Cyano und weiterhin bis zu vierfach mit Fluor, substituiert sein können,
oder

$R^4$ und $R^5$ bilden zusammen mit dem Stickstoffatom, an das sie gebunden sind, einen gesättigten oder partiell ungesättigten, 3- bis 6-gliedrigen monocyclischen oder 6- bis 10-gliedrigen bicyclischen Heterocyclus, der ein oder zwei weitere, gleiche oder verschiedene Heteroatome aus der Reihe N, O, S, SO und/oder $SO_2$ als Ringglieder enthalten kann,
wobei der 3- bis 6-gliedrige monocyclische und der 6- bis 10-gliedrige bicyclische Heterocyclus jeweils mit 1 bis 5 Substituenten unabhängig voneinander ausgewählt aus der Gruppe $(C_1-C_4)$-Alkyl, Difluormethyl, Trifluormethyl, Hydroxy, Hydroxycarbonyl, Oxo, $(C_1-C_3)$-Alkoxy, Difluormethoxy, Trifluormethoxy, Cyano, $(C_1-C_3)$-Alkoxycarbonyl, Aminocarbonyl, Mono-$(C_1-C_3)$-alkylaminocarbonyloxy, $-NHC(=O)R^{13A}$, $-CH_2NHC(=O)R^{13B}$, $-OC(=O)R^{14}$, und weiterhin bis zu vierfach mit Fluor, substituiert sein können,
worin $(C_1-C_4)$-Alkyl ein- oder zweifach, gleich oder verschieden, mit Hydroxy, $(C_1-C_3)$-Alkoxy, und bis zu vierfach mit Fluor, substituiert sein kann,
$R^{13A}$ und $R^{13B}$ unabhängig voneinander $(C_1-C_3)$-Alkyl oder Cyclopropyl darstellen,
und worin
$R^{14}$ $(C_1-C_4)$-Alkyl darstellt,

$R^2$ für eine Gruppe der Formel

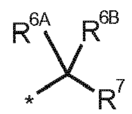

oder

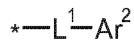

steht, worin
* die Verknüpfungsstelle mit dem N-Atom der Amid-Gruppierung markiert,
$R^{6A}$ Wasserstoff oder $(C_1-C_4)$-Alkyl bedeutet,
$R^{6B}$ Wasserstoff, $(C_1-C_4)$-Alkyl, Cyclopropyl, Monofluormethyl, Difluormethyl, Trifluormethyl, Methoxymethyl oder Trifluormethoxymethyl bedeutet,
$R^7$ $(C_1-C_6)$-Alkyl oder bis zu vierfach mit Fluor substituiertes $(C_3-C_5)$-Cycloalkyl bedeutet, wobei $(C_1-C_6)$-Alkyl mit Amino, Hydroxy, $(C_1-C_6)$-Alkoxy und bis zu fünffach mit Fluor substituiert sein kann,
worin $(C_1-C_6)$-Alkoxy bis zu fünffach mit Fluor substituiert sein kann
$L^1$ eine Bindung oder eine Gruppe der Formel $-C(R^{8A}R^{8B})-(C(R^{9A}R^{9B}))_m-$ bedeutet,
worin
m 0 oder 1 darstellt,
$R^{8A}$ Wasserstoff oder Methyl darstellt,
$R^{8B}$ Wasserstoff, Methyl, Trifluormethyl, Pentafluorethyl oder Trifluormethoxymethyl darstellt,
$R^{9A}$ und $R^{9B}$ unabhängig voneinander Wasserstoff oder Methyl darstellen,
$Ar^2$ Phenyl bedeutet, wobei Phenyl ein- bis dreifach, gleich oder verschieden, mit Fluor, Chlor, $(C_1-C_3)$-Alkyl, Difluormethoxymethyl, Trifluormethoxymethyl und/oder Trifluormethyl substituiert sein kann,
oder
für einen 5- bis 10-gliedrigen monocyclischen, bicyclischen oder tricyclischen Carbocyclus oder Heterocyclus, der ein oder zwei weitere, gleiche oder verschiedene Heteroatome aus der Reihe N und/oder O als Ringglieder enthalten kann, steht,
wobei der 5- bis 10-gliedrige monocyclische, bicyclische oder tricyclische Carbocyclus oder Heterocyclus bis zu dreifach, gleich oder verschieden, mit $(C_1-C_3)$-Alkyl, Trifluormethyl, $(C_1-C_4)$-Alkoxycarbonyl und weiterhin bis zu vierfach mit Fluor substituiert sein können,

$Ar^1$ für eine Gruppe der Formel

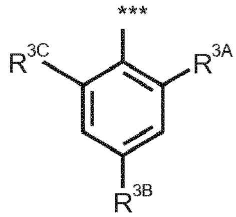

steht, worin
*** die Verknüpfungsstelle mit dem N-Atom markiert,
$R^{3A}$ für Fluor, Chlor, Trifluormethyl oder Methyl steht,
$R^{3B}$ für Wasserstoff oder Fluor steht
und $R^{3C}$ für Wasserstoff, Fluor, Chlor oder Methyl steht, oder
für einen über ein Ring-Kohlenstoffatom angebundenen Pyridinring steht,
wobei der Pyridinring ein oder zweifach mit Fluor, Chlor, Cyano, Methyl oder Trifluormethyl substituiert sein kann,

sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Erfindungsgemäße Verbindungen sind ebenso *N*-Oxide der Verbindungen der Formel (I) sowie deren Salze, Solvate und Solvate der Salze.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Bei einem geeigneten pharmazeutisch unbedenklichen Salz der Verbindungen der vorliegenden Erfindung kann es sich beispielsweise um ein Säureadditionssalz einer Verbindung der vorliegenden Erfindung mit einem ausreichend basischen Stickstoffatom in einer Kette oder einem Ring handeln, wie ein Säureadditionssalz mit einer anorganischen Säure oder "Mineralsäure", beispielsweise Salzsäure, Bromwasserstoffsäure, Iodwasserstoffsäure, Schwefelsäure, Sulfaminsäure, Dischwefelsäure, Phosphorsäure oder Salpetersäure, oder mit einer organischen Säure, wie beispielsweise Ameisensäure, Essigsäure, Acetessigsäure, Brenztraubensäure, Trifluoressigsäure, Propionsäure, Buttersäure, Hexansäure, Heptansäure, Undecansäure, Laurylsäure, Benzoesäure, Salicylsäure, 2-(4-Hydroxybenzoyl)benzoesäure, Camphersäure, Zimtsäure, Cyclopentanpropionsäure, Diglukonsäure, 3-Hydroxy-2-naphthoesäure, Nicotinsäure, Pamoasäure, Pektinsäure, 3-Phenylpropionsäure, Pivalinsäure, 2-Hydroxyethansulfonsäure, Itaconsäure, Trifluormethansulfonsäure, Dodecylschwefelsäure, Ethansulfonsäure, Benzolsulfonsäure, para-Toluolsulfonsäure, Methansulfonsäure, 2-Naphthalinsulfonsäure, Naphthalindisulfonsäure, Camphersulfonsäure, Zitronensäure, Weinsäure, Stearinsäure, Milchsäure, Oxalsäure, Malonsäure, Bernsteinsäure, Äpfelsäure, Adipinsäure, Alginsäure, Maleinsäure, Fumarsäure, D-Glukonsäure, Mandelsäure, Ascorbinsäure, Glukoheptansäure, Glycerophosphorsäure, Asparaginsäure, Sulfosalicylsäure oder Thiocyansäure.

Ein anderes geeignetes pharmazeutisch unbedenkliches Salz einer ausreichend sauren Verbindung der vorliegenden Erfindung ist außerdem ein Alkalisalz, beispielsweise ein Natrium- oder Kaliumsalz, ein Erdalkalisalz, beispielsweise ein Calcium-, Magnesium- oder Strontiumsalz, oder ein Aluminium- oder Zinksalz oder ein Ammoniumsalz, das abgeleitet ist von Ammoniak oder einem organischen primären, sekundären oder tertiären Amin mit 1 bis 20 Kohlenstoffatomen, wie Ethylamin, Diethylamin, Triethylamin, Ethyldiisopropylamin, Monoethanolamin, Diethanolamin, Triethanolamin, Dicyclohexylamin, Dimethylaminoethanol, Diethylaminoethanol, Tris(hydroxymethyl)aminomethan, Procain, Dibenzylamin, *N*-Methylmorpholin, Arginin, Lysin, 1,2-Ethylendiamin, *N*-Methylpiperidin, *N*-Methylglucamin, *N,N*-Dimethylglucamin, *N*-Ethylglucamin, 1,6-Hexandiamin, Glucosamin, Sarcosin, Serinol, 2-Amino-1,3-propandiol, 3-Amino-1,2-propandiol, 4-Amino-1,2,3-butantriol, oder ein Salz mit einem quartären Ammoniumion mit 1 bis 20 Kohlenstoffatomen, wie Tetramethylammonium, Tetraethylammonium, Tetra(*n*-propyl)ammonium, Tetra(*n*-butyl)ammonium, *N*-Benzyl-*N,N,N*-trimethylammonium, Cholin oder Benzalkonium.

Weiterhin ist dem Fachmann bekannt, dass Säureadditionssalze der beanspruchten Verbindungen durch Umsetzung der Verbindungen mit der entsprechenden anorganischen oder organischen Säure nach einer Reihe bekannter Methoden hergestellt werden können. Alternativ dazu werden Alkali- und Erdalkalisalze der sauren erfindungsgemäßen Verbindungen hergestellt, indem man die erfindungsgemäßen Verbindungen nach verschiedenen bekannten Methoden mit der entsprechenden Base umsetzt.

Die vorliegende Erfindung schließt alle möglichen Salze der Verbindungen der vorliegenden Erfindung als einzelne Salze oder als Gemisch dieser Salze in einem beliebigen Verhältnis ein.

Wenn im vorliegenden Text, insbesondere im Experimentellen Teil, für die Synthese von Zwischenprodukten und Beispielen der vorliegenden Erfindung eine Verbindung als Salzform mit der entsprechenden Base oder Säure erwähnt wird, ist die exakte stöchiometrische Zusammensetzung der Salzform, wie sie durch das jeweilige Herstellungs- und/oder Reinigungsverfahren erhalten wurde, in den meisten Fällen nicht bekannt. Sofern nicht anders angegeben, bedeuten Zusätze zu chemischen Namen oder Strukturformeln, die sich auf Salze beziehen, wie beispielsweise "Hydrochlorid", "Trifluoracetat", salz" oder "x HCl", "x $CF_3COOH$", "x $Na^+$" eine Salzform, wobei die Stöchiometrie dieses Salzes nicht angegeben ist. Dies gilt entsprechend für Fälle, in denen Synthesezwischenprodukte oder Beispielverbindungen oder Salze davon nach dem beschriebenen Herstellungs- und/oder Reinigungsverfahren als Solvate, beispielsweise als Hydrate, erhalten wurden.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere sowie ihre jeweiligen Mischungen. Aus solchen Mischungen von Enantiomeren und/ oder Diastereomeren lassen sich die stereoisomer einheitlichen Bestandteile in bekannter Weise isolieren. Vorzugsweise werden hierfür chromatographische Verfahren angewandt, insbesondere die HPLC-Chromatographie an achiralen bzw. chiralen Trennphasen. Im Falle von Carbonsäuren als Zwischen- oder Endprodukten kann alternativ auch eine Trennung über diastereomere Salze mit Hilfe chiraler Amin-Basen erfolgen.

Der Begriff "enantiomerenrein" wird im Rahmen der vorliegenden Erfindung dahingehend verstanden, dass die betreffende Verbindung hinsichtlich der Absolutkonfiguration der chiralen Zentren in einem

Enantiomerenüberschuss von mehr als 95%, bevorzugt von mehr als 98% vorliegt. Der Enantiomerenüberschuss (engl. *enantiomeric excess*, ee-Wert) wird hierbei durch Auswertung des Chromatogramms einer HPLC-Analyse an chiraler Phase nach der folgenden Formel berechnet:

$$ee = \left| \frac{\text{Enantiomer 1 (Flächenprozent)} - \text{Enantiomer 2 (Flächenprozent)}}{\text{Enantiomer 1 (Flächenprozent)} + \text{Enantiomer 2 (Flächenprozent)}} \right| \times 100\%.$$

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ("unnatürlicher Anteil") ausgetauscht ist. Unter dem Ausdruck "unnatürlicher Anteil" ist ein Anteil eines derartigen Isotops zu verstehen, der höher als dessen natürliche Häufigkeit ist. Die in diesem Zusammenhang anzuwendenden natürlichen Häufigkeiten von Isotopen finden sich in "Isotopic Compositions of the Elements 1997", Pure Appl. Chem., 70(1), 217-235, 1998. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie $^{2}H$ (Deuterium), $^{3}H$ (Tritium), $^{13}C$, $^{14}C$, $^{15}N$, $^{17}O$, $^{18}O$, $^{32}P$, $^{33}P$, $^{33}S$, $^{34}S$, $^{35}S$, $^{36}S$, $^{18}F$, $^{36}Cl$, $^{82}Br$, $^{123}I$, $^{124}I$, $^{129}I$ und $^{131}I$. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit $^{3}H$- oder $^{14}C$-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Im Hinblick auf die Behandlung und/oder Prophylaxe der hier angegebenen Störungen enthalten die isotopischen Variante(n) der Verbindungen der allgemeinen Formel (I) vorzugsweise Deuterium ("deuteriumhaltige Verbindungen der allgemeinen Formel (I)"). Isotopische Varianten der Verbindungen der allgemeinen Formel (I), in die ein oder mehrere radioaktive Isotope, wie $^{3}H$ oder $^{14}C$, eingebaut sind, sind beispielsweise bei Arzneistoff- und/oder Substratsgewebeverteilungsstudien von Nutzen. Diese Isotope sind wegen ihrer leichten Einbaubarkeit und Nachweisbarkeit besonders bevorzugt. In eine Verbindung der allgemeinen Formel (I) können Positronen emittierende Isotope wie $^{18}F$ oder $^{11}C$ eingebaut werden. Diese isotopischen Varianten der Verbindungen der allgemeinen Formel (I) eignen sich zur Verwendung bei in-vivo-Bildgebungsanwendungen. Deuteriumhaltige und $^{13}C$-haltige Verbindungen der allgemeinen Formel (I) können im Rahmen prädlinischer oder klinischer Studien bei Massenspektrometrie-Analysen verwendet werden (H. J. Leis et al., Curr. Org. Chem., 1998, 2, 131). Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Isotopische Varianten der Verbindungen der allgemeinen Formel (I) können im Allgemeinen nach dem Fachmann bekannten Verfahren wie in den hier beschriebenen Schemata und/oder Beispielen beschrieben hergestellt werden, indem man ein Reagenz durch eine isotopische Variante des Reagenzes, vorzugsweise ein deuteriumhaltiges Reagenz, ersetzt. Je nach den gewünschten Deuterierungsstellen kann in einigen Fällen Deuterium aus $D_2O$ entweder direkt in die Verbindungen oder in Reagenzien, die für die Synthese derartiger Verbindungen verwendet werden können, eingebaut werden (Esaki et al., Tetrahedron, 2006, 62, 10954; Esaki et al., Chem. Eur. J., 2007, 13, 4052). Ein nützliches Reagenz zum Einbau von Deuterium in Moleküle ist auch Deuteriumgas. Eine schnelle Route zum Einbau von Deuterium ist die katalytische Deuterierung von olefinischen Bindungen (H. J. Leis et al., Curr. Org. Chem., 1998, 2, 131; J. R. Morandi et al., J. Org. Chem., 1969, 34 (6), 1889) und acetylenischen Bindungen (N. H. Khan, J. Am. Chem. Soc., 1952, 74 (12), 3018; S. Chandrasekhar et al., Tetrahedron, 2011, 52, 3865). Zum direkten Austausch von Wasserstoff gegen Deuterium in funktionelle Gruppen enthaltenden Kohlenwasserstoffen können auch Metallkatalysatoren (d.h. Pd, Pt und Rh) in Gegenwart von Deuteriumgas verwendet werden (J. G. Atkinson et al., US-Patent 3966781). Verschiedene deuterierte Reagenzien und Synthesebausteine sind im Handel von Firmen wie beispielsweise C/D/N Isotopes, Quebec, Kanada; Cambridge Isotope Laboratories Inc., Andover, MA, USA; und CombiPhos Catalysts, Inc., Princeton, NJ, USA, erhältlich. Weitere Informationen bezüglich des Standes der Technik im Hinblick auf Deuterium-Wasserstoff-Austausch finden sich beispielsweise in Hanzlik et al., J. Org. Chem., 1990, 55, 3992-3997; R. P. Hanzlik et al., Biochem. Biophys. Res. Commun., 1989, 160, 844; P. J. Reider et al., J. Org. Chem., 1987, 52, 3326-3334; M. Jarman et al., Carcinogenesis ,1993, 16(4), 683-688; J. Atzrodt et al., Angew. Chem., Int. Ed. 2007, 46, 7744; K. Matoishi et al., 2000, J. Chem. Soc, Chem. Commun., 1519-1520; K. Kassahun et al., WO 2012/112363.

Der Begriff "deuteriumhaltige Verbindung der allgemeinen Formel (I)" ist definiert als eine Verbindung der allgemeinen Formel (I), in der ein oder mehrere Wasserstoffatome durch ein oder mehrere Deuteriumatome ersetzt sind und in der die Häufigkeit von Deuterium in jeder deuterierten Position der Verbindung der allgemeinen Formel (I) höher ist als die natürliche Häufigkeit von Deuterium, die etwa 0,015% beträgt. Insbesondere ist in einer deuteriumhaltigen Verbindung der allgemeinen Formel (I) die Häufigkeit von Deuterium in jeder deuterierten Position der Verbindung der allgemeinen Formel (I) höher als 10%, 20%, 30%, 40%, 50%, 60%, 70% oder 80%, vorzugsweise höher als 90%, 95%, 96% oder 97%, noch weiter bevorzugt höher als 98% oder 99% in dieser Position bzw. diesen Positionen. Es versteht sich, dass die Häufigkeit von Deuterium in jeder deuterierten Position von der Häufigkeit von Deuterium in anderen deuterierten Positionen unabhängig ist.

Durch den selektiven Einbau eines oder mehrerer Deuteriumatome in eine Verbindung der allgemeinen Formel (I) können die physikalisch-chemischen Eigenschaften (wie beispielsweise Acidität [A. Streitwieser et al., J. Am. Chem. Soc., 1963, 85, 2759; C. L. Perrin et al., J. Am. Chem. Soc., 2007, 129, 4490], Basizität [C. L. Perrin, et al., J. Am. Chem. Soc., 2003, 125, 15008; C. L. Perrin in Advances in Physical Organic Chemistry, 44, 144; C. L. Perrin et al., J. Am. Chem. Soc., 2005, 127, 9641], Lipophilie [B. Testa et al., Int. J. Pharm., 1984, 19(3), 271]) und/oder das Stoffwechselprofil des Moleküls verändert und Veränderungen des Verhältnisses von Stammverbindung zu Metaboliten oder der gebildeten Mengen von Metaboliten verursacht werden. Derartige Veränderungen können zu bestimmten therapeutischen Vorteilen führen und daher unter bestimmten Umständen bevorzugt sein. Es ist über verringerte Stoffwechselraten und Stoffwechselumschaltung, bei der sich das Verhältnis von Metaboliten ändert, berichtet worden (D. J. Kushner et al., Can. J. Physiol. Pharmacol., 1999, 77, 79; A. E. Mutlib et al., Toxicol. Appl. Pharmacol., 2000, 169, 102). Diese Veränderungen der Exposition gegenüber Stammverbindung und Metaboliten können wichtige Konsequenzen hinsichtlich Pharmakodynamik, Verträglichkeit und Wirksamkeit einer deuteriumhaltigen Verbindung der allgemeinen Formel (I) haben. In einigen Fällen wird durch den Deuteriumersatz die Bildung eines unerwünschten oder toxischen Metaboliten verringert oder eliminiert und die Bildung eines gewünschten Metaboliten verstärkt (z.B. Nevirapin: A. M. Sharma et al., Chem. Res.Toxicol., 2013, 26, 410; Uetrecht et al., Chemical Research in Toxicology, 2008, 21, 9, 1862; Efavirenz: A. E. Mutlib et al., Toxicol. Appl. Pharmacol., 2000, 169, 102). In anderen Fällen besteht der Haupteffekt der Deuterierung darin, die Geschwindigkeit der systemischen Clearance zu verringern. Dadurch wird die biologische Halbwertszeit der Verbindung erhöht. Zu den potentiellen klinischen Vorteilen gehören die Fähigkeit zur Aufrechterhaltung einer ähnlichen systemischen Exposition mit verringerten Spitzenspiegeln und erhöhten Talspiegeln. Dies könnte je nach der Pharmakokinetik/Pharmakodynamik-Beziehung der jeweiligen Verbindung zu geringeren Nebenwirkungen und erhöhter Wirksamkeit führen. Beispiele für diesen Deuterium-Effekt sind Indiplon (A. J. Morales et al., Abstract 285, The 15th North

American Meeting of the International Society of Xenobiotics, San Diego, CA, 12.-16. Oktober, 2008), ML-337 (C. J. Wenthur et al., J. Med. Chem., 2013, 56, 5208) und Odanacatib (K. Kassahun et al., WO2012/112363). Es ist auch noch über weitere Fälle berichtet worden, bei denen verringerte Verstoffwechslungsraten zu einer Erhöhung der Arzneistoffexposition ohne Änderung der Rate der systemischen Clearance führen (z.B. Rofecoxib: F. Schneider et al., Arzneim. Forsch. Drug. Res., 2006, 56, 295; Telaprevir: F. Maltais et al., J. Med. Chem., 2009, 52, 7993). Deuterierte Arzneistoffe, die diesen Effekt zeigen, können verringerte Dosierungsanforderungen haben (z.B. geringere Zahl von Dosen oder niedrigere Dosierung zur Erzielung der gewünschten Wirkung) und/oder zu geringeren Metabolitenbelastungen führen.

Eine Verbindung der allgemeinen Formel (I) kann mehrere potenzielle Angriffsstellen für die Verstoffwechslung aufweisen. Zur Optimierung der oben beschriebenen Effekte auf die physikalisch-chemischen Eigenschaften und das Stoffwechselprofil können deuteriumhaltige Verbindungen der allgemeinen Formel (I) mit einem bestimmten Muster eines oder mehrerer Deuterium-Wasserstoff-Austausche gewählt werden. Insbesondere ist/sind das Deuteriumatom/die Deuteriumatome deuteriumhaltiger Verbindung(en) der allgemeinen Formel (I) an ein Kohlenstoffatom gebunden und/oder steht/stehen in den Positionen der Verbindung der allgemeinen Formel (I), bei denen es sich um Angriffsstellen für Verstoffwechslungsenzyme wie z.B. Cytochrom $P_{450}$ handelt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylsulfonyl, Alkylaminocarbonyloxy und Alkoxycarbonyl stehen für einen linearen oder verzweigten Alkylrest mit in der Regel 1 bis 6, vorzugsweise 1 bis 4 oder 1 bis 3 Kohlenstoffatomen, beispielhaft und vorzugsweise für Methyl, Ethyl, n-Propyl, Isopropyl, tert.-Butyl, iso-Butyl, (2-Methyl-prop-1-yl), n-Pentyl und n-Hexyl.

Alkoxy steht beispielhaft und vorzugsweise für Methoxy, Ethoxy, n-Propoxy, Isopropoxy, tert.-Butoxy, n-Pentoxy und n-Hexoxy.

Alkylaminocarbonyloxy steht für einen Alkylaminocarbonyloxyrest mit einem oder zwei (unabhängig voneinander gewählten) Alkylsubstituenten. ($C_1$-$C_3$)-Alkylaminocarbonyloxy steht beispielsweise für einen Monoalkylaminocarbonyloxyrest mit 1 bis 3 Kohlenstoffatomen oder für einen Dialkylaminocarbonyloxyrest mit jeweils 1 bis 3 Kohlenstoffatomen pro Alkylsubstituent. Beispielhaft und vorzugsweise seien genannt: Methylaminocarbonyloxy, Ethylaminocarbonyloxy, n-Propylaminocarbonyloxy, Isopropylaminocarbonyloxy, tert.-Butylaminocarbonyloxy, n-Pentylaminocarbonyloxy, n-Hexylaminocarbonyloxy, *N,N*-Dimethylaminocarbonyloxy, *N,N*-Diethylaminocarbonyloxy, *N*-Ethyl-*N*-methylaminocarbonyloxy, *N*-Methyl-*N*-n-propylaminocarbonyloxy, *N*-Isopropyl-*N*-n-propyl-aminocarbonyloxy, *N*-tert.-Butyl-*N*-methylaminocarbonyl, *N*-Ethyl-*N*-n-pentylamino-carbonyl und *N*-n-Hexyl-*N*-methylaminocarbonyloxy.

Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.

Alkoxycarbonyl steht beispielhaft und vorzugsweise für Methoxycarbonyl, Ethoxycarbonyl, n-Propoxycarbonyl, Isopropoxycarbonyl und tert.-Butoxycarbonyl.

Carbocyclus steht im Rahmen der Erfindung für einen mono-, poly- oder spirocyclischen, vorzugsweise mono-, oder bicyclischen gesättigten Carbocyclus mit insgesamt 3 bis 6 Ringatomen. Ein monocyclischer, gesättigter Carbocyclus wird synonym mit Cycloalkyl bezeichnet. Beispielhaft seien genannt: Cyclopropyl, Cyclobutyl, Cyclopentyl, Cyclohexyl, Cycloheptyl, Cyclopentenyl, Cyclohexenyl, Cyclohexadienyl, Cycloheptenyl, Cycloheptadienyl, Spiro[2.3]hexyl, Spiro[2.4]heptyl, Spiro[2.5]oktyl, Bicyclo[1.1.1]-pentyl, Bicyclo[2.2.1]heptyl, Bicyclo[2.2.2]oktyl, Tricyclo[3.3.1.13,7]decyl. Bevorzugt ist monocyclisches Cycloalkyl mit 3 bis 5 Kohlenstoffatomen. Beispielhaft und bevorzugt seien genannt: Cyclopropyl, Cyclobutyl, Cyclopentyl, Bicyclo[2.2.1]heptyl, oder Bicyclo[1.1.1]pent-1-yl.

Heterocyclyl steht für einen mono-, poly- oder spirocyclischen, vorzugsweise mono-, bi- oder spirocyclischen nicht-aromatischen heterocyclischen Rest mit in der Regel 3 bis 10 Ringatomen und bis zu 3, vorzugsweise bis zu 2 Heteroatomen und/oder Heterogruppen aus der Reihe N, O, S, SO, $SO_2$. Die Heterocyclyl-Reste können gesättigt oder partiell ungesättigt sein. Bevorzugt sind 4- bis 6-gliedrige monocyclische gesättigte Heterocyclylreste mit einem Stickstoffatom sowie solche mit einem weiteren Heteroatom aus der Reihe N oder O, sowie 6- bis 7-gliedrige bi- oder spirocyclische gesättigte Heterocyclylreste mit einem Stickstoffatom. Beispielhaft und vorzugsweise seien genannt: Azetidinyl, Pyrrolidinyl, Piperidinyl, Piperazinyl, Oxazolidinyl, Imidazolidinyl, Morpholinyl, Tetrahydropyrimidin, Azaspiro[2.4]heptyl oder (2-Oxa-6-azaspiro[3.3]hept-6-yl.

Halogen steht für Fluor, Chlor, Brom und Iod, vorzugsweise für Fluor oder Chlor.

In der Formel der Gruppe, für die $R^1$, $R^2$, $Ar^1$, bzw. $L^1$ stehen kann, steht der Endpunkt der Linie, an dem das Zeichen $\#^1$; *, ** und *** steht, nicht für ein Kohlenstoffatom beziehungsweise eine $CH_2$-Gruppe, sondern ist Bestandteil der Bindung zu dem jeweils bezeichneten Atom, an das $R^1$, $R^2$, $Ar^1$, bzw. $L^1$ gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die

mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Bevorzugt im Rahmen der vorliegenden Erfindung sind Verbindungen der Formel (I), in welcher in welcher

X für Fluor oder Chlor steht,

$R^1$ für Wasserstoff steht,
oder
für $NR^4R^5$ steht,
worin

$R^4$ Wasserstoff, Methyl oder Ethyl bedeutet, und

$R^5$ bis zu vierfach mit Fluor substituiertes ($C_1$-$C_3$)-Alkyl bedeutet,
wobei ($C_1$-$C_3$)-Alkyl mit Hydroxy substituiert sein kann,

oder

$R^4$ und $R^5$ zusammen mit dem Stickstoffatom, an das sie gebunden sind, einen gesättigten 4- bis 6-gliedrigen monocyclischen oder 6- bis 9-gliedrigen bicyclischen Heterocyclus bilden, der ein oder zwei weitere, gleiche oder verschiedene Heteroatome aus der Reihe N und/oder O als Ringglieder enthalten kann,
wobei der 4- bis 6-gliedrige monocyclische und der 6- bis 9-gliedrige bicyclische Heterocyclus jeweils mit 1 bis 4 Substituenten unabhängig voneinander ausgewählt aus der Gruppe ($C_1$-$C_4$)-Alkyl, Difluormethyl, Trifluormethyl, Hydroxy, Oxo, ($C_1$-$C_3$)-Alkoxy, Difluormethoxy, Trifluormethoxy, ($C_1$-$C_3$)-Alkoxycarbonyl, ($C_1$-$C_3$)-Alkylaminocarbonyloxy, $OC(=O)R^{14}$, und weiterhin bis zu vierfach mit Fluor, substituiert sein können,
worin ($C_1$-$C_4$)-Alkyl ein- oder zweifach, gleich oder verschieden, mit Hydroxy, ($C_1$-$C_3$)-Alkoxy, und bis zu vierfach mit Fluor, substituiert sein kann, und worin
$R^{14}$ ($C_1$-$C_4$)-Alkyl darstellt,

$R^2$ für eine Gruppe der Formel

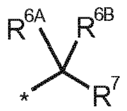

oder

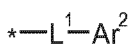

steht, worin

* die Verknüpfungsstelle mit dem N-Atom der Amid-Gruppierung markiert,

$R^{6A}$ Wasserstoff oder ($C_1$-$C_4$)-Alkyl bedeutet,

$R^{6B}$ Methyl, Ethyl, Isopropyl, Cyclopropyl, Monofluormethyl, Difluormethyl, oder Trifluormethyl, bedeutet, und

$R^7$ bis zu fünffach mit Fluor substituiertes ($C_1$-$C_4$)-Alkyl, bis zu vierfach mit Fluor substituiertes ($C_3$-$C_5$)-Cycloalkyl, Methoxymethyl oder Trifluormethoxymethyl bedeutet,

$L^1$ eine Bindung oder eine Gruppe der Formel $-CR^{8A}R^{8B}-$ bedeutet,
worin
$R^{8A}$ Wasserstoff darstellt,
$R^{8B}$ Wasserstoff, Methyl, Trifluormethyl, Pentafluorethyl oder Trifluormethoxymethyl darstellt,

$Ar^2$ Phenyl bedeutet, wobei Phenyl ein- bis dreifach, gleich oder verschieden, mit Fluor oder Chlor, substituiert sein kann,

oder

für einen 5- bis 7-gliedrigen bicyclischen Carbocyclus oder 5- oder 6-gliedrigen monocyclischen Heterocyclus, der ein Stickstoffatom als Ringglied enthält, steht,

wobei der 5- bis 7-gliedrige bicyclische Carbocyclus oder der 5- oder 6-gliedrige monocyclische Heterocyclus jeweils mit ($C_1$-$C_4$)-Alkoxycarbonyl und weiterhin bis zu vierfach mit Fluor substituiert sein können,

$Ar^1$ für eine Gruppe der Formel

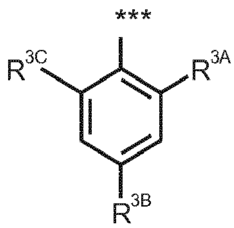

steht, worin

*** die Verknüpfungsstelle mit dem N-Atom markiert,
$R^{3A}$ für Fluor, Chlor, Trifluormethyl oder Methyl steht,
$R^{3B}$ für Wasserstoff oder Fluor steht
und $R^{3C}$ für Wasserstoff, Fluor, Chlor oder Methyl steht,

oder

für einen über ein Ring-Kohlenstoffatom angebundenen Pyridinring steht,

wobei der Pyridinring ein oder zweifach mit Fluor, Chlor oder Cyano substituiert sein kann, sowie ihre Salze, Solvate und Solvate der Salze.

Besonders bevorzugt im Rahmen der vorliegenden Erfindung sind Verbindungen der Formel (I), in welcher

X für Fluor oder Chlor steht,
$R^1$ für $NR^4R^5$ steht,
worin
$R^4$ Methyl oder Ethyl bedeutet, und
$R^5$ Methyl, 2-Hydroxyethyl oder 2-Hydroxypropyl bedeutet, oder
für einen über ein Stickstoff-Atom gebundenen Heterocyclus der Formel

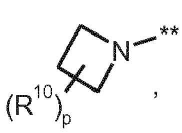 , 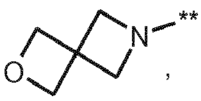 , 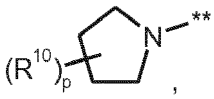 , 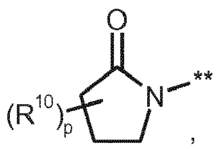 ,

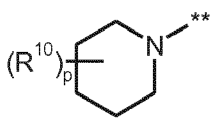 oder 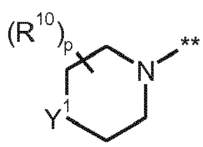

steht, worin
** die Verknüpfungsstelle mit dem Rest des Moleküls markiert,
$R^{10}$ Fluor, Methyl, Hydroxy, Hydroxymethyl, Methoxycarbonyl oder Acetyloxy darstellt,
p die Zahl 0, 1, oder 2 darstellt,
wobei im Fall, dass die Substituenten $R^{10}$ mehrfach auftreten, deren Bedeutungen jeweils gleich oder verschieden sein können,
$Y^1$ -NH-, -N($CH_3$)- oder -O- darstellt,
$R^2$ für eine Gruppe der Formel

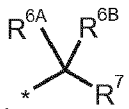, 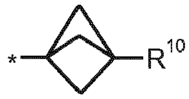, 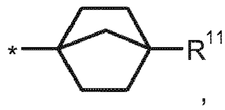,

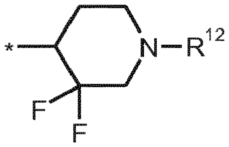 oder 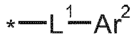

steht, worin
* die Verknüpfungsstelle mit dem N-Atom der Amid-Gruppierung markiert,
$R^{6A}$ Wasserstoff, Methyl oder Ethyl bedeutet,
$R^{6B}$ Methyl, Ethyl, Trifluormethyl, Isopropyl oder Cyclopropyl, bedeutet, und
$R^7$ Methyl, Ethyl, Difluormethyl, Trifluormethyl, 2,2,2-Trifluorethyl, Pentafluorethyl, Isopropyl, iso-Butyl, Methoxymethyl, Trifluormethoxymethyl oder Cyclopropyl bedeutet,
$R^{10}$ Wasserstoff bedeutet,
$R^{11}$ Methoxycarbonyl bedeutet,
$R^{12}$ Wasserstoff oder tert-Butoxycarbonyl bedeutet,
$L^1$ eine Bindung oder eine Gruppe der Formel $-CR^{8A}R^{8B}-$ bedeutet,
worin
$R^{8A}$ Wasserstoff darstellt,
$R^{8B}$ Wasserstoff oder Trifluormethyl darstellt,
$Ar^2$ Phenyl bedeutet, wobei Phenyl ein- bis zweifach, gleich oder verschieden, mit Fluor oder Chlor, substituiert sein kann,
$Ar^1$ für eine Gruppe der Formel

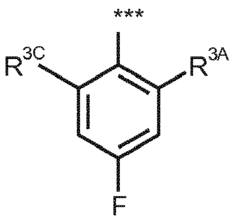, 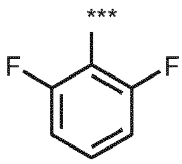 oder 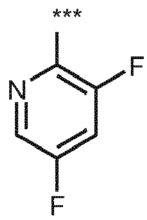

steht, worin
*** die Verknüpfungsstelle mit dem N-Atom markiert,
$R^{3A}$ für Fluor oder Chlor steht,
und $R^{3C}$ für Wasserstoff oder Fluor steht,

sowie ihre Salze, Solvate und Solvate der Salze.

Ganz besonders bevorzugt im Rahmen der vorliegenden Erfindung sind Verbindungen der Formel (I), in welcher

X für Fluor steht,
$R^1$ für einen über ein Stickstoff-Atom gebundenen Heterocyclus der Formel

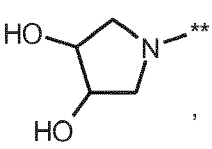, 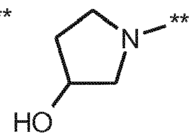 oder 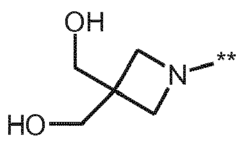

steht, worin
** die Verknüpfungsstelle mit dem Rest des Moleküls markiert,
$R^2$ für eine Gruppe der Formel

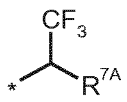, 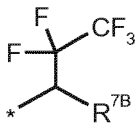, 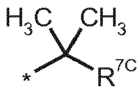,

steht, worin
* die Verknüpfungsstelle mit dem N-Atom der Amid-Gruppierung markiert,
$R^{7A}$ Trifluormethyl, Ethyl oder Cyclopropyl bedeutet,
$R^{7B}$ Methyl oder Ethyl bedeutet,
$R^{7C}$ Trifluormethyl oder Pentafluorethyl bedeutet,

$Ar^1$ für eine Gruppe der Formel

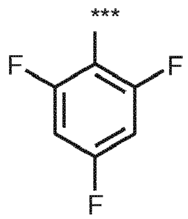 oder 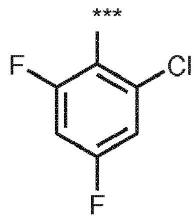

steht, worin
*** die Verknüpfungsstelle mit dem N-Atom markiert,

sowie ihre Salze, Solvate und Solvate der Salze.

Eine weitere besondere Ausführungsform der vorliegenden Erfindung umfasst Verbindungen der Formel (I), in welcher

X für Fluor steht,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

X für Chlor steht,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^1$ für $NR^4R^5$ steht,
worin

$R^4$ Methyl oder Ethyl bedeutet, und
$R^5$ Methyl, 2-Hydroxyethyl oder 2-Hydroxypropyl bedeutet,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

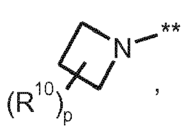, 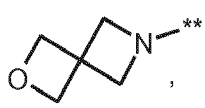, 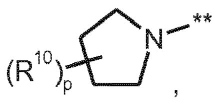, 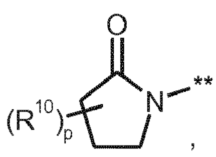,

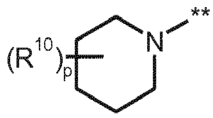 oder 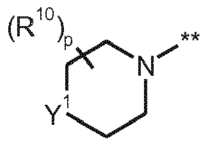

steht, worin

** die Verknüpfungsstelle mit dem Rest des Moleküls markiert,

$R^{10}$ Fluor, Methyl, Hydroxy, Hydroxymethyl, Methoxycarbonyl oder Acetyloxy darstellt,

p die Zahl 0, 1, oder 2 darstellt,

wobei im Fall, dass die Substituenten $R^{10}$ mehrfach auftreten, deren Bedeutungen jeweils gleich oder verschieden sein können,

$y^1$ -NH-, -N($CH_3$)- oder -O- darstellt,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

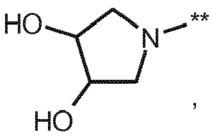 , 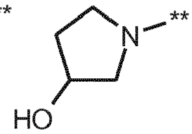 oder 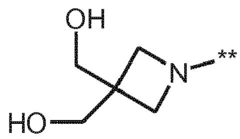

steht, worin

** die Verknüpfungsstelle mit dem Rest des Moleküls markiert,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

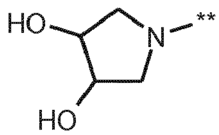

steht, worin

** die Verknüpfungsstelle mit dem Rest des Moleküls markiert, sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^1$ für *trans*-(3*R*,4*R*)-3,4-Dihydroxypyrrolidin-1-yl der Formel

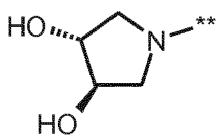

steht, worin

** die Verknüpfungsstelle mit dem Rest des Moleküls markiert,

sowie ihre Salze, Solvate und Solvate der Salze.

Eine weitere besondere Ausuhrungsform der vorliegenden Erfindung umfasst Verbindungen der Formel (I), in welcher

$R^1$ für *cis*-(*R,S*)-3,4-Dihydroxypyrrolidin-1-yl der Formel

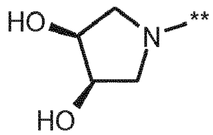

steht, worin
** die Verknüpfungsstelle mit dem Rest des Moleküls markiert,

sowie ihre Salze, Solvate und Solvate der Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

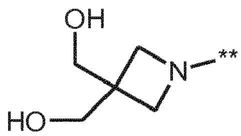

steht, worin
** die Verknüpfungsstelle mit dem Rest des Moleküls markiert,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^2$ für eine Gruppe der Formel

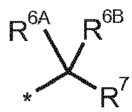

steht, worin
* die Verknüpfungsstelle mit dem N-Atom der Amid-Gruppierung markiert,
$R^{6A}$ Wasserstoff, Methyl oder Ethyl bedeutet,
$R^{6B}$ Methyl, Ethyl, Trifluormethyl, Isopropyl oder Cyclopropyl, bedeutet, und
$R^7$ Methyl, Ethyl, Difluormethyl, Trifluormethyl, 2,2,2-Trifluorethyl, Pentafluorethyl, Isopropyl, iso-Butyl, Methoxymethyl, Trifluormethoxymethyl oder Cyclopropyl bedeutet,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^2$ für eine Gruppe der Formel
$*-L^1-Ar^2$ steht, worin
* die Verknüpfungsstelle mit dem N-Atom der Amid-Gruppierung markiert,
$L^1$ eine Bindung oder eine Gruppe der Formel $-CR^{8A}R^{8B}-$ bedeutet, worin
$R^{8A}$ Wasserstoff darstellt,

$R^{8B}$ Wasserstoff, Methyl oder Trifluormethyl darstellt,
$Ar^2$ für eine Gruppe der Formel

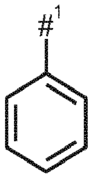 , 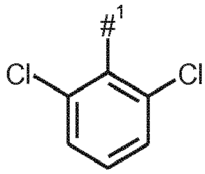 oder 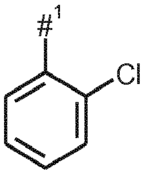

steht, worin
$\#^1$ die Verknüpfungsstelle mit dem Rest des Moleküls markiert,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^2$ für eine Gruppe der Formel

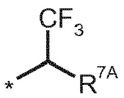 , 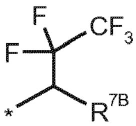 , 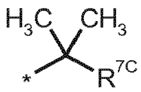 ,

steht, worin
* die Verknüpfungsstelle mit dem N-Atom der Amid-Gruppierung markiert,
$R^{7A}$ Trifluormethyl, Ethyl oder Cyclopropyl bedeutet,
$R^{7B}$ Methyl oder Ethyl bedeutet,
$R^{7C}$ Trifluormethyl oder Pentafluorethyl bedeutet,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^2$ für eine Gruppe der Formel

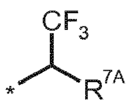

steht, worin
* die Verknüpfungsstelle mit dem N-Atom der Amid-Gruppierung markiert,
$R^{7A}$ Trifluormethyl, Ethyl oder Cyclopropyl bedeutet,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^2$ für eine Gruppe der Formel

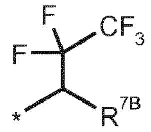

steht, worin
* die Verknüpfungsstelle mit dem N-Atom der Amid-Gruppierung markiert,
$R^{7B}$ Methyl oder Ethyl bedeutet,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^2$ für eine Gruppe der Formel

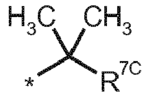

steht, worin

* die Verknüpfungsstelle mit dem N-Atom der Amid-Gruppierung markiert,
$R^{7C}$ Trifluormethyl oder Pentafluorethyl bedeutet,

sowie ihre N-Oxide, Salze, Solvate, Salze der N-Oxide und Solvate der N-Oxide und Salze.

Eine weitere besondere Ausuhrungsform der vorliegenden Erfindung umfasst Verbindungen der Formel (I), in welcher

$R^2$ für (2*S*)-1,1,1-Trifluorbutan-2-yl der Formel

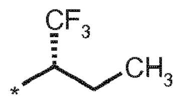

steht, worin
* die Verknüpfungsstelle mit dem N-Atom der Amid-Gruppierung markiert, steht,

sowie ihre Salze, Solvate und Solvate der Salze.

Eine weitere besondere Ausuhrungsform der vorliegenden Erfindung umfasst Verbindungen der Formel (I), in welcher

$R^2$ für (1*S*)-1-Cyclopropyl-2,2,2-trifluorethyl

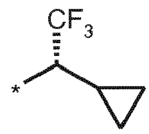

steht, worin
* die Verknüpfungsstelle mit dem N-Atom der Amid-Gruppierung markiert,

sowie ihre Salze, Solvate und Solvate der Salze.

Eine weitere besondere Ausführungsform der vorliegenden Erfindung umfasst Verbindungen der Formel (I),

in welcher

$R^2$ für 1,1,1,3,3,3-Hexafluorpropan-2-yl steht,

sowie ihre Salze, Solvate und Solvate der Salze.

Eine weitere besondere Ausführungsform der vorliegenden Erfindung umfasst Verbindungen der Formel (I), in welcher

$R^2$ für 3,3,4,4,4-Pentafluorbutan-2-yl steht,

sowie ihre Salze, Solvate und Solvate der Salze.

Eine weitere besondere Ausführungsform der vorliegenden Erfindung umfasst Verbindungen der Formel (I), in welcher

$R^2$ für 1,1,1,2,2-Pentafluorpentan-3-yl steht,

sowie ihre Salze, Solvate und Solvate der Salze.

Eine weitere besondere Ausführungsform der vorliegenden Erfindung umfasst Verbindungen der Formel (I), in welcher

$R^2$ für 1,1,1-Trifluor-2-methylpropan-2-yl steht,

sowie ihre Salze, Solvate und Solvate der Salze.

Eine weitere besondere Ausführungsform der vorliegenden Erfindung umfasst Verbindungen der Formel (I), in welcher

$Ar^1$ für eine Gruppe der Formel

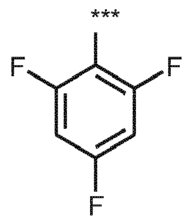

steht, worin

*** die Verknüpfungsstelle mit dem N-Atom markiert,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$Ar^1$ für eine Gruppe der Formel

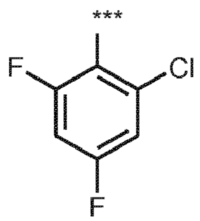

steht, worin
*** die Verknüpfungsstelle mit dem N-Atom markiert,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$Ar^1$ für eine Gruppe der Formel

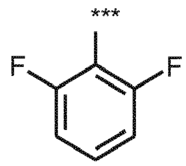

steht, worin
*** die Verknüpfungsstelle mit dem N-Atom markiert,

sowie ihre N-Oxide, Salze, Solvate, Salze der N-Oxide und Solvate der N-Oxide und Salze.
Eine weitere besondere Ausführungsform der vorliegenden Erfindung umfasst Verbindungen der Formel (I), in welcher

$Ar^1$ für eine Gruppe der Formel

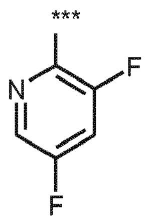

steht, worin

*** die Verknüpfungsstelle mit dem N-Atom markiert,

sowie ihre N-Oxide, Salze, Solvate, Salze der N-Oxide und Solvate der N-Oxide und Salze.
Die in den jeweiligen Kombinationen bzw. bevorzugten Kombinationen von Resten im Einzelnen angegebenen Reste-Definitionen werden unabhängig von den jeweiligen angegebenen Kombinationen der Reste beliebig auch durch Reste-Definitionen anderer Kombinationen ersetzt.
Ganz besonders bevorzugt sind Kombinationen von zwei oder mehreren der oben genannten Vorzugsbereiche und Ausführungsformen.
Die als bevorzugt, besonders bevorzugt und ganz besonders bevorzugt genannten Restedefinitionen gelten sowohl für die Verbindungen der Formel (I) als auch in entsprechender Weise für alle Zwischenprodukte.
Weiterer Gegenstand der Erfindung ist ein Verfahren zur Herstellung von erfindungsgemäßen Verbindungen der Formel (I), dadurch gekennzeichnet, dass man

[A] eine Verbindung der Formel (II-A)

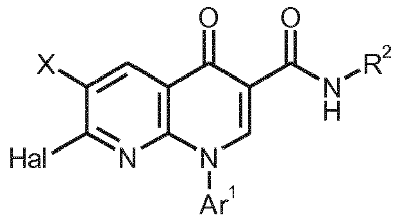

(II-A),

in welcher X, $R^2$ und $Ar^1$ die oben angegebenen Bedeutungen haben,

und

Hal für Fluor, Chlor, Brom oder Iod, bevorzugt für Chlor, steht,

mit einer Verbindung der Formel (III)

$R^1$-H (III),

in welcher $R^1$ die oben angegebene Bedeutung hat, und wobei $R^1$nicht Wasserstoff bedeutet, zum erfindungsgemäßen Carbonsäureamid der Formel (I-A)

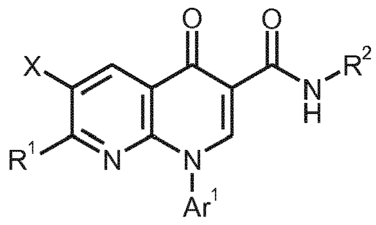

(I-A),

in welcher X, $R^1$, $R^2$ und $Ar^1$ die oben angegebenen Bedeutungen haben, und wobei $R^1$nicht Wasserstoff bedeutet, umsetzt,
oder

[B] eine Verbindung der Formel (IV)

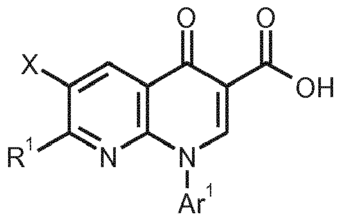

(IV),

in welcher X, $R^1$ und $Ar^1$ die oben angegebenen Bedeutungen haben, mit einer Verbindung der Formel (V)

$R^2$-$NH_2$ (V),

in welcher $R^2$ die oben angegebene Bedeutung hat, zum erfindungsgemäßen Carbonsäureamid der Formel (I)

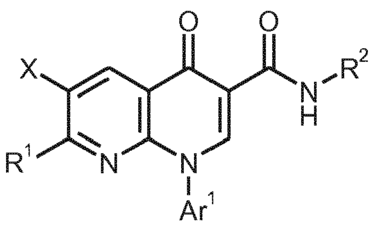

(I),

in welcher X, $R^1$, $R^2$ und $Ar^1$ die oben angegebenen Bedeutungen haben, umsetzt,
und gegebenenfalls die so erhaltenen Verbindungen der Formel (I) in ihre Enantiomere und/oder Diastereomere trennt und/oder mit den entsprechenden (i) Lösungsmitteln und/oder (*ii*) Basen oder Säuren in ihre Solvate, Salze und/oder Solvate der Salze überführt.

Die Umsetzung (II-A) + (III) → (I-A) kann über eine nukleophile Substitutionsreaktion oder eine Übergangsmetall-vermittelte Kupplungsreaktion erfolgen.

Die nukleophile Substitutionsreaktion wird vorzugsweise in Gegenwart einer Base durchgeführt. Als Basen für

den Verfahrensschritt (II-A) + (III) → (I-A) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium- oder Cäsiumcarbonat, Alkali-Alkoholate wie Lithium-, Natrium- oder Kalium-tert.-butylat, Alkalihydride wie Natrium- oder Kaliumhydrid, oder organische Amine wie *N,N*-Diisopropylethylamin (DIPEA), 1,5-Diazabicyclo[4.3.0]non-5-en (DBN) und 1,8-Diazabicyclo[5.4.0]undec-7-en (DBU). Bevorzugt wird *N,N*-Diisopropylethylamin (DIPEA) verwendet. Die Reaktion erfolgt im Allgemeinen in einem Temperaturbereich von 0°C bis +100°C, bevorzugt bei +23°C bis +80°C.

Inerte Lösungsmittel für den Verfahrensschritt (II-A) + (III) → (I-A) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Ethylacetat, Acetonitril, Pyridin, Dimethylsulfoxid, *N,N*-Dimethylformamid (DMF), *N,N'*-Dimethylpropylenharnstoff (DMPU) oder *N*-Methylpyrrolidon (NMP). Ebenso ist es möglich, Gemische der genannten Lösungsmittel zu verwenden. Bevorzugt wird Dimethylformamid (DMF) oder *N*-Methylpyrrolidon (NMP) verwendet.

Die Übergangsmetall-vermittelte Kupplungsreaktion für den Verfahrensschritt (II-A) + (III) → (I-A) wird in einer bevorzugten Ausführungsform in Gegenwart eines Palladiumkatalysators durchgeführt. Geeignete Palladium-Katalysatoren sind beispielsweise Palladium(II)acetat, Palladium(II)chlorid, Bis-(triphenylphosphin)palladium(II)chlorid, Bis(acetonitril)palladium(II)chlorid, Tetrakis(triphenylphosphin)palladium(0), Bis(dibenzylidenaceton)palladium(0), Tris(dibenzylidenaceton)dipalladium(0) oder [1,1'-Bis(diphenylphosphino)ferrocen]palladium(II)chlorid, gegebenenfalls in Kombination mit einem geeigneten Phosphin-Liganden wie zum Beispiel Triphenylphosphin, Tri-*tert.*-butylphosphin, 2-Dicyclohexylphosphino-2',4',6'-triisopropylbiphenyl (X-Phos), 2-Dicyclohexylphosphino-2',6'-dimethoxybiphenyl (S-Phos), 1,2,3,4,5-Pentaphenyl-1'-(di-*tert*.-butylphosphino)ferrocen (Q-Phos), 4,5-Bis(diphenylphosphino)-9,9-dimethylxanthen (Xantphos), 2,2'-Bis(diphenylphosphino)-1,1'-binaphthyl (BINAP), 2-Dicyclohexylphosphino-2'-(*N,N*-dimethylamino)biphenyl oder 2-Di-*tert*.-butylphosphino-2'-(*N,N*-dimethylamino)biphenyl.

Die Palladium-katalysierte Kupplungsreaktion (II-A) + (III) → (I-A) wird in der Regel in Gegenwart einer Base durchgeführt. Als solche eignen sich insbesondere Alkalicarbonate wie Natrium-, Kalium- oder Cäsiumcarbonat, Alkaliphosphate wie Natrium- oder Kaliumphosphat, Alkalifluoride wie Kalium- oder Cäsiumfluorid, oder Alkali-*tert*.-butylate wie Natrium- oder Kalium-*tert*.-butylat. Die Umsetzung erfolgt in einem inerten Lösungsmittel wie beispielsweise Toluol, 1,2-Dimethoxyethan, Tetrahydrofuran, 1,4-Dioxan, Dimethylsulfoxid (DMSO), *N,N*-Dimethylformamid (DMF), *N,N*-Dimethylacetamid (DMA) oder Mischungen hiervon in einem Temperaturbereich von +80°C bis +200°C, bevorzugt bei +80°C bis +150°C, wobei ein Erhitzen mittels Mikrowellenapparatur von Vorteil sein kann.

Bevorzugt wird für diese Kupplungsreaktion ein Katalysator/Ligand/Base-System bestehend aus Palladium(II)acetat, 4,5-Bis(diphenylphosphino)-9,9-dimethylxanthen (Xantphos) und Cäsium- oder Kaliumcarbonat eingesetzt und 1,4-Dioxan als Lösungsmittel verwendet.

Die Kupplungsreaktion (II-A) + (III) → (I-A) kann in einer weiteren bevorzugten Ausführungsform auch mit Hilfe eines Kupfer(I)-Katalysators, wie Kupfer(I)oxid, -bromid oder -iodid, in Gegenwart eines Kupfer-Liganden, wie trans-*N,N*'-Dimethyl-1,2-cyclohexandiamin, 8-Hydroxychinolin oder 1,10-Phenanthrolin, und einer anorganischen oder organischen Carbonat-Base, wie Kalium-, Cäsium- oder Bis(tetraethylammonium)carbonat, durchgeführt werden. Als inerte Lösungsmittel für diese Umsetzung eignen sich insbesondere Toluol, Xylol, 1,4-Dioxan, Acetonitril, Dimethylsulfoxid (DMSO), *N,N*-Dimethylformamid (DMF) oder Gemische hiervon, gegebenenfalls unter Zusatz von Wasser. Bevorzugt wird ein System bestehend aus Kupfer(I)iodid, trans-*N,N*'-Dimethyl-1,2-cyclohexandiamin und Kaliumcarbonat in Dimethylformamid eingesetzt. Die Reaktion erfolgt im Allgemeinen in einem Temperaturbereich von +50°C bis +200°C, bevorzugt bei +60°C bis +150°C.

Die Kupplungsreaktion (IV) + (V) → (I) [Amid-Bildung] kann entweder auf direktem Weg mit Hilfe eines Kondensations- oder Aktivierungsmittels oder über die Zwischenstufe eines aus (IV) erhältlichen Carbonsäurechlorids, Carbonsäureesters oder Carbonsäureimidazolids erfolgen.

Als solche Kondensations- oder Aktivierungsmittel eignen sich beispielsweise Carbodiimide wie *N,N*'-Diethyl-, *N,N'*-Dipropyl-, *N,N*'-Diisopropyl-, *N,N*'-Dicyclohexylcarbodiimid (DCC) oder *N*-(3-Dimethylaminopropyl)-*N'*-ethylcarbodiimid-Hydrochlorid (EDC), Phosgen-Derivate wie *N,N'*-Carbonyldiimidazol (CDI), Chlorameisensäureisopropylester oder Chlorameisensäureisobutylester, 1,2-Oxazolium-Verbindungen wie 2-Ethyl-5-phenyl-1,2-oxazolium-3-sulfat oder 2-*tert*.-Butyl-5-methylisoxazolium-perchlorat, Acylamino-Verbindungen wie 2-Ethoxy-1-ethoxycarbonyl-1,2-dihydrochinolin, α-Chlorenamine wie 1-Chlor-*N,N*,2-trimethylprop-1-en-1-amin, 1,3,5-Triazin-Derivate wie 4-(4,6-Dimethoxy-1,3,5-triazin-2-yl)-4-methylmorpholiniumchlorid, Phosphor-Verbindungen wie *n*-Propanphosphonsäureanhydrid (T3P, PPACA), Cyanophosphonsäurediethylester, Diphenylphosphorylazid (DPPA), Bis-(2-oxo-3-oxazolidinyl)-phosphorylchlorid, Benzotriazol-1-yloxy-tris(dimethylamino)phosphonium-hexafluorophosphat oder Benzotriazol-1-yloxy-tris(pyrrolidino)phosphonium-hexafluorophosphat (PyBOP), oder Uronium-Verbindungen wie *O*-(Benzotriazol-1-yl)-*N,N,N',N'*-tetramethyluronium-tetrafluoroborat (TBTU), *O*-(Benzotriazol-1-yl)-*N,N,N,N*'-tetramethyluronium-hexafluorophosphat (HBTU), *O*-(1*H*-6-Chlorbenzotriazol-1-yl)-1,1,3,3-tetramethyluronium-tetrafluoroborat (TCTU), *O*-(7-Azabenzotriazol-1-yl)-*N,N,N',N'*-tetramethyluronium-hexafluorophosphat (HATU) oder 2-(2-Oxo-1-(2*H*)-pyridyl)-1,1,3,3-tetramethyluronium-tetrafluoroborat (TPTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder tertiäre Aminbasen wie Triethylamin, *N*-Methylmorpholin (NMM), *N*-Methylpiperidin (NMP), *N,N*-Diisopropylethylamin (DIPEA), Pyridin oder 4-*N,N*-Dimethylaminopyridin (DMAP). Als Kondensations- oder Aktivierungsmittel bevorzugt eingesetzt wird *O*-(7-Azabenzotriazol-1-yl)-*N,N,N',N'*-tetramethyluronium-hexafluorophosphat (HATU) in Kombination mit *N,N*-Diisopropylethylamin (DIPEA) sowie *n*-Propanphosphonsäureanhydrid (T3P, PPACA) in Kombination mit *N,N*-Diisolpropylethylamin (DIPEA).

Die Verbindungen der Formel (II-A) können dadurch hergestellt werden, dass eine Carbonsäure-Verbindung der Formel (VI-A)

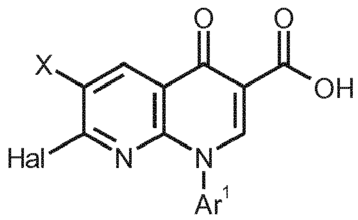

(VI-A),

in welcher X, Hal und $Ar^1$ die oben angegebenen Bedeutungen haben, mit einer Verbindung der Formel (V)

$R^2$-$NH_2$ (V),

in welcher $R^2$ die oben angegebene Bedeutung hat,
zum erfindungsgemäßen Carbonsäureamid der Formel (II-A)

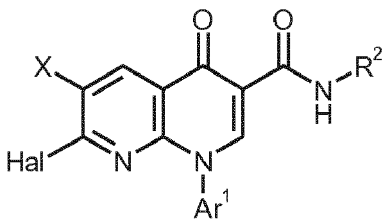

(II-A),

in welcher X, Hal, $R^2$ und $Ar^1$ die oben angegebenen Bedeutungen haben, umgesetzt wird.

Verbindungen der Formel (I-B) können analog zu der Umsetzung (VI-A) + (V) → (II-A) dadurch hergestellt werden, dass eine Carbonsäure-Verbindung der Formel (VI-B)

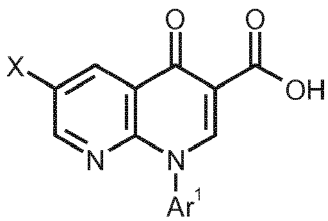

(VI-B),

in welcher X und $Ar^1$ die oben angegebenen Bedeutungen haben,
mit einer Verbindung der Formel (V)

$R^2$-$NH_2$ (V),

in welcher $R^2$ die oben angegebene Bedeutung hat,
zum erfindungsgemäßen Carbonsäureamid der Formel (I-B)

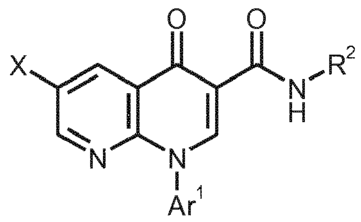

(I-B),

in welcher X, $R^2$ und $Ar^1$ die oben angegebenen Bedeutungen haben, umgesetzt wird.

Die Kupplungsreaktion (VI-A) + (V) → (II-A) oder (VI-B) + (V) → (I-B) [Amid-Bildung] kann entweder auf direktem Weg mit Hilfe eines Kondensations- oder Aktivierungsmittels oder über die Zwischenstufe eines aus (VI) erhältlichen Carbonsäurechlorids, Carbonsäureesters oder Carbonsäureimidazolids analog zu den bereits zur Umsetzung (IV) + (V) → (I) beschriebenen Bedingungen und Reagenzien erfolgen. Wird bei der Kupplungsreaktion zu (II-A) HATU als Aktivierungmittel eingesetzt, ist es möglich, dass entweder ein einzelnes, definiertes Produkt der allgemeinen Formel (II-A) erhalten wird, oder aber ein Gemisch mit einem "HATU-Addukt". Unter einem "HATU-Addukt" wird vorliegend eine Pseudohalogenid-Verbindung bezeichnet, wobei der Substituent Hal in der allgemeinen Formel (II-A) mit der Gruppe 3*H*-[1,2,3]Triazolo[4,5-b]pyridin-3-ol, auch als 1-Hydroxy-7-azabenzotriazol bezeichnet, ersetzt ist. Ein derartiges Gemisch einer Halogenverbindung der allgemeinen Formel (II-A) und einem "HATU-Addukt" kann analog zur beschriebenen Umsetzung ebenfalls direkt als Edukt für die Folgereaktion (nach (I) oder (VIII)) eingesetzt werden.

Bei zweistufiger Reaktionsführung über die aus (VI) erhältlichen Carbonsäurechloride oder Carbonsäureimidazolide wird die Kupplung mit der Amin-Komponente (V) in Gegenwart einer üblichen Base durchgeführt, wie beispielsweise Natrium- oder Kaliumcarbonat, Triethylamin, DIPEA, *N*-Methylmorpholin (NMM), *N*-Methylpiperidin (NMP), Pyridin, 2,6-Dimethylpyridin, 4-*N,N*-Dimethylaminopyridin (DMAP), 1,8-Diazabicyclo[5.4.0]undec-7-en (DBU), 1,5-Diazabicyclo[4.3.0]non-5-en (DBN), Natrium- oder Kaliummethanolat, Natrium- oder Kaliumethanolat, Natrium- oder Kalium-*tert.*-butylat oder Natrium- oder Kaliumhydrid.

Die Carbonsäureimidazolide selbst sind nach bekanntem Verfahren durch Umsetzung von (VI) mit N,N'-Carbonyldiimidazol (CDI) bei erhöhter Temperatur (+60°C bis +150°C) in einem entsprechend höhersiedenden Lösungsmittel wie N,N-Dimethylformamid (DMF) erhältlich. Die Herstellung der Carbonsäurechloride geschieht auf übliche Weise durch Behandlung von (VI) mit Thionylchlorid oder Oxalsäuredichlorid in einem inerten Lösungsmittel wie Dichlormethan oder THF.

Inerte Lösungsmittel für die genannten Kupplungsreaktionen sind - je nach eingesetztem Verfahren-beispielsweise Ether wie Diethylether, Diisopropylether, Methyl-*tert.*-butylether, Tetrahydrofuran, 1,4-Dioxan, 1,2-Dimethoxyethan oder Bis(2-methoxyethyl)ether, Kohlenwasserstoffe wie Benzol, Toluol, Xylol, Pentan, Hexan oder Cyclohexan, Halogenkohlenwasserstoffe wie Dichlormethan, Trichlormethan, Tetrachlormethan, 1,2-Dichlorethan, Trichlorethylen oder Chlorbenzol, oder polar-aprotische Lösungsmittel wie Aceton, Methylethylketon, Ethylacetat, Acetonitril, Butyronitril, Pyridin, Dimethylsulfoxid (DMSO), *N,N*-Dimethylformamid (DMF), *N,N'*-Dimethylpropylenharnstoff (DMPU) oder *N*-Methylpyrrolidinon (NMP). Auch können Gemische solcher Lösungsmittel eingesetzt werden. Bevorzugt wird *N,N*-Dimethylformamid (DMF) und Dichlormethan (DCM) in Kombination mit Triethylamin verwendet. Die Kupplungen werden im Allgemeinen in einem Temperaturbereich von 0°C bis +130°C, bevorzugt bei +20°C bis +30°C durchgeführt.

Die Verbindungen der Formel (IV-A) können in Abhängigkeit vom jeweiligen Substitutionsmuster dadurch hergestellt werden, dass man entweder

[C] eine Verbindung der Formel (VII-A)

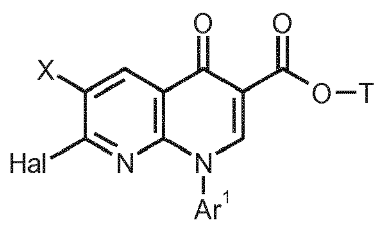

(VII-A),

in welcher X, Hal und $Ar^1$ die oben angegebenen Bedeutungen haben,
und

T für ($C_1$-$C_4$)-Alkyl oder Benzyl steht,

in einem ersten Schritt mit einer Verbindung der Formel (III)

$R^1$-H (III),

in welcher $R^1$ die oben angegebene Bedeutung hat, und wobei $R^1$ nicht Wasserstoff bedeutet, zu einer Verbindung der Formel (VIII-A)

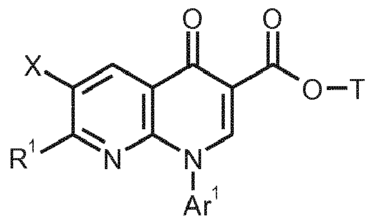

(VIII-A),

in welcher X, T, $R^1$ und $Ar^1$ die oben angegebenen Bedeutungen haben, und wobei $R^1$ nicht Wasserstoff bedeutet, umsetzt, und gegebenenfalls in einem zweiten Schritt den Ester-Rest T zur erfindungsgemäßen Carbonsäure der Formel (IV-A) abspaltet,

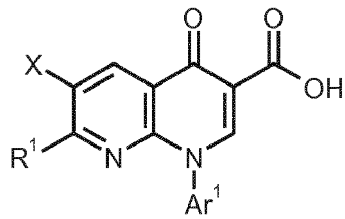

(IV-A),

in welcher X, $R^1$ und $Ar^1$ die oben angegebenen Bedeutungen haben, und wobei $R^1$ nicht Wasserstoff bedeutet, oder

[D] eine Verbindung der Formel (VI-A)

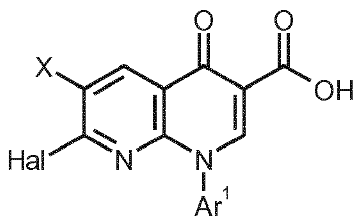

(VI-A),

in welcher X, Hal und $Ar^1$ die oben angegebenen Bedeutungen haben, mit einer Verbindung der Formel (III)

$R^1$-H (III),

in welcher $R^1$ die oben angegebene Bedeutung hat, und wobei $R^1$ nicht Wasserstoff bedeutet, zur erfindungsgemäßen Carbonsäure der Formel (IV-A),

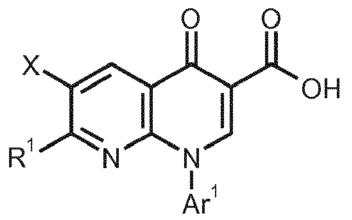

(IV-A),

in welcher X, $R^1$ und $Ar^1$ die oben angegebenen Bedeutungen haben, und wobei $R^1$nicht Wasserstoff bedeutet,

umsetzt.

Die Umsetzung (VII-A) + (III) → (VIII-A) [Weg C] oder die Umsetzung (VI-A) + (III) → (IV-A) [Weg D] kann jeweils über eine nukleophile Substitutionsreaktion oder eine Übergangsmetall-vermittelte Kupplungsreaktion analog zu den bereits zur Umsetzung (II-A) + (III) → (I-A) beschriebenen Bedingungen und Reagenzien erfolgen.

In einer bevorzugten Ausführungsform wird die Umsetzung nach Weg C als nukleophile Substitutionsreaktion in Gegenwart einer Base durchgeführt, bevorzugt wird *N,N*-Diisopropylethylamin (DIPEA) verwendet. Bevorzugt wird Dimethylformamid (DMF), *N*-Methylpyrrolidon (NMP) oder Acetonitril als Lösungsmittel verwendet.

In einer bevorzugten Ausführungsform wird die Umsetzung nach Weg D als Übergangsmetall-vermittelte Kupplungsreaktion in Gegenwart eines geeigneten Palladiumkatalysators durchgeführt. Bevorzugt wird ein System bestehend aus Palladium(II)-acetat in Kombination mit 4,5-Bis(diphenylphosphino)-9,9-dimethylxanthen (Xantphos), Cäsium- oder Kaliumcarbonat und 1,4-Dioxan als Lösungsmittel verwendet.

Die Abspaltung der Ester-Gruppe T im Verfahrensschritt (VIII-A) → (IV-A) wird nach üblichen Methoden durchgeführt, indem man den Ester in einem inerten Lösungsmittel mit einer Säure oder Base behandelt, wobei bei letzterer Variante das zunächst entstehende Salz der Carbonsäure durch nachfolgende Behandlung mit Säure in die freie Carbonsäure überführt wird. Im Falle der *tert*.-Butylester erfolgt die Esterspaltung vorzugsweise mit einer Säure. Benzylester können alternativ auch durch Hydrierung (Hydrogenolyse) in Gegenwart eines geeigneten Katalysators, wie beispielsweise Palladium auf Aktivkohle, abgespalten werden.

Als Lösungsmittel eignen sich für diese Reaktionen Wasser und die für eine Esterspaltung üblichen organischen Lösungsmittel. Hierzu zählen insbesondere Alkohole wie Methanol, Ethanol, *n*-Propanol, Isopropanol, *n*-Butanol oder *tert*.-Butanol, Ether wie Diethylether, Tetrahydrofuran, 1,4-Dioxan oder 1,2-Dimethoxyethan, oder andere Lösungsmittel wie Dichlormethan, Acetonitril, *N,N*-Dimethylformamid oder Dimethylsulfoxid. Ebenso ist es möglich, Gemische dieser Lösungsmittel einzusetzen. Im Falle einer basischen Ester-Hydrolyse werden bevorzugt Gemische von Wasser mit Tetrahydrofuran verwendet.

Als Basen sind die für eine Hydrolyse-Reaktion üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt wird wässrige Salzsäure (18 proz.) in einem Wasser/Tetra-hydrofuran-Gemisch eingesetzt.

Die Esterspaltung wird in der Regel in einem Temperaturbereich von -20°C bis +100°C, bevorzugt bei 23°C bis +120°C durchgeführt.

Die Verbindungen der Formel (VI-A) sowie der Formel (VII-A) können in Abhängigkeit vom jeweiligen Substitutionsmuster dadurch hergestellt werden, dass man in Analogie zu bekannten Verfahren (s. z.B. EP 0607825 A1, S. 25-26) ein 2,6-Dichlomicotinoyl-Acrylsäureester-Derivat der Formel (IX-A)

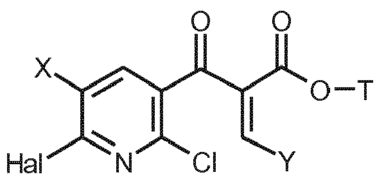

(IX-A),

in welcher X, Hal und T die oben angegebenen Bedeutungen haben,
und

Y für eine Abgangsgruppe wie Dimethylamino, Methoxy oder Ethoxy steht, sowie

in einer ersten Stufe, bevorzugt in Gegenwart einer geeigneten Base, mit einer Aminopyridin-Verbindung der Formel (X)

$Ar^1$-$NH_2$ (X),

in welcher $Ar^1$ die oben angegebene Bedeutungen hat, umsetzt,
und dann in einem zweiten Schritt in Gegenwart einer geeigneten Base zur Ester-Verbindung der Formel (VII-A)

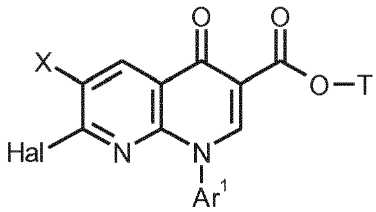
(VII-A),

in welcher X, Hal, $Ar^1$ und T die oben angegebene Bedeutung haben, umsetzt, sowie dann gegebenenfalls unter Hydrolyse-Bedingungen in einem weiteren Schritt die Ester-Verbindung (VII) in die Carbonsäure-Verbindung (VI-A)

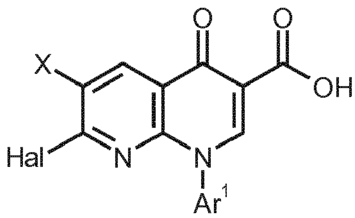
(VI-A),

in welcher X, Hal und $Ar^1$ die oben angegebenen Bedeutungen haben,
unter den in der Literatur bekannten Reaktionsbedingungen überführt.

Verbindungen der Formel (VI-B) sowie der Formel (VII-B) können analog zu der Umsetzung (IX-A) + (X) → (VII-A) → (VI-A) dadurch hergestellt werden, dass man in Analogie zu bekannten Verfahren (s. z.B. EP 0607825 A1, S. 25-26) ein 2,6-Dichlornicotinoyl-Acrylsäureester-Derivat der Formel (IX)

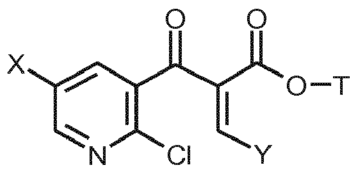
(IX-A),

in welcher X und T die oben angegebenen Bedeutungen haben,
und

Y für eine Abgangsgruppe wie Dimethylamino, Methoxy oder Ethoxy steht, sowie

in einer ersten Stufe, bevorzugt in Gegenwart einer geeigneten Base, mit einer Aminopyridin-Verbindung der Formel (X)

$Ar^1$-$NH_2$ (X),

in welcher $Ar^1$ die oben angegebene Bedeutungen hat, umsetzt,
und dann in einem zweiten Schritt in Gegenwart einer geeigneten Base zur Ester-Verbindung der Formel (VII-B)

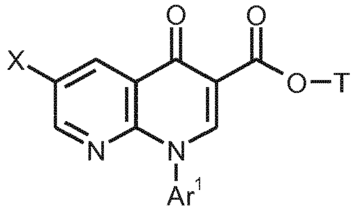

(VII-B),

in welcher X, Ar1 und T die oben angegebene Bedeutung haben,
umsetzt, sowie dann gegebenenfalls unter Hydrolyse-Bedingungen in einem weiteren Schritt die Ester-Verbindung (VII-B) in die Carbonsäure-Verbindung (VI-B)

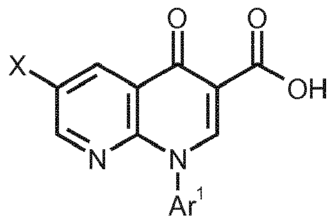

(VI-B),

in welcher X und Ar1 die oben angegebenen Bedeutungen haben,
unter den in der Literatur bekannten Reaktionsbedingungen überführt.

Die Verbindungen der Formel (IX) sind literaturbekannt (siehe z.B. EP 0607825 A1) oder können in Analogie zu literaturbekannten Verfahren hergestellt werden. Die Verbindungen der Formel (III), (V) und (X) sind kommerziell erhältlich oder als solche in der Literatur beschrieben, oder sie können auf für den Fachmann offenkundigem Wege in Analogie zu in der Literatur publizierten Metho-den hergestellt werden. Zahlreiche ausführliche Vorschriften sowie Literaturangaben zur Herstellung der jeweiligen Ausgangsmaterialien befinden sich auch im Experimentellen Teil im Abschnitt zur Herstellung der Ausgangsverbindungen und Intermediate.

Die Auftrennung von Stereoisomeren (Enantio- und/oder Diastereomeren) der erfindungsgemäßen Verbindungen der Formel (I) läßt sich nach üblichen, dem Fachmann geläufigen Methoden erreichen. Vorzugsweise werden hierfür chromatographische Verfahren an achiralen bzw. chiralen Trennphasen angewandt. Eine Trennung der erfindungsgemäßen Verbindungen in die entsprechenden Enantiomere und/ oder Diastereomere kann gegebenenfalls, je nach Zweckmäßigkeit, auch bereits auf der Stufe der Intermediate (II), (IV), oder (VIII) erfolgen, welche dann in separierter Form gemäß der zuvor beschriebenen Reaktionssequenz weiter umgesetzt werden. Für eine solche Auftrennung der Stereoisomere von Intermediaten werden gleichfalls bevorzugt chromatographische Verfahren an achiralen bzw. chiralen Trennphasen angewandt. Alternativ kann auch eine Trennung über diastereomere Salze der Carbonsäuren der Formel (IV) mit chiralen Amin-Basen erfolgen.

Die Herstellung der erfindungsgemäßen Verbindungen kann durch die folgenden Reaktionsschemata beispielhaft veranschaulicht werden:

**Schema 1**

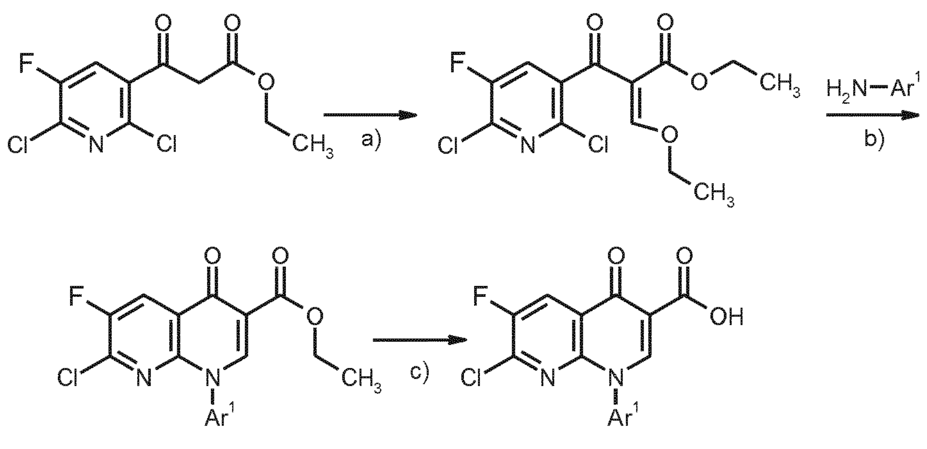

[a): Triethylorthoformiat, Acetanhydrid ; b): DIPEA, DCM, dann $K_2CO_3$; c): 18-proz. Salzsäure, THF, Wasser].

Schema 2

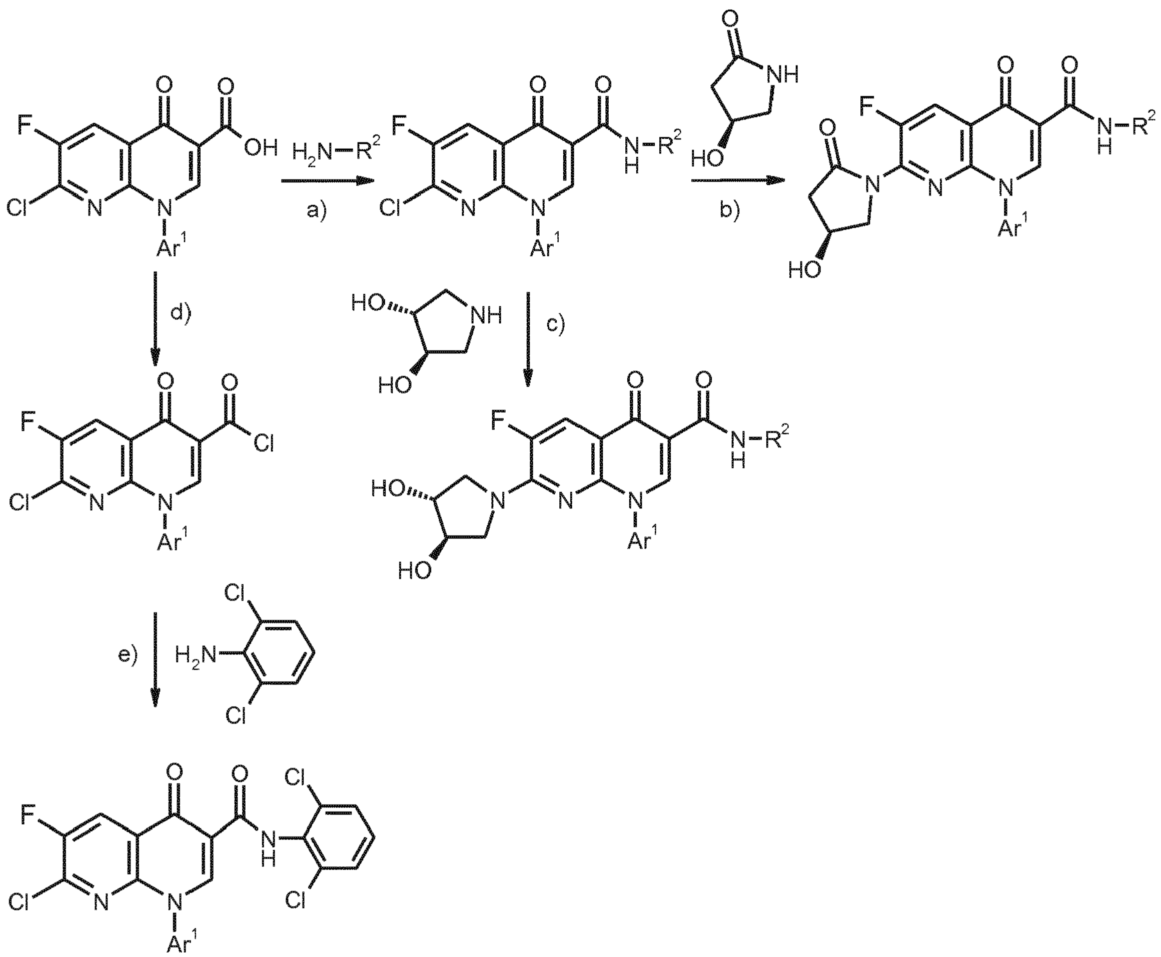


[a): HATU, DIPEA, DMF oder T3P, DIPEA, EtOAc; b): $Pd(OAc)_2$, Xantphos, $K_2CO_3$, 1,4-Dioxan; c): DIPEA, DMF; d): $(COCl)_2$, kat. DMF, THF; e): NaH, DMF oder Triethylamin, DCM].

**Schema 3**

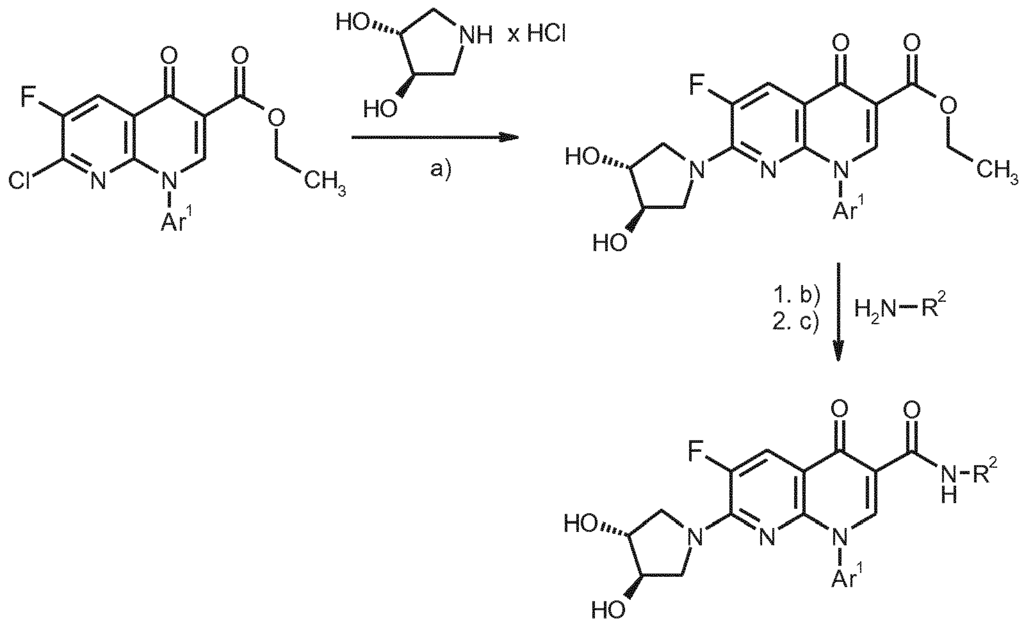


[a): DIPEA, DMF; b): wässr. LiOH, THF oder 18-proz. Salzsäure, THF, Wasser; c): HATU, DIPEA, DMF, RT.]

**Schema 4**

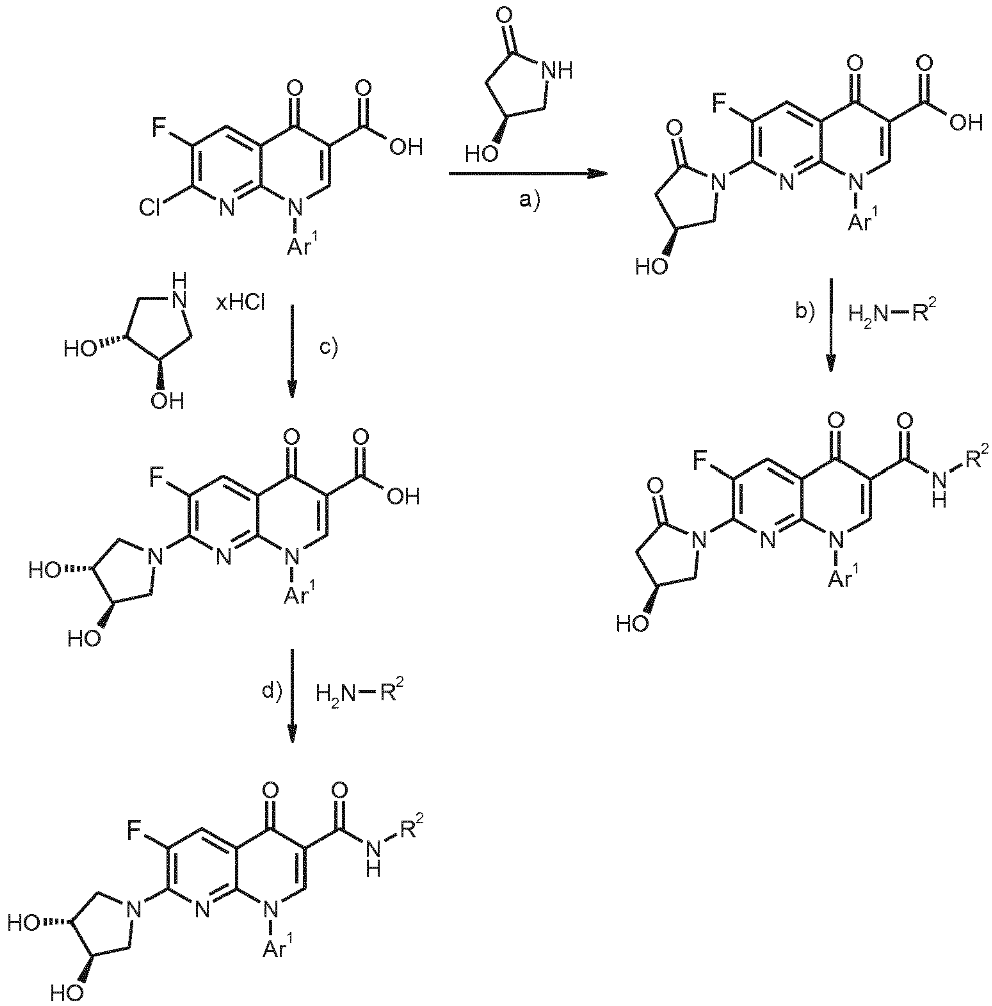


[a): Pd(OAc)$_2$, Xantphos, $K_2CO_3$, 1,4-Dioxan; b) HATU, DIPEA, DMF; c) DIPEA, DMF; d) HATU, DIPEA, DMF;]

Weitere erfindungsgemäße Verbindungen der Formel (I) können, falls zweckmäßig, auch durch Umwandlungen von funktionellen Gruppen einzelner Reste und Substituenten, insbesondere den unter $R^1$ und $R^2$ aufgeführten, hergestellt werden, wobei von anderen, nach obigen Verfahren erhaltenen Verbindungen der Formel (I) oder deren Vorstufen ausgegangen wird. Diese Umwandlungen werden nach üblichen, dem Fachmann geläufigen Methoden durchgeführt und umfassen beispielsweise Reaktionen wie nukleophile oder elektrophile Substitutionsreaktionen, Übergangsmetall-vermittelte Kupplungsreaktionen, Herstellungs- und Additionsreaktionen von Metallorganylen (z.B. Grignard-Verbindungen oder Lithiumorganylen), Oxidations- und Reduktionsreaktionen, Hydrierung, Halogenierung (z.B. Fluorierung, Bromierung), Dehalogenierung, Aminierung, Alkylierung und Acylierung, die Bildung von Carbonsäureestern, Carbonsäureamiden und Sulfonamiden, die Esterspaltung und -hydrolyse sowie die Einführung und Entfernung temporärer Schutzgruppen.

Die Erfindung betrifft in einem weiteren Aspekt Intermediate der allgemeinen Formel (II)

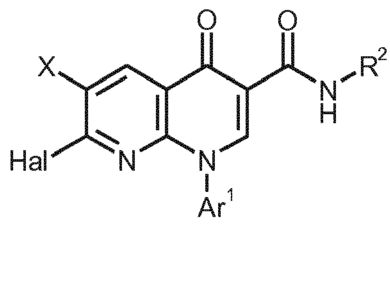

(II),

in welcher X, $R^2$ und $Ar^1$ die oben für Verbindungen der Formel (I) angegebenen Bedeutungen haben
und
Hal für Fluor, Chlor, Brom oder Iod, bevorzugt für Chlor, steht.

Die Erfindung betrifft in einem weiteren Aspekt Intermediate der allgemeinen Formel (IV)

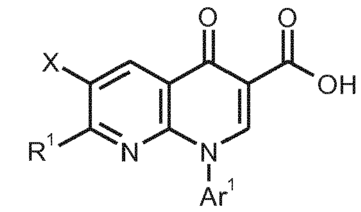
(IV),

in welcher X, $R^1$ und $Ar^1$ die oben für Verbindungen der Formel (I) angegebenen Bedeutungen haben.

Die Erfindung betrifft in einem weiteren Aspekt die Verwendung einer Verbindung der allgemeinen Formel (II)

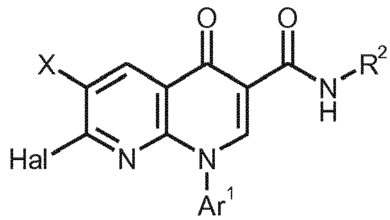
(II),

in welcher X, $R^2$ und $Ar^1$ die oben für Verbindungen der Formel (I) angegebenen Bedeutungen haben
und
Hal für Fluor, Chlor, Brom oder Iod, bevorzugt für Chlor, steht.
oder
einer Verbindung der allgemeinen Formel (IV)

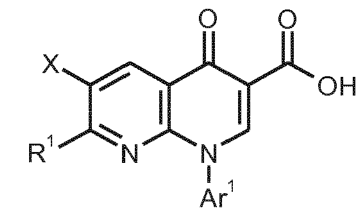
(IV),

in welcher X, $R^1$ und $Ar^1$ die oben für Verbindungen der Formel (I) angegebenen Bedeutungen haben,
zur Herstellung einer Verbindung der allgemeinen Formel (I) wie oben definiert.

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren. Die erfindungsgemäßen Verbindungen besitzen wertvolle pharmakologische Eigenschaften und können zur Behandlung und/ oder Prophylaxe von Erkrankungen bei Menschen und Tieren verwendet werden.

Die erfindungsgemäßen Verbindungen stellen positive allosterische Modulatoren des muskarinergen M2-Rezeptors dar und eignen sich daher zur Behandlung und/oder Prävention von Erkrankungen und pathologischen Prozessen, insbesondere kardiovaskulärer Erkrankungen und/oder Nierenerkrankungen, bei denen im Zuge einer Fehlregulation des autonomen Nervensystems bzw. eines Ungleichgewichtes zwischen der Aktivität des sympathischen und parasympathischen Teils des autonomen Nervensystems der M2-Rezeptor involviert ist.

Gegenstand der vorliegenden Erfindung sind positive allosterische Modulatoren des muskarinergen M2-Rezeptors. Allosterische Modulatoren weisen gegenüber klassischen, orthosterischen Liganden deutliche Unterschiede auf. Der Effekt eines allosterischen Modulators ist selbstlimitierend, wenn er in hohen Konzentrationen die Bindung des Agonisten stabilisiert. Darüber hinaus kann sich die Wirkung eines allosterischen Modulators nur in der Anwesenheit des endogenen Liganden entfalten. Der allosterische Modulator selbst hat keinen direkten Einfluss auf die Rezeptoraktivierung. Daraus ergibt sich eine räumliche und zeitliche Spezifität der allosterischen Wirkung. Die als Kooperativität bezeichnete gegenseitige Beeinflussung von allosterischen und orthosterischen Liganden hinsichtlich Affinität und intrinsischer Aktivität wird von den beiden Liganden bestimmt. Im Falle eines positiven allosterischen Modulators werden die Effekte des orthosterischen Liganden verstärkt (positive Kooperativität). Aufgrund ihrer Fähigkeit, Rezeptorkonformationen in Gegenwart eines orthosterischen Liganden zu modulieren, können allosterische Liganden eine Feinabstimmung pharmakologischer Effekte hervorrufen.

Im Sinne der vorliegenden Erfindung sind unter Erkrankungen des Herzkreislauf-Systems beziehungsweise kardiovaskulären Erkrankungen beispielsweise die folgenden Erkrankungen zu verstehen: akute und chronische Herzinsuffizienz, arterielle Hypertonie, koronare Herzerkrankung, stabile und instabile Angina pectoris, myokardiale Ischämie, Myokardinfarkt, Schock, Atherosklerose, Herzhypertrophie, Herzfibrose, atriale und ventrikuläre Arrhythmien, Tachykardie, transitorische und ischämische Attacken, Hirnschlag, Präeklampsie, entzündliche kardiovaskuläre Erkrankungen, periphere und kardiale Gefäßerkrankungen, periphere Durchblutungsstörungen, arterielle pulmonale Hypertonie, Spasmen der Koronararterien und peripherer Arterien, Thrombosen, thromboembolische Erkrankungen, Ödembildung wie zum Beispiel pulmonales Ödem, Hirnödem, renales Ödem oder Herzinsuffizienz-bedingtes Ödem, sowie Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädi-gungen (Vasculitis), Reperfusionsschäden, arterielle und venöse Thrombosen, Mikroalbuminurie, Herzmuskelschwäche, endotheliale Dysfunktion, periphere und kardiale Gefäßerkranlcungen, periphere Durchblutungsstörungen, Herzinsuffizienz-bedingtes Ödem, erhöhte Spiegel von Fibrinogen und von LDL geringer Dichte sowie erhöhte Konzentrationen von Plasminogenaktivator-Inhibitor 1 (PAI 1).

Im Sinne der vorliegenden Erfindung umfasst der Begriff Herzinsuffizienz auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, Herzinsuffizienz bei erhaltener systolischer Pumpfunktion (HFpEF), diastolische Herzinsuffizienz sowie Herzinsuffizienz bei reduzierter systolischer Pumpfunktion (HFrEF systolische Herzinsuffizienz).

Im Sinne der vorliegenden Erfindung umfasst der Begriff atriale und ventrikuläre Arrhythmien auch spezifischere oder verwandte Krankheitsformen wie: Vorhofflimmern, paroxysmales Vorhofflimmern intermittierendes Vorhofflimmern, permanentes Vorhofflimmern, Vorhofflattern, Sinusarrhythmie, Sinustachykardie, passive Heterotopie, aktive Heterotopie, Ersatzsystolen, Extrasystolen, Reizleitungsstörungen, Sick-sinus-Syndrom, hypersensitiver Karotissinus, Tachykardien, AV-Knoten Reenhytachykardie, atriventrikuläre Reentrytachykardie, WPW-Syndrom (Wolff-Parkinson-White), Mahaim-Tachykardie, verborgene akzessorische Leitungsbahn, permanente junktionale Re-entrytachykardie, fokale atriale Tachykardie, junktional ektope Tachykardie, atriale Reentrytachykardie, Kammertachykardie, Kammerflattern, Kammerflimmern, plötzlicher Herztod.

Im Sinne der vorliegenden Erfindung umfasst der Begriff koronare Herzerkrankung auch spezifischere oder verwandte Krankheitsformen wie: ischämische Herzkrankheit, stabile Angina pectoris, akutes Koronarsyndrom, instabile Angina pectoris, NSTEMI (nicht-ST-Streckenhebungsinfarkt), STEMI (ST-Streckenhebungsinfarkt), ischämische Herzmuskelschädigung, Herzrhythmusstörungen, und Myokardinfarkt.

Die erfindungsgemäßen Verbindungen sind weiter geeignet für die Prophylaxe und/oder Behandlung der polyzystischen Nierenkrankheit (PCKD) und des Syndroms der inadäquaten ADH-Sekretion (SIADH).

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen.

Im Sinne der vorliegenden Erfindung umfasst der Begriff akute Niereninsuffizienz akute Erscheinungsformen der Nierenerkrankung, des Nierenversagens und/ oder der Niereninsuffizienz mit und ohne Dialysepflicht, wie auch zugrundeliegende oder verwandte Nierenerkrankungen wie renale Hypoperfusion, intradialytische Hypotonie, Volumenmangel (z.B. Dehydratation, Blutverlust), Schock, akute Glomerulonephritis, hämolytisch-urämisches Syndrom (HUS), vaskuläre Kathastrophe (arterielle oder venöse Thrombose oder Embolie), Cholesterinembolie, akute Bence-Jones-Niere bei Plasmozytom, akute supravesikal oder subvesikale Abflussbehinderungen, immunlogische Nierenerkrankungen wie Nierentransplant-atabstoßung, Immunkomplex-induzierte Nierenerkrankungen, tubuläre Dilatation, Hyper-phosphatämie und/ oder akute Nierenerkrankungen, die durch die Notwendigkeit zur Dialyse charakterisiert werden können, sowie bei Teilresektionen der Niere, Dehydratation durch forcierte Diurese, unkontrolliertem Blutdruckanstieg mit maligner Hypertonie, Harnwegs-obstruktion und -infekt und Amyloidose sowie Systemerkrankungen mit glomerulärer Beteiligung, wie rheumatologisch-immunologische Systemerkrankungen, wie beispielsweise Lupus erythematodes, Nierenarterienthrombose, Nierenvenenthrombose, Analgetikanephropathie und renal-tubuläre Azidose, sowie Röntgen-Kontrastmittel- sowie Medikamenten-induzierte akute interstitielle Nierenerkrankungen.

Im Sinne der vorliegenden Erfindung umfasst der Begriff chronische Niereninsuffizienz chronische Erscheinungsformen der Nierenerkrankung, des Nierenversagens und/ oder der Niereninsuffizienz mit und ohne Dialysepflicht, wie auch zugrundeliegende oder verwandte Nierenerkrankungen wie renale Hypoperfusion, intradialytische Hypotonie, obstruktive Uropathie, Glomerulopathien, glomeruläre und tubuläre Proteinurie, renale Ödeme, Hämaturie, primäre, sekundäre sowie chronische Glomerulonephritis, membranöse und membrano-proliferative Glomerulonephritis, Alport-Syndrom, Glomerulosklerose, tubulointerstitielle Erkrankungen, nephropathische Erkrankungen wie primäre und angeborene Nierenerkrankung, Nierenentzündung, immunlogische Nierenerkrankungen wie Nierentransplantatabstoßung, Immunkomplex-induzierte Nierenerkrankungen, diabetische und nicht-diabetische Nephro-pathie, Pyelonephritis, Nierenzysten, Nephrosklerose, hypertensive Nephrosklerose und nephrotisches Syndrom, welche diagnostisch beispielsweise durch abnorm verminderte Kreatinin- und/ oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/ oder die Notwendigkeit zur Dialyse charakterisiert werden können, sowie bei Nierenzellkarzinomen, nach Teilresektionen der Niere, Dehydratation durch forcierte Diurese, unkontrollierter Blutdruckanstieg mit maligner Hypertonie, Harnwegsobstruktion und -infekt und Amyloidose sowie Systemerkrankungen mit glomerulärer Beteiligung, wie rheumatologisch-immunologische Systemerkrankungen, wie beispielsweise Lupus erythematodes, sowie Nierenarterienstenose, Nierenarterienthrombose, Nierenvenenthrombose, Analgetikanephropathie und renal-tubuläre Azidose zu verstehen. Weiterhin Röntgen-Kontrastmittel- sowie Medikamenten-induzierte chronische interstitielle Nierenerkrankungen, Metabolisches Syndrom und Dyslipidämie. Die vorliegende Erfindung umfasst auch die Verwendung der erfindungsgemäßen Verbindungen zur Behandlung und/ oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von pulmonaler arterieller Hypertonie (PAH) und anderen Formen der pulmonalen Hypertonie (PH), der chronisch-obstruktiven Lungenerkrankung (COPD), des akuten Atemwegssyndroms (ARDS), der akuten Lungenschädigung (ALI), der alpha-1-Antitrypsin-Defizienz (AATD), der Lungenfibrose, des Lungenemphysems (z.B. durch Zigarettenrauch induziertes Lungenemphysem), der zystischen Fibrose (CF), von akutem Koronarsyndrom (ACS), Herzmuskelentzündungen (Myokarditis) und anderen autoimmunen Herzerkrankungen (Perikarditis, Endokarditis, Valvolitis, Aortitis, Kardiomyopathien), kardiogenem Schock, Aneurysmen, Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen.

Die erfindungsgemäßen Verbindungen können weiterhin verwendet werden zur Behandlung und/ oder Prophylaxe von asthmatischen Erkrankungen unterschiedlicher Schweregrade mit intermittierendem oder persistierendem Verlauf (refraktives Asthma, bronchiales Asthma, allergisches Asthma, intrinsisches Asthma, extrinsisches Asthma, durch Medikamente oder durch Staub induziertes Asthma), von verschiedenen Formen der Bronchitis (chronische Bronchitis, infektiöse Bronchitis, eosinophile Bronchitis), von Bronchiolitis obliterans, Bronchiektasie, Pneumonie, idiopathischer interstitieller Pneumonie, Farmerlunge und verwandten Krankheiten, Husten- und Erkältungskrankheiten (chronischer entzündlicher Husten, iatrogener Husten), Nasenschleimhautentzündungen (einschließlich medikamentöse Rhinitis, vasomotorische Rhinitis und jahreszeitabhängige, allergische Rhinitis, z.B. Heuschnupfen) und von Polypen.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lemleistung oder Gedächtnisleistung nach kognitiven Störungen, wie sie insbesondere bei Situationen/Krankheiten/Syndromen auftreten wie "Mild cognitive impairment", altersassoziierte Lern- und Gedächtnisstörungen, altersassoziierte Gedächtnisverluste, vaskuläre Demenz, Schädel-Hirn-Trauma, Schlaganfall, Demenz, die nach Schlaganfällen auftritt ("post stroke dementia"), posttraumatisches Schädel-Hirn-Trauma, allgemeine Konzentrationsstörungen, Konzentrationsstörungen bei Kindern mit Lern- und Gedächtnisproblemen, Alzheimer'sche Krankheit, Demenz mit Lewy-Körperchen, Demenz mit Degeneration der Frontallappen einschließlich des Pick's-Syndroms, Parkinson'sche Krankheit, progressiver nuclear palsy, Demenz mit corticobasaler Degeneration, Amyolateralsklerose (ALS), Huntington'sche Krankheit, Demyelinisation, Multiple Sklerose, thalamische Degeneration, Creutzfeld-Jacob-Demenz, HIV-Demenz, Schizophrenie mit Demenz oder Korsakoff-Psychose. Sie eignen sich auch zur Behandlung und/oder Prävention von Erkrankungen des Zentralnervensystems wie Angst-, Spannung- und Depressionszuständen, bipolarer Störung, zentral-nervös bedingten Sexuaidysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Behandlung und/oder Prophylaxe von urologischen Erkrankungen wie: Harninkontinenz, insbesondere Streßinlcontinenz, Dranginkontinenz, Reflexinkontinenz und Überlaufinkontinenz, Detrusor-hyperaktivität, neurogene Detrusorhyperaktivität, idiopathische Detrusorhyperaktivität, benigne Prostatahyperplasie (BPH-Syndrom), Symptome des unteren Harntraktes (LUTS = lower urinary tract symptoms).

Die erfindungsgemäßen Verbindungen eignen sich weiterhin zur Behandlung und/oder Prävention von gastroenterologischen Erkrankungen, wie Ösophaguskrankheiten, Erbrechen, Achalasie, gastroösophageale Refluxkranheit, Magenkrankheiten, wie Gastritis, Darmerkrankungen, wie Diarrhö, Ostipation, Malassimilationssyndrom, Gallensäureverlustsyndrom, Morbus Crohn, Colitis ulcerosa, mikroskopische Colitis, und Reizdarmsyndrom.

Die erfindungsgemäßen Verbindungen eignen sich weiterhin zur Behandlung und/oder Prävention von Schmerzzuständen, wie beispielsweise Menstruationstörungen, Dysmenorrhoe, Endometriose, Frühgeburt, Tokolyse.

Aufgrund ihres biochemischen und pharmakologischen Eigenschaftsprofils eignen sich die erfindungsgemäßen Verbindungen insbesondere zur Behandlung und/oder Prävention von Herzinsuffizienz, koronarer Herzerkrankung, atrialen und ventrikulären Arrhythmien, Niereninsuffizienz und Nephropathien.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Die erfindungsgemäßen Verbindungen eignen sich weiterhin zur Behandlung und/oder Prävention von ophthalmologischen Erkrankungen, wie beispielsweise von Glaukom, altersbedingter Makuladegeneration (AMD), trockener (nicht-exsudativer) AMD, feuchter (exsudativer, neovaskulärer) AMD, choroidaler Neovaskularisation (CNV), diabetiseher Retinopathie, atrophischen Veränderungen des retinalen Pigmentepithels (RPE), hypertrophischen Veränderungen des retinalen Pigmentepithels, Makulaödem, diabetischem Makulaödem, Netzhautvenenverschluss, choroidalem Netzhautvenenverschluss, Makulaödem auf grund von Netzhautvenenverschluss, Angiogenese an der Vorderseite des Auges wie kornealer Angiogenese beispielsweise nach Keratitis, Hornhauttransplantation oder Keratoplastik, kornealer Angiogenese auf grund von Hypoxie (durch extensives Tragen von Kontaktlinsen), Pterygium conjunctivae, subretinalem Ödem und intraretinalem Ödem. Des weiteren eignen sich die erfindungsgemäßen Verbindungen zur Behandlung und/oder Prävention von erhöhtem und hohem Augeninnendruck als Folge von traumatischem Hyphaema, periorbitalem Ödem, postoperativer viskoelastischer Retention oder intraokularer Entzündung.

Außerdem sind die erfindungsgemäßen Verbindungen zur Behandlung und/ oder Prophylaxe von Hepatitis, Neoplasma, Osteoporose, Glaukom und Gastroparese geeignet.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerz, Neuralgien und Tinnitus eingesetzt werden.

Die zuvor genannten, gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Weiterer Gegenstand der vorliegenden Erfindung ist somit die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Arzneimittel, enthaltend mindestens eine der erfindungsgemäßen Verbindungen, zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung sind die erfindungsgemäßen Verbindungen zur Verwendung in einem Verfahren zur Behandlung und/oder Prävention von Erkrankungen, insbesondere der zuvor genannten Erkrankungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als hierfür geeignete Kombinationswirkstoffe seien beispielhaft und vorzugsweise genannt:

- den Blutdruck senkende Wirkstoffe, beispielhaft und vorzugsweise aus der Gruppe der Calcium-Antagonisten, Angiotensin AII-Antagonisten, ACE-Inhibitoren, NEP-Inhibitoren, Vasopeptidase-Inhibitoren, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika;

- antiarrhythmische Wirkstoffe, wie beispielsweise und vorzugsweise Natriumkanalblocker, Betarezeptorenblocker, Kaliumkanalblocker, Kalziumantagonisten, If-Kanalblocker, Digitalis, Parasympatholytika (Vagoliytika), Sympathomimetika und andere Antiarrhythmika wie Adenosin, Adenosinrezeptor Agonisten sowie Vernakalant.

- positiv-inotrope Wirkstoffe, wie z.B. Herzglykoside (Dogoxin), beta-adrenerge und dopaminerge Agonisten, wie Isoprenalin, Adrenalin, Noradrenalin, Dopamin oder Dobutamin;

- Vasopressin-Rezeptor-Antagonisten, wie beispielsweise und vorzugsweise Conivaptan, Tolvaptan, Lixivaptan, Mozavaptan, Satavaptan, SR-121463, RWJ 676070 oder BAY 86-8050, sowie die in WO 2010/105770, WO2011/104322 und WO 2016/071212 beschriebenen Verbindungen;

- Natriuretische Peptide, wie z.B. atriales natriuretisches Peptid (ANP), natriuretisches Peptid Typ B (BNP, Nesiritid) natriuretisches Peptid Typ C (CNP) oder Urodilatin;

- Aktivatoren des kardialen Myosins, wie z.B. Omecamtiv Mecarbil (CK-1827452);

- Calcium-Sensitizer, wie z.B. Levosimendan

- den Energiestoffwechsel des Herzens beeinflussende Verbindungen, wie beispielhaft und vorzugsweise Etomoxir, Dichloracetat, Ranolazine oder Trimetazidine, volle oder partielle Adenosin A1 Receptor Agonisten wie GS-9667 (früher bekannt als CVT-3619), Capadenoson, Neladenoson und BAY 1067197;

- Verbindungen die die Herzfrequenz beinflüssen, wie z.B. Ivabradin

- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, Udenafil, Desantafil, Avanafil, Mirodenafil, Lodenafil or PF-00489791;

- antithrombotisch wirkende Mittel, beispielhaft und vorzugsweise aus der Gruppe der Thrombozytenaggregationshemmer, der Antikoagulantien oder der profibrinolytischen Substanzen;

- bronchodilatorisch wirkende Mittel, beispielhaft und vorzugsweise aus der Gruppe der betaadrenergen Rezeptor-Agonisten, wie insbesondere Albuterol, Isoproterenol, Metaproterenol, Terbutalin, Formoterol oder Salmeterol, oder aus der Gruppe der Anticholinergika, wie insbesondere Ipratropiumbromid;

- anti-inflammatorisch wirkende Mittel, beispielhaft und vorzugsweise aus der Gruppe der Glucocorticoide, wie insbesondere Prednison, Prednisolon, Methylprednisolon, Triamcinolon, Dexamethason, Beclomethason, Betamethason, Flunisolid, Budesonid oder Fluticason sowie die nichtsteroidale anti-inflammatorische Wirkstoffe (NSAIDs), wie insbesondere Acetylsalicylsäure (Aspirin), Ibuprofen and Naproxen, 5-Aminosalicylic Säure-Derivate, Leukotrien-Antagonists , TNF-alpha-Inhibitoren und Chemokin-Receptor Antagonisten, wie CCR1 2 und/or 5 Inhibitoren;

- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma-und/oder PPAR-8-Agonisten, Cholesterin-Absorptionshemmer, Lipase-Inhibitoren, polymeren Gallensäureadsorber, Gallensäure-Reabsoptionshemmer und Lipoprotein(a)-Antagonisten.

- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren;

- Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Chymase, Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12) und Neutrophil-Elastase (HNE), wie z.B. Sivelestat oder DX-890;

- Verbindungen, die die Bindung von Serotonin an dessen Rezeptor blockieren, beispielhaft und vorzugsweise Antagonisten des 5-$HT_{2b}$-Rezeptors;

- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;

- NO-unabhängige, jedoch Häm-abhängige Stimulatoren der löslichen Guanylatcyclase, wie insbesondere die in WO 00/06568, WO 00/06569, WO 02/42301, WO 03/095451, WO 2011/147809, WO 2012/004258, WO 2012/028647 und WO 2012/059549 beschriebenen Verbindungen;

- NO- und Häm-unabhängige Aktivatoren der löslichen Guanylatcyclase, wie insbesondere die in WO 01/19355, WO 01/19776, WO 01/19778, WO 01/19780, WO 02/070462 und WO 02/070510 beschriebenen Verbindungen;

- Verbindungen, die die Synthese von cGMP steigern, wie beispielsweise sGC Modulatoren, wie beispielhaft und vorzugsweise Riociguat, Cinaciguat, Vericiguat oder BAY 1101042;

- Prostacyclin-Analoga, wie beispielhaft und vorzugsweise Iloprost, Beraprost, Treprostinil oder Epoprostenol;

- Verbindungen, die die lösliche Epoxidhydrolase (sEH) inhibieren, wie beispielsweise *N,N'*-Di-cyclohexylharnstoff, 12-(3-Adamantan-1-yl-ureido)-dodecansäure oder 1-Adamantan-1-yl-3-{5-[2-(2-ethoxyethoxy)ethoxy]pentyl}-harnstoff;

- den Glucosestoffwechsel verändernde Wirkstoffe, wie beispielsweise Insuline, Biguanide, Thiazolidinedione, Sulfonylharnstoffe, Acarbose, DPP4 Inhibitoren, GLP-1 Analoga oder SGLT-1 Inhibitor.

Bei einer bevorzugten Ausführungsform der Erfindung werden die erfindungsgemäßen Verbindungen in Kombination mit einem Kinase-Inhibitor, wie beispielhaft und vorzugsweise Bortezomib, Canertinib, Erlotinib, Gefitinib, Imatinib, Lapatinib, Lestaurtinib, Lonafarnib, Nintedanib, Dasatinib, Nilotinib, Bosutinib, Axitinib, Telatinib, Imatinib, Brivanib, Pazopanib, Pegaptinib, Pelitinib, Semaxanib, Sorafenib, Regorafenib, Sunitinib, Tandutinib, Tipifarnib, Vatalanib, Fasudil, Lonidamin, Leflunomid, BMS-3354825 oder Y-27632, eingesetzt.
Bei einer bevorzugten Ausführungsform der Erfindung werden die erfindungsgemäßen Verbindungen in Kombination mit einem Serotonin-Rezeptor-Antagonisten, wie beispielhaft und vorzugsweise PRX-08066, eingesetzt.
Unter antithrombotisch wirkenden Mittel werden vorzugsweise Verbindungen aus der Gruppe der Thrombozytenaggregationshemmer, der Antikoagulantien oder der profibrinolytischen Substanzen verstanden.
Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.
Bei einer bevorzugten Ausführungsform der Erfindung werden die erfindungsgemäßen Verbindungen in Kombination mit einem Thrombin-Inhibitor, wie beispielhaft und vorzugsweise Dabigatran, Ximelagatran, Melagatran, Bivalirudin oder Clexane, verabreicht.
Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.
Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Edoxaban (DU-176b), Apixaban,

Otamixaban, Fidexaban, Razaxaban, Fondaparinux, Idraparinux, PMD-3112, Y*N*-150, KFA-1982, EMD-503982, MC*N*-17, mLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika ver-standen.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, Irbesartan, Olmesartan, Eprosartan oder Azilsartan oder einem dualen Angiotensin AII-Antagonisten/NEP-Inhibitor, wie beispielsweise und vorzugsweise Entresto (LCZ696, Valsartan/Sacubitril), verabreicht.

Bei einer bevorzugten Ausführungsform der Erfindung werden die erfindungsgemäßen Verbindungen in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan, Avosentan, Macitentan, Atrasentan oder Sitaxsentan, verabreicht.

Bei einer bevorzugten Ausführungsform der Erfindung werden die erfindungsgemäßen Verbindungen in Kombination mit einem TGFbeta Antagonisten, wie beispielhaft und vorzugsweise Pirfenidone oder Fresolimumab, verabreicht.

Bei einer bevorzugten Ausführungsform der Erfindung werden die erfindungsgemäßen Verbindungen in Kombination mit einem TNFalpha Antagonisten, wie beispielhaft und vorzugsweise Adalimumab, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit HIF-PH Inhibitoren, wie beispielhaft und vorzugsweise Molidustat oder Roxadustat verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, Finerenon verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095, SB-772077, GSK-269962A oder BA-1049, verabreicht.

Bei einer bevorzugten Ausführungsform der Erfindung werden die erfindungsgemäßen Verbindungen in Kombination mit einem Diuretikum, wie beispielhaft und Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-8-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), Anacetrapib, JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-8-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Bei einer bevorzugten Ausführungsform der Erfindung werden die erfindungsgemäßen Verbindungen in Kombination mit sGC Modulatoren, wie beispielhaft und vorzugsweise Riociguat, Cinaciguat, Vericiguat oder BAY 1101042 verabreicht.

Bei einer bevorzugten Ausführungsform der Erfindung werden die erfindungsgemäßen Verbindungen in Kombination mit einem den Glucosestoffwechsel verändernden Wirkstoff, wie beispielhaft und vorzugsweise Insulin, einem Sulfonylharnstoff, Acarbose, DPP4 Inhibitoren, GLP-1 Analoga oder SGLT-1 Inhibitor, verabreicht.

Besonders bevorzugt sind Kombinationen der erfindungsgemäßen Verbindungen mit einem oder mehreren weiteren Wirkstoffen ausgewählt aus der Gruppe bestehend aus den Blutdruck senkende Wirkstoffe, antiarrhythmische Wirkstoffe, Vasopressin-Rezeptor-Antagonisten, PDE 5-Inhibitoren, Thrombozytenaggregationshemmer, sGC-Aktivatoren und sGC-Stimulatoren.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat oder Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees,

Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Dosieraerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale und die parenterale Applikation, insbesondere die orale, die intravenöse und die intrapulmonale (inhalative) Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

## A. Beispiele

**Abkürzungen und Akronyme:**

| | |
|---|---|
| AAV | allgemeine Arbeitsvorschrift |
| abs. | absolut |
| aq. | wässrig, wässrige Lösung |
| br. | breit (bei NMR-Signal) |
| Bsp. | Beispiel |
| Bu | Butyl |
| C | Konzentration |
| ca. | circa, ungefähr |
| cat. | katalytisch |
| CDI | Carbonyldiimidazol |
| CI | chemische Ionisation (bei MS) |
| d | Dublett (bei NMR) |
| d | Tag(e) |

(fortgesetzt)

| | |
|---|---|
| DCM | Dichlormethan |
| dd | Dublett von Dublett (bei NMR) |
| de | Diastereomerenüberschuss |
| DEA | Diethylamin |
| dest. | destilliert |
| DIPEA | N,N-Diisopropylethylamin |
| DMAP | 4-N,N-Dimethylaminopyridin |
| DMF | N,N-Dimethylformamid |
| DMSO | Dimethylsulfoxid |
| dt | Dublett von Triplett (bei NMR) |
| d. Th. | der Theorie (bei chemischer Ausbeute) |
| ee | Enantiomerenüberschuss |
| EI | Elektronenstoß-Ionisation (bei MS) |
| ent | enantiomerenrein, Enantiomer |
| Äq. | Äquivalent(e) |
| ESI | Elektrospray-Ionisation (bei MS) |
| Et | Ethyl |
| GC | Gaschromatographie |
| GC/MS | Gaschromatographie-gekoppelte Massenspektrometrie |
| h | Stunde(n) |
| HATU | O-(7-Azabenzotriazol-1-yl)-N,N,N',N'-tetramethyluroniumhexafluoro-phosphat |
| HPLC | Hochdruck-, Hochleistungsflüssigchromatographie |
| konz. | konzentriert (bei Lösung) |
| LC | Flüssigchromatographie |
| LC/MS | Flüssigchromatographie-gekoppelte Massenspektrometrie |
| Lit. | Literatur(stelle) |
| m | Multiplett (bei NMR) |
| M | molar (bei Lösung) |
| Me | Methyl |
| min | Minute(n) |
| MS | Massenspektrometrie |
| NMR | Kernresonanzspektrometrie |
| q (oder quart) | Quartett (bei NMR) |
| qd | Quartett von Dublett (bei NMR) |
| quant. | quantitativ (bei chemischer Ausbeute) |
| quintt | Quinttett (bei NMR) |
| rac | racemisch, Racemat |
| RP | reverse phase (Umkehrphase, bei HPLC) |
| RT | Raumtemperatur |

(fortgesetzt)

| | |
|---|---|
| Rt | Retentionszeit (bei HPLC, LC/MS) |
| S | Singulett (bei NMR) |
| sept | Septett (bei NMR) |
| SFC | superkritische Flüssigchromatographie |
| t | Triplett (bei NMR) |
| tBu | tert.-Butyl |
| td | Triplett von Dublett (bei NMR) |
| TFA | Trifluoressigsäure |
| THF | Tetrahydrofuran |
| UV | Ultraviolett-Spektrometrie |
| vgl. | vergleiche |
| v/v | Volumen zu Volumen-Verhältnis (einer Lösung) |
| Xantphos | 9,9-Dimethyl-4,5-bis-(diphenylphosphino)-xanthen |
| zus. | zusammen |
| **HPLC- und LC/MS-Methoden:** | |

Methode 1:

Instrument: Waters ACQUITY SQD UPLC System; Säule: Waters Acquity UPLC HSS T3 1.8 μ 50 x 1 mm; Eluent A: 1 l Wasser + 0.25 ml 99%ige Ameisensäure , Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 208 - 400 nm.

Methode 2:

Instrument MS: Waters (Micromass) QM; Instrument HPLC: Agilent 1100 Serie; Säule : Agient ZORBAX Extend-C18 3.0x50mm 3.5-Micron; Eluent A: 1 l Wasser + 0.01 mol Ammoniumcarbonat, Eluent B: 1 l Acetonitril, Gradient: 0.0 min 98% A → 0.2min 98% A → 3.0 min 5% A→ 4.5 min 5% A ; Ofen: 40°C; Fluss: 1.75 ml/min; UV-Detektion: 210 nm

Methode 3:

Gerätetyp MS: Thermo Scientific FT-MS; Gerätetyp UHPLC+: Thermo Scientific UltiMate 3000; Säule: Waters, HSST3, 2.1 x 75 mm, C18 1.8 μm; Eluent A: 1 l Wasser + 0.01% Ameisensäure; Eluent B: 1 l Acetonitril + 0.01% Ameisensäure; Gradient: 0.0 min 10% B → 2.5 min 95% B → 3.5 min 95% B; Ofen: 50°C; Fluss: 0.90 ml/min; UV-Detektion: 210 nm/ Optimum Integration Path 210-300 nm.

Methode 4:

Instrument: Waters ACQUITY SQD UPLC System; Säule: Waters Acquity UPLC HSS T3 1.8 μ 50 x 1 mm; Eluent A: 1 l Wasser + 0.25 ml 99%ige Ameisensäure , Eluent B: 1 l Acetonitril + 0.25 ml 99%ige Ameisensäure; Gradient: 0.0 min 95% A → 6.0 min 5% A → 7.5 min 5% A Ofen: 50°C; Fluss: 0.35 ml/min; UV-Detektion: 210 - 400 nm.

Methode 5:

Instrument: Agilent MS Quad 6150;HPLC: Agilent 1290; Säule: Waters Acquity UPLC HSS T3 1.8 μ 50 x 2.1 mm; Eluent A: 1 l Wasser + 0.25 ml 99%ige Ameisensäure , Eluent B: 1 l Acetonitril + 0.25 ml 99%ige Ameisensäure; Gradient: 0.0 min 90% A → 0.3 min 90% A → 1.7 min 5% A → 3.0 min 5% A Ofen: 50°C; Fluss: 1,20 ml/min; UV-Detektion: 205 - 305 nm.

Methode 6:

Instrument: Thermo DFS, Trace GC Ultra; Säule: Restek RTX-35, 15 m x 200 μm x 0.33 μm; konstanter Fluss mit Helium: 1.20 ml/min; Ofen: 60°C; Inlet: 220°C; Gradient: 60°C, 30°C/min → 300°C (3.33 min halten).

**Weitere Angaben:**

Die Prozentangaben in den folgenden Beispiel- und Testbeschreibungen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Bei Aufreinigungen von erfindungsgemäßen Verbindungen per präparativer HPLC nach den beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen ausreichend basische bzw. säure Funktionalitäten enthalten. Ein solches Salz kann durch verschiedene dem Fachmann bekannte Methoden in die entsprechende freie Base bzw. Säure überführt werden.

Reinheitsangaben beziehen sich in der Regel auf entsprechende Peak-Integrationen im LC/MS-Chromatogramm, können aber zusätzlich auch unter Zuhilfenahme des 1H-NMR-Spektrums ermittelt worden sein. Wenn keine Reinheit angegeben ist, handelt es sich in der Regel um eine 100%-Reinheit laut automatischer Peak-Integration im LC/MS-Chromatogramm oder die Reinheit wurde nicht explizit ermittelt.

Angaben zu Ausbeuten in % d. Th. sind in der Regel reinheitskorrigiert, sofern eine Reinheit <100% angegeben ist. Bei lösungsmittelhaltigen oder verunreinigten Chargen kann die Ausbeute formal ">100%" betragen; in diesen Fällen ist die Ausbeute nicht lösungsmittel- bzw. reinheitskorrigiert.

Die nachfolgenden Beschreibungen der Kopplungsmuster von 1H-NMR-Signalen wurden teilweise direkt den Vorschlägen des ACD SpecManagers (ACD/Labs Release 12.00, Product version 12.5) entnommen und nicht notwendigerweise streng hinterfragt. Zusätzlich zu diesen $^1$H-NMR-Angaben liegen unter Umständen zusätzliche verbreitete Signale - bedingt durch die vorhandene Moleküldynamik (insbesondere im Bereich von 2.50 - 4.20 ppm) - vor, welche nicht separat angegeben sind. Teilweise wurden die Vorschläge des SpecManagers manuell angepasst. Manuell angepasste bzw. zugewiesene Beschreibungen orientieren sich in der Regel an dem optischen Erscheinungsbild der betreffenden Signale und entsprechen nicht notwendigerweise einer strengen, physikalisch korrekten Interpretation. In der Regel bezieht sich die Angabe zur chemischen Verschiebung auf das Zentrum des betreffenden Signals. Bei breiten Multipletts erfolgt die Angabe eines Intervalls. Durch Lösungsmittel oder Wasser verdeckte Signale wurden entweder tentativ zugeordnet oder sind nicht aufgeführt. Stark verbreiterte Signale - z.B. verursacht durch schnelle Rotation von Molekülteilen oder aufgrund von austauschenden Protonen - wurden ebenfalls tentativ zugeordnet (oft als breites Multiplett oder breites Singulett bezeichnet) oder sind nicht aufgeführt.

Die $^1$H-NMR-Daten ausgewählter Beispiele werden in Form von $^1$H-NMR-Peaklisten notiert. Zu jedem Signalpeak wird erst der δ-Wert in ppm und dann die Signalintensität in runden Klammem aufgeführt. Die δ-Wert-Signalintensitäts-Zahlenpaare von verschiedenen Signalpeaks werden durch Kommata voneinander getrennt aufgelistet. Die Peakliste eines Beispieles hat daher die Form: $\delta_1$ (Intensität$_1$), $\delta_2$ (Intensität$_2$), ... , $\delta_i$ (Intensität$_1$), ... , $\delta_n$ (Intensität$_n$).

Die Intensität scharfer Signale korreliert mit der Höhe der Signale in einem gedruckten Beispiel eines NMR-Spektrums in cm und zeigt im Vergleich mit anderen Signalen die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden. Die Listen der $^1$H-NMR-Peaks sind ähnlich den klassischen $^1$H-NMR-Ausdrucken und enthalten somit gewöhnlich alle Peaks, die bei einer klassischen NMR-Interpretation aufgeführt werden. Darüber hinaus können sie wie klassische $^1$H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen. Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%). Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen. Ein Experte, der die Peaks der Zielverbindungen mit bekannten Verfahren (MestreC, ACD-Simulation, oder unter Verwendung von empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen $^1$H-NMR-Interpretation. Eine detaillierte Beschreibung der Darstellung von NMR-Daten in Form von Peaklisten kann der Publikation "Citation of NMR Peaklist Data within Patent Applications" entnommen werden (vgl. Research Disclosure Database Number 605005, 2014, 1. August 2014 oder http://www.researchdisclosure.com/searching-disclosures). In der Peak Picking Routine, die in der Research Disclosure Database Number 605005 beschrieben ist, kann der Parameter "MinimumHeight" zwischen 1%

und 4% eingestellt werden. Abhängig von der Art der chemischen Struktur und/oder abhängig von der Konzentration der zu vermessenden Verbindung kann es sinnvoll sein, den Parameter "MinimumHeight" auf Werte <1% einzustellen. Schmelzpunkte und Schmelzbereiche, soweit angegeben, sind nicht korrigiert.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

## Allgemeine Arbeitsvorschriften

### AAV1

Eine Lösung der entsprechenden Carbonsäure (1 Äq.) in DMF (0.08-0.12M) wurde mit N,N-Diisopropylethylamin (1.4-1.5 Äq. bzw. 2.4-3.0 Äq. wenn das Amin als Hydrochlorid eingesetzt wurde) und HATU (1.0-1.65 Äq.) versetzt und die Mischung für 30 min bei RT gerührt. Anschließend wurde das entsprechende Amin (1.04-1.5 Äq.) zugegeben und die Mischung für 0.15-2h bei Raumtemperatur nachgerührt. Die Reaktion wurde dann durch die Zugabe von Wasser und 1M wässriger Salzsäure beendet. Der Niederschlag wurde abfiltriert, in DCM aufgenommen, über Magnesiumsulfat getrocknet, filtriert und das Lösungsmittel unter reduziertem Druck entfernt. Alternativ wurde nach dem Ansäuern mit Essigsäureethylester extrahiert, die vereinigten organischen Phasen über Magnesiumsulfat oder Natriumsulfat getrocknet, filtriert und das Lösungsmittel unter reduziertem Druck entfernt. Das Rohprodukt wurde anschließend entweder mittels Nonnalphasen-Chromatographie (Kieselgel, Eluent: Cyclohexan-Essigsäureethylester-Gemische oder Dichlormethan-Methanol-Gemische) oder präparativer RP-HPLC (Wasser-Acetonitril-Gradient) gereinigt. Alternativ wurde die Reaktionsmischung mit wenig Acetonitril, Wasser und Ameisensäure verdünnt und die erhaltene Rohlösung mittels RP-HPLC (Wasser-Acetonitril-Gradient) gereinigt. Weitere Alternativen bei der Aufarbeitung sind falls durchgeführt bei dem jeweiligen Experiment beschrieben.

### AAV2

Kalium- oder Cäsiumcarbonat (1.5-2.5 Äq.) wurden im Reaktionsgefäß im Vakuum ausgeheizt. Es wurde auf RT abgekühlt und mit Argon geflutet. Palladiumacetat (0.1-0.36 Äq.), 9,9-Dimethyl-4,5-bis-(diphenylphosphino)-xanthen (Xantphos, 0.18-0.36 Äq.) und Dioxan (0.04-0.12M) wurden zugegeben und die Suspension wurde für 10 min bei Raumtemperatur im Argonstrom entgast. Anschließend wurde das entsprechende Amid (1.0-10 Äq.) und das entsprechende 7-Chlor-4-oxo-1,4-dihydro-1,8-naphthyridin (1.0 Äq.) zugegeben. Es wurde für 1h (oder bis zum vollständigen Umsatz nach analytischer HPLC oder Dünnschichtchromatographie mit entsprechenden Laufinittelgemischen) bei 80-110°C gerührt. Es wurde dann auf RT abgekühlt und alle flüchtigen Komponenten unter reduziertem Druck entfernt oder alternativ das Reaktionsgemisch auf Wasser gegossen, der pH-Wert mit 1M wässriger Salzsäure auf pH 1 eingestellt, mit Essigsäureethylester extrahiert, die vereinigten organischen Phasen mit gesättigter, wässriger Natriumchlorid-Lösung gewaschen, über Magnesiumsulfat getrocknet, filtriert und das Lösungsmittel unter reduziertem Druck entfernt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient) gereinigt. Alternativ wurde die Reaktionsmischung mit wenig Acetonitril, Wasser und Ameisensäure oder TFA verdünnt und die erhaltene Rohlösung mittels RP-HPLC (Wasser-Acetonitril-Gradient) gereinigt. Weitere Alternativen bei der Aufarbeitung sind, falls abweichend durchgeführt, bei dem jeweiligen Experiment beschrieben.

### AAV3

Eine Lösung des entsprechenden 7-Chlor-4-oxo-1,4-dihydro-1,8-naphthyridins in DMF (0.10-0.22M) wurde mit dem entsprechenden Amin (1.2 Äq.) und DIPEA (1.5-3.5 Äq.) versetzt. Die Reaktionslösung wurde über Nacht bei RT gerührt. Das Rohprodukt wurde anschließend entweder nach wässriger Aufarbeitung und Extraktion mit entsprechendem organischen Lösungsmittel mittels NormalphasenChromatographie (Eluent: Cyclohexan-Essigsäureethylester-Gemische oder Dichlormethan-Methanol-Gemische) oder präparativer RP-HPLC (Wasser-Acetonitril-Gradient) gereinigt. Alternativ wurde die Reaktionsmischung mit wenig Acetonitril, Wasser und Ameisensäure verdünnt und die erhaltene Rohlösung mittels RP-HPLC (Wasser-Acetonitril-Gradient) gereinigt. Weitere Alternativen bei der Aufarbeitung sind falls durchgeführt bei dem jeweiligen Experiment beschrieben.

**AUSGANGSVERBINDUNGEN UND INTERMEDIATE:**

**Beispiel 1A**

7-Chlor-1-(2,6-difluorphenyl)-6-fluor-4-oxo-1,4-dihydro-1,8-naphthyridin-3-carbonsäureethylester

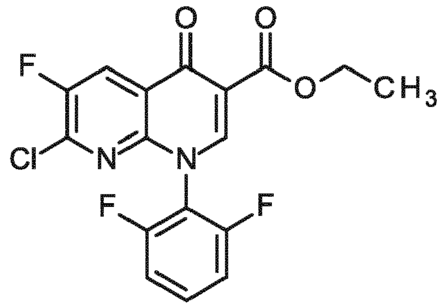

Eine Lösung aus 6.00 g (17.8 mmol) 2-[(2,6-Dichlor-5-fluorpyridin-3-yl)carbonyl]-3-ethoxyacrylsäure-ethylester (Darstellung beschrieben in US4840954 A, Beispiel G, Stufe 1, Seite 7) und 3.23 g (24.9 mmol) 2,6-Difluoranilin in 30 ml Dichlormethan wurde mit 21.8 ml (125 mmol) DIPEA versetzt und für 4 h bei RT gerührt. Anschließend wurden 2.47 g (17.8 mmol) Kaliumcarbonat zugegeben und über Nacht unter Rückfluss erhitzt. Die Mischung wurde mit 200 ml Dichlormethan verdünnt und zweimal mit 150 ml 1M wässriger Salzsäure gewaschen. Die organische Phase wurde über Natriumsulfat getrocknet, filtriert und das Lösungsmittel unter reduziertem Druck entfernt. Es wurde mit 80 ml *tert.*-Butylmethylether verdünnt, der Niederschlag abgesaugt und mit 10 ml *tert.*-Butylmethylether gewaschen. Es wurden 3.22 g (45% d. Th., 95.7% Reinheit) der Titelverbindung erhalten.
LC-MS (Methode 1): $R_t$ = 0.96 min; MS (ESIpos): m/z = 383 [M+H]+.
$^1$H-NMR (400 MHz, DMSO-d6): $\delta$ [ppm] = 8.95 (s, 1H), 8.57 (d, 1H), 7.80-7.71 (m, 1H), 7.50-7.43 (m, 2H), 4.25 (q, 2H), 1.26 (t, 3H).

**Beispiel 2A**

7-Chlor-1-(2,6-difluorphenyl)-6-fluor-4-oxo-1,4-dihydro-1,8-naphthyridin-3-carbonsäure

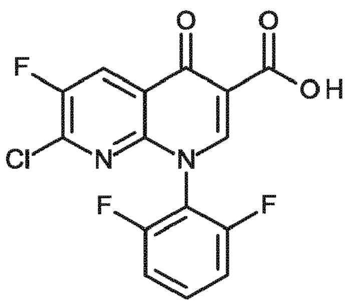

3.22 g (8.41 mmol) 7-Chlor-1-(2,6-difluorphenyl)-6-fluor-4-oxo-1,4-dihydro-1,8-naphthyridin-3-carbonsäureethylester wurden in 25.2 ml Wasser vorgelegt, mit 25.2 ml 36 proz. wässriger Salzsäure und 25.2 ml THF versetzt und 4 h bei 110°C nachgerührt. Das Reaktionsgemisch wurde auf RT abgekühlt, der Niederschlag abgesaugt, zweimal mit 30 ml Wasser gewaschen und am Hochvakuum getrocknet. Es wurden 4.1 g (quantitativ, 96.8% Reinheit) der Titelverbindung erhalten.
LC-MS (Methode 1): $R_t$ = 0.96 min; MS (ESIpos): m/z = 355 $[M+H]^+$.
$^1$H-NMR (400 MHz, DMSO-d6): $\delta$ [ppm] = 13.70 (s, 1H), 9.25 (s, 1H), 8.76 (d, 1H), 7.80-7.72 (m, 1H), 7.51-7.43 (m, 2H).

**Beispiel 3A**

7-Chlor-1-(2,6-difluorphenyl)-6-fluor-4-oxo-*N*-[(2*S*)-1,1,1-trifluorbutan-2-yl]-1,4-dihydro-1,8-naphthyridin-3-carbonsäureamid

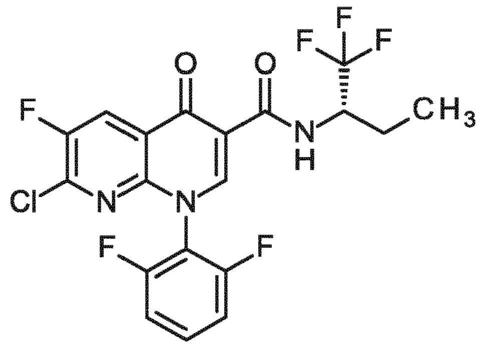

Gemäß AAV1 wurden 1.00 g (2.82 mmol) 7-Chlor-1-(2,6-difluorphenyl)-6-fluor-4-oxo-1,4-dihydro-1,8-naphthyridin-3-carbonsäure mit 553 mg (3.38 mmol) (2*S*)-1,1,1-Trifluorbutan-2-amin-Hydrochlorid in Gegenwart von 1.29 g (3.38 mmol) HATU und 1.96 ml (11.3 mmol) DIPEA in 20 ml DMF zur Reaktion gebracht. Es wurden für 1 min nachgerührt und die Reaktionslösung auf ein Gemisch aus Wasser, 1M wässrige Salzsäure und Essigsäureethylester gegeben. Die Phasen wurden getrennt und die wässrige Phase viermal mit 50 ml Essigsäureethylester extrahiert. Die vereinigten organischen Phasen wurden mit gesättigter wässriger Natriumchlorid-Lösung gewaschen, über Natriumsulfat getrocknet, filtriert und das Lösungsmittel unter reduziertem Druck entfernt. Das Rohprodukt wurde in wenig Essigsäureethylester gelöst und mittels Normalphasenchromatographie (Cyclohexan-Essigsäureethylester, 5:1) gereinigt. Die Fraktionen wurden vereinigt, im Vakuum eingeengt und der Rückstand über Nacht aus Acetonitril lyophilisiert. Es wurden 331 mg (25% d. Th., 100% Reinheit) der Titelverbindung erhalten.
LC-MS (Methode 3): $R_t$ = 2.32 min; MS (ESIpos): m/z = 464 $[M+H]^+$.
$^1$H-NMR (400 MHz, DMSO-d6): $\delta$ [ppm] = 9.84 (d, 1H), 9.12 (s, 1H), 8.72 (d, 1H), 7.80-7.72 (m, 1H), 7.51-7.44 (m, 2H), 4.85-4.71 (m, 1H), 1.96-1.83 (m, 1H), 1.75-1.61 (m, 1H), 0.98 (t, 3H).

**Beispiel 4A**

7-Chlor-1-(2,4,6-trifluorphenyl)-6-fluor-4-oxo-1,4-dihydro-1,8-naphthyridin-3-carbonsäureethylester

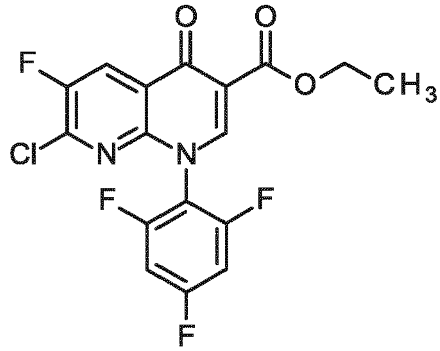

Eine Lösung aus 12.0 g (35.7 mmol) 2-[(2,6-Dichlor-5-fluorpyridin-3-yl)carbonyl]-3-ethoxyacrylsäure-ethylester (US4840954 A, Beispiel G, Stufe 1, Seite 7) und 7.35 g (49.9 mmol) 2,4,6-Trifluoranilin in 60 ml Dichlormethan wurde mit 43.5 ml (250 mmol) DIPEA versetzt und für 4 h bei RT gerührt. Anschließend wurden 4.93 g (35.7 mmol) Kaliumcarbonat zugegeben und über Nacht unter Rückfluss erhitzt. Die Mischung wurde dann mit 200 ml Dichlormethan verdünnt und dreimal mit 150 ml 1M wässriger Salzsäure gewaschen. Die organische Phase wurde über Natriumsulfat

getrocknet, filtriert und das Lösungsmittel unter reduziertem Druck entfernt. Es wurde mit 100 ml *tert.*-Butylmethylether verdünnt, der Niederschlag abgesaugt und mit dreimal 20 ml *tert.*-Butylmethylether gewaschen und am Hochvakuum getrocknet. Es wurden 8.80 g (58% d. Th., 94.6% Reinheit) der Titelverbindung erhalten.
LC-MS (Methode 1): $R_t$ = 1.01 min; MS (ESIpos): m/z = 401 [M+H]+.
$^1$H-NMR (400 MHz, DMSO-d6): δ [ppm] = 8.97 (s, 1H), 8.56 (d, 1H), 7.67-7.56 (m, 2H), 4.26 (q, 2H), 1.28 (t, 3H).

**Beispiel 5A**

7-Chlor-6-fluor-4-oxo-1-(2,4,6-trifluorphenyl)-1,4-dihydro-1,8-naphthyridin-3-carbonsäure

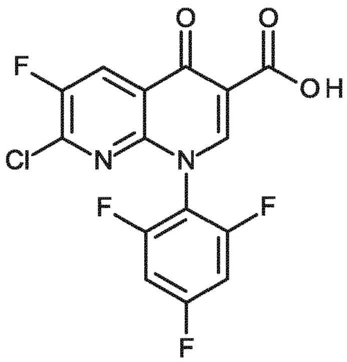

8.80 g (21.9 mmol) 7-Chlor-1-(2,4,6-trifluorphenyl)-6-fluor-4-oxo-1,4-dihydro-1,8-naphthyridin-3-carbonsäureethylester wurden in 66.2 ml Wasser vorgelegt, mit 66.2 ml 36 proz. wässriger Salzsäure und 66.2 ml THF versetzt und 4 h bei 110°C nachgerührt. Das Reaktionsgemisch wurde auf RT abgekühlt, der Niederschlag abgesaugt, viermal mit 40 ml Wasser gewaschen und am Hochvakuum getrocknet. Es wurden 7.37 g (89% d. Th., 99% Reinheit) der Titelverbindung erhalten.
LC-MS (Methode 3): $R_t$ = 1.84 min; MS (ESIpos): m/z = 373 $[M+H]^+$.
$^1$H-NMR (400 MHz, DMSO-d6): δ [ppm] = 13.67 (s, 1H), 9.28 (s, 1H), 8.76 (d, 1H), 7.68-7.59 (m, 2H).

**Beispiel 6A**

7-[(3*R*,4*R*)-3,4-Dihydroxypyrrolidin-1-yl]-6-fluor-4-oxo-1-(2,4,6-trifluorphenyl)-1,4-dihydro-1,8-naphthyridin-3-carbonsäure

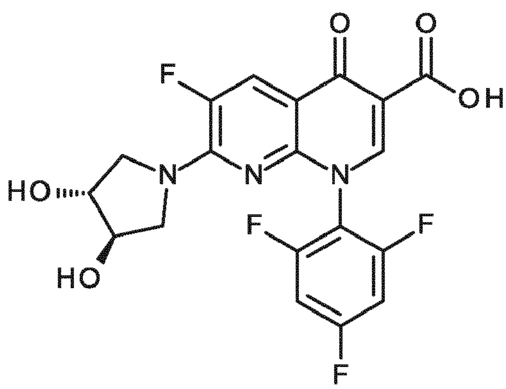

Zu einer Lösung von 3.60 g (9.66 mmol) 7-Chlor-1-(2,4,6-trifluorphenyl)-6-fluor-4-oxo-1,4-dihydro-1,8-naphthyridin-3-carbonsäure und 1.48 g (10.6 mmol) (3*R*,4*R*)-Pyrrolidin-3,4-diol-Hydrochlorid in 50 ml DMF wurde bei RT 5.89 ml (33.8 mmol) DIPEA gegeben. Es wurde für 1 h bei RT nachgerührt. Die Reaktionsmischung wurde dann mit 150 ml Wasser und 100 ml wässrige 1M Salzsäure versetzt und der entstandene Niederschlag abgesaugt. Der Niederschlag

wurde mit Wasser gewaschen und am Hochvakuum getrocknet. Es wurden 3.96 g (93% d. Th., 100% Reinheit) der Titelverbindung erhalten.
LC-MS (Methode 3): $R_t$ = 1.23 min; MS (ESIpos): m/z = 440 [M+H]+.
$^1$H-NMR (500 MHz, DMSO-d6): δ [ppm] = 15.01 (s, 1H), 9.05 (s, 1H), 8.07 (d, 1H), 7.64-7.54 (m, 2H), 5.30-5.14 (m, 2H), 4.09-3.64 (m, 4H), 3.28-3.21 (m, 0.6H, teilweise unter der Wasser Resonanz), 3.15-3.01 (m, 1H).

**Beispiel 7A**

6-Fluor-7-[(4*S*)-4-hydroxy-2-oxopyrrolidin-1-yl]-4-oxo-1-(2,4,6-trifluorphenyl)-1,4-dihydro-1,8-naphthyridin-3-carbonsäure

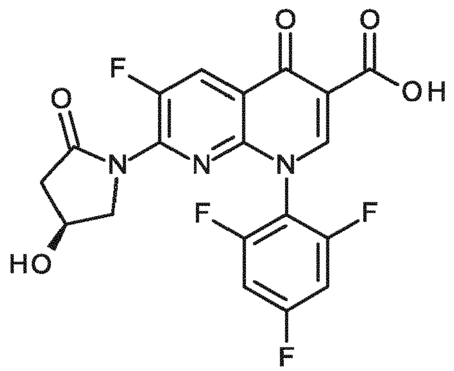

Gemäß AAV2 wurden 100 mg (268 mmol) 7-Chlor-1-(2,4,6-trifluorphenyl)-6-fluor-4-oxo-1,4-dihydro-1,8-naphthyridin-3-carbonsäure mit 32.6 mg (322 μmol) (4*S*)-4-Hydroxypyrrolidin-2-on in Gegenwart von 92.7 mg (671 μmol) Kaliumcarbonat, 6.0 mg (27 μmol) Palladiumacetat und 33 mg (54 μmol) Xantphos in 2.4 ml Dioxan bei 90°C für 1 h zur Reaktion gebracht. Die Reaktionsmischung wurde mit 1 ml wässriger 1M Salzsäure und 1 ml DMSO verdünnt und direkt mittels präp. HPLC (Acetonitril-Wasser mit Ameisensäure, C18 RP-HPLC) gereinigt. Es wurden 61.7 mg (42% d. Th., 80% Reinheit) der Titelverbindung erhalten.
LC-MS (Methode 3): $R_t$ = 1.19 min; MS (ESIpos): m/z = 438 [M+H]$^+$.

**Beispiel 8A**

7-[(3*S*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure

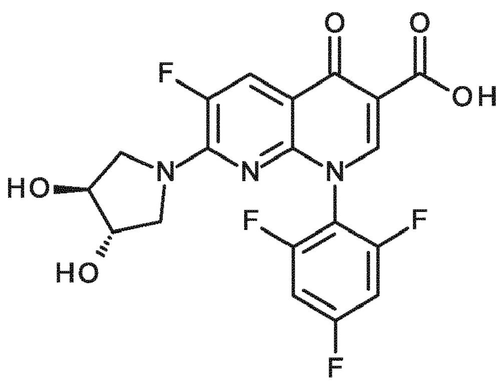

Zu einer Lösung von 240 mg (644 μmol) 7-Chlor-1-(2,4,6-trifluorphenyl)-6-fluor-4-oxo-1,4-dihydro-1,8-naphthyridin-3-carbonsäure und 73.0 mg (708 μmol) (3*S*,4*S*)-Pyrrolidin-3,4-diol in 3.3 ml DMF wurde bei RT 280 μl (1.61 mmol) DIPEA gegeben. Es wurde für 1h bei RT nachgerührt. Die Reaktionsmischung wurde mit 0.4 ml wässriger 1M Salzsäure und 1 ml Acetonitril verdünnt und direkt mittels präp. HPLC (Acetonitril-Wasser mit Ameisensäure, C18 RP-HPLC) gereinigt. Es wurden 232 mg (74% d. Th., 94.4% Reinheit) der Titelverbindung erhalten.
LC-MS (Methode 1): $R_t$ = 0.69 min; MS (ESIpos): m/z = 440 [M+H]+.
$^1$H-NMR (500 MHz, DMSO-d6): δ [ppm] = 15.01 (s, 1H), 9.05 (s, 1H), 8.07 (d, 1H), 7.64-7.55 (m, 2H), 5.33-5.10 (m, 2H), 4.10-3.63 (m, 4H), 3.29-3.20 (m, 0.8H, teilweise unter der Wasser Resonanz), 3.15-3.00 (m, 1H).

**Beispiel 9A**

7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure

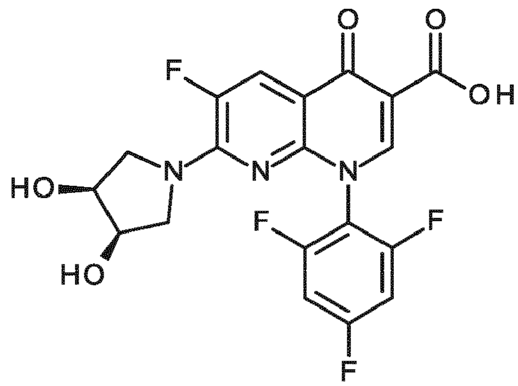

Zu einer Lösung von 250 mg (671 μmol) 7-Chlor-1-(2,4,6-trifluorphenyl)-6-fluor-4-oxo-1,4-dihydro-1,8-naphthyridin-3-carbonsäure und 103 mg (738 μmol) *cis*-Pyrrolidin-3,4-diol Hydrochlorid in 3.5 ml DMF wurde bei RT 409 μl (2.35 mmol) DIPEA gegeben. Es wurde für 1 h bei RT nachgerührt. Die Reaktionsmischung wurde mit 7 ml wässriger 1M Salzsäure angesäuert, mit 15 ml Wasser versetzt und der Niederschlag abgesaugt. Der Rückstand wurde mit Wasser gewaschen und lyophiliisert. Es wurden 256 mg (86% d. Th., 99% Reinheit) der Titelverbindung erhalten.
LC-MS (Methode 1): $R_t$ = 0.71 min; MS (ESIpos): m/z = 440 [M+H]+.
$^1$H-NMR (500 MHz, DMSO-d6): δ [ppm] = 15.0 (s, 1H), 9.05 (s, 1H), 8.05 (d, 1H), 7.63-7.54 (m, 2H), 5.15-4.89 (m, 2H), 4.13-3.86 (m, 3H), 3.61 (br. s, 1H), 3.21 (br. s, 1H), 3.04 (br. s, 1H).

**Beispiel 10A**

6-Fluor-7-[(3S)-3-hydroxypyrrolidin-1-yl]-4-oxo-1-(2,4,6-trifluorphenyl)-1,4-dihydro-1,8-naphthyridin-3-carbonsäure

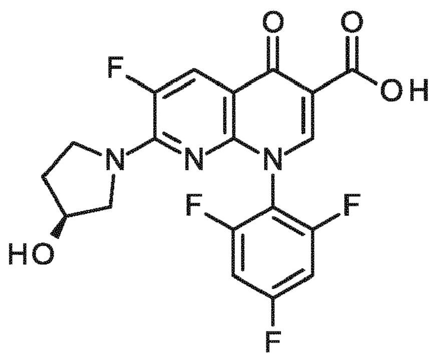

7-Chlor-6-fluor-4-oxo-1-(2,4,6-trifluorphenyl)-1,4-dihydro-1,8-naphthyridin-3-carbonsäure (800 mg, 2.15 mmol) wurde in 8 in DMF vorgelegt, mit (3S)-Pyrrolidin-3-ol (206 mg, 2.36 mmol) und N,N-Diisopropylethylamin (1.3 ml, 7.5 mmol) versetzt und das Gemisch wurde 2 h bei RT gerührt. Das Reaktionsgemisch wurde auf Wasser gegeben, mit 1M Salzsäure und Ethylacetat versetzt. Die organische Phase wurde abgetrennt und die die wässrige Phase wurde dreimal

mit Ethylacetat extrahiert. Die vereinten organischen Phasen wurden einmal mit ges. Natriumchlorid-Lösung gewaschen, über Natriumsulfat getrocknet und eingeengt. Das Produkt wurde mit Acetonitril verrührt, abfilltriert mit etwas kaltem Acetonitril nachgewaschen und getrocknet. Es wurden 770 mg (85% d. Th., 100% Reinheit) der Titelverbindung erhalten.
LC-MS (Methode 1): $R_t$ = 0.82 min; MS (ESIpos): m/z = 424 $[M+H]^+$
$^1$H-NMR (400 MHz, DMSO-d6) δ [ppm]: -0.008 (2.22), 0.008 (2.03), 1.909 (0.87), 2.074 (16.00), 3.222 (0.71), 3.875 (0.53), 4.309 (0.50), 5.024 (1.35), 7.565 (2.70), 7.586 (4.97), 7.608 (2.81), 8.037 (5.77), 8.068 (5.70), 9.043 (10.89), 15.025 (9.55).

**Beispiel 11A**

N-[(1S)-1-Cyclopropyl-2,2,2-trifluorethyl]-6-fluor-4-oxo-7-(3H-[1,2,3]triazolo[4,5-b]pyridin-3-yloxy)-1-(2,4,6-trifluorphenyl)-1,4-dihydro-1,8-naphthyridin-3-carboxamid

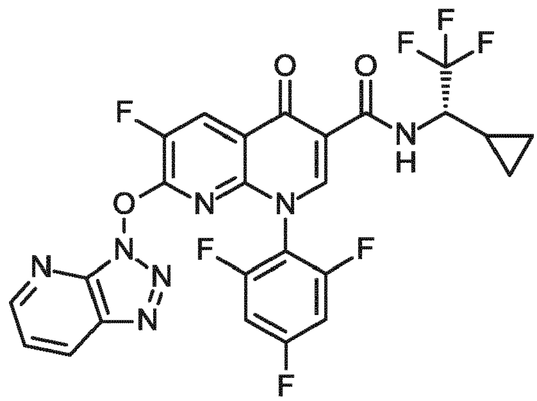

Gemäß AAV1 wurden 500 mg (1.34 mmol) 7-Chlor-1-(2,6-difluorphenyl)-6-fluor-4-oxo-1,4-dihydro-1,8-naphthyridin-3-carbonsäure mit 283 mg (1.61 mmol) (1*S*)-1-Cyclopropyl-2,2,2-trifluorethanamin Hydrochlorid in Gegenwart von 612 mg (1.61 mmol) HATU und 935 μl (5.37 mmol) DIPEA in 10 ml DMF zur Reaktion gebracht. Es wurde für 1 h bei RT nachgerührt und die Reaktionslösung auf ein Gemisch aus Wasser und Essigsäureethylester gegeben. Die Phasen wurden getrennt und die wässrige Phase viermal mit 50 ml Essigsäureethylester extrahiert. Die organischen Phasen wurden vereinigt, mit 50ml pH-7 Puffer und zweimal mit 50ml gesättigter wässriger Natriumchlorid-Lösung gewaschen, über Natriumsulfat getrocknet, filtriert und eingeengt. Die Substanz wurde in Essigsäureethylester gelöst und auf Kieselgel aufgezogen und mittels Normalphasenchromatographie (Cyclohexan-Essigsäureethylester Gradient) gereinigt. Die Fraktionen wurden vereinigt, im Vakuum eingeengt und der Rückstand über Nacht aus Acetonitril lyophilisiert. Es wurden 534 mg (66% d. Th., 99% Reinheit) der Titelverbindung erhalten.
LC-MS (Methode 3): $R_t$ = 2.21 min; MS (ESIpos): m/z = 594 $[M+H]^+$.
$^1$H-NMR (400 MHz, DMSO-d6): δ [ppm] = 10.01 (d, 1H), 8.96 (s, 1H), 8.88 (d, 1H), 8.74 (dd, 1H), 8.63 (dd, 1H), 7.65 (dd, 1H), 7.05-6.97 (m, 2H), 4.42-4.37 (m, 1H), 1.28-1.17 (m, 1H), 0.71-0.51 (m, 3H), 0.36-0.28 (m, 1H).

**Beispiel 12A**

6-Fluor-4-oxo-7-(3H-[1,2,3]triazolo[4,5-b]pyridin-3-yloxy)-N-[(2S)-1,1,1-trifluorbutan-2-yl]-1-(2,4,6-trifluorphenyl)-1,4-dihydro-1,8-naphthyridin-3-carboxamid

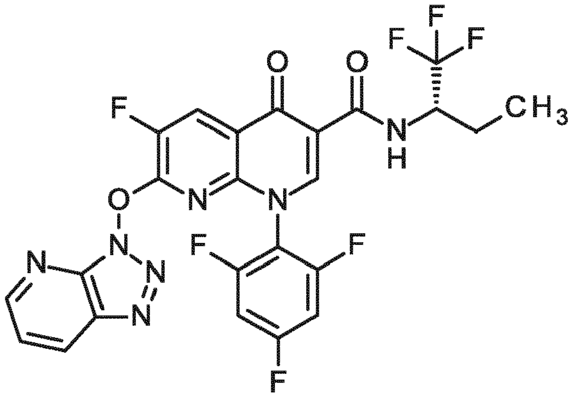

Gemäß AAV1 wurden 500 mg (1.34 mmol) 7-Chlor-1-(2,6-difluorphenyl)-6-fluor-4-oxo-1,4-dihydro-1,8-naphthyridin-3-carbonsäure mit 263 mg (1.61 mmol) (S)-1,1,1-Trifluorbutan-2-amin Hydrochlorid in Gegenwart von 612 mg (1.61 mmol) HATU und 935 µl (5.37 mmol) DIPEA in 9.5 ml DMF zur Reaktion gebracht. Es wurde für 1 h bei RT nachgerührt und die Reaktionslösung auf ein Gemisch aus Wasser und Essigsäureethylester gegeben. Die Phasen wurden getrennt und die wässrige Phase viermal mit 50 ml Essigsäureethylester extrahiert. Die organischen Phasen wurden vereinigt, mit 50 ml Puffer pH7 und zweimal mit 50 ml gesättigter wässriger Natriumchlorid-Lösung gewaschen, über Natriumsulfat getrocknet, filtriert und eingeengt. Die Substanz wurde in Essigsäureethylester gelöst und auf Kieselgel aufgezogen und mittels Normalphasenchromatographie (Cyclohexan-Essigsäureethylester Gradient) gereinigt. Die Fraktionen wurden vereinigt, im Vakuum eingeengt und der Rückstand über Nacht aus Acetonitril lyophilisiert. Es wurden 522 mg (66% d. Th., 99% Reinheit) der Titelverbindung erhalten.
LC-MS (Methode 3): $R_t$ = 2.19 min; MS (ESIpos): m/z = 582 $[M+H]^+$.
$^1$H-NMR (400 MHz, DMSO-d6): δ [ppm] = 9.85 (d, 1H), 8.97 (s, 1H), 8.87 (d, 1H), 8.74 (dd, 1H), 8.63 (dd, 1H), 7.65 (dd, 1H), 7.06-6.96 (m, 2H), 4.81-4.66 (m, 1H), 1.94-1.81 (m, 1H), 1.73-1.59 (m, 1H), 0.96 (t, 3H).

**Beispiel 13A**

tert-Butyl-4-[6-{[(1S)-1-cyclopropyl-2,2,2-trifluorethyl]carbamoyl}-3-fluor-5-oxo-8-(2,4,6-trifluorphenyl)-5,8-dihydro-1,8-naphthyridin-2-yl]piperazin-1-carboxylat

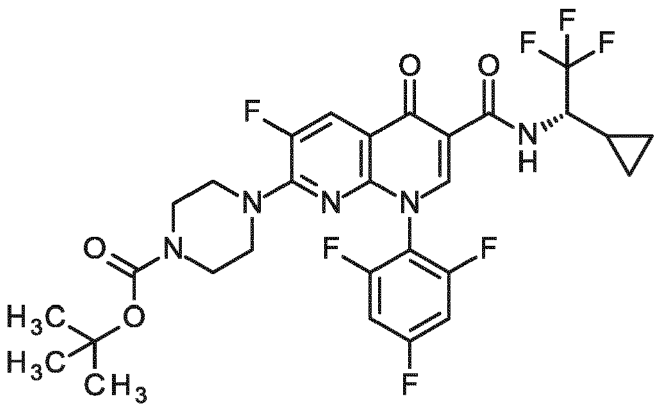

7-Chlor-N-[(1S)-1-cyclopropyl-2,2,2-trifluorethyl]-6-fluor-4-oxo-1-(2,4,6-trifluorphenyl)-1,4-dihydro-1,8-naphthyridin-3-carboxamid (60.0 mg, 122 µmol) wurde in 1.2 ml Acetonitril vorgelegt, mit tert-Butyl-piperazin-1-carboxylat (45.3 mg, 243 µmol) und N,N-Diisopropylethylamin (74 µl, 430 µmol) versetzt und 2 h bei Raumtemperatur gerührt. Die Reaktionslösung wurde im Vakuum eingeengt und ohne weitere Reinigung in die Folgestufe eingesetzt. Es wurden 113 mg der Zielverbindung (quantitative Ausbeute, Reinheit ca. 69%) erhalten.
LC-MS (Methode 3): $R_t$ = 2.61 min; MS (ESIpos): m/z = 644 $[M+H]^+$

**Beispiel 14A**

tert-Butyl-(2S)-4-[6-{[(1S)-1-cyclopropyl-2,2,2-trifluorethyl]carbamoyl}-3-fluor-5-oxo-8-(2,4,6-trifluorphenyl)-5,8-dihydro-1,8-naphthyridin-2-yl]-2-methylpiperazin-1-carboxylat

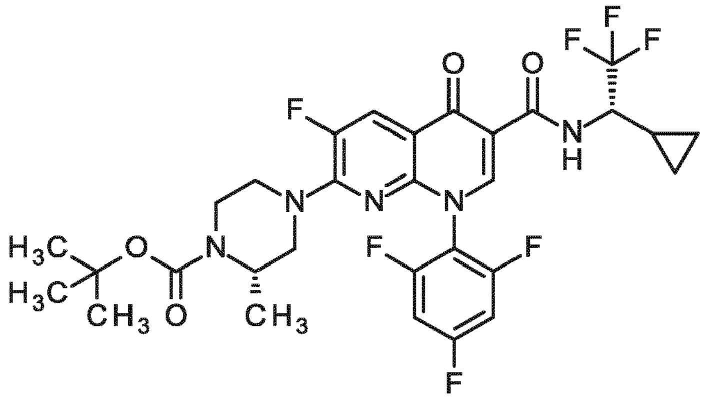

7-Chlor-N-[(1S)-1-cyclopropyl-2,2,2-trifluorethyl]-6-fluor-4-oxo-1-(2,4,6-trifluorphenyl)-1,4-dihydro-1,8-naphthyridin-3-carboxamid (60.0 mg, 122 μmol) wurde in 1.2 ml DMF vorgelegt, mit tert-Butyl-(2S)-2-methylpiperazin-1-carboxylat (34.1 mg, 170 μmol) und N,N-Diisopropylethylamin (74 μl, 430 μmol) versetzt und bei Raumtemperatur für 1 h gerührt. Die Reaktionslösung wurde in Ethylacetat auf-genommen und dreimal mit einer halbgesättigten-Ammoniumchlorid-Lsg. ausgeschüttelt. Die vereinigten wässrigen Phasen wurden einmal mit Ethylacetat reextrahiert. Die vereinigten organischen Phasen wurden über Natriumsulfat getrocknet, abfiltriert und im Vakuum eingeengt. Es wurden 82 mg der Zielverbindung (91% d. Th., Reinheit 90%) erhalten.
LC-MS (Methode 3): $R_t$ = 2.64 min; MS (ESIpos): m/z = 658 $[M+H]^+$

**Beispiel 15A**

7-Chlor-1-(3,5-difluorpyridin-2-yl)-6-fluor-4-oxo-1,4-dihydro-1,8-naphthyridin-3-carbonsäureethylester

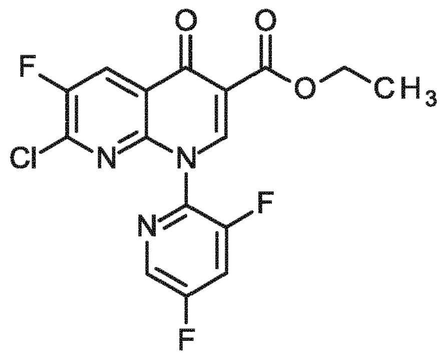

Eine Lösung aus 6.00 g (17.8 mmol) 2-[(2,6-Dichlor-5-fluorpyridin-3-yl)carbonyl]-3-ethoxyacrylsäure-ethylester ( US4840954, 1989, Beispiel G, Stufe 1, Seite 7) und 3.25 g (25.0 mmol) 2-Amino-3,5-difluorpyridin in 30 ml Dichlormethan wurde mit 21.8 ml (125 mmol) DIPEA versetzt und für 4 h bei RT gerührt. Anschließend wurden 2.47 g (17.8 mmol, 1 Äq.) Kaliumcarbonat zugegeben und über Nacht unter Rückfluss erhitzt. Es wurde dann ein weiteres Äquivalent Kaliumcarbonat hinzugegeben und weiter unter Rückfluss über Nacht erhitzt. Es wurde dann ein weiteres Äquivalent Kaliumcarbonat hinzugegeben und weiter für 3d unter Rückfluss erhitzt. Die Mischung wurde mit 200 ml Dichlormethan verdünnt und zweimal mit 200 ml 1M wässriger Salzsäure gewaschen. Die organische Phase wurde über Natriumsulfat getrocknet, filtriert und das Lösungsmittel unter reduziertem Druck entfernt. Es wurde mit 80 ml *tert.*-Butylmethylether verdünnt, der Niederschlag abgesaugt, mit 10 ml *tert.*-Butylmethylether gewaschen und am Hochvakuum getrocknet.

Es wurden 3.73 g (54% d. Th., 99% Reinheit) der Titelverbindung erhalten.
LC-MS (Methode 1): $R_t$ = 0.93 min; MS (ESIpos): m/z = 384 [M+H]+.
$^1$H-NMR (400 MHz, DMSO-d6): δ [ppm] = 8.92 (s, 1H), 8.66 (d, 1H), 8.56 (d, 1H), 8.44-8.37 (m, 1H), 4.26 (q, 2H), 1.28 (t, 3H).

**Beispiel 16A**

7-Chlor-1-(3,5-difluoropyridin-2-yl)-6-fluor-4-oxo-1,4-dihydro-1,8-naphthyridin-3-carbonsäure

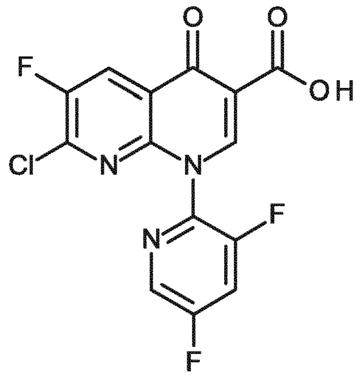

3.60 g (9.38 mmol) 7-Chlor-1-(3,5-difluorpyridin-2-yl)-6-fluor-4-oxo-1,4-dihydro-1,8-naphthyridin-3-carbonsäureethylester wurden in 28.3 ml Wasser vorgelegt, mit 28.3 ml 36 proz. wässriger Salzsäure und 28.3 ml THF versetzt und 4 h bei 110°C nachgerührt. Es wurden dann nacheinander zweimal je 28.3 ml 36 proz. wässriger Salzsäure zugegeben und für 2 d bei 110°C gerührt. Das Reaktionsgemisch wurde auf RT abgekühlt, der Niederschlag abgesaugt, mit Wasser gewaschen und am Hochvakuum getrocknet. Es wurden 3.25 g (96% d. Th., 99% Reinheit) der Titelverbindung erhalten.
LC-MS (Methode 1): $R_t$ = 0.90 min; MS (ESIpos): m/z = 356 $[M+H]^+$.
$^1$H-NMR (400 MHz, DMSO-d6): δ [ppm] = 13.71 (s, 1H), 9.18 (s, 1H), 8.76 (d, 1H), 8.68 (dd, 1H), 8.46-8.39 (m, 1H).

**Beispiel 17A**

1-(3,5-Difluorpyridin-2-yl)-7-[(3*R*,4*R*)-3,4-dihydroxypyrrolidin-1-yl]-6-fluor-4-oxo-1,4-dihydro-1,8-naphthyridin-3-carbonsäure

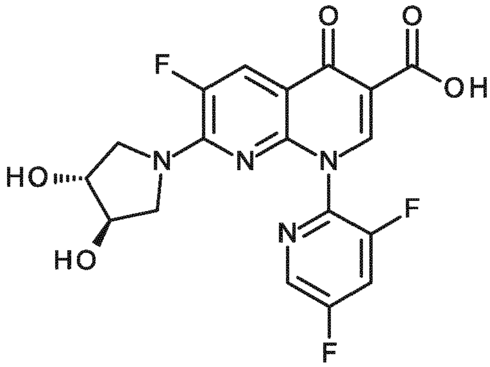

Zu einer Lösung von 1.50 g (4.22 mmol) 7-Chlor-1-(3,5-difluoropyridin-2-yl)-6-fluor-4-oxo-1,4-dihydro-1,8-naphthyridin-3-carbonsäure und 648 mg (4.64 mmol) (3*R*,4*R*)-Pyrrolidin-3,4-diol-Hydrochlorid in 21 ml DMF wurde bei RT 2.57 ml (14.8 mmol) DIPEA gegeben. Es wurde für 2 h bei RT nachgerührt. Es wurde mit wässriger 1M Salzsäure angesäuert und dann mit 100 ml Wasser und 50 ml Essigsäureethylester verdünnt. Die Phasen wurden getrennt und die wässrige Phase zweimal mit 50 ml Essigsäureethylester extrahiert. Die vereinigten organischen Phasen wurden

zweimal mit 50 ml einer pH7-Puffer-Lösung und einmal mit 50 ml gesättigter wässriger Natriumchlorid-Lösung gewaschen, über Natriumsulfat getrocknet, filtriert und im Vakuum eingeengt. Der Rückstand wurde mit 20 ml *tert.*-Butylmethylether verrührt, dekantiert und der Niederschlag am Hochvakuum getrocknet. Es wurden 1.41 g (78% d. Th., 99% Reinheit) der Titelverbindung erhalten.
LC-MS (Methode 3): $R_t$ = 1.07 min; MS (ESIpos): m/z = 423 [M+H]+.
$^1$H-NMR (500 MHz, DMSO-d6): δ [ppm] = 15.02 (s, 1H), 8.96 (s, 1H), 8.66-8.61 (m, 1H), 8.41-8.34 (m, 1H), 8.07 (d, 1H), 5.34-5.06 (m, 2H), 4.14-3.59 (m, 4H), 3.44-3.20 (m, 1H, teilweise unter der Wasser Resonanz), 3.19-3.01 (m, 1H).

**Beispiel 18A**

1-(3,5-Difluorpyridin-2-yl)-6-fluor-7-[(3S)-3-hydroxypyrrolidin-1-yl]-4-oxo-1,4-dihydro-1,8-naphthyridin-3-carbonsäure

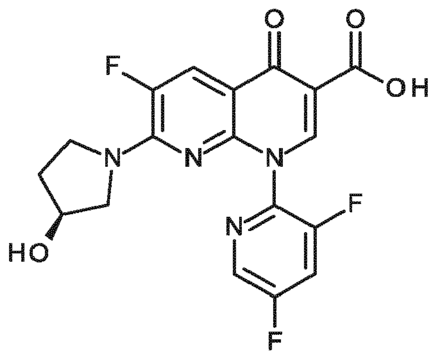

Gemäß AAV3 wurden 300 mg (843 μmol) 7-Chlor-1-(3,5-difluoropyridin-2-yl)-6-fluor-4-oxo-1,4-dihydro-1,8-naphthyridin-3-carbonsäure in 3.1 ml DMF mit 81 mg (928 μmol) (S)-3-Pyrrolidinol und 0.514 ml (2.95 mmol) DIPEA versetzt und 1 h bei RT gerührt. Anschließend wurden nochmals 20 mg (232 μmol) (S)-3-Pyrrolidinol hinzugegeben und das Gemisch wurde 1 h bei RT gerührt. Die Reaktionsmischung wurde mit Wasser verdünnt und direkt mittels präparativer HPLC (Acetonitril-Wasser mit Ameisensäure, C18 RP-HPLC) gereinigt. Es wurden 244 mg (72% d. Th., 100% Reinheit) der Titelverbindung erhalten.
LC-MS (Methode 3): $R_t$ = 1.36 min; MS (ESIpos): m/z = 407 $[M+H]^+$.

**Beispiel 19A**

1-(3,5-Difluorpyridin-2-yl)-6-fluor-7-(3-hydroxyazetidin-1-yl)-4-oxo-1,4-dihydro-1,8-naphthyridin-3-carbonsäure

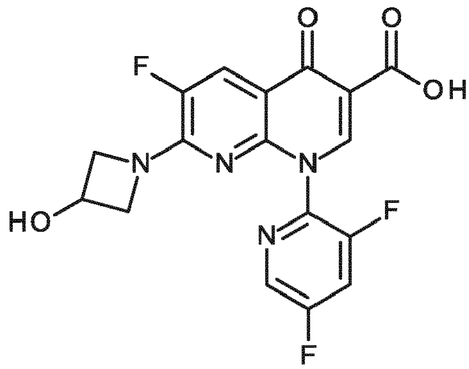

Zu einer Lösung von 500 mg (1.41 mmol) 7-Chlor-1-(3,5-difluoropyridin-2-yl)-6-fluor-4-oxo-1,4-dihydro-1,8-naphthyridin-3-carbonsäure und 169 mg (1.55 mmol) 3-Hydroxyazetidin-Hydrochlorid in 7 ml DMF wurde bei RT 857 μl (4.92 mmol) DIPEA hinzugegeben. Es wurde für 2.5 h bei RT nachgerührt. Es wurde mit wässriger 1M Salzsäure angesäuert, mit 30 ml Wasser und 30 ml Essigsäureethylester verdünnt. Der Niederschlag wurde abgesaugt (erste

Produktfraktion). Die Phasen wurden getrennt und die wässrige Phase zweimal mit 15 ml Essigsäureethylester extrahiert. Die vereinigten organischen Phasen wurden zweimal mit 15 ml Puffer pH 7 und einmal mit 15 ml gesättigter wässriger Natriumchlorid-Lösung gewaschen, über Natriumsulfat getrocknet, filtriert und im Vakuum eingeengt. Der Rückstand wurde mit 10 ml *tert*.-Butylmethylether verrührt, dekantiert und der Niederschlag am Hochvakuum getrocknet (zweite Produktfraktion). Es wurden kombiniert 476 mg (86% d. Th., 99% Reinheit) der Titelverbindung erhalten.
LC-MS (Methode 3): $R_t$ = 1.35 min; MS (ESIpos): m/z = 393 $[M+H]^+$.
$^1$H-NMR (500 MHz, DMSO-d6): $\delta$ [ppm] = 14.98 (s, 1H), 8.95 (s, 1H), 8.62 (d, 1H), 8.39-8.31 (m, 1H), 8.05 (d, 1H), 5.80 (d, 1H), 4.81-3.50 (m, 5H).

## Beispiel 20A

1-(3,5-Difluorpyridin-2-yl)-6-fluor-7-(3-hydroxy-3-methylazetidin-1-yl)-4-oxo-1,4-dihydro-1,8-naphthyridin-3-carbonsäure

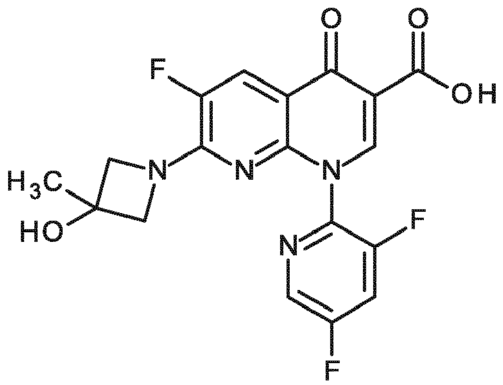

Zu einer Lösung von 500 mg (1.41 mmol) 7-Chlor-1-(3,5-difluoropyridin-2-yl)-6-fluor-4-oxo-1,4-dihydro-1,8-naphthyridin-3-carbonsäure und 191 mg (1.55 mmol) 3-Methylazetidin-3-ol-Hydrochlorid in 7 ml DMF wurde bei RT 857 µl (4.92 mmol) DIPEA hinzugegeben. Es wurde für 2 h bei RT nachgerührt. Es wurde mit wässriger 1M Salzsäure angesäuert, mit 40 ml Wasser verdünnt und der Niederschlag abgesaugt. Der Niederschlag wurde dreimal mit 5 ml Wasser gewaschen und am Hochvakuum getrocknet. Es wurden 534 mg (93% d. Th., 99% Reinheit) der Titelverbindung erhalten.
LC-MS (Methode 3): Rt = 1.43 min; MS (ESIpos): m/z = 407 $[M+H]^+$.
$^1$H-NMR (500 MHz, DMSO-d6): $\delta$ [ppm] = 14.98 (s, 1H), 8.95 (s, 1H), 8.62 (d, 1H), 8.39-8.32 (m, 1H), 8.06 (d, 1H), 5.72 (s, 1H), 4.48-3.49 (m, 4H), 1.38 (s, 3H).

## Beispiel 21A

Ethyl-(2Z)-2-[(2,6-dichlor-5-fluorpyridin-3-yl)carbonyl]-3-ethoxyacrylat

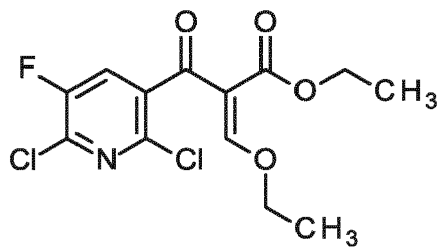

Ethyl-3-(2,6-dichlor-5-fluorpyridin-3-yl)-3-oxopropanoat (19.5 g, 69.6 mmol) und Orthoameisensäuretriethylester (23.1 ml, 140 mmol) wurden in Essigsäureanhydrid (46 ml, 490 mmol) vorgelegt und die Mischung bei 140°C über Nacht gerührt. Das Reaktionsgemisch wurde dann im Vakuum eingeengtim Vakuum eingeengt und ohne weitere Aufarbeitung für die Folgestufen weiter umgesetzt. Man ging dabei von quantitativen Umsatz aus.
LC-MS (Methode 1): $R_t$ = 1.00 min; MS (ESIpos): m/z = 336 $[M+H]^+$

**Beispiel 22A**

Ethyl-7-chlor-1-(2-chlor-4,6-difluorphenyl)-6-fluor-4-oxo-1,4-dihydro-1,8-naphthyridin-3-carboxylat (Atropisomerengemisch)

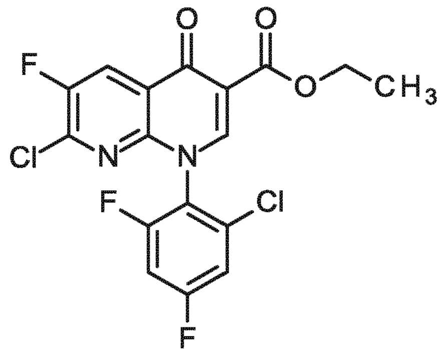

Unter Argon wurden Ethyl-(2Z)-2-[(2,6-dichlor-5-fluorpyridin-3-yl)carbonyl]-3-ethoxyacrylat (24.0 g, 71.4 mmol) und 2-Chlor-4,6-difluoranilin (16.3 g, 100 mmol) in 120 ml Dichlormethan vorgelegt und bei Raumtemperatur mit N,N-Diisopropylethylamin (87 ml, 500 mmol) versetzt. Die Reaktionslösung wurde 4 h bei Raumtemperatur gerührt. Anschließend wurde Kaliumcarbonat (9.87 g, 71.4 mmol) hinzugegeben und über Nacht unter Rückfluss gerührt Das Reaktionsgemisch wurde abgekühlt, mit 300 ml Dichlormethan verdünnt und dreimal mit je 180 ml 1 M Salzsäure gewaschen. Die organische Phase wurde über Natriumsulfat getrocknet, filtriert und im Vakuum eingeengt. Die so erhaltene Suspension wurde in 150 ml *tert.*-Butylmethylether verrührt. Die Lösung wurde im Vakuum eingeengt. Das erhaltene Rohprodukt wurde mittels Kieselgelchromatographie gereinigt (Laufmittel: Cyclohexan/Ethylacetat 10/1 nach 5/1 nach 2/1). Es wurden 13.75g der Zielverbindung (46% d. Th., Reinheit 99%) erhalten.
LC-MS (Methode 3): $R_t$ = 1.98 min; MS (ESIpos): m/z = 417 $[M+H]^+$

**Beispiel 23A**

7-Chlor-1-(2-chlor-4,6-difluorphenyl)-6-fluor-4-oxo-1,4-dihydro-1,8-naphthyridin-3-carbonsäure (Atropisomerengemisch)

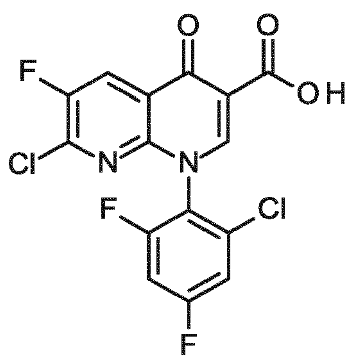

Ethyl-7-chlor-1-(2-chlor-4,6-difluorphenyl)-6-fluor-4-oxo-1,4-dihydro-1,8-naphthyridin-3-carboxylat (6.00 g, 99 % Reinheit, 14.2 mmol) wurde in 43 ml THF suspendiert. Wasser (43 ml) und konz. Salzsäure (43 ml) wurden hinzugeben und das Gemisch wurde 4 h bei 110°C Badtemperatur rühren gelassen. Das organische Lösungsmittel wurde weitestgehend im Vakuum eingeengt. Die Suspension wurde mit 20 ml Wasser versetzt und der ausgefallene Niederschlag

abfiltriert. Es wurden 5.12 g der Zielverbindung (92% d. Th., Reinheit 99%) erhalten.
LC-MS (Methode 3): $R_t$ = 1.93 min; MS (ESIpos): m/z = 389 $[M+H]^+$
$^1$H-NMR (400 MHz, DMSO-d6) δ [ppm]: -0.008 (3.01), 0.008 (2.85), 2.327 (0.79), 2.671 (0.75), 7.752 (1.44), 7.758 (2.19), 7.774 (2.10), 7.782 (6.12), 7.794 (2.31), 7.805 (5.86), 7.816 (1.78), 8.760 (8.43), 8.778 (8.40), 9.250 (16.00), 13.654 (2.73).

**Beispiel 24A**

7-Chlor-1-(2-chlor-4,6-difluorphenyl)-N-[(1S)-1-cyclopropyl-2,2,2-trifluorethyl]-6-fluor-4-oxo-1,4-dihydro-1,8-naphthyridin-3-carboxamid (Atropisomerengemisch)

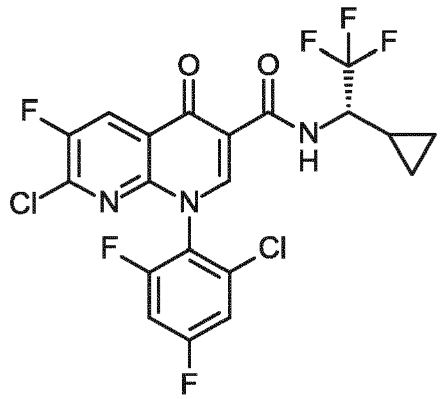

7-Chlor-1-(2-chlor-4,6-difluorphenyl)-6-fluor-4-oxo-1,4-dihydro-1,8-naphthyridin-3-carbonsäure (Atropisomerengemisch, 1.50 g, 3.85 mmol) wurde in 34 ml DMF vorgelegt. HATU (1.47 g, 3.85 nmol) und N,N-Diisopropylethylamin (1.6 ml, 9.3 mmol) hinzugeben und 30 min bei Raumtemperatur vorgerührt. Anschließend wurde (1S)-1-Cyclopropyl-2,2,2-trifluorethanamin-Hydrochlorid (745 mg, 4.24 mmol) hinzugeben und 2 min bei Raumtemperatur nachrühren gelassen. Die Reaktion wurde ohne Reaktionskontrolle direkt aufgearbeitet. Der Ansatz wurde auf 340 ml Wasser gegeben. Der dabei ausgefallene Feststoff wurde abfiltriert und im Hochvakuum getrocknet. Es wurden 2.13 g der Zielverbindung (62% d. Th., Reinheit 57%) erhalten.
LC-MS (Methode 3): $R_t$ = 2.46 min; MS (ESIpos): m/z = 510 $[M+H]^+$

**Beispiel 25A**

1-(2-Chlor-4,6-difluorphenyl)-7-[(3*R*,4*R*)-3,4-dihydroxypyrrolidin-1-yl]-6-fluor-4-oxo-1,4-dihydro-1,8-naphthyridin-3-carbonsäure (Atropisomerengemisch)

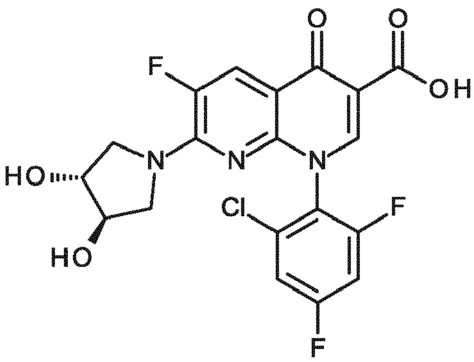

Zu einer Lösung von 500 mg (1.29 mmol) 7-Chlor-1-(2-chlor-4,6-difluorphenyl)-6-fluor-4-oxo-1,4-dihydro-1,8-naphthyridin-3-carbonsäure (Atropisomerengemisch) und 648 mg (4.64 mmol) (3*R*,4*R*)-Pyrrolidin-3,4-diol-Hydrochlorid in 21 ml DMF wurde bei RT 2.57 ml (14.8 mmol) DIPEA gegeben. Es wurde für 12 h bei RT nachgerührt. Es wurde in

100 ml Wasser eingerührt und der Niederschlag abgesaugt. Der Niederschlag wurde mit Wasser gewaschen und am Hochvakuum getrocknet. Es wurden 463 mg (78% d. Th., 99% Reinheit) der Titelverbindung erhalten.
LC-MS (Methode 3): $R_t$ = 1.30 min; MS (ESIpos): m/z = 456 [M+H]+.
$^1$H-NMR (400 MHz, DMSO-d6): δ [ppm] = 15.04 (s, 1H), 9.01 (s, 1H), 8.07 (d, 1H), 7.80-7.69 (m, 1H),), 5.22 (br. s, 2H), 4.09-3.64 (m, 4H), 3.28-3.17 (m, 1H), 3.11-2.94 (m, 1H).

**Beispiel 26A**

1-(2,6-Difluorphenyl)-7-[(3R,4R)-3,4-dihydroxypyrrolidin-1-yl]-6-fluor-4-oxo-1,4-dihydro-1,8-naphthyridin-3-carbonsäure

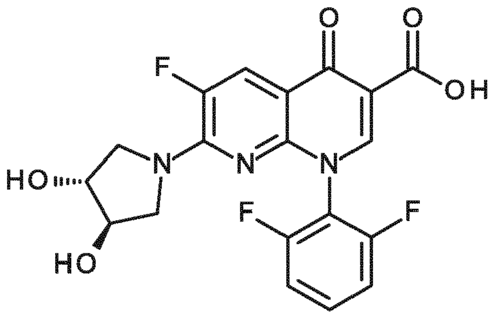

Zu einer Lösung von 1.00 g (2.82 mmol) 7-Chlor-1-(2,6-difluorphenyl)-6-fluor-4-oxo-1,4-dihydro-1,8-naphthyridin-3-carbonsäure und 433 mg (3.10 mmol) (3*R*,4*R*)-Pyrrolidin-3,4-diol-Hydrochlorid in 15.4 ml DMF wurde bei RT 1.72 ml (9.87 mmol) DIPEA gegeben. Es wurde für 2 h bei RT nachgerührt. Anschließend wurde das Gemisch mit wässriger 1M Salzsäure angesäuert, mit 200 ml Wasser und 100 ml Essigsäureethylester verdünnt. Die Phasen wurden getrennt und die wässrige Phase zweimal mit 50 ml Essigsäureethylester extrahiert. Die vereinigten organischen Phasen wurden zweimal mit 25 ml Puffer pH 7, einmal mit 50 ml gesättigter wässriger Natriumchlorid-Lösung gewaschen, über Magnesiumsulfat getrocknet, filtriert und im Vakuum eingeengt. Es wurden 1.03 g (87% d. Th., 100% Reinheit) der Titelverbindung erhalten.
LC-MS (Methode 3): $R_t$ = 1.19 min; MS (ESIpos): m/z = 422 $[M+H]^+$.
$^1$H-NMR (400 MHz, DMSO-d6): δ [ppm] = 15.04 (s, 1H), 9.01 (s, 1H), 8.08 (d, 1H), 7.78-7.68 (m, 1H),), 7.47-7.39 (m, 2H), 5.28-5.14 (m, 2H), 4.09-3.62 (m, 4H), 3.26-3.15 (m, 1H), 3.08-2.96 (m, 1H).

**Beispiel 27A**

Ethyl-(2Z)-3-ethoxy-2-[(2,5,6-trichlorpyridin-3-yl)carbonyl]acrylat

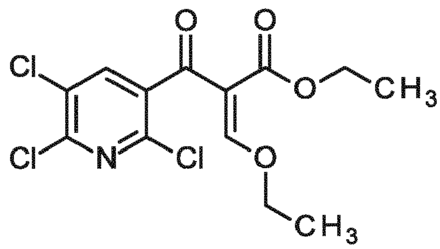

Ethyl-3-oxo-3-(2,5,6-trichlorpyridin-3-yl)propanoat (1.6 g, 5.40 mmol) und (Diethoxymethoxy)ethan (1.80 ml, 1.3 mmol) vorgelegt und Essigsäureanhydrid (3.31 ml, 35.1 mmol) zugegeben. Es wurde über Nacht bei 140 °C gerührt. Das Gemisch wurde eingedampft und ohne weitere Reinigung weiter umgesetzt (Annahme 100% Umsatz).

**Beispiel 28A**

Ethyl-6,7-dichlor-4-oxo-1-(2,4,6-trifluorphenyl)-1,4-dihydro-1,8-naphthyridin-3-carboxylat

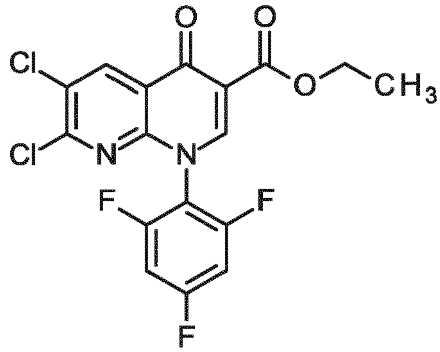

Unter Argon wurde Ethyl-(2Z)-3-ethoxy-2-[(2,5,6-trichlorpyridin-3-yl)carbonyl]acrylat (Annahme: 1.90 g, 5.39 mmol) aus der Vorstufe und 2,4,6-Trifluoranilin (1.11 g, 7.54 mmol) in 50 ml Dichlormethan vorgelegt. Es wurde N,N-Diisopropylethylamin (6.6 ml, 38 mmol) zugegeben und 4 h bei RT gerührt. Dann wurde Kaliumcarbonat (745 mg, 5.39 mmol) zugegeben und bei Rückfluss über Nacht gerührt. Das Reaktionsgemisch wurde mit 120 ml Dichlormethan verdünnt und mit zweimal 40 ml 1M Salzsäure gewaschen, getrocknet und eingeengt. Der Rückstand wurde über Kieselgel gereinigt (Laufinittel Cyclohexan/Ethylacetat = 4:1). Die produkthaltige Fraktionen wurden eingeengt. Es wurden 0.298 g (13% d. Th., 100% Reinheit) der Titelverbindung erhalten.
LC-MS (Methode 1): $R_t$ = 1.08 min; MS (ESIpos): m/z = 417 $[M+H]^+$.

**Beispiel 29A**

6,7-Dichlor-4-oxo-1-(2,4,6-trifluorphenyl)-1,4-dihydro-1,8-naphthyridin-3-carbonsäure

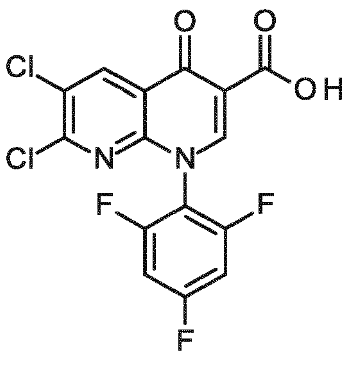

292 mg Ethyl-6,7-dichlor-4-oxo-1-(2,4,6-trifluorphenyl)-1,4-dihydro-1,8-naphthyridin-3-carboxylat (700 µmol) wurden in THF (4.0 ml, 49 mmol), Ethanol (2.0 ml, 34 mmol) und Wasser (1.0 ml) vorgelegt und bei RT mit konz. Salzsäure sauer gestellt (ca. 2 ml) und anschließend bei 110°C 4 d gerührt. Der Niederschlag wurde abfiltriert, mit Wasser gewaschen und über Nacht am Hochvakuum getrocknet. Es wurden 253 mg (90% d. Th., 97% Reinheit) der Titelverbindung erhalten.
LC-MS (Methode 3): $R_t$ = 1.99 min; MS (ESIpos): m/z = 389 $[M+H]^+$.

**Beispiel 30A**

6-Chlor-7-[(3R,4R)-3,4-dihydroxypyrrolidin-1-yl]-4-oxo-1-(,2,4,6-trifluorphenyl)-1,4-dihydro-1,8-naphthyridin-3-carbonsäure

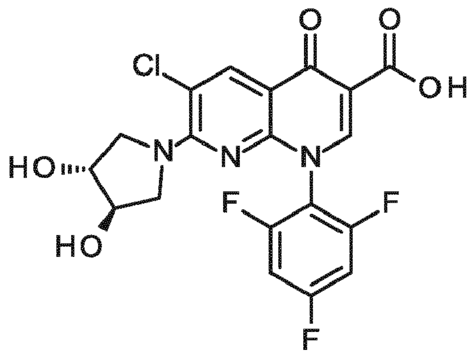

253 mg 6,7-Dichlor-4-oxo-1-(2,4,6-trifluorphenyl)-1,4-dihydro-1,8-naphthyridin-3-carbonsäure (97 % Reinheit, 631 μmol) wurden in DMF (6.0 ml, 78 mmol) gelöst. Es wurden (3R,4R)-Pyrrolidin-3,4-diol-hydrochlorid (99.8 mg, 97 % Reinheit, 694 μmol) und N,N-Diisopropylethylamin (384 μl, 2.2 mmol) zugegeben und 1 h bei RT gerührt. Es wurde mit 20 ml Wasser, 5 ml 1M Salzsäure und 20 ml Ethylacetat verdünnt. Die organische Phasen wurden getrennt und die wässrige Phase wurde dreimal mit 20 ml Ethylacetat extrahiert. Die vereinten org. Phasen wurden zweimal mit 20 ml Puffer (pH 7) und 20 ml ges. wässrige Natriumchlorid-Lsg. gewaschen, über Magnesiumsulfat getrocknet, filtriert und eingeengt. Es wurden 172 mg (57% d. Th., 95% Reinheit) der Titelverbindung erhalten.
LC-MS (Methode 3): $R_t$ = 1.33 min; MS (ESIpos): m/z = 456 $[M+H]^+$
$^1$H-NMR (400 MHz, DMSO-d6) δ [ppm]: -0.150 (0.50), 0.008 (4.41), 0.146 (0.53), 1.157 (0.75), 1.175 (1.58), 1.193 (1.14), 1.211 (0.80), 1.229 (0.63), 1.263 (0.54), 1.988 (2.86), 2.327 (0.86), 2.366 (0.56), 2.670 (1.04), 2.710 (0.65), 2.731 (7.25), 2.891 (9.16), 3.940 (5.63), 4.003 (0.85), 4.021 (1.18), 4.038 (1.09), 4.056 (0.63), 4.176 (0.54), 4.194 (0.51), 5.210 (10.86), 5.216 (10.51), 5.754 (0.55), 7.582 (5.35), 7.604 (9.75), 7.626 (5.44), 7.952 (1.08), 8.314 (15.84), 9.065 (16.00), 14.776 (6.50).

**Beispiel 31A**

7-Chlor-6-fluor-4-oxo-1-(2,4,6-trifluorphenyl)-1,4-dihydro-1,8-naphthyridin-3-carbonylchlorid

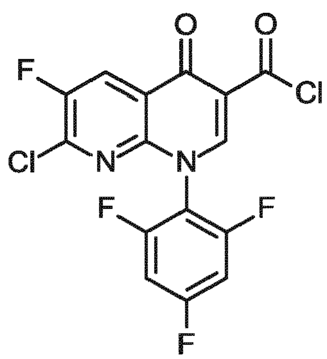

Zu einer Lösung von 300 mg (805 μmol) 7-Chlor-6-fluor-4-oxo-1-(2,4,6-trifluorphenyl)-1,4-dihydro-1,8-naphthyridin-3-carbonsäure in 6 ml THF wurden 180 μl (2.40 mmol) Thionylchlorid gegeben und 3 h unter Rückfluss nachgerührt und anschließend alle flüchtigen Komponenten unter reduziertem Druck entfernt. Das Rohprodukt wurde ohne weitere Aufarbeitung im nächsten Schritt eingesetzt (quantitative Umsetzung wurde angenommen).

**Beispiel 32A**

7-Chlor-*N*-(2,6-dichlorphenyl)-6-fluor-4-oxo-1-(2,4,6-trifluorphenyl)-1,4-dihydro-1,8-naphthyridin-3-carboxamid

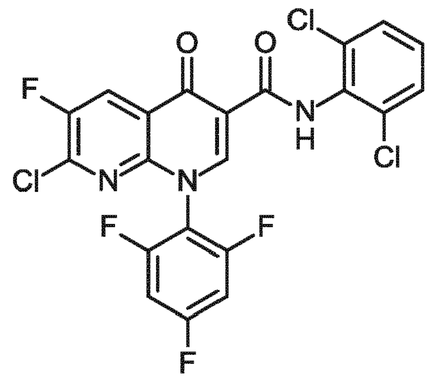

Zu einer Lösung von 314 mg (803 μmol) 7-Chlor-6-fluor-4-oxo-1-(2,4,6-trifluorphenyl)-1,4-dihydro-1,8-naphthyridin-3-carbonylchlorid in 20 ml Dichlormethan wurden bei RT 340 μl (2.40 mmol) Triethylamin und 156 mg (963 μmol) 2,6-Dichloranilin gegeben. Es wurde für 30 min bei RT und über Nacht bei 50°C nachgerührt. Die Reaktionsmischung wurde eingeengt und in Dichlormethan aufgenommen, zweimal mit 1 M wässriger Salzsäure gewaschen, über Magnesiumsulfat getrocknet, filtriert und das Lösungsmittel unter reduziertem Druck entfernt. Das Rohprodukt wurde mittels präparativer HPLC (Säule: Acetonitril / Wasser / 0.1% Ameisensäure) gereinigt. Es wurden 255 mg (61% d. Th., 100% Reinheit) der Titelverbindung erhalten.
LC-MS (Methode 1): $R_t$ = 1.28 min; MS (ESIpos): m/z = 516 $[M+H]^+$
$^1$H-NMR (400 MHz, DMSO-d6) δ [ppm]: -0.149 (0.50), -0.008 (5.02), 0.008 (4.03), 0.146 (0.49), 1.245 (0.63), 1.260 (0.75), 1.275 (0.44), 2.073 (11.19), 2.328 (0.67), 2.367 (0.63), 2.524 (2.42), 2.670 (0.76), 2.710 (0.70), 2.891 (0.41), 7.381 (3.22), 7.402 (6.06), 7.422 (4.60), 7.596 (16.00), 7.608 (4.71), 7.616 (13.09), 7.629 (7.60), 7.652 (4.03), 8.767 (6.42), 8.786 (6.40), 9.250 (10.90), 11.287 (9.23).

**Beispiel 33A**

Ethyl-2-[(2,5-dichlorpyridin-3-yl)carbonyl]-3-(dimethylamino)acrylat

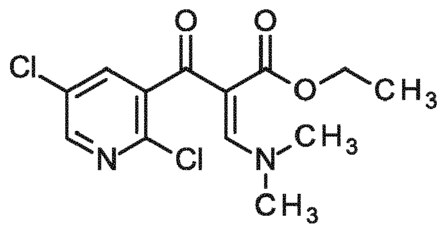

2.0 g (10.42 mmol) 2,5-Dichlornicotinsäure in 27 ml Dichlormethan wurden bei RT mit 1.34 ml (15.39 mmol) Oxalylchlorid und 4 Tropfen DMF versetzt und 1.5 h bei RT gerührt. Anschließend wurde die klare Lösung eingedampft, mit Toluol versetzt und erneut eingedampft (zweimal). Das erhaltene Zwischenprodukt wurde in 67 ml Toluol gelöst, mit 2.17 ml (15.60 mmol) Triethylamin und 1.94 g (13.54 mmol) Ethyl-(2E)-3-(dimethylamino)acrylat versetzt. Das Gemisch wurde 2.5 h bei 90°C gerührt, abgekühlt, abfiltriert und zur Trockene eingedampft. Das Rohprodukt wurde wurde mittels Silicagelchromatographie (Lösungsmittel: Cyclohexan / Ethylacetat = 1:1) gereinigt. Es wurden 4.10 g (quantitative Ausbeute, ca. 95% Reinheit) der Titelverbindung erhalten.
LC-MS (Method 1): $R_t$ = 0.76 min; MS (ESIpos): m/z = 317 $[M+H]^+$

**Beispiel 34A**

Ethyl-6-chlor-1-(2,4-difluorphenyl)-4-oxo-1,4-dihydro-1,8-naphthyridin-3-carboxylat

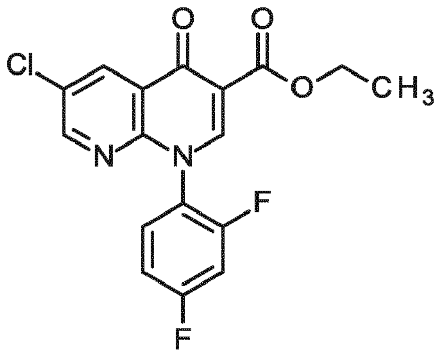

Zu einer Lösung von 2.00 g (6.31 mmol) Ethyl-2-[(2,5-dichlorpyridin-3-yl)carbonyl]-3-(dimethylamino)acrylat in 15 ml Ethanol wurden 770 µl (7.60 mmol) 2,4-Difluoranilin in 3.8 ml THF gegeben und die Reaktionsmischung über Nacht bei RT gerührt. Anschließend wurde das Lösungsmittel unter reduziertem Druck entfernt, der Rückstand in 20 ml DMF aufgenommen und mit 1.31 g (9.48 mmol) Kaliumcarbonat versetzt. Die Suspension wurde dann für 1 h bei 100°C gerührt, anschließend auf RT abgekühlt und auf 50 ml Wasser gegeben. Der Niederschlag wurde abfiltriert und dreimal mit Wasser gewaschen. Es wurden 1.06 g (46% d. Th., 91% Reinheit) der Titelverbindung erhalten, welche ohne weitere Reinigung in die Folgestufe eingesetzt wurde.
LC-MS (Methode 1): $R_t$ = 0.99 min; MS (ESIpos): m/z = 365 $[M+H]^+$
$^1H$ NMR (400 MHz, DMSO-$d_6$): δ ppm = 8.80 (d, 1 H), 8.78 (s, 1 H), 8.59 (d, 1 H), 7.80 - 7.88 (m, 1 H), 7.57 - 7.65 (m, 1 H), 7.31 - 7.39 (m, 1 H), 4.24 (q, 2 H), 1.28 (t, 3 H).

**Beispiel 35A**

6-Chlor-1-(2,4-difluorphenyl)-4-oxo-1,4-dihydro-1,8-naphthyridin-3-carbonsäure

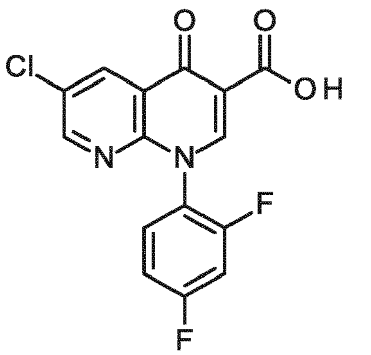

Zu einer Suspension von 1.10 g (3.02 mmol) Ethyl-6-chlor-1-(2,4-difluorphenyl)-4-oxo-1,4-dihydro-1,8-naphthyridin-3-carboxylat in 10 ml THF und 3.6 ml Wasser wurden 127 mg (3.02 mmol) Lithiumhydroxid Monohydrat gegeben und die Reaktionsmischung für 1 h bei Raumtemperatur gerührt. Anschließend wurde mit 20 ml THF und 20 mL Wasser verdünnt und der pH-Wert mit 1M wässr. Salzsäure auf pH 1 eingestellt. Essigsäureethylester wurde hinzugegeben und die wässrige Phase wurde dreimal mit Essigsäureethylester extrahiert. Die organische Phase wurde über Natriumsulfat getrocknet, filtriert und das Lösungsmittel im Vakuum entfernt. Es wurden 0.90 g (86% d. Th., 97% Reinheit) der Titelverbindung erhalten.
LC-MS (Methode 1): $R_t$ = 0.96 min; MS (ESIpos): m/z = 337 $[M+H]^+$
$^1H$ NMR (400 MHz, DMSO-$d_6$): δ ppm = 13.98 (br s, 1 H), 9.10 (s, 1 H), 8.95 (d, 1 H), 8.80 (d, 1 H), 7.80 - 7.89 (m, 1

H), 7.58 - 7.67 (m, 1 H), 7.26 - 7.47 (m, 1 H).

**Beispiel 36A**

6-Chlor-1-(2,4-difluorphenyl)-4-oxo-1,4-dihydro-1,8-naphthyridin-3-carbonylchlorid

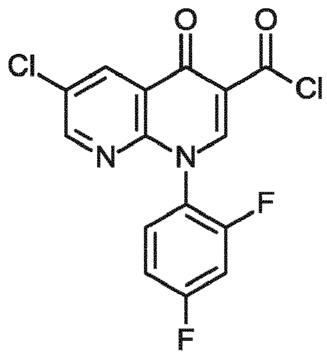

Zu einer Lösung von 150 mg (446 µmol) 6-Chlor-1-(2,4-difluorphenyl)-4-oxo-1,4-dihydro-1,8-naphthyridin-3-carbonsäure in 3 ml THF wurden 58 µl (670 µmol) Oxalsäuredichlorid und DMF (katalytische Mengen) gegeben. Die Reaktionsmischung wurde 1 h bei Raumtemperatur und eine weitere Stunde unter Rückfluss gerührt. Anschließend wurden alle flüchtigen Komponenten unter reduziertem Druck entfernt. Das Rohprodukt wurde ohne weitere Aufarbeitung im nächsten Schritt eingesetzt (quantitative Umsetzung wurde angenommen).

**Beispiel 37A**

7-Chlor-N-[(1S)-1-cyclopropyl-2,2,2-trifluorethyl]-6-fluor-4-oxo-1-(2,4,6-trifluorphenyl)-1,4-dihydro-1,8-naphthyridin-3-carboxamid

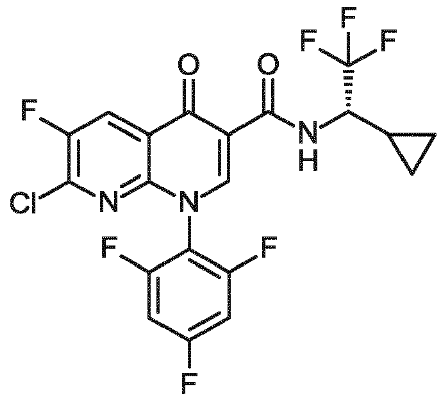

7-Chlor-6-fluor-4-oxo-1-(2,4,6-trifluorphenyl)-1,4-dihydro-1,8-naphthyridin-3-carbonsäure (100 mg, 268 µmol) wurde in 2.5 ml Acetonitril vorgelegt, (1S)-1-Cyclopropyl-2,2,2-trifluorethanaminhydrochlorid (51.8 mg, 295 µmol) und N,N-Diisopropylethylamin (190 µl, 1.1 mmol) wurden hinzugegeben. Anschließend wurde mit T3P-Lösung (Propanphosphonsäure-cyclo-anhydrid, 50% in Ethylacetat, 190 µl, 320 µmol) versetzt. Die Reaktionslösung wurde über Nacht bei Raumtemperatur gerührt. Das Reaktionsgemisch wurde anschließend mit Wasser versetzt und der ausgefallene Feststoff abfiltriert und im Hochvakuum getrocknet. Es wurden 145 mg der Zielverbindung (quantitative Ausbeute) erhalten.
LC-MS (Methode 3): $R_t$ = 2.42 min; MS (ESIpos): m/z = 494 $[M+H]^+$
$^1$H-NMR (400 MHz, DMSO-d6) δ [ppm]: -0.150 (0.74), 0.146 (0.69), 0.335 (4.38), 0.348 (3.99), 0.359 (2.50), 0.567 (5.66), 0.579 (6.91), 0.590 (7.00), 0.624 (1.67), 0.651 (2.50), 0.670 (4.11), 0.687 (2.47), 1.224 (2.32), 1.237 (3.75), 1.245 (3.07),

1.257 (3.55), 1.268 (2.06), 2.328 (1.49), 2.366 (1.19), 2.669 (1.43), 2.710 (1.01), 4.370 (2.03), 4.391 (3.66), 4.411 (3.61), 4.433 (1.94), 5.754 (2.89), 7.602 (6.41), 7.624 (12.45), 7.647 (6.50), 8.709 (9.33), 8.728 (9.33), 9.157 (16.00), 9.972 (6.97), 9.996 (6.88).

**Beispiel 38A**

*N*-Benzyl-1,1,1,2,2-pentafluorbutan-3-amin (Racemat)

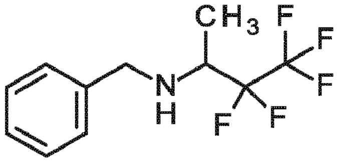

Zu einer Lösung von 2.00 g (12.2 mmol) 3,3,4,4,4-Pentafluorobutan-2-on in 10 ml Dichlormethan wurden bei 0°C 5.40 ml (18.3 mmol) Titantetraisopropoxid und 2.66 ml (24.4 mmol) Benzylamin gegeben. Es wurde 90 min bei RT nachgerührt bevor erneut auf 0°C abgekühlt wurde. Anschließend wurde 2.14 g (34.1 mmol) Natriumcyanoborhydrid, 36 ml Methanol und 3Å Molsieb zugegeben. Es wurde auf RT erwärmt und 2d nachgerührt. Die Reaktionslösung wurde mit wenig Wasser und Essigsäureethylester versetzt und filtriert. Das Filtrat wurde zweimal mit gesättigter wässriger Natriumhydrogencarbonat-Lösung und einmal mit gesättigter wässriger Natriumchlorid-Lösung gewaschen. Die organische Phase wurde über Natriumsulfat getrocknet, filtriert und das Lösungsmittel unter reduziertem Druck entfernt. Der Rückstand wurde zweimal mittels Normalphasenchromatographie (Essigsäureethylester/Cyclohexan 1/20) gereinigt und 1.65 g (48% d. Th., 91% Reinheit) der Titelverbindung erhalten.
LC-MS (Methode 6): $R_t$ = 2.17 min; MS (ESIpos): m/z = 254 $[M+H]^+$.
$^1$H NMR (500 MHz, DMSO-d6): δ [ppm] = 7.28-7.36 (m, 4H), 7.20-7.27 (m, 1H), 3.83 (dd, 1H), 3.72 (dd, 1H), 3.22-3.30 (m, 1H), 2.43-2.48 (m, 1H), 1.20 (d, 3H).

**Beispiel 39A**

1,1,1,2,2-Pentafluorbutan-3 -amin-Hydrochlorid (Racemat)

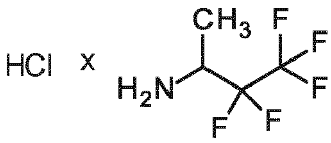

Zu einer Lösung von 1.50 g (5.92 mmol) *N*-Benzyl-1,1,1,2,2-pentafluorpentan-3-amin in 27.4 ml Methanol wurden 150 mg Palladium auf Kohle (10%) gegeben und für 6h bei Normaldruck und Raumtemperatur hydriert. Anschließend wurde die Reaktionsmischung durch einen Milliporefilter filtriert und das Lösungsmittel unter reduziertem Druck entfernt. Die Vorlage mit dem abdestillierten Lösungsmittel wurde anschließend in einen Kolben überführt und mit 4N wässriger Salzsäure in Dioxan versetzt und erneut eingeengt. Der Rückstand wurde mit Diethylether verrührt, der Niederschlag abgesaugt und im Hochvakuum getrocknet. Es wurden 456 mg (39% d. Th., 100% Reinheit) der Titelverbindung erhalten.
$^1$H NMR (500 MHz, DMSO-d6): δ [ppm] = 9.21 (br. s, 3H), 4.40-4.29 (m, 1H), 1.41 (d, 3H).

**Beispiel 40A**

*N*-Benzyl-1,1,1,2,2-pentafluorpentan-3-amin (Racemat)

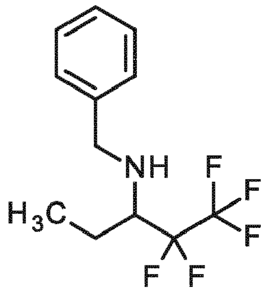

Zu einer Lösung von 2.00 g (11.4 mmol) 1,1,1,2,2-Pentafluorpentan-3-on in 10 ml Dichlormethan wurden bei 0°C 5.03 ml (17.0 nmol) Titantetraisopropoxid und 2.48 ml (22.7 mmol) Benzylamin gegeben. Es wurde 90 min bei RT nachgerührt bevor erneut auf 0°C abgekühlt wurde. Anschließend wurden 2.00 g (31.8 mmol) Natriumcyanoborhydrid, 36 ml Methanol und 3Å Molsieb zugegeben. Es wurde auf RT erwärmt und 2d nachgerührt. Die Reaktionslösung wurde dann mit wenig Wasser und Essigsäureethylester versetzt und filtriert. Das Filtrat wurde zweimal mit gesättigter wässriger Natriumhydrogencarbonat-Lösung und einmal mit gesättigter wässriger Natriumchlorid-Lösung gewaschen. Die organische Phase wurde über Natriumsulfat getrocknet, filtriert und das Lösungsmittel unter reduziertem Druck entfernt. Der Rückstand wurde mittels Normalphasenchromatographie (Essigsäureethylester/Cyclohexan 1/20) gereinigt und 989 mg (25% d. Th., 76% Reinheit) der Titelverbindung erhalten.
LC-MS (Methode 1): $R_t$ = 1.27 min; MS (ESIpos): m/z = 268 $[M+H]^+$
$^1H$ NMR (400 MHz, DMSO-d6): δ [ppm] = 7.21-7.36 (m, 5H), 3.73-3.85 (m, 2H), 3.05-3.20 (m, 1H), 1.63-1.75 (m, 1H), 1.49-1.61 (m, 1H), 1.15-1.20 (m, 1H), 0.96 (t, 3H).

**Beispiel 41A**

1,1,1,2,2-Pentafluorpentan-3-aminhydrochlorid (Racemat)

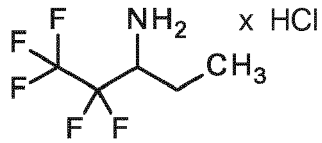

Zu einer Lösung von 980 mg (2.75 mmol, 75% Reinheit) der Verbindung aus Beispiel 40A in 11.3 ml Methanol wurden 75 mg Palladium auf Kohle (10%) gegeben und für 6 h bei Normaldruck und Raumtemperatur hydriert. Anschließend wurde die Reaktionsmischung durch einen Milliporefilter filtriert und das Lösungsmittel unter reduziertem Druck entfernt. Die Vorlage mit dem abdestillierten Lösungsmittel wurde anschließend in einen Kolben überführt und mit 4 M wässriger Salzsäure in Dioxan versetzt und erneut eingeengt. Der Rückstand wurde mit Diethylether verrührt, der Niederschlag abgesaugt und im Hochvakuum getrocknet. Es wurden 379 mg (65% d. Th., 100% Reinheit) der Titelverbindung erhalten.
$^1H$ NMR (400 MHz, DMSO-d6): δ [ppm] = 8.97 (br. s, 3H), 4.16-4.28 (m, 1H), 1.67-1.94 (m, 2H), 1.05 (t, 3H).

AUSFÜHRUNGSBEISPIELE:

**Beispiel 1**

1-(2,6-Difluorphenyl)-7-[(3*R*,4*R*)-3,4-dihydroxypyrrolidin-1-yl]-6-fluor-4-oxo-*N*-(4,4,4-trifluor-2-methylbutan-2-yl)-1,4-dihydro-1,8-naphthyridin-3-carbonsäureamid

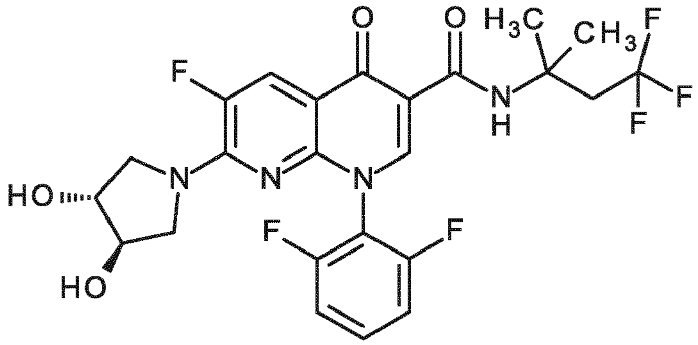

Gemäß AAV1 wurden 99.9 mg (237 µmol) 1-(2,6-Difluorphenyl)-7-[(3R,4R)-3,4-dihydroxypyrrolidin-1-yl]-6-fluor-4-oxo-1,4-dihydro-1,8-naphthyridin-3-carbonsäure mit 40.1 mg (284 µmol) 4,4,4-Trifluor-2-methylbutan-2-amin in Gegenwart von 108 mg (284 µmol) HATU und 103 µl (593 µmol) DIPEA in 2.4 ml DMF zur Reaktion gebracht. Das Reaktionsgemisch wurde direkt mittels präparativer HPLC [bei UV max: 265nm, Säule: Chromatorex C18, 10 µm, 125x30 mm, Lösungsmittel: Acetonitril / 0.05% Ameisensäure-Gradient; (0 bis 3 min. 10% Acetonitril bis 15 min. 90% Acetonitril und weitere 3 min. 90% Acetonitril)] gereinigt. Die Produktfraktionen wurden vereinigt, vom Lösungsmittel befreit und lyophilisiert. Es wurden 107 mg (82% d. Th., 99% Reinheit) der Titelverbindung erhalten.
LC-MS (Methode 3): $R_t$ = 1.76 min; MS (ESIpos): m/z = 545 $[M+H]^+$.
$^1H$ NMR (400 MHz, DMSO-d6): δ [ppm] = 10.10 (s, 1H), 8.67 (s, 1H), 8.00 (d, 1H), 7.77-7.66 (m, 1H), 7.47-7.36 (m, 2H), 5.18 (br. s, 2H), 4.09-3.51 (br. m, 4H), 3.27-2.86 (m, 4H).

## Beispiel 2

*N*-[(1*S*)-1-Cyclopropyl-2,2,2-trifluorethyl]-1-(2,6-difluorphenyl)-7-[(3*R*,4*R*)-3,4-dihydroxypyrrolidin-1-yl]-6-fluor-4-oxo-1,4-dihydro-1,8-naphthyridin-3-carbonsäureamid

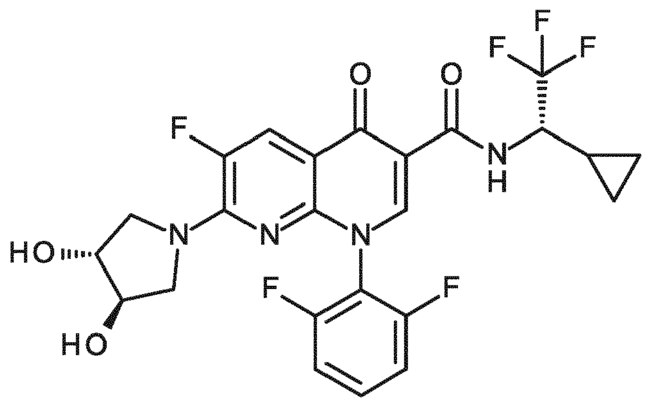

Gemäß AAV1 wurden 99.9 mg (237 µmol) 1-(2,6-Difluorphenyl)-7-[(3R,4R)-3,4-dihydroxypyrrolidin-1-yl]-6-fluor-4-oxo-1,4-dihydro-1,8-naphthyridin-3-carbonsäure mit 49.9 mg (284 µmol) (1*S*)-1-Cyclopropyl-2,2,2-trifluorethanamin Hydrochlorid in Gegenwart von 108 mg (284 µmol) HATU und 103 µl (593 µmol) DIPEA in 2.4 ml DMF zur Reaktion gebracht. Das Reaktionsgemisch wurde anschließend direkt mittels präparativer HPLC [bei UV max: 265nm, Säule: Chromatorex C18, 10 µm, 125x30 mm, Lösungsmittel: Acetonitril / 0.05% Ameisensäure-Gradient; (0 bis 3 min. 10% Acetonitril bis 15 min. 90% Acetonitril und weitere 3 min. 90% Acetonitril)] gereinigt. Die Produktfraktionen wurden vereinigt, vom Lösungsmittel befreit und lyophilisiert. Es wurden 100 mg (77% d. Th., 99% Reinheit) der Titelverbindung erhalten.
LC-MS (Methode 3): $R_t$ = 1.77 min; MS (ESIpos): m/z = 543 $[M+H]^+$.
$^1H$ NMR (400 MHz, DMSO-d6): δ [ppm] = 10.49 (d, 1H), 8.78 (s, 1H), 8.02 (d, 1H), 7.76-7.67 (m, 1H), 7.46-7.38 (m, 2H), 5.19 (br. s, 2H), 4.45-4.32 (m, 1H), 4.11-3.53 (br. m, 4H), 3.27-2.89 (m, 2H), 1.27-1.16 (m, 1H), 0.70-0.49 (m, 3H), 0.38-0.28 (m, 1H).

**Beispiel 3**

1-(2,6-Difluorphenyl)-7-[(3*R*,4*R*)-3,4-dihydroxypyrrolidin-1-yl]-6-fluor-4-oxo-*N*-[(2*S*)-1,1,1-trifluorbutan-2-yl]-1,4-dihydro-1,8-naphthyridin-3-carbonsäureamid

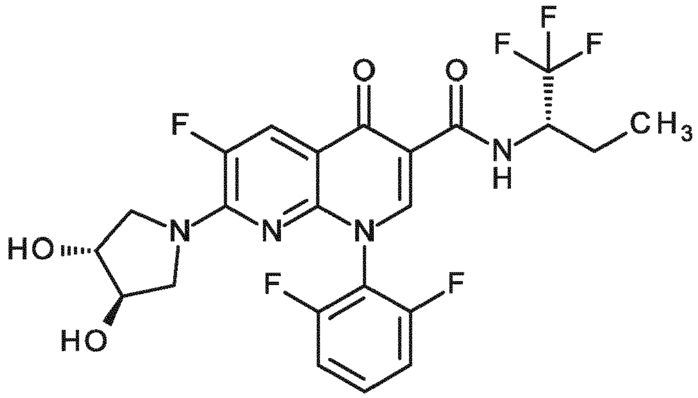

Gemäß AAV1 wurden 100 mg (237 μmol) 1-(2,6-Difluorphenyl)-7-[(3R,4R)-3,4-dihydroxypyrrolidin-1-yl]-6-fluor-4-oxo-1,4-dihydro-1,8-naphthyridin-3-carbonsäure mit 46.6 mg (285 μmol) (2*S*)-1,1,1-Trifluorbutan-2-amin Hydrochlorid in Gegenwart von 108 mg (285 μmol) HATU und 103 μl (593 μmol) DIPEA in 2.4 ml DMF zur Reaktion gebracht. Das Reaktionsgemisch wurde dann mit 2 ml wässriger Salzsäure verdünnt und mittels präparativer HPLC [bei UV max: 265nm, Säule: Chromatorex C18, 10 μm, 125x30 mm, Lösungsmittel: Acetonitril / 0.05% Ameisensäure-Gradient; (0 bis 3 min. 10% Acetonitril bis 15 min. 90% Acetonitril und weitere 3 min. 90% Acetonitril)] gereinigt. Die Produktfraktionen wurden vereinigt, vom Lösungsmittel befreit und lyophilisiert. Es wurden 32.7 mg (26% d. Th., 100% Reinheit) der Titelverbindung erhalten.
LC-MS (Methode 1): $R_t$ = 0.92 min; MS (ESIpos): m/z = 531 $[M+H]^+$.
$^1H$ NMR (400 MHz, DMSO-d6): δ [ppm] = 10.36 (d, 1H), 8.79 (s, 1H), 8.02 (d, 1H), 7.77-7.67 (m, 1H), 7.47-7.38 (m, 2H), 5.19 (br. s, 2H), 4.81-4.67 (m, 1H), 4.10-3.56 (br. m, 4H), 3.27-2.90 (m, 2H), 1.94-1.82 (m, 1H), 1.71-1.58 (m, 1H), 0.97 (t, 1H).

**Beispiel 4**

1-(2,6-Difluorphenyl)-6-fluor-7-[(2-hydroxyethyl)(methyl)amino]-4-oxo-*N*-[(2*S*)-1,1,1-trifluorbutan-2-yl]-1,4-dihydro-1,8-naphthyridin-3-carbonsäureamid

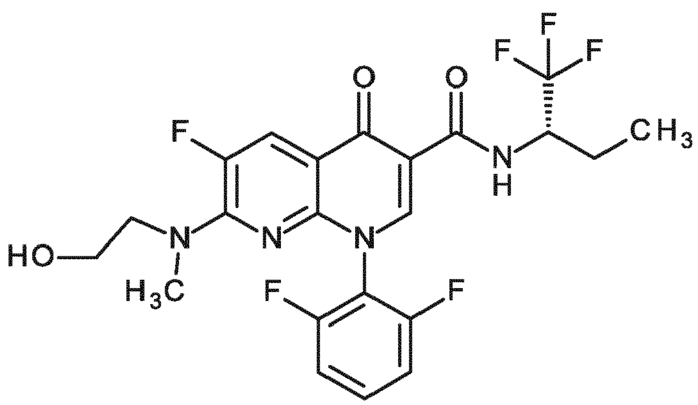

Gemäß AAV3 wurden 50.0 mg (108 μmol) 7-Chlor-1-(2,6-difluorphenyl)-6-fluor-4-oxo-*N*-[(2*S*)-1,1,1-trifluorbutan-2-yl]-1,4-dihydro-1,8-naphthyridin-3-carbonsäureamid mit 8.91 mg (119 μmol) 2-(Methylamino)ethanol in Gegenwart von 66 μl (0.38 mmol) DIPEA in 0.5 ml DMF zur Reaktion gebracht. Es wurde dann mit Acetonitril, Wasser und 0.2 ml

wässriger Salzsäure verdünnt und die Rohlösung mittels präparativer HPLC (Acetonitril-Wasser mit Ameisensäure, C18 RP-HPLC) gereinigt. Die Produktfraktionen wurden verreinigt, im Vakuum eingeengt und über Nacht aus Acetonitril/Wasser lyophilisiert. Es wurden 37.9 mg (70% d. Th., 100% Reinheit) der Titelverbindung erhalten.
LC-MS (Methode 3): $R_t$ = 1.97 min; MS (ESIpos): m/z = 503 $[M+H]^+$.
$^1$H NMR (400 MHz, DMSO-d6): δ [ppm] = 10.33 (d, 1H), 8.81 (s, 1H), 8.01 (d, 1H), 7.75-7.65 (m, 1H), 7.45-7.37 (m, 2H), 4.80-4.67 (m, 2H), 3.51-3.35 (m, 4H), 3.05 (s, 3H), 1.94-1.82 (m, 1H), 1.71-1.58 (m, 1H), 0.97 (t, 3H).

**Beispiel 5**

*N*-(Bicyclo[1.1.1]pent-1-yl)-1-(2,6-difluorphenyl)-7-[(3*R*,4*R*)-3,4-dihydroxypyrrolidin-1-yl]-6-fluor-4-oxo-1,4-dihydro-1,8-naphthyridin-3-carbonsäureamid

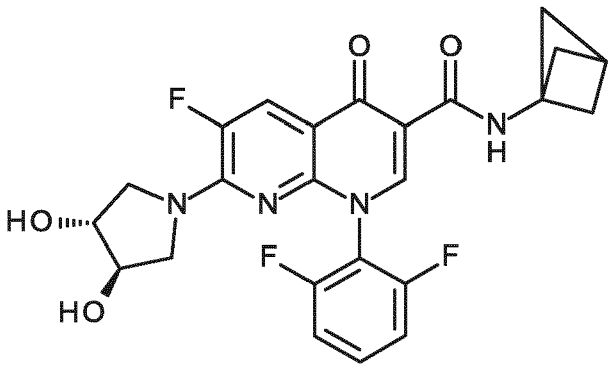

Gemäß AAV1 wurden 100 mg (237 μmol) 1-(2,6-Difluorphenyl)-7-[(3R,4R)-3,4-dihydroxypyrrolidin-1-yl]-6-fluor-4-oxo-1,4-dihydro-1,8-naphthyridin-3-carbonsäure mit 34.1 mg (285 μmol) Bicyclo[1.1.1]pentan-1-amin Hydrochlorid in Gegenwart von 108 mg (285 μmol) HATU und 103 μl (593 μmol) DIPEA in 2.4 ml DMF zur Reaktion gebracht. Das Reaktionsgemisch wurde dann mit 2 ml wässriger Salzsäure verdünnt und zweimal mittels präparativer HPLC [bei UV max: 265nm, Säule: Chromatorex C18, 10 μm, 125x30 mm, Lösungsmittel: Acetonitril / 0.05% Ameisensäure-Gradient; (0 bis 3 min. 10% Acetonitril bis 15 min. 90% Acetonitril und weitere 3 min. 90% Acetonitril)] gereinigt. Die Produktfraktionen wurden vereinigt, vom Lösungsmittel befreit und lyophilisiert. Es wurden 3 mg (2% d. Th., 100% Reinheit) der Titelverbindung erhalten.
LC-MS (Methode 3): $R_t$ = 1.65 min; MS (ESIpos): m/z = 487 $[M+H]^+$.

**Beispiel 6**

7-[(3*R*,4*R*)-3,4-Dihydroxypyrrolidin-1-yl]-6-fluor-4-oxo-*N*-[(2*S*)-1,1,1-trifluorbutan-2-yl]-1-(2,4,6-trifluorphenyl)-1,4-dihydro-1,8-naphthyridin-3-carbonsäureamid

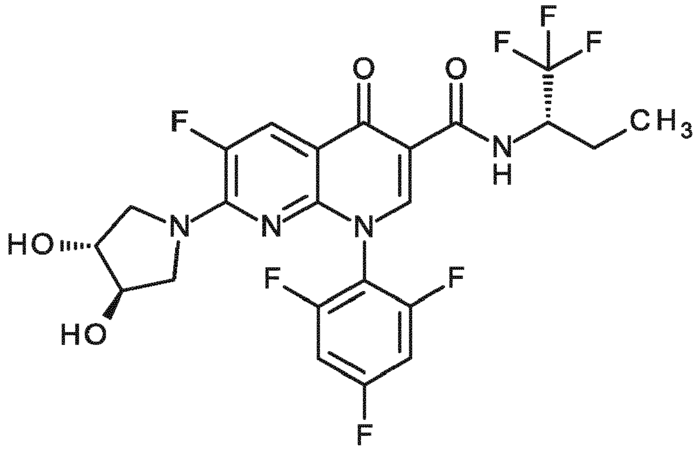

Gemäß AAV3 wurden 417 mg (717 µmol) 6-Fluor-4-oxo-7-(1-[1,2,3]triazol[4,5-*b*]pyridin-1-yloxy)-*N*-[(2*S*)-1,1,1-trifluorbutan-2-yl]-1-(2,4,6-trifluorphenyl)-1,4-dihydro-1,8-naphthyridin-3-carbonsäureamid mit 120 mg (861 µmol) (3*R*,4*R*)-Pyrrolidin-3,4-diol Hydrochlorid in Gegenwart von 437 µl (2.51 mmol) DIPEA in 7.25 ml DMF zur Reaktion gebracht. Die Reaktionslösung wurde dann auf 80 ml Wasser geben, mit 2 ml wässriger1M Salzsäure angesäuert, der Niederschlag abgesaugt und mit Wasser gewaschen. Der Rückstand wurde in 6 ml Acetonitril aufgenommen mittels präparativer HPLC (Acetonitril-Wasser mit Ameisensäure, C18 RP-HPLC) gereinigt. Die Produktfraktionen wurden vereinigt, im Vakuum eingeengt und der Rückstand über Nacht aus Acetonitril/Wasser lyophilisiert. Es wurden 296 mg (74% d. Th., 99% Reinheit) der Titelverbindung erhalten.
LC-MS (Methode 3): $R_t$ = 1.78 min; MS (ESIpos): m/z = 549 $[M+H]^+$.
$^1H$ NMR (400 MHz, DMSO-d6): δ [ppm] = 10.34 (d, 1H), 8.84 (s, 1H), 8.02 (d, 1H), 7.62-7.53 (m, 2H), 5.20 (br. s, 2H), 4.82-4.67 (m, 1H), 4.13-3.54 (br. m, 4H), 3.28-2.95 (m, 2H), 1.94-1.81 (m, 1H), 1.72-1.57 (m, 1H), 0.97 (t, 1H).

**Beispiel 7**

*N*-[(1*S*)-1-Cyclopropyl-2,2,2-trifluorethyl]-7-[(3*R*,4*R*)-3,4-dihydroxypyrrolidin-1-yl]-6-fluor-4-oxo-1-(2,4,6-trifluorphenyl)-1,4-dihydro-1,8-naphthyridin-3-carbonsäureamid

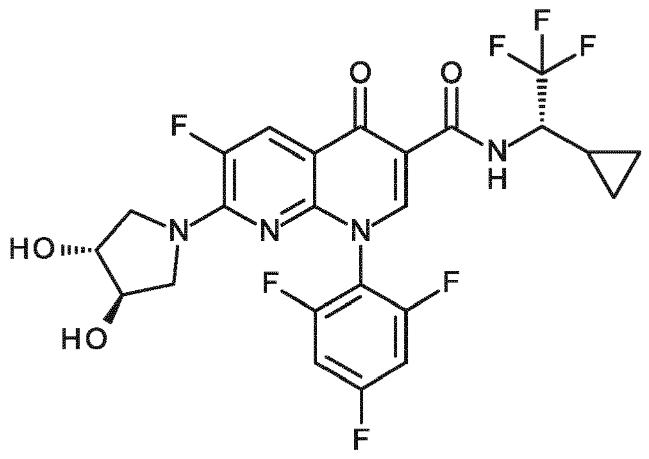

Gemäß AAV1 wurden 1.00 g (2.28 mmol) 7-[(3*R*,4*R*)-3,4-Dihydroxypyrrolidin-1-yl]-6-fluor-4-oxo-1-(2,4,6-trifluorphenyl)-1,4-dihydro-1,8-naphthyridin-3-carbonsäure mit 480 mg (2.73 mmol) (1*S*)-1-Cyclopropyl-2,2,2-trifluorethanamin Hydrochlorid in Gegenwart von 1.04 g (2.73 mmol) HATU und 991 µl (5.69 mmol) DIPEA in 23 ml DMF zur Reaktion gebracht. Es wurde anschließend mit wässriger 1M Salzsäure angesäuert und mit 200 ml Wasser und 100 ml Essigsäureethylester verdünnt. Die Phasen wurden getrennt und die wässrige Phase zweimal mit 60 ml Essigsäureethylester extrahiert. Die vereinigten organischen Phasen wurden mit 50 ml Puffer pH 7 sowie mit 50 ml gesättigter wässriger Natriumchlorid-Lösung gewaschen, über Magnesiumsulfat getrocknet, filtriert und im Vakuum eingeengt. Der Rückstand

wurde mittels Normalphasenchromatographie (Cyclohexan-Essigsäureethylester) gereinigt, die Fraktionen verreinigt, im Vakuum eingeengt und über Nacht aus Acetonitril/Wasser lyophilisiert. Es wurden 1.05 g (83% d. Th., 100% Reinheit) der Titelverbindung erhalten.
LC-MS (Methode 3): $R_t$ = 1.81 min; MS (ESIpos): m/z = 561 $[M+H]^+$.
$^1H$ NMR (400 MHz, DMSO-d6): $\delta$ [ppm] = 10.48 (d, 1H), 8.83 (s, 1H), 8.02 (d, 1H), 7.62-7.52 (m, 2H), 5.20 (br. s, 2H), 4.45-4.31 (m, 1H), 4.11-3.55 (br. m, 4H), 3.29-2.95 (m, 2H), 1.26-1.14 (m, 1H), 0.70-0.48 (m, 3H), 0.38-0.28 (m, 1H).

**Beispiel 8**

7-[(3*R*,4*R*)-3,4-Dihydroxypyrrolidin-1-yl]-6-fluor-4-oxo-*N*-[3,3,4,4,4-pentafluorobutan-2-yl]-1-(2,4,6-trifluorphenyl)-1,4-dihydro-1,8-naphthyridin-3-carbonsäureamid (Diastereomerengemisch)

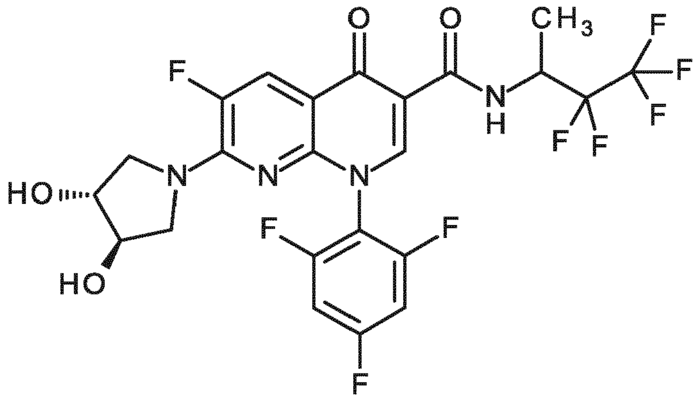

Gemäß AAV1 wurden 2.77 g (6.31 mmol) 7-[(3*R*,4*R*)-3,4-Dihydroxypyrrolidin-1-yl]-6-fluor-4-oxo-1-(2,4,6-trifluorphenyl)-1,4-dihydro-1,8-naphthyridin-3-carbonsäure mit 1.51 g (7.57 mmol) 3,3,4,4,4-Pentafluorbutan-2-amin Hydrochlorid (Racemat) in Gegenwart von 2.88 g (7.57 mmol) HATU und 3.84 ml (22.1 mmol) DIPEA in 30 ml DMF zur Reaktion gebracht. Die Reaktionslösung wurde anschließend in eine Mischung aus 3 ml wässriger 1M Salzsäure und 300 ml Eiswasser eingetropft. Der entstandene Niederschlag wurde abfiltriert, getrocknet und mittels Normalphasenchromatographie (Cyclohexan-Essigsäureethylester) gereinigt. Es wurden 2.40 g (65% d. Th., 100% Reinheit) der Titelverbindung erhalten.
LC-MS (Methode 3): $R_t$ = 1.84 min; MS (ESIpos): m/z = 585 $[M+H]^+$.
2.40 g der Titelverbindung (Diastereomerengemisch) wurde durch chirale HPLC in die Diastereomere getrennt (präparative SFC: Säule Daicel Chiralpak AD, 5 μm, 250x30 mm; Eluent: 85% Kohlendioxid, 15% iso-Propanol; Temperatur: 38°C; Fluss: 130 ml/min; Druck: 140 bar; UV-Detektion: 210 nm.)
Man erhielt (in der Reihenfolge der Elution von der Säule) 1.15 g Diastereomer 1 aus Beispiel 9 (99% de) $R_t$ = 3.23 min, 1.09 g Diastereomer 2 aus Beispiel 10 (94% de) $R_t$ = 4.79 min.
[Analytische HPLC: Säule Daicel Chiralpak AD-3, 3 μm, 100x4.6 mm; Eluent: 90% Kohlendioxid, 10% iso-Propanol; Temperatur: 60°C; Fluss: 3.0 ml/min; Druck: 130 bar; UV-Detektion: 220nm].
Diastereomer 1 wurde mittels Normalphasenchromatographie (Cyclohexan-Essigsäureethylester) nachgereinigt. Es wurden 903 mg (24% d. Th., 99% Reinheit) der Verbindung aus Beispiel 9 erhalten. Diastereomer 2 wurde mittels Normalphasenchromatographie (Cyclohexan-Essigsäureethylester) nachgereinigt. Es wurden 912 mg (25% d. Th., 99% Reinheit) der Verbindung aus Beispiel 10 erhalten.

**Beispiel 9**

7-[(3*R*,4*R*)-3,4-Dihydroxypyrrolidin-1-yl]-6-fluor-4-oxo-*N*-[3,3,4,4,4-pentafluorobutan-2-yl]-1-(2,4,6-trifluorphenyl)-1,4-dihydro-1,8-naphthyridin-3-carbonsäureamid (Diastereomer 1)

LC-MS (Methode 3): $R_t$ = 1.84 min; MS (ESIpos): m/z = 585 $[M+H]^+$.

$^1H$ NMR (400 MHz, DMSO-d6): $\delta$ [ppm] = 10.46 (d, 1H), 8.84 (s, 1H), 8.01 (d, 1H), 7.62-7.53 (m, 2H), 5.20 (br. s,

2H), 5.10-4.93 (m, 1H), 4.11-3.55 (br. m, 4H), 3.29-2.95 (m, 2H), 1.39 (d, 3H).

**Beispiel 10**

7-[(3*R*,4*R*)-3,4-Dihydroxypyrrolidin-1-yl]-6-fluor-4-oxo-*N*-[3,3,4,4,4-pentafluorobutan-2-yl]-1-(2,4,6-trifluophenyl)-1,4-dihydro-1,8-naphthyridin-3-carbonsäureamid (Diastereomer 2)

LC-MS (Methode 3): $R_t$ = 1.84 min; MS (ESIpos): m/z = 585 $[M+H]^+$.

$^1$H NMR (400 MHz, DMSO-d6): δ [ppm] = 10.47 (d, 1H), 8.84 (s, 1H), 8.01 (d, 1H), 7.62-7.54 (m, 2H), 5.20 (br. s, 2H), 5.10-4.93 (m, 1H), 4.11-3.57 (br. m, 4H), 3.29-2.96 (m, 2H), 1.39 (d, 3H).

Die folgenden Ausführungsbeispiele wurden analog zu Beispiel 8 gemäß AAV1 hergestellt:

| Beispiel | IUPAC-Name Struktur LC-MS (Methode): Retentionszeit; detektierte Masse $^1$H-NMR eingesetztes Amin (Ausbeute, Reinheit) |
|---|---|
| 11 | 7-[(3R,4R)-3,4-Dihydroxypyrrolidin-1-yl]-6-fluor-4-oxo-N-[(2S)-1,1,1-trifluor-4-methylpentan-2-yl]-1-(2,4,6-trifluorphenyl)-1,4-dihydro-1,8-naphthyridin-3-carboxamid 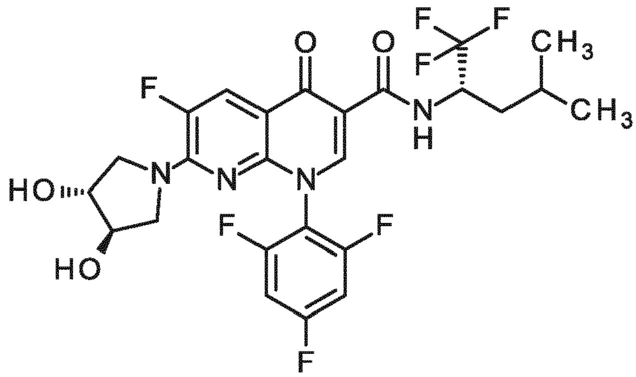 LC-MS (Methode 1): $R_t$ = 1.07 min; MS (ESIpos): m/z = 577 $[M+H]^+$ $^1$H-NMR (400 MHz, DMSO-d6) δ [ppm]: 0.008 (1.63), 0.882 (15.82), 0.898 (15.97), 0.940 (15.69), 0.956 (16.00), 1.527 (0.65), 1.562 (2.61), 1.590 (2.10), 1.640 (2.07), 1.650 (3.07), 1.676 (3.98), 1.703 (1.66), 2.328 (0.72), 2.366 (0.49), 2.524 (2.17), 2.670 (0.75), 2.710 (0.47), 3.070 (0.79), 3.696 (0.83), 3.904 (1.68), 4.017 (1.18), 4.815 (1.32), 4.838 (1.35), 4.857 (0.78), 5.201 (2.98), 7.554 (2.23), 7.558 (2.44), 7.575 (4.26), 7.580 (4.31), 7.597 (2.46), 7.993 (6.91), 8.025 (6.83), 8.847 (12.23), 10.316 (4.89), 10.340 (4.71). (2S)-1,1,1-Trifluor-4-methylpentan-2-aminhydrochlorid (75% d.Th., 99% Reinheit) |
| 12 | N-(Bicyclo[1.1.1]pent-1-yl)-7-[(3R,4R)-3,4-dihydroxypyrrolidin-1-yl]-6-fluor-4-oxo-1-(2,4,6-trifluorphenyl)-1,4-dihydro-1,8-naphthyridin-3-carboxamid 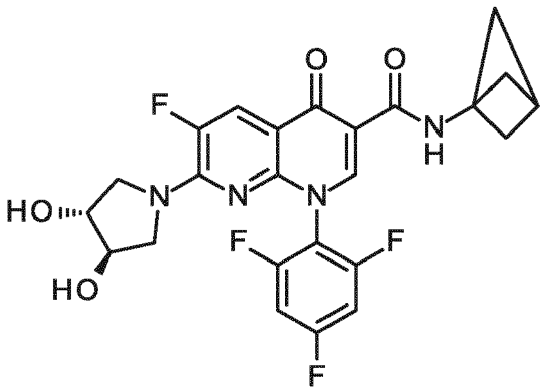 |

(fortgesetzt)

| Beispiel | IUPAC-Name Struktur LC-MS (Methode): Retentionszeit; detektierte Masse $^1$H-NMR eingesetztes Amin (Ausbeute, Reinheit) |
|---|---|
| | LC-MS (Methode 1): $R_t$ = 0.91 min; MS (ESIpos): m/z = 505 [M+H]$^+$ $^1$H-NMR (400 MHz, DMSO-d6) δ [ppm]: 2.074 (0.98), 2.094 (16.00), 2.477 (2.52), 2.519 (0.42), 5.188 (0.90), 7.557 (0.60), 7.579 (1.03), 7.599 (0.60), 7.949 (1.39), 7.981 (1.36), 8.696 (2.25), 10.195 (1.71). Bicyclo[1.1.1]pentan-1-aminhydrochlorid (69% d. Th., 100% Reinheit) |
| 13 | 7-[(3R,4R)-3,4-Dihydroxypyrrolidin-1-yl]-6-fluor-4-oxo-N-(4,4,4-trifluor-2-methylbutan-2-yl)-1-(2,4,6-trifluorphenyl)-1,4-dihydro-1,8-naphthyridin-3-carboxamid 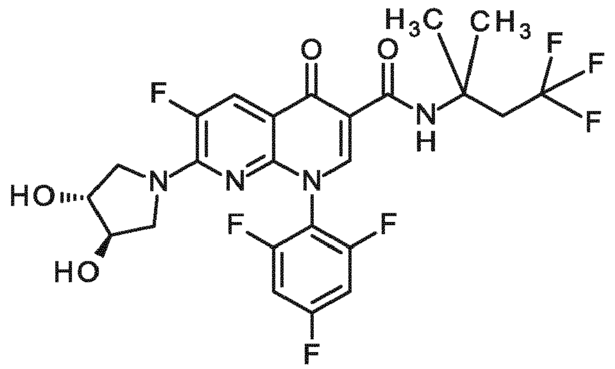 LC-MS (Methode 3): $R_t$ = 1.8 min; MS (ESIpos): m/z = 563 [M+H]$^+$ $^1$H-NMR (400 MHz, DMSO-d6) δ [ppm]: 1.480 (16.00), 2.902 (0.72), 2.932 (2.07), 2.963 (2.01), 2.992 (0.69), 3.908 (0.59), 5.192 (1.67), 7.552 (1.20), 7.573 (2.17), 7.595 (1.21), 7.980 (2.69), 8.012 (2.63), 8.724 (4.96), 10.086 (3.27). 4,4,4-Trifluor-2-methylbutan-2-aminhydrochlorid (92% d. Th., 100% Reinheit) |
| 14 | 7-[(3R,4R)-3,4-Dihydroxypyrrolidin-1-yl]-6-fluor-4-oxo-N-[(2R)-1,1,1-trifluor-4-methylpentan-2-yl]-1-(2,4,6-trifluorphenyl)-1,4-dihydro-1,8-naphthyridin-3-carboxamid 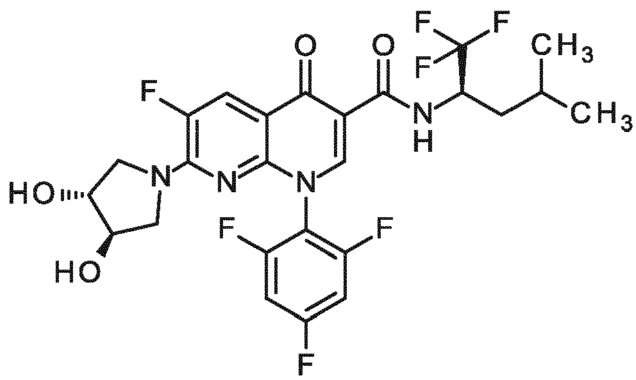 LC-MS (Methode 1): $R_t$ = 1.07 min; MS (ESIpos): m/z = 577 [M+H]$^+$ $^1$H-NMR (400 MHz, DMSO-d6) δ [ppm]: -0.008 (2.52), 0.008 (2.37), 0.882 (15.72), 0.898 (15.79), 0.940 (15.66), 0.956 (16.00), 1.528 (0.63), 1.534 (0.59), 1.555 (1.28), 1.563 (2.58), 1.571 (1.29), 1.591 (2.06), 1.640 (1.99), 1.650 (2.98), 1.676 (3.84), 1.704 (1.62), 1.713 (1.08), 2.329 (0.42), 2.524 (1.33), 2.671 (0.45), 3.070 (0.76), 3.694 (0.77), 3.912 (1.65), 4.018 (1.13), 4.816 (1.26), 4.839 (1.29), 4.858 (0.73), 5.201 (4.77), 7.556 (3.83), 7.578 (6.91), 7.599 (3.80), 7.994 (7.11), 8.026 (6.94), 8.848 (12.19), 10.318 (4.80), 10.342 (4.57). (2R)-1,1,1-Trifluor-4-methylpentan-2-aminhydrochlorid (73% d. Th., 97% Reinheit) |
| 15 | 7-[(3R,4R)-3,4-Dihydroxypyrrolidin-1-yl]-6-fluor-N-[(2R)-3-methylbutan-2-yl]-4-oxo-1-(2,4,6-trifluorphenyl)-1,4-dihydro-1,8-naphthyridin-3-carboxamid |

(fortgesetzt)

| Beispiel | IUPAC-Name Struktur LC-MS (Methode): Retentionszeit; detektierte Masse $^{1}$H-NMR eingesetztes Amin (Ausbeute, Reinheit) |
|---|---|
| | 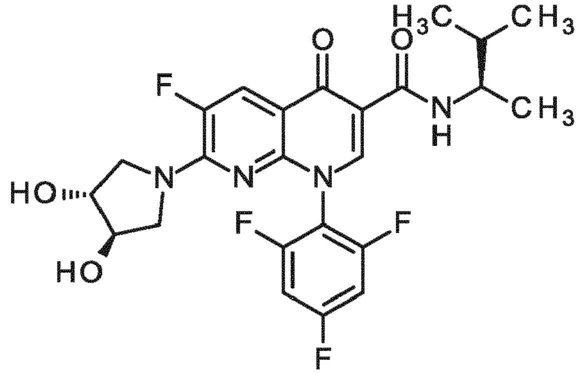<br>LC-MS (Methode 3): $R_t$ = 1.67 min; MS (ESIpos): m/z = 509 $[M+H]^+$ $^{1}$H-NMR (400 MHz, DMSO-d6) δ [ppm]: -0.008 (2.45), 0.008 (1.92), 0.900 (14.25), 0.918 (16.00), 0.924 (14.85), 0.941 (14.39), 1.098 (14.84), 1.115 (14.90), 1.731 (0.46), 1.747 (1.23), 1.764 (1.75), 1.778 (1.69), 1.795 (1.13), 1.811 (0.41), 2.328 (0.43), 2.519 (1.74), 2.524 (1.27), 2.671 (0.43), 3.070 (0.46), 3.269 (0.67), 3.276 (0.53), 3.680 (0.48), 3.887 (2.02), 3.903 (2.66), 3.908 (2.44), 3.921 (2.64), 3.938 (2.04), 3.954 (1.12), 5.191 (3.01), 7.550 (2.38), 7.571 (4.17), 7.592 (2.37), 7.992 (5.69), 8.024 (5.64), 8.711 (9.43), 9.868 (3.38), 9.890 (3.30). (2R)-3-Methylbutan-2-amin (75 % d. Th., 99% Reinheit) |
| 16 | 7-[(3R,4R)-3,4-Dihydroxypyrrolidin-1-yl]-6-fluor-N-[(2S)-3-methylbutan-2-yl]-4-oxo-1-(2,4,6-trifluorphenyl)-1,4-dihydro-1,8-naphthyridin-3-carboxamid<br>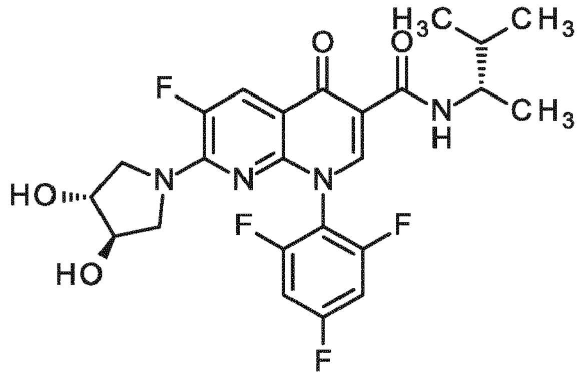<br>LC-MS (Methode 3): $R_t$ = 1.67 min; MS (ESIpos): m/z = 509 $[M+H]^+$ $^{1}$H-NMR (400 MHz, DMSO-d6) δ [ppm]: 0.008 (1.40), 0.901 (14.31), 0.918 (16.00), 0.925 (14.98), 0.942 (14.43), 1.099 (14.84), 1.116 (14.86), 1.731 (0.49), 1.748 (1.30), 1.765 (1.85), 1.778 (1.73), 1.795 (1.14), 3.063 (0.51), 3.680 (0.53), 3.888 (2.11), 3.904 (2.84), 3.922 (2.79), 3.939 (2.15), 5.194 (4.39), 7.550 (2.81), 7.572 (4.99), 7.593 (2.71), 7.994 (6.12), 8.026 (6.00), 8.713 (11.30), 9.870 (3.57), 9.892 (3.45). (2S)-3-Methylbutan-2-amin (77 % d. Th., 100% Reinheit) |
| 17 | 7-[(3R,4R)-3,4-Dihydroxypyrrolidin-1-yl]-6-fluor-N-[(2S)-1-methoxy-3-methylbutan-2-yl]-4-oxo-1-(2,4,6-trifluorphenyl)-1,4-dihydro-1,8-naphthyridin-3-carboxamid |

(fortgesetzt)

| Beispiel | IUPAC-Name Struktur LC-MS (Methode): Retentionszeit; detektierte Masse $^1$H-NMR eingesetztes Amin (Ausbeute, Reinheit) |
|---|---|
| | 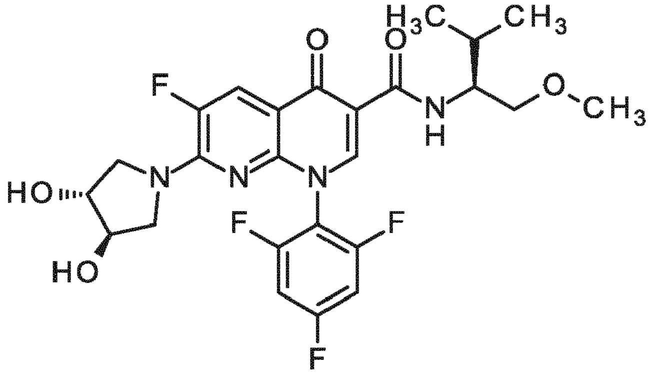 LC-MS (Methode 3): $R_t$ = 1.58 min; MS (ESIpos): m/z = 539 $[M+H]^+$ $^1$H-NMR (400 MHz, DMSO-d6) δ [ppm]: -0.008 (1.71), 0.008 (1.47), 0.912 (10.53), 0.929 (10.84), 1.903 (0.49), 1.920 (0.75), 1.936 (0.74), 1.953 (0.46), 3.269 (16.00), 3.352 (0.72), 3.365 (0.83), 3.377 (1.17), 3.390 (1.12), 3.439 (1.10), 3.453 (1.22), 3.464 (0.76), 3.477 (0.70), 3.919 (0.46), 3.965 (0.52), 3.980 (0.91), 3.994 (0.99), 4.002 (1.00), 4.017 (0.87), 5.192 (1.07), 7.553 (0.86), 7.573 (1.56), 7.594 (0.86), 8.000 (2.37), 8.031 (2.31), 8.723 (4.24), 9.926 (1.34), 9.949 (1.29). (2S)-1-Methoxy-3-methylbutan-2-aminhydrochlorid (87 % d. Th., 99% Reinheit) |
| 18 | 7-[(3R,4R)-3,4-Dihydroxypyrrolidin-1-yl]-6-fluor-4-oxo-1-(2,4,6-trifluorphenyl)-N-[(2S)-1,1,1-trifluopropan-2-yl]-1,4-dihydro-1,8-naphthyridin-3-carboxamid 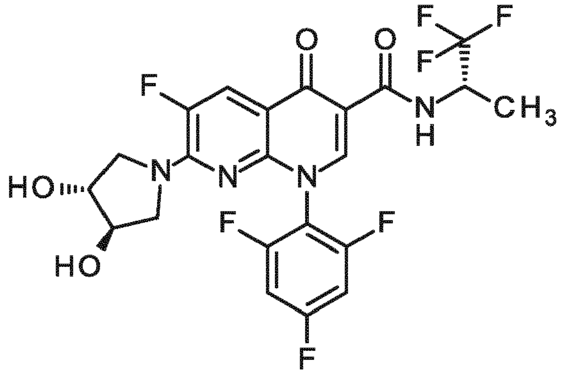 LC-MS (Methode 1): $R_t$ = 0.89 min; MS (ESIpos): m/z = 535 $[M+H]^+$ $^1$H-NMR (400 MHz, DMSO-d6) δ [ppm]: -0.008 (3.40), 1.366 (15.93), 1.383 (16.00), 2.328 (0.75), 2.367 (0.46), 2.670 (0.72), 2.710 (0.46), 3.065 (0.82), 3.692 (0.82), 3.906 (1.71), 4.011 (1.21), 4.842 (0.45), 4.861 (1.18), 4.882 (1.82), 4.902 (1.87), 4.920 (1.20), 5.199 (4.80), 7.555 (2.86), 7.577 (5.42), 7.598 (2.83), 7.990 (8.04), 8.022 (7.89), 8.837 (14.74), 10.383 (5.19), 10.406 (4.90). (2S)-1,1,1-Trifluorpropan-2-amin (77 % d. Th., 99% Reinheit) |
| 19 | N-[(R)-1-Cyclopropylethyl]-7-[(3R,4R)-3,4-dihydroxypyrrolidin-1-yl]-6-fluor-4-oxo-1-(2,4,6-trifluorphenyl)-1,4-dihydro-1,8-naphthyridin-3-carboxamid 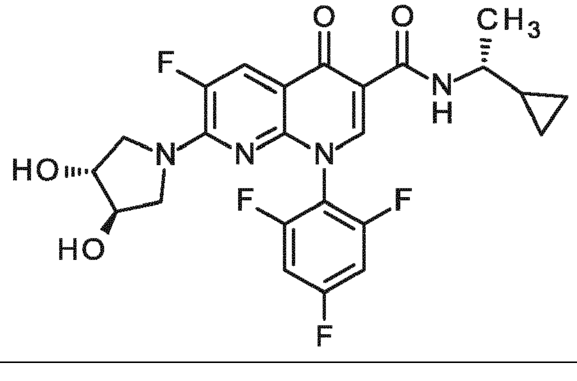 |

(fortgesetzt)

| Beispiel | IUPAC-Name Struktur LC-MS (Methode): Retentionszeit; detektierte Masse $^1$H-NMR eingesetztes Amin (Ausbeute, Reinheit) |
|---|---|
| | LC-MS (Methode 3): $R_t$ = 1.63 min; MS (ESIpos): m/z = 507 [M+H]$^+$ $^1$H-NMR (400 MHz, DMSO-d6) δ [ppm]: -0.150 (0.68), -0.008 (6.67), 0.008 (5.02), 0.146 (0.68), 0.218 (1.10), 0.229 (1.68), 0.241 (2.46), 0.249 (2.36), 0.261 (1.43), 0.266 (1.39), 0.278 (2.30), 0.287 (2.75), 0.299 (1.94), 0.310 (1.26), 0.322 (0.55), 0.394 (0.65), 0.402 (0.65), 0.414 (1.85), 0.425 (2.27), 0.435 (2.59), 0.447 (3.11), 0.461 (2.75), 0.469 (2.01), 0.482 (1.59), 0.491 (0.62), 0.940 (0.45), 0.952 (0.87), 0.960 (1.30), 0.972 (2.27), 0.980 (1.46), 0.992 (2.14), 1.004 (1.10), 1.012 (0.74), 1.215 (15.87), 1.231 (16.00), 2.327 (1.13), 2.366 (1.00), 2.523 (3.85), 2.670 (1.23), 2.710 (1.10), 3.064 (0.55), 3.482 (0.42), 3.498 (1.39), 3.518 (2.56), 3.535 (2.49), 3.553 (1.33), 3.571 (0.49), 3.679 (0.62), 3.917 (1.39), 5.189 (4.15), 7.546 (3.17), 7.568 (5.73), 7.589 (3.21), 7.975 (6.25), 8.007 (6.19), 8.708 (10.85), 9.864 (4.05), 9.884 (3.92). (1R)-1-Cyclopropylethanamin (76 % d. Th., 100% Reinheit) |
| 20 | N-[(1S)-1-Cyclopropylethyl]-7-[(3R,4R)-3,4-dihydroxypyrrolidin-1-yl]-6-fluor-4-oxo-1-(2,4,6-trifluorphenyl)-1,4-dihydro-1,8-naphthyridin-3-carboxamid 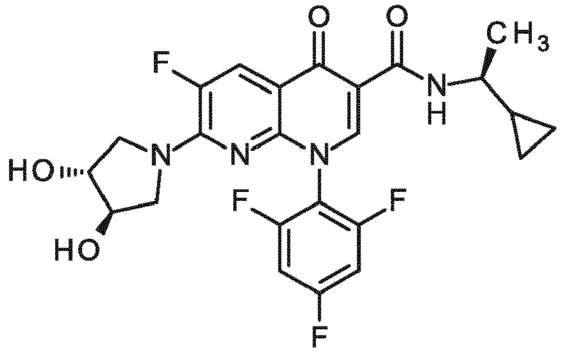 LC-MS (Methode 3): $R_t$ = 1.62 min; MS (ESIpos): m/z = 507 [M+H]$^+$ $^1$H-NMR (400 MHz, DMSO-d6) δ [ppm]: -0.149 (0.42), -0.008 (4.00), 0.008 (2.96), 0.146 (0.42), 0.207 (0.46), 0.218 (1.12), 0.229 (1.67), 0.241 (2.46), 0.249 (2.39), 0.261 (1.42), 0.266 (1.39), 0.278 (2.32), 0.287 (2.75), 0.299 (1.93), 0.310 (1.19), 0.321 (0.56), 0.394 (0.60), 0.402 (0.67), 0.414 (1.84), 0.425 (2.23), 0.435 (2.56), 0.448 (3.05), 0.456 (1.96), 0.461 (2.72), 0.470 (1.98), 0.482 (1.60), 0.491 (0.60), 0.502 (0.44), 0.940 (0.44), 0.952 (0.88), 0.960 (1.26), 0.972 (2.25), 0.980 (1.44), 0.984 (1.40), 0.992 (2.16), 1.005 (1.09), 1.012 (0.74), 1.215 (15.91), 1.232 (16.00), 2.328 (0.61), 2.367 (0.60), 2.524 (2.23), 2.670 (0.65), 2.710 (0.58), 3.073 (0.54), 3.484 (0.40), 3.501 (1.35), 3.520 (2.49), 3.537 (2.46), 3.556 (1.30), 3.573 (0.44), 3.673 (0.56), 3.909 (1.32), 5.190 (4.47), 7.547 (2.51), 7.568 (4.49), 7.589 (2.51), 7.976 (6.25), 8.008 (6.18), 8.709 (10.70), 9.864 (4.05), 9.884 (3.89). (1S)-1-Cyclopropylethanamin (77 % d. Th., 100% Reinheit) |
| 21 | N-(Dicyclopropylmethyl)-7-[(3R,4R)-3,4-dihydroxypyrrolidin-1-yl]-6-fluor-4-oxo-1-(2,4,6-trifluorphenyl)-1,4-dihydro-1,8-naphthyridin-3-carboxamid 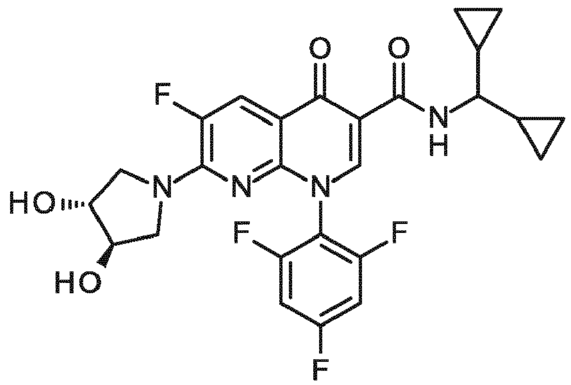 |

(fortgesetzt)

| Beispiel | IUPAC-Name Struktur LC-MS (Methode): Retentionszeit; detektierte Masse $^1$H-NMR eingesetztes Amin (Ausbeute, Reinheit) |
|---|---|
| | LC-MS (Methode 1): $R_t$ = 0.94 min; MS (ESIpos): m/z = 533 [M+H]$^+$ $^1$H-NMR (400 MHz, DMSO-d6) δ [ppm]: -0.149 (1.11), -0.008 (9.56), 0.008 (7.59), 0.146 (1.11), 0.299 (15.57), 0.311 (15.27), 0.322 (4.14), 0.370 (2.56), 0.393 (6.91), 0.415 (5.85), 0.452 (4.99), 0.472 (6.31), 0.498 (1.92), 1.016 (2.82), 1.029 (5.25), 1.036 (3.41), 1.049 (4.99), 1.061 (2.60), 2.328 (2.22), 2.367 (1.11), 2.670 (2.13), 2.710 (1.07), 3.221 (2.22), 3.239 (4.44), 3.261 (4.74), 3.280 (2.86), 3.903 (1.79), 5.189 (5.16), 7.545 (3.50), 7.568 (6.27), 7.588 (3.58), 7.988 (9.09), 8.020 (8.75), 8.709 (16.00), 9.892 (5.03), 9.914 (4.82). 1,1-Dicyclopropylmethanamin (61% d. Th., 99% Reinheit) |
| 22 | N-(1,1-Difluor-2-methylpropan-2-yl)-7-[(3R,4R)-3,4-dihydroxypyrrolidin-1-yl]-6-fluor-4-oxo-1-(2,4,6-trifluorphenyl)-1,4-dihydro-1,8-naphthyridin-3-carboxamid 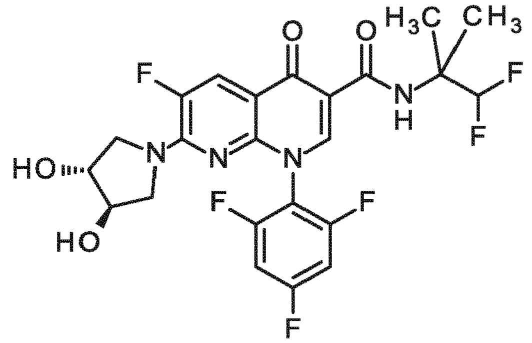 LC-MS (Methode 1): $R_t$ = 0.93 min; MS (ESIpos): m/z = 531 [M+H]$^+$ $^1$H-NMR (400 MHz, DMSO-d6) δ [ppm]: -0.008 (1.57), 0.008 (1.50), 1.434 (16.00), 2.073 (0.74), 2.328 (0.48), 2.670 (0.52), 3.910 (0.55), 5.192 (1.49), 6.277 (0.88), 6.420 (1.62), 6.562 (0.73), 7.554 (1.10), 7.577 (1.94), 7.597 (1.10), 7.987 (2.44), 8.019 (2.41), 8.750 (4.28), 10.232 (3.14). 1,1-Difluor-2-methylpropan-2-aminhydrochlorid (57 % d. Th., 100% Reinheit) |
| 23 | 7-[(3R,4R)-3,4-Dihydroxypyrrolidin-1-yl]-6-fluor-4-oxo-N-(1,1,1-trifluor-2-methylpropan-2-yl)-1-(2,4,6-trifluophenyl)-1,4-dihydro-1,8-naphthyridin-3-carboxamid 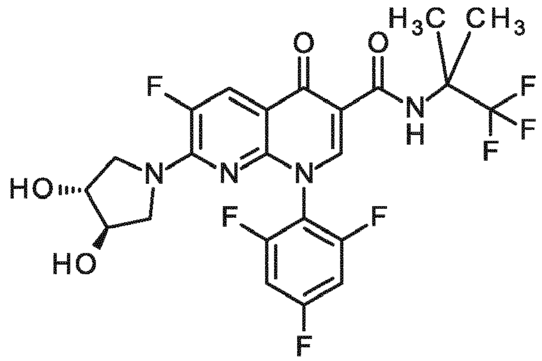 LC-MS (Methode 3): $R_t$ = 1.77 min; MS (ESIpos): m/z = 549 [M+H]$^+$ $^1$H-NMR (400 MHz, DMSO-d6) δ [ppm]: -0.008 (0.89), 0.008 (0.56), 1.633 (16.00), 2.520 (0.82), 2.524 (0.74), 3.908 (0.50), 5.194 (1.18), 7.557 (0.97), 7.579 (1.58), 7.600 (0.89), 8.008 (2.21), 8.040 (2.14), 8.775 (3.54), 10.561 (2.95). 1,1,1 -Trifluor-2-methylpropan-2-aminhydrochlorid (63% d. Th., 100% Reinheit) |
| 24 | 7-[(3R,4R)-3,4-Dihydroxypyrrolidin-1-yl]-N-(2,4-dimethylpentan-3-yl)-6-fluor-4-oxo-1-(2,4,6-trifluorphenyl)-1,4-dihydro-1,8-naphthyridin-3-carboxamid |

(fortgesetzt)

| Beispiel | IUPAC-Name Struktur LC-MS (Methode): Retentionszeit; detektierte Masse $^1$H-NMR eingesetztes Amin (Ausbeute, Reinheit) |
|---|---|
| | 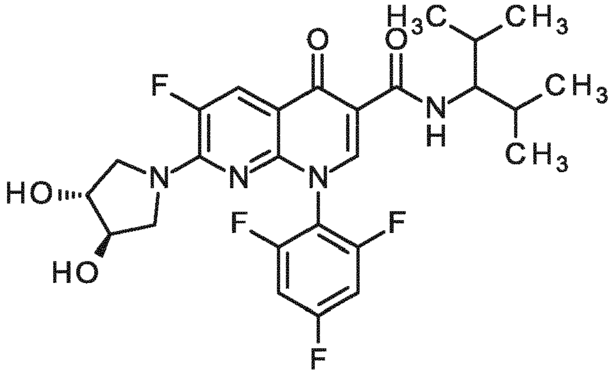 LC-MS (Methode 3): $R_t$ = 1.87 min; MS (ESIpos): m/z = 537 $[M+H]^+$ $^1$H-NMR (400 MHz, DMSO-d6) δ [ppm]: -0.008 (0.92), 0.008 (0.71), 0.865 (13.39), 0.877 (15.46), 0.881 (16.00), 0.893 (13.11), 1.810 (0.43), 1.827 (1.26), 1.844 (2.06), 1.860 (2.00), 1.877 (1.16), 2.524 (0.55), 3.640 (0.71), 3.656 (1.27), 3.666 (0.90), 3.672 (0.89), 3.681 (1.31), 3.697 (0.75), 3.911 (0.57), 5.198 (1.63), 7.550 (1.24), 7.572 (2.16), 7.592 (1.23), 8.013 (3.06), 8.045 (2.98), 8.727 (5.38), 9.761 (1.66), 9.786 (1.59). 2,4-Dimethylpentan-3-amin (57% d. Th., 100% Reinheit) |
| 25 | N-(2-Cyclopropylpropan-2-yl)-7-[(3R,4R)-3,4-dihydroxypyrrolidin-1-yl]-6-fluor-4-oxo-1-(2,4,6-trifluorphenyl)-1,4-dihydro-1,8-naphthyridin-3-carboxamid 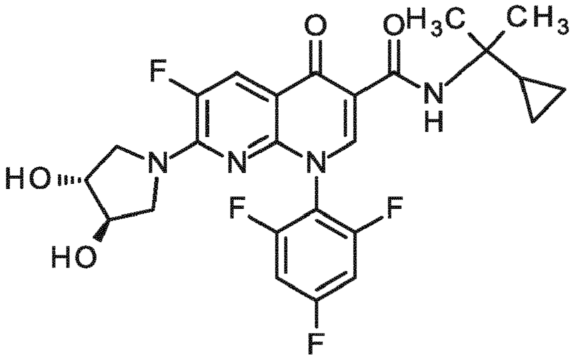 LC-MS (Methode 3): $R_t$ = 1.79 min; MS (ESIpos): m/z = 521 $[M+H]^+$ $^1$H-NMR (400 MHz, DMSO-d6) δ [ppm]: 0.397 (5.62), 0.414 (4.00), 1.290 (0.69), 1.311 (16.00), 1.325 (0.79), 5.187 (1.10), 7.550 (0.76), 7.572 (1.33), 7.593 (0.73), 7.993 (1.77), 8.024 (1.73), 8.680 (3.16), 9.863 (2.07). 2-Cyclopropylpropan-2-amin (95 % d. Th., 100% Reinheit) |
| 26 | 7-[(3R,4R)-3,4-Dihydroxypyrrolidin-1-yl]-6-fluor-4-oxo-N-[(2)-1-(trifluormethoxy)butan-2-yl]-1-(2,4,6-trifluorphenyl)-1,4-dihydro-1,8-naphthyridin-3-carboxamid (Diastereomerengemisch) 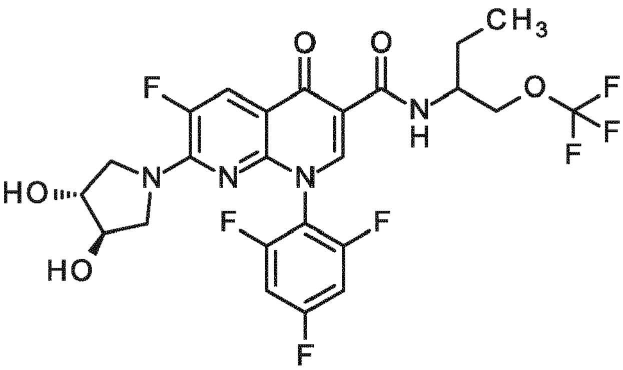 |

(fortgesetzt)

| Beispiel | IUPAC-Name Struktur LC-MS (Methode): Retentionszeit; detektierte Masse $^1$H-NMR eingesetztes Amin (Ausbeute, Reinheit) |
|---|---|
| | LC-MS (Methode 3): $R_t$ = 1.78 min; MS (ESIpos): m/z = 579 $[M+H]^+$ $^1$H-NMR (400 MHz, DMSO-d6) δ [ppm]: -0.008 (2.02), 0.008 (1.73), 0.922 (6.79), 0.941 (16.00), 0.959 (7.44), 1.550 (0.56), 1.568 (0.97), 1.585 (1.26), 1.603 (1.40), 1.622 (0.89), 1.633 (0.55), 1.651 (1.04), 1.663 (1.19), 1.669 (1.15), 1.682 (1.27), 1.698 (0.74), 1.716 (0.46), 2.074 (1.63), 2.328 (0.45), 2.524 (1.32), 2.671 (0.43), 3.069 (0.51), 3.685 (0.52), 3.911 (1.21), 4.148 (2.09), 4.162 (2.31), 4.176 (3.98), 4.183 (4.11), 4.194 (4.10), 4.211 (2.53), 5.193 (3.51), 7.552 (2.57), 7.574 (4.50), 7.595 (2.53), 7.995 (6.24), 8.026 (6.06), 8.762 (10.71), 9.985 (2.62), 10.005 (2.44). 1-(Trifluormethoxy)butan-2-aminhydrochlorid (Racemat) (54% d. Th., 100% Reinheit) |
| 27 | 7-[(3R,4R)-3,4-Dihydroxypyrrolidin-1-yl]-6-fluor-4-oxo-N-[(3)-1,1,1,2,2-pentafluorpentan-3-yl]-1-(2,4,6-trifluorphenyl)-1,4-dihydro-1,8-naphthyridin-3-carboxamid (Diastereomerengemisch) 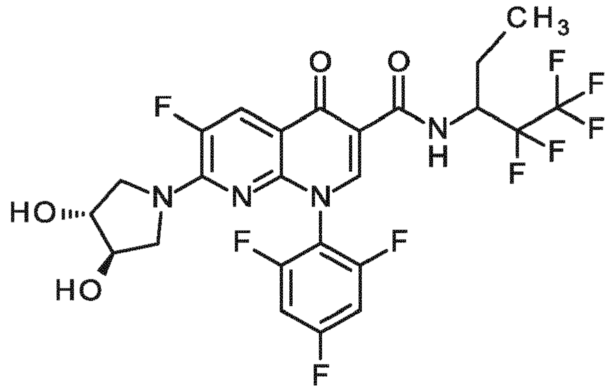 LC-MS (Methode 3): $R_t$ = 1.93 min; MS (ESIpos): m/z = 599 $[M+H]^+$ $^1$H-NMR (400 MHz, DMSO-d6) δ [ppm]: 0.944 (7.18), 0.962 (16.00), 0.981 (7.74), 1.619 (0.90), 1.638 (1.31), 1.654 (1.50), 1.663 (1.39), 1.673 (1.34), 1.681 (1.41), 1.699 (1.00), 1.922 (1.31), 2.329 (0.58), 2.672 (0.66), 3.079 (0.81), 3.693 (0.86), 3.905 (1.75), 4.012 (1.22), 4.852 (1.12), 4.879 (1.06), 5.208 (3.80), 7.557 (3.27), 7.579 (5.89), 7.599 (3.25), 8.005 (7.21), 8.037 (7.04), 8.850 (14.56), 10.377 (4.69), 10.402 (4.39). 1,1,1,2,2-Pentafluorpentan-3-aminhydrochlorid (Racemat) (85% d. Th., 99% Reinheit) |
| 28 | 7-[(3R,4R)-3,4-Dihydroxypyrrolidin-1-yl]-6-fluor-N-(2-methylpentan-3-yl)-4-oxo-1-(2,4,6-trifluorphenyl)-1,4-dihydro-1,8-naphthyridin-3-carboxamid (Diastereomerengemisch) - 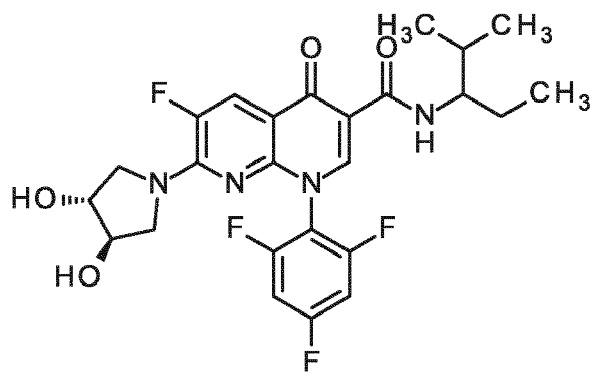 LC-MS (Methode 3): $R_t$ = 1.80 min; MS (ESIpos): m/z = 523 $[M+H]^+$ $^1$H-NMR (400 MHz, DMSO-d6) δ [ppm]: 0.008 (1.53), 0.851 (4.76), 0.869 (11.65), 0.878 (11.15), 0.888 (7.46), 0.896 (16.00), 0.915 (10.32), 1.380 (0.55), 1.397 (0.79), 1.414 (1.00), 1.436 (1.06), 1.454 (0.71), 1.538 (0.78), 1.550 (0.92), 1.568 (1.02), 1.584 (0.72), 1.602 (0.49), 1.794 (0.89), 1.810 (1.24), 1.824 (1.23), 1.840 (0.80), 2.328 (0.45), 3.680 (0.42), 3.764 (0.53), 3.777 (0.93), 3.788 (1.29), 3.799 (1.63), 3.811 (1.29), 3.822 (0.87), 3.834 (0.59), 3.907 (0.94), 5.199 (2.67), 7.551 (2.01), 7.573 (3.53), 7.594 (1.96), 7.999 (4.29), 8.031 (4.21), 8.716 (8.19), 9.768 (2.51), 9.792 (2.39). 2-Methylpentan-3-aminhydrochlorid (Racemat) (31% d. Th., 100% Reinheit) |

**Beispiel 29**

7-[(3R,4R)-3,4-Dihydroxypyrrolidin-1-yl]-6-fluor-N-(2-methylpentan-3-yl)-4-oxo-1-(2,4,6-trifluorphenyl)-1,4-dihydro-1,8-naphthyridin-3-carboxamid (Diastereomer 1)

37 mg 7-[(3R,4R)-3,4-Dihydroxypyrrolidin-1-yl]-6-fluor-N-(2-methylpentan-3-yl)-4-oxo-1-(2,4,6-trifluorphenyl)-1,4-dihydro-1,8-naphthyridin-3-carboxamid (Diastereomerengemisch) wurde durch chirale HPLC in die Diastereomere getrennt (präparative HPLC: Säule Daicel® Chiralpak OX-H, 5 μm, 250 x 20 mm; Eluent: 80% n-Heptan / 20% Isopropanol; Fluss 15 ml/min; Temperatur: 35°C, Detektion: 265 nm).
Diastereomer 1: 13 mg (>99% de)
$R_t$ = 6.27 min [analytische HPLC: Säule Daicel® Chiralpak OX-H, 1ml/min; 5 μm, 250 x 4.6 mm; Eluent: 75% iso-Hexan/ 25% Isopropanol + 0.2 % DEA; Detektion: 265 nm].
LC-MS (Methode 3): $R_t$ = 1.80 min; MS (ESIpos): m/z = 523 $[M+H]^+$

**Beispiel 30**

7-[(3R,4R)-3,4-Dihydroxypyrrolidin-1-yl]-6-fluor-N-(2-methylpentan-3-yl)-4-oxo-1-(2,4,6-trifluophenyl)-1,4-dihydro-1,8-naphthyridin-3-carboxamid (Diastereomer 2)

37 mg 7-[(3R,4R)-3,4-Dihydroxypyrrolidin-1-yl]-6-fluor-N-(2-methylpentan-3-yl)-4-oxo-1-(2,4,6-trifluophenyl)-1,4-dihydro-1,8-naphthyridin-3-carboxamid (Diastereomerengemisch) wurde durch chirale HPLC in die Diastereomere getrennt (präparative HPLC: Säule Daicel® Chiralpak OX-H, 5 μm, 250 x 20 mm; Eluent: 80% n-Heptan / 20% Isopropanol; Fluss 15 ml/min; Temperatur: 35°C, Detektion: 265 nm).
Diastereomer 2: 13 mg (>99% de)
$R_t$ = 7.35 min [analytische HPLC: Säule Daicel® Chiralpak OX-H, 1ml/min; 5 μm, 250 x 4.6 mm; Eluent: 75% iso-Hexan/ 25% Isopropanol + 0.2 % DEA; Detektion: 265 nm].
LC-MS (Methode 3): $R_t$ = 1.80 min; MS (ESIpos): m/z = 523 $[M+H]^+$

**Beispiel 31**

7-[(3R,4R)-3,4-Dihydroxypyrrolidin-1-yl]-6-fluor-4-oxo-N-[(2)-1-(trifluormethoxy)butan-2-yl]-1-(2,4,6-trifluorphenyl)-1,4-dihydro-1,8-naphthyridin-3-carboxamid (Diastereomer 1)

218 mg 7-[(3R,4R)-3,4-Dihydroxypyrrolidin-1-yl]-6-fluor-4-oxo-N-[(2)-1-(trifluormethoxy)butan-2-yl]-1-(2,4,6-trifluorphenyl)-1,4-dihydro-1,8-naphthyridin-3-carboxamid (Diastereomerengemisch) wurde durch chirale HPLC in die Diastereomere getrennt (präparative HPLC: Säule Daicel® Chiralpak ID, 5 μm, 250 x 20 mm; Eluent: 85% n-Heptan / 15% Isopropanol; Fluss 15 ml/min; Temperatur: 30°C, Detektion: 220 nm).
Diastereomer 1: 63.7 mg (99% de)
$R_t$ = 5.50 min [analytischeHPLC:Säule Daicel® Chiralpak ID, 1ml/min; 5 μm, 250 x 4.6 mm; Eluent: 80% iso-Hexan/ 20% Propanol; Detektion: 220 nm].
LC-MS (Methode 3): $R_t$ = 1.78 min; MS (ESIpos): m/z = 579 $[M+H]^+$

**Beispiel 32**

7-[(3R,4R)-3,4-Dihydroxypyrrolidin-1-yl]-6-fluor-4-oxo-N-[(2)-1-(trifluormethoxy)butan-2-yl]-1-(2,4,6-trifluorphenyl)-1,4-dihydro-1,8-naphthyridin-3-carboxamid (Diastereomer 2)

218 mg 7-[(3R,4R)-3,4-Dihydroxypyrrolidin-1-yl]-6-fluor-4-oxo-N-[(2)-1-(trifluormethoxy)butan-2-yl]-1-(2,4,6-trifluorphenyl)-1,4-dihydro-1,8-naphthyridin-3-carboxamid (Diastereomerengemisch) wurde durch chirale HPLC in die Diastereomere getrennt (präparative HPLC: Säule Daicel® Chiralpak ID, 5 μm, 250 x 20 mm; Eluent: 85% n-Heptan / 15% Isopropanol; Fluss 15 ml/min; Temperatur: 30°C, Detektion: 220 nm).
Diastereomer 2: 64.2 mg (97.6% de)
$R_t$ = 6.23 min [analytische HPLC:Säule Daicel® Chiralpak ID, 1ml/min; 5 μm, 250 x 4.6 mm; Eluent: 80% iso-Hexan/ 20% Propanol; Detektion: 220 nm].
LC-MS (Methode 3): $R_t$ = 1.78 min; MS (ESIpos): m/z = 579 $[M+H]^+$

**Beispiel 33**

7-[(3R,4R)-3,4-Dihydroxypyrrolidin-1-yl]-6-fluor-4-oxo-N-[(3)-1,1,1,2,2-pentafluorpentan-3-yl]-1-(2,4,6-trifluorphenyl)-1,4-dihydro-1,8-naphthyridin-3-carboxamid (Diastereomer 1)

292 mg 7-[(3R,4R)-3,4-Dihydroxypyrrolidin-1-yl]-6-fluor-4-oxo-N-[(3)-1,1,1,2,2-pentafluorpentan-3-yl]-1-(2,4,6-trifluorphenyl)-1,4-dihydro-1,8-naphthyridin-3-carboxamid (Diastereomerengemisch) wurde durch chirale HPLC in die Diastereomere getrennt (präparative HPLC: Säule Daicel® Chiralpak IA, 5 μm, 250 x 20 mm; Eluent: 85% n-Heptan / 15% Isopropanol; Fluss 15 ml/min; Temperatur: 30°C, Detektion: 220 nm).
Diastereomer 1: 111.6 mg (>99% de)
$R_t$ = 6.10 min [analytische HPLC:Säule Daicel® Chiralpak IA, 1ml/min; 5 μm, 250 x 4.6 mm; Eluent: 80% iso-Hexan/ 20% Isopropanol; Detektion: 265 mn].
LC-MS (Methode 3): $R_t$ = 1.93 min; MS (ESIpos): m/z = 599 $[M+H]^+$

**Beispiel 34**

7-[(3R,4R)-3,4-Dihydroxypyrrolidin-1-yl]-6-fluor-4-oxo-N-[(3)-1,1,1,2,2-pentafluorpentan-3-yl]-1-(2,4,6-trifluorphenyl)-1,4-dihydro-1,8-naphthyridin-3-carboxamid (Diastereomer 2)

292 mg 7-[(3R,4R)-3,4-Dihydroxypyrrolidin-1-yl]-6-fluor-4-oxo-N-[(3)-1,1,1,2,2-pentafluopentan-3-yl]-1-(2,4,6-trifluorphenyl)-1,4-dihydro-1,8-naphthyridin-3-carboxamid (Diastereomerengemisch) wurde durch chirale HPLC in die Diastereomere getrennt (präparative HPLC: Säule Daicel® Chiralpak IA, 5 μm, 250 x 20 mm; Eluent: 85% n-Heptan / 15% Isopropanol; Fluss 15 ml/min; Temperatur: 30°C, Detektion: 220 nm).
Diastereomer 2: 110.1 mg 99.5% de)
$R_t$ = 6.76 min [analytische HPLC:Säule Daicel® Chiralpak IA, 1ml/min; 5 μm, 250 x 4.6 mm; Eluent: 80% iso-Hexan/ 20% Isopropanol; Detektion: 265 nm].
LC-MS (Methode 3): $R_t$ = 1.93 min; MS (ESIpos): m/z = 599 $[M+H]^+$

**Beispiel 35**

(3R,4R)-1-[3-Fluor-5-oxo-6-{[(2S)-1,1,1-trifluorbutan-2-yl]carbamoyl}-8-(2,4,6-trifluorphenyl)-5,8-dihydro-1,8-naphthyridin-2-yl]-4-hydroxypyrrolidin-3-yl-acetat

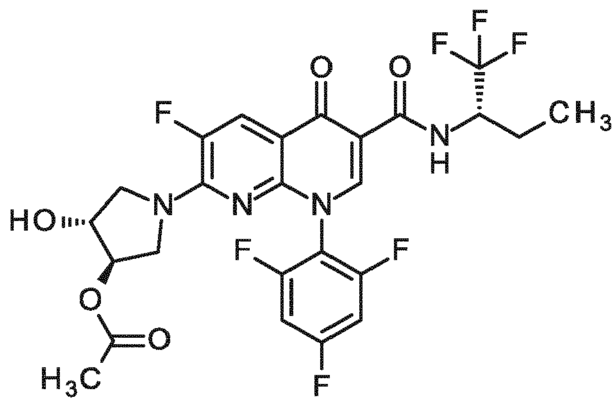

(7-[(3R,4R)-3,4-Dihydroxypyrrolidin-1-yl]-6-fluor-4-oxo-N-[(2S)-1,1,1-trifluorbutan-2-yl]-1-(2,4,6-trifluorphenyl)-1,4-dihydro-1,8-naphthyridin-3-carbonsäureamid (60.0 mg, 109 μmol) wurde in Dichlormethan (1.0 ml) gelöst und mit Dimethylaminopyridin (1.34 mg, 10.9 μmol) versetzt. Es wurde bei 0°C Acetylchlorid (5.4 μl, 77 μmol) zugetropft und 3 h bei RT gerührt. Das Reaktionsgemisch wurde eingeengt und der Rückstand wurde in Acetonitril aufgenommen und mittels präparativer HPLC (Acetonitril-Wasser mit Ameisensäure, C18 RP-HPLC) gereinigt. Die Produktfraktionen wurden vereinigt, eingeengt und über Nacht aus Acetonitril/Wasser lyophilisiert. Es wurden 25.9 mg (39% d. Th., 99% Reinheit) der Titelverbindung erhalten.
LC-MS (Methode 3): $R_t$ = 2.07 min; MS (ESIpos): m/z = 591 $[M+H]^+$
$^1$H-NMR (400 MHz, DMSO-d6) δ [ppm]: -0.149 (0.90), -0.008 (7.72), 0.008 (6.90), 0.146 (0.88), 0.952 (2.34), 0.971

(5.26), 0.989 (2.57), 1.625 (0.47), 1.642 (0.53), 1.651 (0.53), 1.661 (0.51), 1.668 (0.53), 1.685 (0.41), 1.852 (0.41), 1.871 (0.49), 1.881 (0.58), 1.897 (0.45), 1.990 (16.00), 2.328 (0.68), 2.523 (1.81), 2.670 (0.68), 2.710 (0.41), 4.139 (0.45), 4.738 (0.51), 4.951 (0.41), 5.607 (0.94), 7.555 (1.38), 7.577 (2.51), 7.599 (1.40), 8.036 (2.20), 8.067 (2.20), 8.858 (5.18), 10.300 (1.75), 10.324 (1.68).

Die folgenden Reaktionen wurden analog zu Beispiel 1 gemäß AAV1 hergestellt:

| Beispiel | IUPAC-Name Struktur LC-MS (Methode): Retentionszeit; detektierte Masse $^1$H-NMR eingesetztes Amin (Ausbeute, Reinheit) |
|---|---|
| 36 | 7-[(3R,4R)-3,4-Dihydroxypyrrolidin-1-yl]-6-fluor-4-oxo-N-[(2R)-1,1,1-trifluorbutan-2-yl]-1-(2,4,6-trifluorphenyl)-1,4-dihydro-1,8-naphthyridin-3-carboxamid 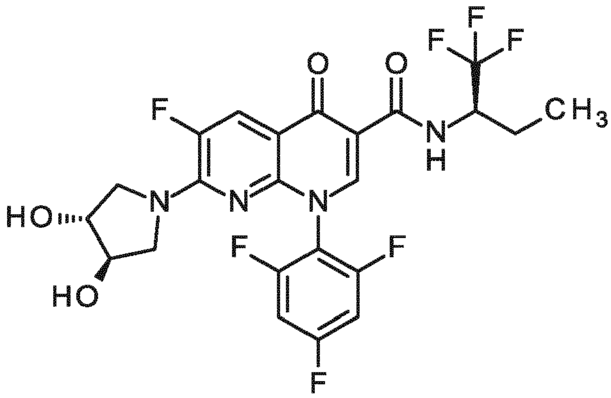 LC-MS (Methode 3): $R_t$ = 1.78 min; MS (ESIpos): m/z = 549 $[M+H]^+$ $^1$H-NMR (400 MHz, DMSO-d6) δ [ppm]: -0.149 (0.60), -0.008 (5.32), 0.008 (4.91), 0.147 (0.57), 0.950 (7.28), 0.969 (16.00), 0.987 (7.85), 1.604 (1.06), 1.622 (1.40), 1.629 (1.28), 1.639 (1.74), 1.647 (1.55), 1.657 (1.47), 1.664 (1.70), 1.682 (1.28), 1.851 (1.32), 1.860 (1.51), 1.868 (1.47), 1.879 (1.74), 1.885 (1.51), 1.895 (1.32), 1.904 (1.13), 1.914 (0.98), 2.328 (1.36), 2.366 (0.94), 2.524 (4.68), 2.670 (1.43), 2.710 (0.98), 3.067 (0.79), 3.691 (0.87), 3.906 (1.81), 4.012 (1.25), 4.735 (1.43), 4.754 (1.36), 5.200 (4.83), 7.558 (3.89), 7.580 (6.87), 7.601 (3.89), 7.999 (7.58), 8.030 (7.51), 8.840 (13.17), 10.329 (5.21), 10.353 (5.02). (2R)-1,1,1-Trifluorbutan-2-aminhydrochlorid (69% d. Th., 99% Reinheit) |
| 37 | 6-Chlor-N-[(1S)-1-cyclopropyl-2,2,2-trifluorethyl]-7-[(3R,4R)-3,4-dihydroxypyrrolidin-1-yl]-4-oxo-1-(2,4,6-trifluophenyl)-1,4-dihydro-1,8-naphthyridin-3-carboxamid 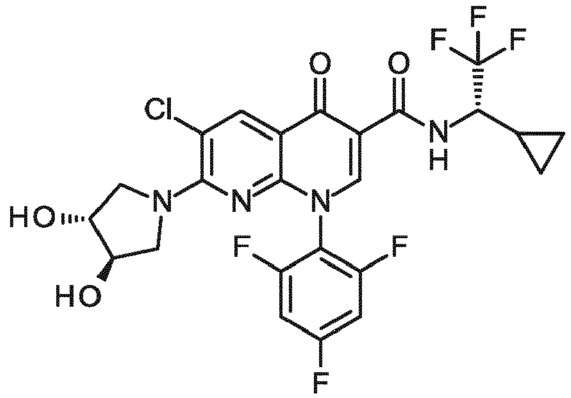 LC-MS (Methode 3): $R_t$ = 1.89 min; MS (ESIpos): m/z = 577.11 $[M+H]^+$ $^1$H-NMR (400 MHz, DMSO-d6) δ [ppm]: -0.008 (1.73), 0.008 (1.41), 0.320 (1.55), 0.330 (2.59), 0.342 (2.48), 0.353 (1.79), 0.365 (1.00), 0.522 (1.67), 0.534 (2.52), 0.547 (2.55), 0.553 (2.84), 0.571 (2.95), 0.580 (2.20), 0.591 (1.98), 0.601 (1.63), 0.615 (0.97), 0.630 (1.27), 0.639 (1.31), 0.650 (2.61), 0.660 (2.01), 0.667 (1.80), 0.685 (0.98), 0.693 (0.58), 1.170 (0.48), 1.182 (1.01), 1.190 (1.45), 1.203 (2.41), 1.212 (1.79), 1.223 (2.40), 1.235 (1.31), 1.244 (0.87), 2.329 (0.60), 2.367 (0.41), 2.524 (2.02), 2.671 (0.70), 2.711 (0.47), 3.683 (0.57), 3.930 (5.71), 4.342 (1.32), 4.363 (2.25), 4.384 (2.19), 4.405 (1.19), 5.188 (9.80), 5.196 (9.81), 7.564 (3.82), 7.585 (6.78), 7.607 (3.80), 8.284 (16.00), 8.856 (13.94), 10.356 (5.25), 10.379 (5.07). (1S)-1-Cyclopropyl-2,2,2-trifluorethanaminhydrochlorid (81% d. Th., 99% Reinheit) |

(fortgesetzt)

| Beispiel | IUPAC-Name Struktur LC-MS (Methode): Retentionszeit; detektierte Masse $^1$H-NMR eingesetztes Amin (Ausbeute, Reinheit) |
|---|---|
| 38 | 6-Chlor-7-[(3R,4R)-3,4-dihydroxypyrrolidin-1-yl]-4-oxo-N-[(2S)-1,1,1-trifluorbutan-2-yl]-1-(2,4,6-trifluorphenyl)-1,4-dihydro-1,8-naphthyridin-3-carboxamid 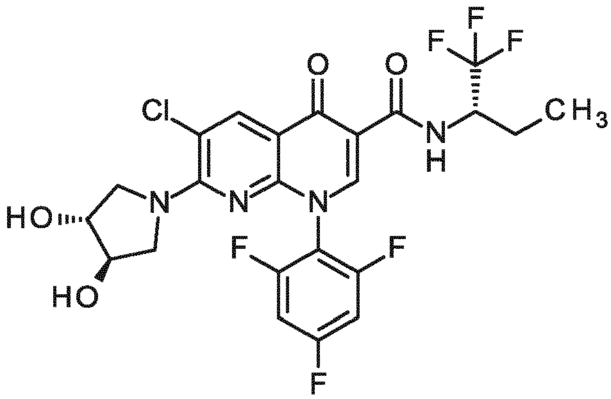 LC-MS (Methode 3): $R_t$ = 1.86 min; MS (ESIpos): m/z = 565 $[M+H]^+$ $^1$H-NMR (400 MHz, DMSO-d6) δ [ppm]: -0.008 (1.70), 0.008 (1.59), 0.951 (7.25), 0.970 (16.00), 0.988 (7.86), 1.609 (1.08), 1.626 (1.45), 1.633 (1.27), 1.644 (1.73), 1.652 (1.56), 1.662 (1.48), 1.669 (1.66), 1.687 (1.24), 1.832 (0.43), 1.850 (1.31), 1.860 (1.52), 1.869 (1.52), 1.879 (1.75), 1.885 (1.54), 1.895 (1.33), 1.904 (1.13), 1.913 (0.96), 2.328 (0.61), 2.367 (0.50), 2.524 (1.95), 2.671 (0.63), 2.711 (0.53), 3.671 (0.56), 3.930 (5.75), 4.735 (1.45), 4.750 (1.34), 5.185 (13.17), 5.192 (13.00), 7.566 (4.26), 7.588 (7.94), 7.610 (4.20), 8.279 (13.40), 8.865 (12.98), 10.212 (5.29), 10.236 (4.98). (2S)-1,1,1-Trifluorbutan-2-aminhydrochlorid (72% d. Th., 99% Reinheit) |
| 39 | 1-(3,5-Difluorpyridin-2-yl)-7-[(3R,4R)-3,4-dihydroxypyrrolidin-1-yl]-6-fluor-4-oxo-N-[(2)-3,3,4,4,4-pentafluorbutan-2-yl]-1,4-dihydro-1,8-naphthyridin-3-carboxamid (Diastereomerengemisch) 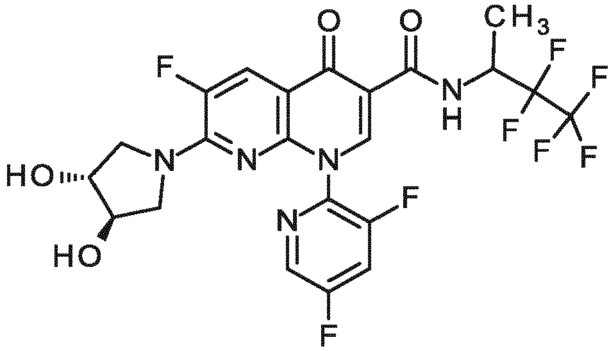 LC-MS (Methode 3): $R_t$ = 1.71 min; MS (ESIpos): m/z = 568 $[M+H]^+$ $^1$H-NMR (400 MHz, DMSO-d6) δ [ppm]: -0.149 (0.43), -0.008 (4.17), 0.008 (3.72), 0.146 (0.45), 1.389 (16.00), 1.405 (15.88), 2.328 (0.93), 2.367 (0.71), 2.524 (2.99), 2.670 (0.91), 2.711 (0.68), 3.067 (0.88), 3.691 (1.06), 3.918 (3.12), 4.976 (1.14), 4.997 (1.93), 5.018 (2.30), 5.041 (2.36), 5.063 (2.17), 5.084 (1.55), 5.102 (1.09), 5.200 (5.07), 7.997 (7.54), 8.028 (7.69), 8.329 (2.76), 8.351 (5.01), 8.373 (2.71), 8.616 (11.67), 8.622 (10.91), 8.837 (7.39), 8.844 (8.07), 10.451 (7.34), 10.475 (7.07). 3,3,4,4,4-Pentafluorbutan-2-aminhydrochlorid (Racemat) (78% d. Th., 99% Reinheit) |
| 40 | 1-(2-Chlor-4,6-difluorphenyl)-7-[(3R,4R)-3,4-dihydroxypyrrolidin-1-yl]-6-fluor-4-oxo-N-[(2S)-1,1,1-trifluorbutan-2-yl]-1,4-dihydro-1,8-naphthyridin-3-carboxamid (Atropisomerengemisch) |

(fortgesetzt)

| Beispiel | IUPAC-Name Struktur LC-MS (Methode): Retentionszeit; detektierte Masse $^1$H-NMR eingesetztes Amin (Ausbeute, Reinheit) |
|---|---|
| | 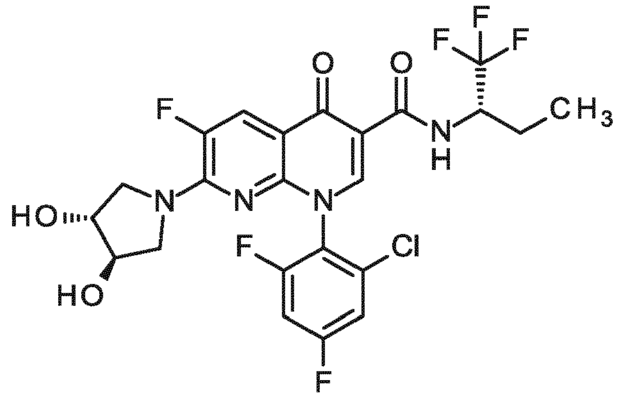 LC-MS (Methode 3): $R_t$ = 1.83 min; MS (ESIpos): m/z = 565 $[M+H]^+$ $^1$H-NMR (400 MHz, DMSO-d6) $\delta$ [ppm]: 0.008 (1.02), 0.949 (4.31), 0.958 (5.08), 0.967 (9.96), 0.977 (9.85), 0.985 (5.50), 0.995 (4.62), 1.603 (0.66), 1.614 (0.80), 1.621 (1.07), 1.638 (1.64), 1.648 (1.55), 1.657 (1.71), 1.664 (1.33), 1.674 (1.24), 1.681 (0.93), 1.692 (0.76), 1.852 (1.27), 1.862 (1.49), 1.870 (1.55), 1.880 (1.71), 1.898 (1.31), 1.905 (1.13), 1.915 (0.91), 2.328 (0.73), 2.366 (0.58), 2.524 (2.35), 2.670 (0.78), 2.710 (0.62), 3.018 (0.87), 3.220 (0.93), 3.693 (0.95), 3.891 (1.82), 4.013 (1.37), 4.734 (1.57), 4.750 (1.47), 5.202 (4.19), 7.686 (0.84), 7.693 (1.27), 7.709 (1.69), 7.717 (2.44), 7.728 (3.11), 7.732 (3.15), 7.740 (3.53), 7.751 (3.53), 7.763 (2.35), 8.004 (7.55), 8.035 (7.54), 8.794 (16.00), 10.347 (3.64), 10.351 (3.73), 10.371 (3.57), 10.375 (3.55). (2S)-1,1,1-Trifluorobutan-2-aminhydrochlorid (83% d. Th., 100% Reinheit) |
| 41 | 1-(2-Chlor-4,6-difluorphenyl)-7-[(3R,4R)-3,4-dihydroxypyrrolidin-1-yl]-6-fluor-4-oxo-N-(1,1,1-trifluor-2-methylpropan-2-yl)-1,4-dihydro-1,8-naphthyridin-3-carboxamid (Atropisomerengemisch) 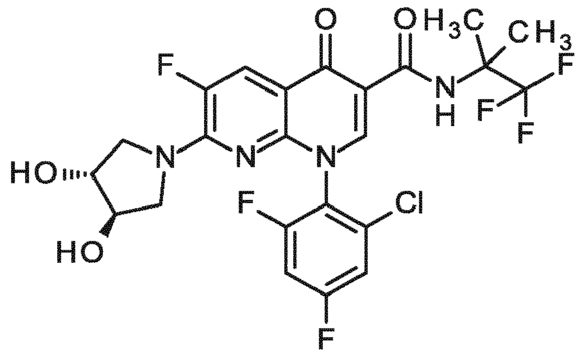 LC-MS (Methode 3): $R_t$ = 1.85 min; MS (ESIpos): m/z = 565 $[M+H]^+$ $^1$H-NMR (400 MHz, DMSO-d6) $\delta$ [ppm]: -0.008 (1.27), 0.008 (0.95), 1.634 (16.00), 2.524 (0.91), 3.894 (0.45), 5.193 (1.23), 7.709 (0.41), 7.717 (0.65), 7.726 (0.67), 7.732 (0.70), 7.739 (0.91), 7.749 (0.72), 7.764 (0.59), 8.012 (1.99), 8.044 (1.97), 8.723 (3.93), 10.582 (2.93). 1,1,1-Trifluor-2-methylpropan-2-amin (96 % d. Th., 99% Reinheit) |
| 42 | 1-(2-Chlor-4,6-difluorphenyl)-N-[(1S)-1-cyclopropyl-2,2,2-trifluorethyl]-7-[(3R,4R)-3,4-dihydroxypyrrolidin-1-yl]-6-fluor-4-oxo-1,4-dihydro-1,8-naphthyridin-3-carboxamid (Atropisomerengemisch) |

(fortgesetzt)

| Beispiel | IUPAC-Name Struktur LC-MS (Methode): Retentionszeit; detektierte Masse $^1$H-NMR eingesetztes Amin (Ausbeute, Reinheit) |
|---|---|
| | 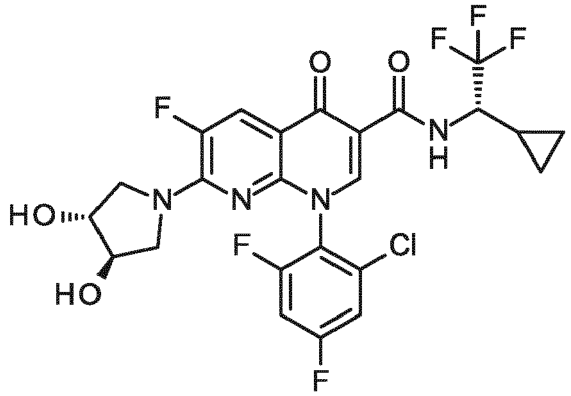<br>LC-MS (Methode 3): $R_t$ = 1.86 min; MS (ESIpos): m/z = 577 $[M+H]^+$ $^1$H-NMR (400 MHz, DMSO-d6) δ [ppm]: -0.149 (0.75), -0.008 (7.24), 0.008 (5.85), 0.146 (0.80), 0.317 (1.19), 0.328 (2.21), 0.340 (2.61), 0.351 (2.36), 0.363 (1.54), 0.374 (0.62), 0.526 (2.24), 0.543 (2.26), 0.555 (2.21), 0.565 (2.36), 0.575 (2.54), 0.586 (2.24), 0.596 (1.92), 0.610 (1.29), 0.624 (1.02), 0.634 (1.34), 0.644 (1.92), 0.655 (2.24), 0.668 (1.99), 0.677 (1.49), 1.167 (0.47), 1.179 (1.00), 1.187 (1.42), 1.199 (2.44), 1.209 (1.87), 1.220 (2.44), 1.232 (1.34), 1.241 (0.85), 1.253 (0.42), 2.327 (1.12), 2.366 (0.77), 2.523 (3.66), 2.665 (0.95), 2.670 (1.24), 2.710 (0.82), 3.015 (0.85), 3.221 (0.90), 3.687 (0.95), 3.894 (1.79), 4.013 (1.37), 4.340 (0.80), 4.359 (1.64), 4.378 (2.12), 4.399 (1.64), 4.418 (0.75), 5.199 (4.33), 7.684 (0.85), 7.691 (1.39), 7.701 (1.37), 7.707 (1.59), 7.715 (2.76), 7.724 (2.84), 7.730 (2.86), 7.737 (3.66), 7.747 (3.09), 7.761 (2.36), 8.008 (7.12), 8.039 (7.07), 8.785 (16.00), 10.486 (4.95), 10.510 (4.70).<br>(1S)-1-Cyclopropyl-2,2,2-trifluorethanaminhydrochlorid (81% d. Th., 99% Reinheit) |
| 43 | 1-(2-Chlor-4,6-difluophenyl)-N-[(1S)-1-cyclopropylethyl]-7-[(3R,4R)-3,4-dihydroxypyrrolidin-1-yl]-6-fluor-4-oxo-1,4-dihydro-1,8-naphthyridin-3-carboxamid (Atropisomerengemisch)<br>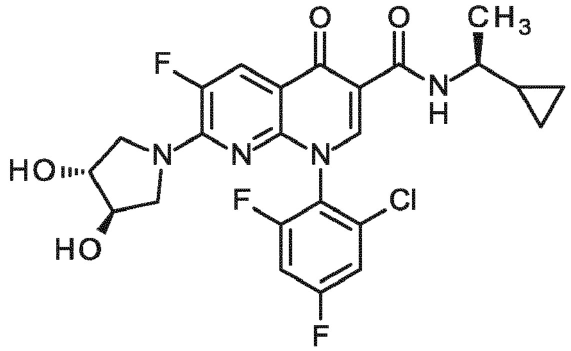<br>LC-MS (Methode 3): $R_t$ = 1.67 min; MS (ESIpos): m/z = 523 $[M+H]^+$ $^1$H-NMR (400 MHz, DMSO-d6) δ [ppm]: -0.008 (6.44), 0.008 (2.37), 0.217 (1.40), 0.228 (2.02), 0.240 (2.94), 0.251 (2.63), 0.264 (1.75), 0.277 (2.10), 0.285 (2.72), 0.292 (2.28),<br>0.305 (1.58), 0.401 (0.92), 0.412 (1.75), 0.422 (2.76), 0.433 (3.20), 0.443 (3.59), 0.453 (3.51), 0.459 (2.85), 0.467 (3.11), 0.479 (1.88), 0.487 (1.23), 0.959 (1.27), 0.964 (1.45), 0.971 (2.02), 0.977 (2.10), 0.984 (1.93), 0.991 (1.93), 0.996 (1.71), 1.004 (1.14), 1.213 (12.01), 1.220 (11.79), 1.230 (11.88), 1.236 (10.48), 2.328 (1.01), 2.366 (0.75), 2.519 (5.57), 2.670 (1.01), 2.710 (0.70), 3.008 (0.70), 3.488 (1.14), 3.507 (2.24), 3.524 (2.98), 3.540 (2.10), 3.560 (1.05), 3.669 (0.75), 3.894 (1.75), 5.186 (5.92), 7.676 (1.01), 7.682 (1.40), 7.693 (1.71), 7.698 (1.84), 7.706 (2.54), 7.717 (3.07), 7.722 (3.07), 7.729 (3.42), 7.739 (2.98), 7.751 (2.15), 7.980 (7.80), 8.011 (7.63), 8.653 (16.00), 9.881 (3.29), 9.887 (3.20), 9.902 (3.16), 9.906 (2.94). (S)-1-Cyclopropylethanamin (84 % d. Th., 99% Reinheit) |
| 44 | 1-(2-Chlor-4,6-difluorphenyl)-N-[(1R)-1-cyclopropylethyl]-7-[(3R,4R)-3,4-dihydroxypyrrolidin-1-yl]-6-fluor-4-oxo-1,4-dihydro-1,8-naphthyridin-3-carboxamid (Atropisomerengemisch) |

(fortgesetzt)

| Beispiel | IUPAC-Name Struktur LC-MS (Methode): Retentionszeit; detektierte Masse $^1$H-NMR eingesetztes Amin (Ausbeute, Reinheit) |
|---|---|
| | 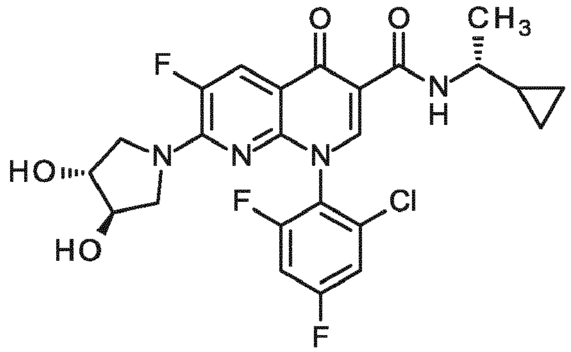 LC-MS (Methode 3): $R_t$ = 1.67 min; MS (ESIpos): m/z = 523 $[M+H]^+$ $^1$H-NMR (400 MHz, DMSO-d6) δ [ppm]: -0.008 (1.78), 0.008 (1.48), 0.218 (1.23), 0.229 (2.01), 0.240 (2.94), 0.252 (2.71), 0.265 (1.58), 0.279 (2.08), 0.286 (2.81), 0.294 (2.36), 0.307 (1.73), 0.328 (0.45), 0.392 (0.43), 0.402 (0.78), 0.413 (1.63), 0.423 (2.69), 0.434 (3.19), 0.445 (3.77), 0.454 (3.59), 0.459 (2.86), 0.467 (3.19), 0.480 (2.03), 0.487 (1.31), 0.501 (0.65), 0.959 (1.18), 0.964 (1.36), 0.972 (1.93), 0.977 (2.11), 0.984 (1.88), 0.992 (2.01), 0.997 (1.81), 1.004 (1.23), 1.010 (1.05), 1.213 (11.53), 1.220 (12.21), 1.229 (12.16), 1.236 (11.68), 2.328 (0.53), 2.367 (0.48), 2.524 (1.93), 2.671 (0.60), 2.711 (0.50), 3.006 (0.70), 3.227 (0.78), 3.486 (1.16), 3.505 (2.36), 3.522 (3.27), 3.539 (2.36), 3.558 (1.18), 3.575 (0.40), 3.677 (0.78), 3.898 (1.76), 5.188 (5.70), 7.676 (0.85), 7.684 (1.66), 7.692 (1.36), 7.700 (1.63), 7.707 (3.09), 7.715 (2.56), 7.730 (3.99), 7.738 (2.84), 7.749 (2.66), 7.754 (2.44), 7.979 (8.26), 8.011 (8.16), 8.653 (16.00), 9.886 (3.74), 9.904 (3.77). (R)-1-Cyclopropylethanamin (91% d. Th., 99% Reinheit) |

## Beispiel 45

1-(3,5-Difluorpyridin-2-yl)-7-[(3R,4R)-3,4-dihydroxypyrrolidin-1-yl]-6-fluor-4-oxo-N-[(2)-3,3,4,4,4-pentafluorbutan-2-yl]-1,4-dihydro-1,8-naphthyridin-3-carboxamid (Diastereomer 1)

486 mg 1-(3,5-Difluorpyridin-2-yl)-7-[(3R,4R)-3,4-dihydroxypyrrolidin-1-yl]-6-fluor-4-oxo-N-[(2)-3,3,4,4,4-pentafluorbutan-2-yl]-1,4-dihydro-1,8-naphthyridin-3-carboxamid (Diastereomerengemisch) wurden durch chirale HPLC in die Diastereomere getrennt (präparative HPLC: Säule Daicel® Chiralpak IE, 5 μm, 250 x 20 mm; Eluent: 70% n-Heptan/ 30% Isopropanol + 0.2% Diethylamin; Fluss 15 ml/min; Temperatur: 25°C, Detektion: 270 mn).
Diastereomer 1: 172.5 mg (>99% de)
$R_t$ = 4.82 min [analytische HPLC: Säule Daicel® Chiralpak IE, 1ml/min; 3 μm, 50 x 4.6 mm; Eluent: 80% iso-Hexan/ 20% Isopropanol + 0.2% Diethylamin; Detektion: 220 nm].

## Beispiel 46

1-(3,5-Difluorpyridin-2-yl)-7-[(3R,4R)-3,4-dihydroxypyrrolidin-1-yl]-6-fluor-4-oxo-N-[(2)-3,3,4,4,4-pentafluorbutan-2-yl]-1,4-dihydro-1,8-naphthyridin-3-carboxamid (Diastereomer 2)

486 mg 1-(3,5-Difluorpyridin-2-yl)-7-[(3R,4R)-3,4-dihydroxypyrrolidin-1-yl]-6-fluor-4-oxo-N-[(2)-3,3,4,4,4-pentafluorbutan-2-yl]-1,4-dihydro-1,8-naphthyridin-3-carboxamid (Diastereomerengemisch) wurden durch chirale HPLC in die Diastereomere getrennt (präparative HPLC: Säule Daicel® Chiralpak IE, 5 μm, 250 x 20 mm; Eluent: 70% n-Heptan / 30% Isopropanol + Diethylamin; Fluss 15 ml/min; Temperatur: 25°C, Detektion: 270 nm).
Diastereomer 2: 160.3 mg (>99% de)
$R_t$ = 7.11 min [analytische HPLC: Säule Daicel® Chiralpak IE, 1ml/min; 3 μm, 50 x 4.6 mm; Eluent: 80% iso-Hexan/ 20% Isopropanol + 0.2% Diethylamin; Detektion: 220 nm].

**Beispiel 47**

1-(2-Chlor-4,6-difluorphenyl)-7-[(3R,4R)-3,4-dihydroxypyrrolidin-1-yl]-6-fluor-4-oxo-N-[(2S)-1,1,1-trifluorbutan-2-yl]-1,4-dihydro-1,8-naphthyridin-3-carboxamid (Atropisomer 1)

103 mg 1-(2-Chlor-4,6-difluorphenyl)-7-[(3R,4R)-3,4-dihydroxypyrrolidin-1-yl]-6-fluor-4-oxo-N-[(2S)-1,1,1-trifluorbutan-2-yl]-1,4-dihydro-1,8-naphthyridin-3-carboxamid (Atropisomerengemisch) wurden durch chirale HPLC in die Atropisomere getrennt (präparative HPLC: Säule Daicel® Chiralpak IG, 5 μm, 250 x 20 nm; Eluent: 75% n-Heptan / 25% Isopropanol + 0.2% Diethylamin; Fluss 15 ml/min; Temperatur: 30°C, Detektion: 265 mn).
Atropisomer 1: 38 mg (>99% de)
$R_t$ = 4.71 min [analytische HPLC: Säule Daicel® Chiralpak IG, 1ml/min; 5 μm, 250 x 4.6 mm; Eluent: 70% iso-Hexan/ 30% Isopropanol + 0.2% Diethylamin; Detektion: 265 nm].

**Beispiel 48**

1-(2-Chlor-4,6-difluorphenyl)-7-[(3R,4R)-3,4-dihydroxypyrrolidin-1-yl]-6-fluor-4-oxo-N-[(2S)-1,1,1-trifluorbutan-2-yl]-1,4-dihydro-1,8-naphthyridin-3-carboxamid (Atropisomer 2)

103 mg 1-(2-Chlor-4,6-difluorphenyl)-7-[(3R,4R)-3,4-dihydroxypyrrolidin-1-yl]-6-fluor-4-oxo-N-[(2S)-1,1,1-trifluorbutan-2-yl]-1,4-dihydro-1,8-naphthyridin-3-carboxamid (Atropisomerengemisch) wurden durch chirale HPLC in die Atropisomere (präparative HPLC: Säule Daicel® Chiralpak IG, 5 μm, 250 x 20 mm; Eluent: 75% n-Heptan / 25% Isopropanol + 0.2% Diethylamin; Fluss 15 ml/min; Temperatur: 30°C, Detektion: 265 nm).
Atropisomer 2: 40 mg (>99% de)
$R_t$ = 5.95 min [analytische HPLC: Säule Daicel® Chiralpak IG, 1ml/min; 5 μm, 250 x 4.6 mm; Eluent: 70% iso-Hexan/ 30% Isopropanol + 0.2% Diethylamin; Detektion: 265 nm].

**Beispiel 49**

1-(2-Chlor-4,6-difluorphenyl)-7-[(3R,4R)-3,4-dihydroxypyrrolidin-1-yl]-6-fluor-4-oxo-N-(1,1,1-trifluor-2-methylpropan-2-yl)-1,4-dihydro-1,8-naphthyridin-3-carboxamid (Atropisomer 1)

119 mg 1-(2-Chlor-4,6-difluorphenyl)-7-[(3R,4R)-3,4-dihydroxypyrrolidin-1-yl]-6-fluor-4-oxo-N-(1,1,1-trifluor-2-methylpropan-2-yl)-1,4-dihydro-1,8-naphthyridin-3-carboxamid (Atropisomerengemisch) wurden durch chirale HPLC in die Atropisomere getrennt (präparative HPLC: Säule YMC Chiralart Amylose SA, 5 μm, 250 x 30 mm; Eluent: 80% n-Heptan/ 20% Isoproanol + 0.2% Diethylamin; Fluss 30 ml/min; Temperatur: 30°C, Detektion: 265 nm).
Atropisomer 1: 26 mg (>99% de)
$R_t$ = 4.86 min [analytische HPLC: Säule YMC Chiralart Amylose SA, 1ml/min; 5 μm, 250 x 4.6 mm; Eluent: 70% n-Heptan/ 30% Isoproanol, 0.2% Diethylamin; Detektion: 265 nm].

**Beispiel 50**

1-(2-Chlor-4,6-difluorphenyl)-7-[(3R,4R)-3,4-dihydroxypyrrolidin-1-yl]-6-fluor-4-oxo-N-(1,1,1-trifluor-2-methylpropan-2-yl)-1,4-dihydro-1,8-naphthyridin-3-carboxamid (Atropisomer 2)

119 mg 1-(2-Chlor-4,6-difluorphenyl)-7-[(3R,4R)-3,4-dihydroxypyrrolidin-1-yl]-6-fluor-4-oxo-N-(1,1,1-trifluor-2-methylpropan-2-yl)-1,4-dihydro-1,8-naphthyridin-3-carboxamid (Atropisomerengemisch) durch chirale HPLC in die Diastereomere getrennt (präparative HPLC: Säule YMC Chiralart Amylose SA, 5 μm, 250 x 30 mm; Eluent: 80% n-Heptan/ 20% Isoproanol + 0.2% Diethylamin; Fluss 30 ml/min; Temperatur: 30°C, Detektion: 265 nm).
Atropisomer 2: 25 mg (99% de)
$R_t$ = 5.42 min [analytische HPLC: Säule YMC Chiralart Amylose SA, 1ml/min; 5 μm, 250 x 4.6 mm; Eluent: 70% n-Heptan/ 30% Isoproanol, 0.2% Diethylamin; Detektion: 265 nm].

**Beispiel 51**

1-(2-Chlor-4,6-difluorphenyl)-N-[(1S)-1-cyclopropyl-2,2,2-trifluorethyl]-7-[(3R,4R)-3,4-dihydroxypyrrolidin-1-yl]-6-fluor-4-oxo-1,4-dihydro-1,8-naphthyridin-3-carboxamid (Atropisomer 1)

103 mg 1-(2-Chlor-4,6-difluorphenyl)-N-[(1S)-1-cyclopropyl-2,2,2-trifluorethyl]-7-[(3R,4R)-3,4-dihydroxypyrrolidin-1-yl]-6-fluor-4-oxo-1,4-dihydro-1,8-naphthyridin-3-carboxamid (Atropisomerengemisch) wurden durch chirale HPLC in die Atropisomere getrennt (präparative HPLC: Säule Daicel® Chiralpak AD-H, 5 μm, 250 x 20 mm; Eluent: 80% n-Heptan / 20% Ethanol; Fluss 25 ml/min; Temperatur: 40°C, Detektion: 210 nm).
Atropisomer 1: 30 mg (99% de)
$R_t$ = 6.04 min [analytische HPLC:Säule Daicel® Chiralpak AI, 1ml/min; 5 μm, 250 x 4.6 mm; Eluent: 80% iso-Hexan/ 20% Ethanol; Detektion: 235 nm].

**Beispiel 52**

1-(2-Chlor-4,6-difluorphenyl)-N-[(1S)-1-cyclopropyl-2,2,2-trifluorethyl]-7-[(3R,4R)-3,4-dihydroxypyrrolidin-1-yl]-6-fluor-4-oxo-1,4-dihydro-1,8-naphthyridin-3-carboxamid (Atropisomer 2)

103 mg 1-(2-Chlor-4,6-difluorphenyl)-N-[(1S)-1-cyclopropyl-2,2,2-trifluorethyl]-7-[(3R,4R)-3,4-dihydroxypyrrolidin-1-yl]-6-fluor-4-oxo-1,4-dihydro-1,8-naphthyridin-3-carboxamid (Atropisomerengemisch) wurden durch chirale HPLC in die Atropisomere getrennt (präparative HPLC: Säule Daicel® Chiralpak AD-H, 5 μm, 250 x 20 mm; Eluent: 80% n-Heptan / 20% Ethanol; Fluss 25 ml/min; Temperatur: 40°C, Detektion: 210 nm).
Atropisomer 2: 30 mg (89% de)
$R_t$ = 7.33 min [analytische HPLC:Säule Daicel® Chiralpak AI, 1ml/min; 5 μm, 250 x 4.6 mm; Eluent: 80% iso-Hexan/ 20% Ethanol; Detektion: 235 nm].

**Beispiel 53**

N-[(1S)-1-Cyclopropyl-2,2,2-trifluorethyl]-6-fluor-7-(3-hydroxy-3-methylazetidin-1-yl)-4-oxo-1-(2,4,6-trifluorphenyl)-1,4-dihydro-1,8-naphthyridin-3-carboxamid

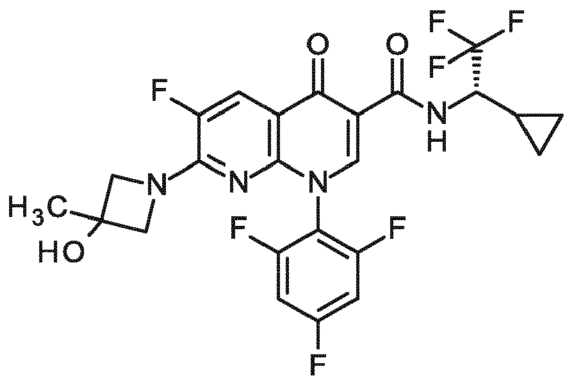

50 mg N-[(1S)-1-Cyclopropyl-2,2,2-trifluorethyl]-6-fluor-4-oxo-7-(3H-[1,2,3]triazolo[4,5-b]pyridin-3-yloxy)-1-(2,4,6-trifluorphenyl)-1,4-dihydro-1,8-naphthyridin-3-carboxamid (84.3 μmol) wurden in DMF (980 μl) gelöst. Es wurden 3-Methylazetidin-3-ol-hydrochlorid (20.8 mg, 169 μmol) und N,N-Diisopropylethylamin (51 μl, 290 μmol) zugegeben und 2 h bei RT gerührt. Die Reaktionsmischung wurde dann mit 0.3 ml 1 N Salzsäure und 1 ml Acetonitril versetzt und mittels präparativer HPLC (Acetonitril-Wasser mit Ameisensäure, C18 RP-HPLC) gereinigt. Die Produktfraktionen wurden vereinigt, eingeengt und über Nacht aus Acetonitril/Wasser lyophilisiert. Es wurden 36.2 mg (78% d. Th., 99% Reinheit) der Titelverbindung erhalten.
LC-MS (Methode 1): $R_t$ = 1.13 min; MS (ESIpos): m/z = 545 $[M+H]^+$
$^1$H-NMR (400 MHz, DMSO-d6) δ [ppm]: -0.008 (2.92), 0.008 (2.80), 0.314 (0.84), 0.325 (1.33), 0.337 (1.27), 0.349 (1.02), 0.360 (0.50), 0.512 (0.90), 0.522 (1.35), 0.535 (1.27), 0.545 (1.38), 0.564 (1.40), 0.574 (1.14), 0.585 (1.02), 0.594 (0.87), 0.608 (0.52), 0.625 (0.77), 0.634 (0.73), 0.645 (1.26), 0.656 (1.01), 0.667 (0.95), 1.177 (0.55), 1.185 (0.79), 1.198 (1.31), 1.206 (0.92), 1.218 (1.38), 1.230 (0.71), 1.382 (16.00), 2.328 (0.67), 2.367 (0.45), 2.670 (0.60), 2.711 (0.41), 3.896 (0.45), 4.350 (0.73), 4.372 (1.28), 4.394 (1.17), 4.413 (0.68), 5.673 (9.48), 7.535 (2.55), 7.557 (4.79), 7.579 (2.54), 8.000 (4.69), 8.028 (4.62), 8.835 (8.40), 10.440 (2.87), 10.464 (2.65).

**Beispiel 54**

N-[(1S)-1-Cyclopropyl-2,2,2-trifluorethyl]-6-fluor-7-(3-hydroxyazetidin-1-yl)-4-oxo-1-(2,4,6-trifluophenyl)-1,4-dihydro-1,8-naphthyridin-3-carboxamid

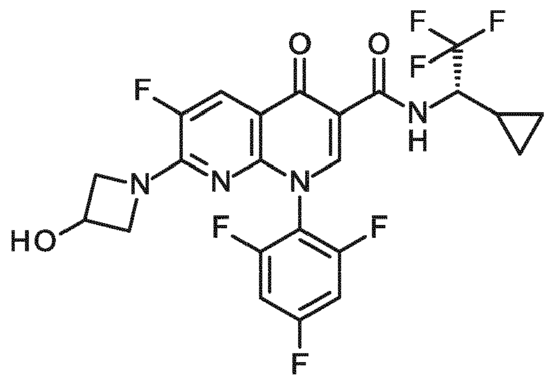

N-[(1S)-1-50 mg Cyclopropyl-2,2,2-trifluorethyl]-6-fluor-4-oxo-7-(3H-[1,2,3]triazolo[4,5-b]pyridin-3-yloxy)-1-(2,4,6-trifluorphenyl)-1,4-dihydro-1,8-naphthyridin-3-carboxamid (84.3 μmol) wurden in DMF (980 μl) gelöst. Es wurden Azetidin-3-ol-hydrochlorid (18.5 mg, 169 μmol) und N,N-Diisopropylethylamin (51 μl, 290 μmol) zugegeben und 2 h bei RT gerührt. Die Reaktionsmischung wurde dann mit 0.3 ml 1 N Salzsäure und 1 ml Acetonitril versetzt und mittels präparativer HPLC (Acetonitril-Wasser mit Ameisensäure, C18 RP-HPLC) gereinigt. Die Produktfraktionen wurden vereinigt, eingeengt und über Nacht aus Acetonitril/Wasser lyophilisiert. Es wurden 32.2 mg (71% d. Th., 99% Reinheit) der Titelverbindung erhalten.
LC-MS (Methode 1): $R_t$ = 1.08 min; MS (ESIpos): m/z = 531 $[M+H]^+$
$^1$H-NMR (400 MHz, DMSO-d6) δ [ppm]: -0.149 (0.55), -0.008 (6.99), 0.008 (4.19), 0.146 (0.49), 0.314 (2.27), 0.324 (3.51), 0.337 (3.32), 0.348 (2.57), 0.360 (1.23), 0.511 (2.49), 0.522 (3.55), 0.534 (3.29), 0.545 (3.63), 0.563 (3.70), 0.573 (2.93), 0.584 (2.64), 0.594 (2.21), 0.608 (1.40), 0.624 (1.96), 0.634 (1.95), 0.644 (3.27), 0.655 (2.74), 0.660 (2.57), 0.667 (2.47), 0.676 (1.25), 0.689 (0.81), 1.164 (0.76), 1.176 (1.47), 1.185 (2.06), 1.197 (3.32), 1.205 (2.49), 1.217 (3.15), 1.229 (1.72), 1.238 (1.19), 1.250 (0.49), 2.328 (0.85), 2.366 (0.70), 2.524 (4.17), 2.670 (0.85), 2.710 (0.57), 3.821 (1.08), 4.330 (1.23), 4.350 (2.42), 4.371 (3.53), 4.392 (3.34), 4.412 (1.87), 4.501 (0.94), 4.517 (2.23), 4.528 (3.61), 4.544 (3.31), 4.555 (1.74), 4.571 (0.57), 5.741 (9.80), 5.757 (9.35), 7.532 (5.86), 7.555 (10.75), 7.577 (5.65), 7.992 (9.11), 8.020 (8.88), 8.832 (16.00), 10.439 (6.76), 10.462 (6.37).

**Beispiel 55**

N-[(1S)-1-Cyclopropyl-2,2,2-trifluorethyl]-6-fluor-7-[(2-hydroxyethyl)(methyl)amino]-4-oxo-1-(2,4,6-trifluorphenyl)-1,4-dihydro-1,8-naphthyridin-3-carboxamid

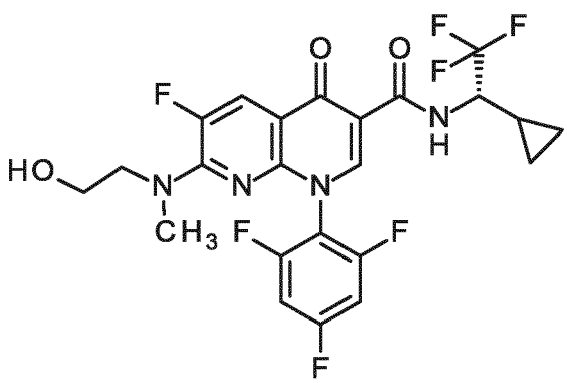

80 mg N-[(1S)-1-Cyclopropyl-2,2,2-trifluorethyl]-6-fluor-4-oxo-7-(3H-[1,2,3]triazolo[4,5-b]pyridin-3-yloxy)-1-(2,4,6-trifluorphenyl)-1,4-dihydro-1,8-naphthyridin-3-carboxamid (135 µmol) wurden in DMF (980 µl) gelöst. Es wurden 2-(Methylamino)ethanol (20.3 mg, 270 µmol) und N,N-Diisopropylethylamin (82 µl, 470 µmol) zugegeben und 2 h bei RT gerührt. Die Reaktionsmischung wurde mit 0.2 ml 1 N Salzsäure und 2 ml Acetonitril versetzt und mittels präparativer HPLC (Acetonitril-Wasser mit Ameisensäure, C18 RP-HPLC) gereinigt. Die Produktfraktionen wurden vereinigt, eingeengt und über Nacht aus Acetonitril/Wasser lyophilisiert. Es wurden 45.1 mg (62% d. Th., 99% Reinheit) der Titelverbindung erhalten.
LC-MS (Methode 1): $R_t$ = 1.09 min; MS (ESIpos): m/z = 533 $[M+H]^+$
$^1$H-NMR (400 MHz, DMSO-d6) δ [ppm]: -0.149 (0.80), -0.008 (7.20), 0.146 (0.77), 0.319 (1.97), 0.329 (3.20), 0.341 (3.09), 0.353 (2.45), 0.365 (1.17), 0.513 (2.13), 0.525 (3.31), 0.538 (2.91), 0.548 (3.25), 0.567 (3.33), 0.577 (2.75), 0.588 (2.40), 0.598 (2.05), 0.612 (1.25), 0.626 (1.68), 0.636 (1.63), 0.647 (2.93), 0.657 (2.53), 0.663 (2.40), 0.670 (2.37), 0.679 (1.15), 0.691 (0.80), 1.166 (0.59), 1.178 (1.23), 1.187 (1.81), 1.199 (3.04), 1.208 (2.21), 1.219 (2.99), 1.231 (1.63), 1.240 (1.12), 1.252 (0.48), 2.327 (1.49), 2.366 (1.23), 2.523 (5.39), 2.669 (1.60), 2.710 (1.20), 3.076 (9.76), 3.442 (6.83), 3.470 (5.87), 4.331 (0.40), 4.351 (1.63), 4.373 (2.85), 4.393 (2.80), 4.414 (1.49), 4.713 (2.96), 4.725 (6.51), 4.738 (3.01), 7.539 (5.52), 7.561 (10.56), 7.583 (5.60), 7.994 (9.63), 8.028 (9.44), 8.849 (16.00), 10.436 (6.37), 10.459 (6.16).

**Beispiel 56**

N-(Dicyclopropylmethyl)-1-(3,5-difluorpyridin-2-yl)-6-fluor-7-(3-hydroxy-3-methylazetidin-1-yl)-4-oxo-1,4-dihydro-1,8-naphthyridin-3-carboxamid

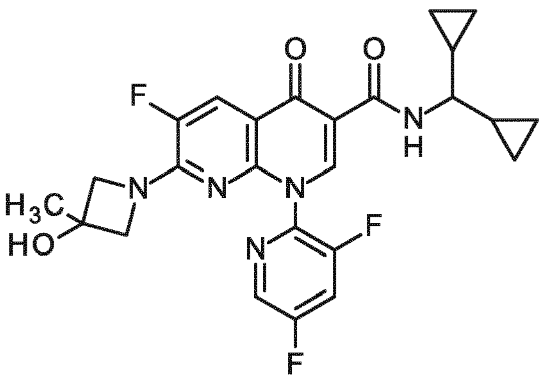

50 mg 1-(3,5-Difluorpyridin-2-yl)-6-fluor-7-(3-hydroxy-3-methylazetidin-1-yl)-4-oxo-1,4-dihydro-1,8-naphthyridin-3-carbonsäure (123 µmol) wurden in DMF (980 µl) gelöst. Es wurden HATU (56.2 mg, 148 µmol), N,N-Diisopropylethylamin (54 µl, 308 µmol) und 1,1-Dicyclopropylmethanamin (15.1 mg, 135 µmol) zugegeben und 2 h bei RT gerührt. Die Reaktionsmischung wurde mit 0.1 ml 1 M Salzsäure und 1 ml Acetonitril versetzt und mittels präparativer HPLC (Acetonitril-Wasser mit Ameisensäure, C18 RP-HPLC) gereinigt. Die Produktfraktionen wurden vereinigt, eingeengt und über Nacht aus Acetonitril/Wasser lyophilisiert. Es wurden 48.7 mg (78% d. Th., 99% Reinheit) der Titelverbindung erhalten.
LC-MS (Methode 1): $R_t$ = 1.04 min; MS (ESIpos): m/z = 500 $[M+H]^+$
$^1$H-NMR (400 MHz, DMSO-d6) δ [ppm]: -0.008 (3.14), 0.008 (2.56), 0.300 (6.52), 0.387 (1.96), 0.397 (2.30), 0.416 (1.48), 0.455 (1.94), 0.475 (2.68), 1.031 (2.06), 1.044 (2.01), 1.382 (16.00), 2.323 (0.44), 2.328 (0.58), 2.524 (1.88), 2.670 (0.60), 3.235 (1.02), 3.254 (2.27), 3.276 (2.41), 3.928 (0.72), 5.676 (6.09), 7.985 (4.45), 8.014 (4.40), 8.292 (1.04), 8.298 (1.18), 8.316 (1.67), 8.319 (1.81), 8.337 (1.07), 8.343 (1.16), 8.591 (5.22), 8.597 (4.96), 8.753 (9.26), 9.856 (2.93), 9.878 (2.87).

**Beispiel 57**

6-Fluor-7-[(2S)-2-(hydroxymethyl)piperidin-1-yl]-4-oxo-N-[(2S)-1,1,1-trifluorbutan-2-yl]-1-(2,4,6-trifluorphenyl)-1,4-dihydro-1,8-naphthyridin-3-carboxamid

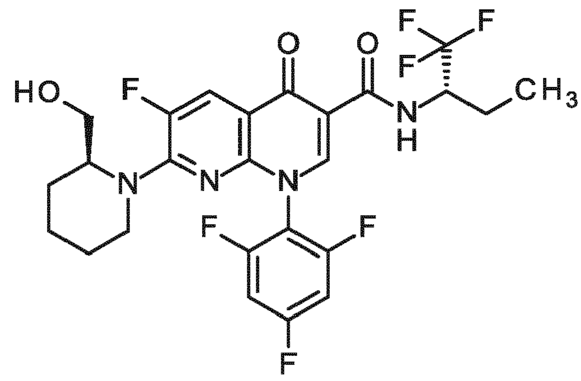

50 mg 6-Fluor-4-oxo-7-(3H-[1,2,3]triazolo[4,5-b]pyridin-3-yloxy)-N-[(2S)-1,1,1-trifluorbutan-2-yl]-1-(2,4,6-trifluorphenyl)-1,4-dihydro-1,8-naphthyridin-3-carboxamid (86.0 µmol) wurden in DMF (980 µl) gelöst. Es wurden (2S)-Piperidin-2-ylmethanol (19.8 mg, 172 µmol) und N,N-Diisopropylethylamin (52 µl, 300 µmol) zugegeben und 2 h bei RT gerührt. Die Reaktionsmischung wurde dann mit 0.3 ml 1 M Salzsäure und 1 ml Acetonitril versetzt und mittels präparativer HPLC (Acetonitril-Wasser mit Ameisensäure, C18 RP-HPLC) gereinigt. Die Produktfraktionen wurden vereinigt, eingeengt und über Nacht aus Acetonitril/Wasser lyophilisiert. Es wurden 37.3 mg (77% d. Th., 99% Reinheit) der Titelverbindung erhalten.
LC-MS (Methode 3): $R_t$ = 2.25 min; MS (ESIpos): m/z = 561 $[M+H]^+$
$^1$H-NMR (400 MHz, DMSO-d6) δ [ppm]: -0.149 (0.55), -0.008 (4.87), 0.008 (4.03), 0.146 (0.52), 0.948 (7.19), 0.967 (16.00), 0.985 (7.82), 1.344 (1.09), 1.376 (1.34), 1.471 (1.90), 1.530 (3.44), 1.549 (5.64), 1.577 (2.47), 1.606 (1.34), 1.624 (1.56), 1.631 (1.36), 1.641 (1.83), 1.649 (1.65), 1.659 (1.59), 1.666 (1.77), 1.684 (1.47), 1.703 (0.91), 1.723 (1.95), 1.740 (1.83), 1.832 (0.43), 1.851 (1.31), 1.861 (1.54), 1.869 (1.56), 1.879 (1.74), 1.886 (1.54), 1.896 (1.34), 1.905 (1.15), 1.914 (0.98), 2.367 (0.70), 2.519 (3.11), 2.524 (2.47), 2.711 (0.66), 2.925 (1.07), 2.955 (1.99), 2.988 (1.07), 3.479 (1.00), 3.495 (1.47), 3.506 (2.47), 3.520 (2.63), 3.536 (1.90), 3.559 (1.20), 3.574 (2.02), 3.588 (1.77), 3.616 (0.68), 3.855 (1.79), 3.888 (1.68), 4.288 (2.04), 4.662 (3.01), 4.676 (6.53), 4.689 (2.97), 4.737 (1.41), 4.758 (1.32), 7.531 (1.43), 7.535 (1.41), 7.550 (3.99), 7.555 (4.15), 7.573 (4.28), 7.578 (3.88), 7.597 (1.36), 8.001 (8.41), 8.036 (8.14), 8.869 (13.96), 10.274 (5.15), 10.298 (4.94).

**Beispiel 58**

N-[(1S)-1-Cyclopropyl-2,2,2-trifluorethyl]-6-fluor-7-[(2S)-2-(hydroxymethyl)piperidin-1-yl]-4-oxo-1-(2,4,6-trifluorphenyl)-1,4-dihydro-1,8-naphthyridin-3-carboxamid

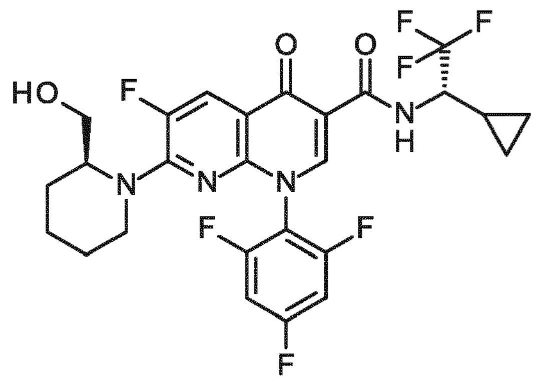

50 mg N-[(1S)-1-Cyclopropyl-2,2,2-trifluorethyl]-6-fluor-4-oxo-7-(1H-[1,2,3]triazolo[4,5-b]pyridin-1-yloxy)-1-(2,4,6-trifluorphenyl)-1,4-dihydro-1,8-naphthyridin-3-carbonsäureamid 50 mg N-[(1S)-1-Cyclopropyl-2,2,2-trifluorethyl]-6-fluor-4-oxo-7-(3H-[1,2,3]triazolo[4,5-b]pyridin-3-yloxy)-1-(2,4,6-trifluorphenyl)-1,4-dihydro-1,8-naphthyridin-3-carboxamid (84.3 µmol) wurden in DMF (980 µl) gelöst. Es wurden (2S)-Piperidin-2-ylmethanol (19.4 mg, 169 µmol) und N,N-Diisopropylethylamin (51 µl, 290 µmol) zugegeben und 2 h bei RT gerührt. Die Reaktionsmischung wurde mit 0,3 ml 1 N Salzsäure und 1 ml Acetonitril versetzen und mittels präparativer HPLC (Acetonitril-Wasser mit Ameisensäure, C18 RP-HPLC) gereinigt. Die Produktfraktionen wurden vereinigt, eingeengt und über Nacht aus Acetonitril/Wasser

lyophilisiert. Es wurden 36.5 mg (75% d. Th., 99% Reinheit) der Titelverbindung erhalten.
LC-MS (Methode 1): $R_t$ = 1.21 min; MS (ESIpos): m/z = 573 $[M+H]^+$
$^1$H-NMR (400 MHz, DMSO-d6) δ [ppm]: -0.149 (0.72), -0.008 (6.54), 0.008 (5.08), 0.147 (0.62), 0.318 (1.64), 0.330 (2.51), 0.342 (2.47), 0.353 (1.96), 0.516 (1.76), 0.528 (2.59), 0.539 (2.36), 0.550 (2.63), 0.568 (2.76), 0.579 (2.15), 0.589 (2.00), 0.599 (1.66), 0.613 (1.02), 0.628 (1.49), 0.637 (1.40), 0.648 (2.51), 0.659 (2.08), 0.664 (1.98), 0.670 (1.96), 1.189 (1.51), 1.201 (2.61), 1.209 (1.87), 1.221 (2.57), 1.233 (1.44), 1.377 (1.34), 1.472 (2.00), 1.532 (3.55), 1.551 (5.84), 1.577 (2.55), 1.723 (1.98), 1.740 (1.87), 2.328 (1.13), 2.367 (0.70), 2.524 (3.19), 2.670 (1.08), 2.711 (0.70), 2.924 (1.10), 2.954 (2.10), 2.987 (1.15), 3.479 (1.00), 3.495 (1.47), 3.506 (2.59), 3.520 (2.74), 3.536 (1.95), 3.574 (2.08), 3.587 (1.85), 3.859 (1.95), 3.892 (1.79), 4.286 (2.17), 4.352 (1.30), 4.373 (2.30), 4.394 (2.32), 4.414 (1.25), 4.662 (2.95), 4.675 (6.42), 4.688 (2.87), 7.533 (1.59), 7.547 (3.89), 7.553 (4.38), 7.569 (4.48), 7.576 (3.85), 7.590 (1.53), 8.006 (8.95), 8.041 (8.61), 8.860 (16.00), 10.414 (5.44), 10.437 (5.10).

**Beispiel 59**

N-[(1S)-1-Cyclopropyl-2,2,2-trifluorethyl]-6-fluor-7-[(4S)-4-hydroxy-2-oxopyrrolidin-1-yl]-4-oxo-1-(2,4,6-trifluorphenyl)-1,4-dihydro-1,8-naphthyridin-3-carboxamid

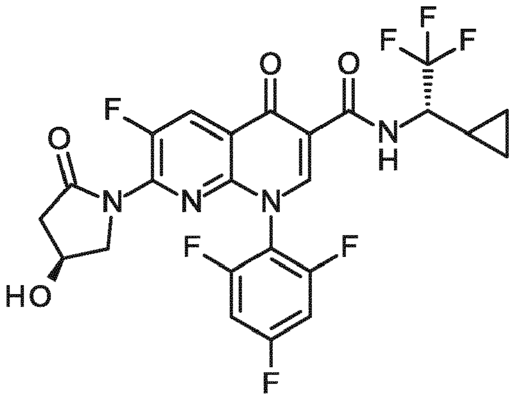

Gemäß AAV 1 wurden 61.7 mg (80 % Reinheit, 113 μmol) 6-Fluor-7-[(4S)-4-hydroxy-2-oxopyrrolidin-1-yl]-4-oxo-1-(2,4,6-trifluorphenyl)-1,4-dihydro-1,8-naphthyridin-3-carbonsäure mit 29.7 mg (1S)-1-Cyclopropyl-2,2,2-trifluorethanaminhydrochlorid (169 μmol) in Gegenwart von 64.4 mg (169 μmol) HATU und 98 μl (560 μmol) DIPEA in 3.0 ml DMF zur Reaktion gebracht. Das Reaktionsgemisch wurde mit 0.5 ml wässriger Salzsäure verdünnt und mittels präparativer HPLC [bei UV max: 265nm, Säule: Chromatorex C18, 10 μm, 125x30 mm, Lösungsmittel: Acetonitril / 0.05% Ameisensäure-Gradient; (0 bis 3 min. 10% Acetonitril bis 15 min. 90% Acetonitril und weitere 3 min. 90% Acetonitril)] gereinigt. Die Produktfraktionen wurden vereinigt, vom Lösungsmittel befreit und lyophilisiert. Es wurden 27.2 mg (43% d. Th., 100% Reinheit) der Titelverbindung erhalten.
LC-MS (Methode 3): $R_t$ = 1.80 min; MS (ESIpos): m/z = 559 $[M+H]^+$

**Beispiel 60**

N-tert-Butyl-7-(dimethylamino)-6-fluor-4-oxo-1-(2,4,6-trifluorphenyl)-1,4-dihydro-1,8-naphthyridin-3-carboxamid

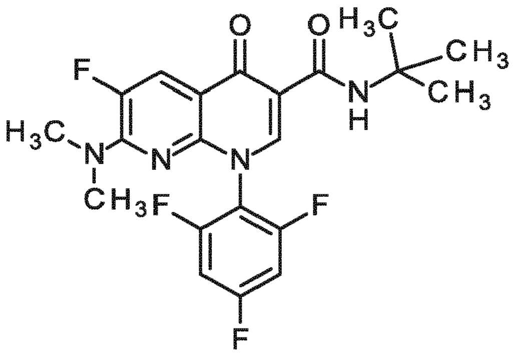

7-Chlor-6-fluor-4-oxo-1-(2,4,6-trifluorphenyl)-1,4-dihydro-1,8-naphthyridin-3-carbonsäure (120 mg, 322 μmol), wurde in 2.4 ml DMF vorgelegt, mit HATU (147 mg, 386 μmol) und N,N-Diisopropylethylamin (200 μl, 1.1 mmol) versetzt und 30 min bei Raumtemperatur gerührt. 2-Methylpropan-2-amin (41 μl, 390 μmol) wurde hinzugeben und 5 min bei Raumtemperatur gerührt. Nach 5 min wurde das Reaktionsgemisch mit Wasser versetzt. Die entstandene Suspension wurde über Nacht stehen gelassen. Am nächsten Morgen war ein absaugbarer Feststoff entstanden. Dieser Rückstand wurde mittels Säulenchromatographie gereinigt (Kieselgel; Laufmittel: Dichlormethan/Methanol-Gradient: 100/0 nach 100/1). Es wurden 23 mg (16% d.Th.) der Zielverbindung erhalten.
LC-MS (Methode 3): $R_t$ = 2.32 min; MS (ESIpos): m/z = 437 $[M+H]^+$

## Beispiel 61

7-(Dimethylamino)-6-fluor-N-(2-methylbutan-2-yl)-4-oxo-1-(2,4,6-trifluorphenyl)-1,4-dihydro-1,8-naphthyridin-3-carboxamid

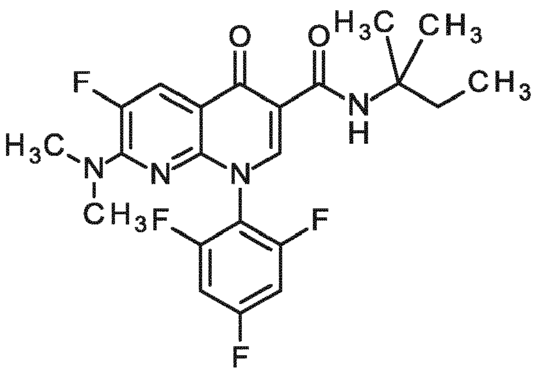

7-Chlor-6-fluor-4-oxo-1-(2,4,6-trifluorphenyl)-1,4-dihydro-1,8-naphthyridin-3-carbonsäure (120 mg, 322 μmol), wurde in 2.4 ml DMF vorgelegt, mit HATU (147 mg, 386 μmol) und N,N-Diisopropylethylamin (200 μl, 1.1 mmol) versetzt und 30 min bei Raumtemperatur gerührt. 2-Methylbutan-2-amin (45 μl, 390 μmol) wurde hinzugeben und 5 min bei Raumtemperatur gerührt. Nach 5 min wurde das Reaktionsgemisch mit Wasser versetzt. Die entstandene Suspension wurde über Nacht stehen gelassen. Am nächsten Morgen war ein absaugbarer Feststoff entstanden. Dieser Rückstand wurde mittels Säulenchromatographie gereinigt (Kieselgel; Laufmittel: Dichlormethan/Methanol-Gradient: 100/0 nach 100/1). Es wurden 19 mg (13% d.Th.) der Zielverbindung erhalten.
LC-MS (Methode 3): $R_t$ = 2.42 min; MS (ESIpos): m/z = 451 $[M+H]^+$

## Beispiel 62

7-(Dimethylamino)-6-fluor-4-oxo-N-(1,1,1-trifluor-2-methylpropan-2-yl)-1-(2,4,6-trifluorphenyl)-1,4-dihydro-1,8-naphthyridin-3-carboxamid

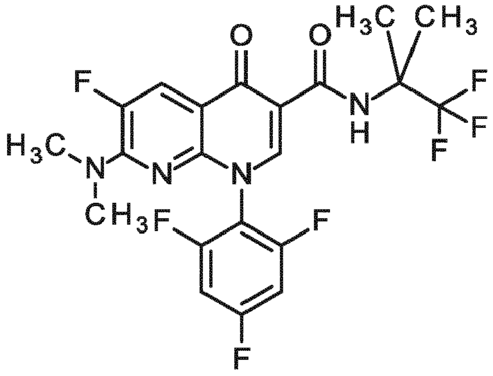

7-Chlor-6-fluor-4-oxo-1-(2,4,6-trifluorphenyl)-1,4-dihydro-1,8-naphthyridin-3-carbonsäure (120 mg, 322 μmol), wurde in 2.4 ml DMF vorgelegt, mit HATU (147 mg, 386 μmol) und N,N-Diisopropylethylamin (200 μl, 1.1 mmol) versetzt und 30 min bei Raumtemperatur gerührt. 1,1,1-Trifluor-2-methylpropan-2-amin (49.1 mg, 386 μmol) wurde hinzugeben und 5 min bei Raumtemperatur gerührt. Nach 5 min wurde das Reaktionsgemisch mit Wasser versetzt. Die entstandene Suspension wurde über Nacht stehen gelassen. Am nächsten Morgen war ein absaugbarer Feststoff entstanden. Dieser Rückstand wurde mittels Säulenchromatographie gereinigt (Kieselgel; Laufmittel: Dichlormethan/Methanol-Gradient: 100/0 nach 100/1). Es wurden 30 mg (19% d.Th.) der Zielverbindung erhalten.
LC-MS (Methode 3): $R_t$ = 2.42 min; MS (ESIpos): m/z = 491 $[M+H]^+$

**Beispiel 63**

7-(Dimethylamino)-6-fluor-4-oxo-N-(4,4,4-trifluor-2-methylbutan-2-yl)-1-(2,4,6-trifluorphenyl)-1,4-dihydro-1,8-naphthyridin-3-carboxamid

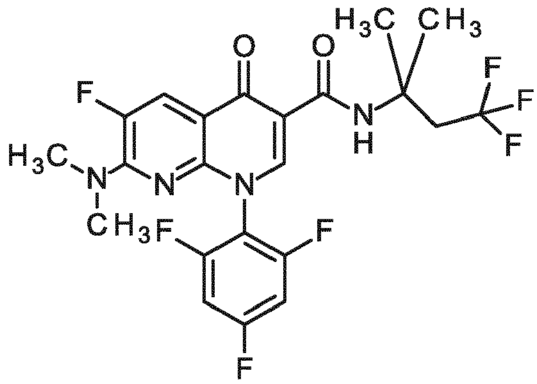

7-Chlor-6-fluor-4-oxo-1-(2,4,6-trifluorphenyl)-1,4-dihydro-1,8-naphthyridin-3-carbonsäure (120 mg, 322 μmol), wurde in 2.4 ml DMF vorgelegt, mit HATU (147 mg, 386 μmol) und N,N-Diisopropylethylamin (200 μl, 1.1 mmol) versetzt und 30 min bei Raumtemperatur gerührt. 4,4,4-Trifluor-2-methylbutan-2-amin-Hydrochlorid (68.6 mg, 386 μmol) wurde hinzugeben und 5 min bei Raumtemperatur gerührt. Nach 5 min wurde das Reaktionsgemisch mit Wasser versetzt. Die entstandene Suspension wurde über Nacht stehen gelassen. Am nächsten Morgen war ein absaugbarer Feststoff entstanden. Dieser Rückstand wurde mittels Säulenchromatographie gereinigt (Kieselgel; Laufmittel: Dichlormethan/Methanol-Gradient: 100/0 nach 100/1). Es wurden 24 mg (15% d.Th.) der Zielverbindung erhalten.
LC-MS (Methode 3): $R_t$ = 2.42 min; MS (ESIpos): m/z = 505 $[M+H]^+$

**Beispiel 64**

N-[(1S)-1-Cyclopropyl-2,2,2-trifluorethyl]-6-fluor-7-{[(2S)-2-hydroxypropyl](methyl)amino}-4-oxo-1-(2,4,6-trifluorphenyl)-1,4-dihydro-1,8-naphthyridin-3-carboxamid

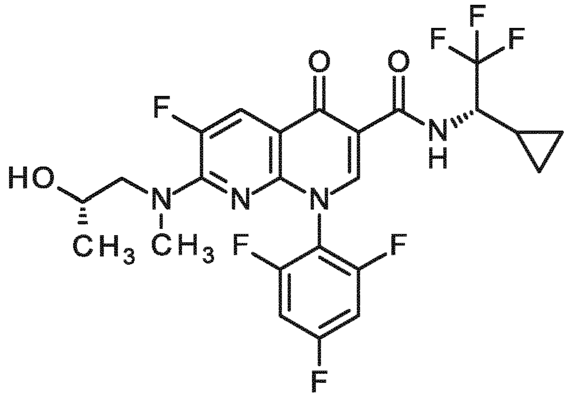

7-Chlor-N-[(1S)-1-cyclopropyl-2,2,2-trifluorethyl]-6-fluor-4-oxo-1-(2,4,6-trifluorphenyl)-1,4-dihydro-1,8-naphthyridin-3-carboxamid (60.0 mg, 122 µmol) wurde in 1.2 ml DMF vorgelegt, mit (2S)-1-(Methylamino)propan-2-ol (21.7 mg, 243 µmol) und N,N-Diisopropylethylamin (74 µl, 430 µmol) versetzt und 2 h bei Raumtemperatur gerührt. Die Reaktionslösung wurde mit Acetonitril/Wasser versetzt und mittels präparativer HPLC gereinigt (RP18 Säule, Laufmittel: Acetonitril/Wasser-Gradient unter Zusatz von 0.1% TFA). Die Produktfraktionen wurden vereinigt und im Vakuum eingeengt. Der Rückstand wurde in Dichlormethan aufgenommen und zweimal mit gesättigter, wässriger Natriumhydrogencarbonat-Lösung extrahiert. Die vereinigten wässrigen Phasen wurden einmal mit Dichlormethan reextrahiert. Die vereinigten organischen Phasen wurden über Natriumsulfat getrocknet, filtriert und im Vakuum eingeengt. Es wurden 47 mg der Zielverbindung (70% d. Th.) erhalten.
LC-MS (Methode 3): $R_t$ = 2.15 min; MS (ESIpos): m/z = 547 $[M+H]^+$
$^1$H-NMR (400 MHz, DMSO-$d_6$) δ [ppm]: -0.149 (0.68), -0.059 (5.17), -0.008 (4.04), 0.008 (3.41), 0.146 (0.41), 0.318 (1.61), 0.328 (2.49), 0.340 (2.44), 0.352 (1.95), 0.364 (0.96), 0.512 (1.71), 0.523 (2.54), 0.535 (2.29), 0.547 (2.57), 0.555 (1.96), 0.566 (2.64), 0.576 (2.19), 0.586 (1.99), 0.597 (1.62), 0.611 (1.01), 0.625 (1.41), 0.636 (1.41), 0.646 (2.39), 0.656 (2.07), 0.662 (2.01), 0.670 (2.00), 0.678 (1.03), 0.690 (0.76), 0.834 (7.90), 0.849 (7.89), 1.166 (0.69), 1.178 (1.23), 1.186 (1.66), 1.198 (2.72), 1.207 (1.98), 1.219 (2.71), 1.231 (1.99), 1.251 (0.59), 2.074 (0.65), 2.329 (0.46), 2.671 (0.42), 3.160 (4.79), 3.460 (2.29), 3.490 (1.88), 3.705 (1.53), 4.354 (1.31), 4.375 (2.27), 4.396 (2.20), 4.417 (1.17), 4.738 (5.75), 4.750 (5.64), 5.755 (2.37), 7.561 (3.16), 7.582 (5.63), 7.603 (3.10), 7.989 (8.41), 8.023 (8.17), 8.841 (16.00), 10.441 (5.22), 10.464 (5.04).

## Beispiel 65

N-[(1S)-1-Cyclopropyl-2,2,2-trifluorethyl]-7-[ethyl(2-hydroxypropyl)amino]-6-fluor-4-oxo-1-(2,4,6-trifluorphenyl)-1,4-dihydro-1,8-naphthyridin-3-carboxamid (Diastereomerengemisch)

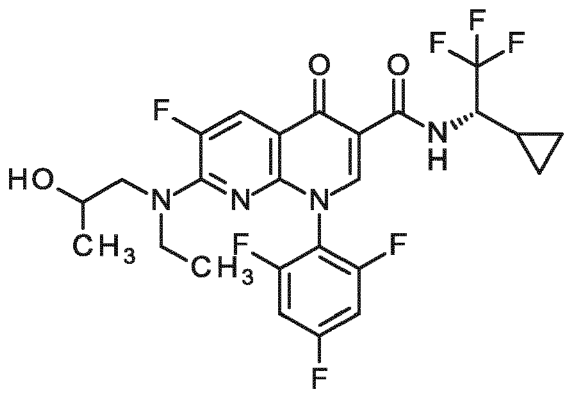

7-Chlor-N-[(1S)-1-cyclopropyl-2,2,2-trifluorethyl]-6-fluor-4-oxo-1-(2,4,6-trifluorphenyl)-1,4-dihydro-1,8-naphthyridin-3-carboxamid (80.0 mg, 162 µmol) wurde in 1.6 ml Acetonitril vorgelegt, mit 1-(Ethylamino)propan-2-ol (33.4 mg, 324 µmol; Racemat) und N,N-Diisopropylethylamin (99 µl, 570 µmol) versetzt und 2 h bei Raumtemperatur gerührt. Die Reaktionslösung wurde dann im Vakuum eingeengt. Der Rückstand wurde in Ethylacetat aufgenommen und mit

Wasser versetzt. Die wässrige Phase wurde mit 1 M Salzsäure sauer gestellt und zweimal extrahiert. Die organische Phase wurde einmal mit gesättigter, wässriger Natriumhydrogencarbonat-Lösung ausgeschüttelt, einmal mit gesättigter, wässriger Natriumchlorid- Lösung gewaschen, über Natriumsulfat getrocknet, filtriert und im Vakuum eingeengt. Es wurden 76 mg der Zielverbindung (82% d. Th.) erhalten.
LC-MS (Methode 3): $R_t$ = 2.23 min; MS (ESIpos): m/z = 561 $[M+H]^+$
$^1$H-NMR (400 MHz, DMSO-$d_6$) $\delta$ [ppm]: -0.149 (0.87), -0.008 (7.43), 0.008 (7.19), 0.146 (0.84), 0.329 (2.28), 0.342 (2.25), 0.514 (1.80), 0.525 (2.61), 0.549 (2.52), 0.568 (2.61), 0.577 (2.19), 0.588 (2.07), 0.626 (1.32), 0.647 (2.43), 0.851 (6.17), 1.013 (5.51), 1.157 (1.05), 1.175 (2.52), 1.185 (1.74), 1.197 (2.94), 1.206 (2.22), 1.217 (2.82), 1.238 (1.89), 1.988 (3.09), 2.328 (1.86), 2.367 (0.93), 2.670 (1.86), 2.711 (1.05), 3.061 (0.93), 3.418 (2.19), 3.455 (2.55), 3.575 (1.14), 3.710 (1.59), 4.021 (0.84), 4.039 (0.81), 4.350 (1.20), 4.370 (2.22), 4.391 (2.22), 4.412 (1.17), 4.736 (4.04), 4.748 (3.96), 7.566 (3.06), 7.588 (5.51), 7.607 (3.12), 7.996 (8.21), 8.031 (8.03), 8.843 (16.00), 10.439 (5.21), 10.463 (5.00).

**Beispiel 66**

N-[(1S)-1-Cyclopropyl-2,2,2-trifluorethyl]-7-[ethyl(2-hydroxypropyl)amino]-6-fluor-4-oxo-1-(2,4,6-trifluorphenyl)-1,4-dihydro-1,8-naphthyridin-3-carboxamid (Diastereomer 1)

69 mg N-[(1S)-1-Cyclopropyl-2,2,2-trifluorethyl]-7-[ethyl(2-hydroxypropyl)amino]-6-fluor-4-oxo-1-(2,4,6-trifluorphenyl)-1,4-dihydro-1,8-naphthyridin-3-carboxamid (Diastereomerengemisch) wurde durch chirale HPLC in die Diastereomere getrennt (präparative HPLC: Säule Daicel® Chiralpak AD-H, 5 μm, 250 x 20 mm; Eluent: 80% n-Heptan / 20% Isopropanol; Fluss 15 ml/min; Temperatur: 25°C, Detektion: 210 nm).
Diastereomer 1: 30 mg (>99% de)
$R_t$ = 1.37 min [analytische HPLC:Säule Daicel® Chiralpak AD, 1ml/min; 3 μm, 50 x 4.6 mm; Eluent: 80% iso-Hexan/ 20% Isopropanol; Detektion: 220 nm].
LC-MS (Methode 3): $R_t$ = 2.25 min; MS (ESIpos): m/z = 561 $[M+H]^+$
$^1$H-NMR (400 MHz, DMSO-$d_6$) $\delta$ [ppm]: -0.008 (1.84), 0.008 (1.52), 0.321 (1.83), 0.331 (2.88), 0.343 (2.85), 0.355 (2.22), 0.367 (1.10), 0.503 (0.76), 0.515 (1.97), 0.526 (2.98), 0.539 (2.67), 0.550 (2.97), 0.557 (2.12), 0.568 (3.14), 0.578 (2.48), 0.589 (2.26), 0.599 (1.84), 0.613 (1.18), 0.627 (1.69), 0.637 (1.61), 0.648 (2.73), 0.659 (2.37), 0.664 (2.19), 0.670 (2.15), 0.680 (1.11), 0.684 (1.10), 0.692 (0.80), 0.852 (6.50), 0.863 (6.50), 1.012 (5.79), 1.165 (0.66), 1.177 (1.26), 1.185 (1.75), 1.198 (2.95), 1.206 (2.09), 1.218 (2.89), 1.230 (1.55), 1.238 (1.05), 1.250 (0.45), 2.328 (0.82), 2.333 (0.60), 2.367 (0.56), 2.519 (3.02), 2.524 (2.34), 2.666 (0.58), 2.670 (0.80), 2.675 (0.58), 2.710 (0.51), 3.075 (0.94), 3.419 (2.44), 3.454 (2.73), 3.578 (1.05), 3.708 (1.64), 4.347 (1.48), 4.368 (2.54), 4.389 (2.50), 4.410 (1.34), 4.735 (4.60), 4.747 (4.46), 7.566 (3.23), 7.587 (5.73), 7.606 (3.23), 7.996 (9.12), 8.031 (8.91), 8.843 (16.00), 10.440 (6.11), 10.463 (5.86).

**Beispiel 67**

N-[(1S)-1-Cyclopropyl-2,2,2-trifluorethyl]-7-[ethyl(2-hydroxypropyl)amino]-6-fluor-4-oxo-1-(2,4,6-trifluophenyl)-1,4-dihydro-1,8-naphthyridin-3-carboxamid (Diastereomer 2)

69 mg N-[(1S)-1-Cyclopropyl-2,2,2-trifluorethyl]-7-[ethyl(2-hydroxypropyl)amino]-6-fluor-4-oxo-1-(2,4,6-trifluorphenyl)-1,4-dihydro-1,8-naphthyridin-3-carboxamid (Diastereomerengemisch) wurde durch chirale HPLC in die Diastereomere getrennt (präparative HPLC: Säule Daicel® Chiralpak AD-H, 5 μm, 250 x 20 mm; Eluent: 80% n-Heptan / 20% Isopropanol; Fluss 15 ml/min; Temperatur: 25°C, Detektion: 210 nm).
Diastereomer 2: 30 mg (>99% de)
$R_t$ = 2.31 min [analytische HPLC:Säule Daicel® Chiralpak AD, 1ml/min; 3 μm, 50 x 4.6 mm; Eluent: 80% iso-Hexan/ 20% Isopropanol; Detektion: 220 nm].

LC-MS (Methode 3): $R_t$ = 2.25 min; MS (ESIpos): m/z = 561 $[M+H]^+$
$^1$H-NMR (400 MHz, DMSO-$d_6$) $\delta$ [ppm]: 0.318 (1.80), 0.328 (2.83), 0.340 (2.58), 0.514 (1.98), 0.525 (3.01), 0.537 (2.55), 0.548 (2.78), 0.567 (2.79), 0.576 (2.35), 0.588 (2.12), 0.626 (1.52), 0.647 (2.48), 0.657 (2.17), 0.851 (6.26), 1.013 (5.57), 1.177 (1.33), 1.185 (1.71), 1.198 (2.72), 1.218 (2.67), 1.230 (1.43), 2.328 (1.15), 2.671 (1.04), 3.063 (1.04), 3.420 (2.39), 3.453 (2.67), 3.585 (1.11), 3.711 (1.63), 4.350 (1.49), 4.371 (2.39), 4.393 (2.19), 4.413 (1.31), 4.737 (4.38), 4.749 (4.05), 7.567 (3.32), 7.588 (5.50), 7.607 (2.95), 7.997 (7.99), 8.032 (7.81), 8.844 (16.00), 10.440 (5.11), 10.463 (5.00).

**Beispiel 68**

1-(2-Chlor-4,6-difluorphenyl)-N-[(1S)-1-cyclopropyl-2,2,2-trifluorethyl]-6-fluor-7-(2-oxa-6-azaspiro[3.3]hept-6-yl)-4-oxo-1,4-dihydro-1,8-naphthyridin-3-carboxamid Trifluoracetat (Atropisomerengemisch)

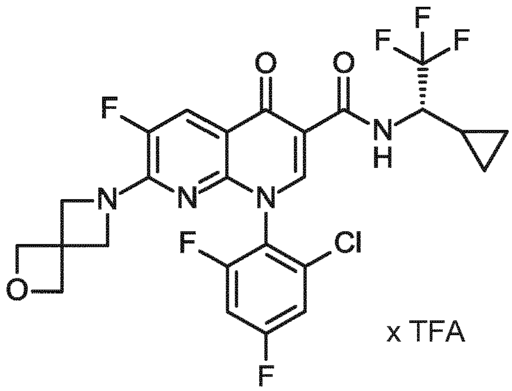

x TFA

7-Chlor-1-(2-chlor-4,6-difluorphenyl)-N-[(1S)-1-cyclopropyl-2,2,2-trifluorethyl]-6-fluor-4-oxo-1,4-dihydro-1,8-naphthyridin-3-carboxamid (Atropisomerengemisch, Reinheit 57%, 90.0 mg, 176 μmol) wurde in 1.7 ml DMF vorgelegt, mit Ethandisäure-2-oxa-6-azaspiro[3.3]heptan (1:1) (46.7 mg, 247 μmol) und N,N-Diisopropylethylamin (150 μl, 880 μmol) versetzt und über Nacht bei Raumtemperatur gerührt. Die Reaktionslösung wurde mit Acetonitril/Wasser versetzt und mittels präparativer HPLC gereinigt (RP18 Säule, Laufmittel: Acetonitril/Wasser-Gradient unter Zusatz von 0.1% TFA). (Fraktion 1). Die Produktfraktionen wurden vereinigt und im Vakuum eingeengt. Der Rückstand wurde in Dichlormethan aufgenommen und zweimal mit gesättigter, wässriger Natriumhydrogencarbonat-Lösung extrahiert. Die vereinigten wässrigen Phasen wurden einmal mit Dichlormethan reextrahiert. Die vereinigten organischen Phasen wurden über Natriumsulfat getrocknet, filtriert und im Vakuum eingeengt. Es wurden 74 mg der Zielverbindung (60% d. Th., Reinheit 98%) erhalten.
LC-MS (Methode 3): $R_t$ = 2.28 min; MS (ESIpos): m/z = 573 $[M-TFA+H]^+$
$^1$H-NMR (500 MHz, $DMSO-d_6$) δ [ppm]: 0.006 (0.44), 0.313 (0.41), 0.322 (0.73), 0.331 (0.87), 0.341 (0.77), 0.350 (0.49), 0.524 (0.72), 0.536 (0.69), 0.544 (0.68), 0.552 (0.50), 0.561 (0.59), 0.571 (0.77), 0.580 (0.71), 0.587 (0.60), 0.597 (0.45), 0.635 (0.46), 0.644 (0.64), 0.652 (0.79), 0.663 (0.77), 0.672 (0.54), 1.188 (0.50), 1.197 (0.83), 1.205 (0.69), 1.213 (0.78), 1.222 (0.48), 2.073 (6.12), 2.519 (0.49), 4.339 (0.49), 4.355 (0.83), 4.372 (0.98), 4.388 (0.77), 4.649 (16.00), 7.680 (0.58), 7.686 (0.77), 7.699 (1.02), 7.704 (1.35), 7.718 (0.63), 7.724 (1.34), 7.745 (1.11), 7.999 (2.73), 8.022 (2.68), 8.777 (6.49), 10.453 (1.61), 10.471 (1.53).

**Beispiel 69**

7-[3,3-Bis(hydroxymethyl)azetidin-1-yl]-1-(2-chlor-4,6-difluorphenyl)-N-[(1S)-1-cyclopropyl-2,2,2-trifluorethyl]-6-fluor-4-oxo-1,4-dihydro-1,8-naphthyridin-3-carboxamid (Atropisomerengemisch)

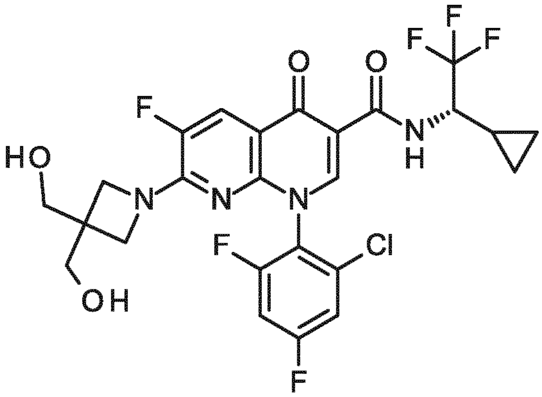

1-(2-Chlor-4,6-difluorphenyl)-N-[(1S)-1-cyclopropyl-2,2,2-trifluorethyl]-6-fluor-7-(2-oxa-6-azaspiro-[3.3]hept-6-yl)-4-oxo-1,4-dihydro-1,8-naphthyridin-3-carboxamid Trifluoracetat (Atropisomerengemisch, 70.0 mg, 102 μmol) wurde in Trifluoressigsäure (640 μl, 8.3 mmol) vorgelegt, mit 640 μl Wasser und 0.2 ml Acetonitril versetzt und für 4 Tage bei Raumtemperatur gerührt. Die Reaktionslösung wurde mittels präparativer HPLC gereinigt (RP18 Säule, Laufmittel: Acetonitril/Wasser-Gradient unter Zusatz von 0.1% TFA). Die Produktfraktionen wurden im Vakuum eingeengt, der Rückstand wurde in Dichlormethan aufgenommen und zweimal mit gesättigter, wässriger Natriumhydrogencarbonatlösung gewaschen. Die vereinigten wässrigen Phasen wurde einmal mit Dichlormethan reextrahiert. Die vereinigten organischen Phasen wurden über Natriumsulfat getrocknet, filtriert und im Vakuum eingeengt. Es wurden 60 mg der Zielverbindung (98% d. Th., Reinheit 98%) erhalten.
LC-MS (Methode 3): $R_t$ = 1.91 min; MS (ESIpos): m/z = 591 $[M+H]^+$
$^1$H-NMR (400 MHz, DMSO-$d_6$) δ [ppm]: -0.149 (0.88), -0.008 (7.82), 0.008 (6.52), 0.146 (0.86), 0.312 (0.99), 0.323 (1.77), 0.334 (2.13), 0.346 (1.93), 0.358 (1.22), 0.523 (1.69), 0.538 (1.80), 0.561 (1.93), 0.572 (2.02), 0.583 (1.74), 0.593 (1.49), 0.606 (1.08), 0.621 (0.83), 0.631 (1.13), 0.641 (1.55), 0.652 (1.82), 0.667 (1.60), 0.675 (1.19), 0.687 (0.69), 1.162 (0.44), 1.175 (0.80), 1.183 (1.16), 1.195 (1.99), 1.205 (1.55), 1.215 (1.96), 1.227 (1.22), 1.235 (1.13), 2.073 (0.77), 2.328 (1.16), 2.366 (0.80), 2.523 (4.37), 2.670 (1.30), 2.710 (0.94), 3.465 (15.72), 3.479 (16.00), 4.118 (0.91), 4.333 (0.66), 4.353 (1.33), 4.373 (1.77), 4.392 (1.35), 4.411 (0.64), 4.831 (5.11), 4.844 (12.10), 4.858 (5.06), 7.667 (1.27), 7.673 (1.82), 7.690 (2.10), 7.697 (3.45), 7.713 (3.43), 7.720 (3.87), 7.731 (2.57), 7.742 (1.71), 7.967 (6.38), 7.996 (6.33), 8.758 (14.51), 10.481 (4.03), 10.505 (3.90).

**Beispiel 70**

7-[3,3-Bis(hydroxymethyl)azetidin-1-yl]-1-(2-chlor-4,6-difluorphenyl)-N-[(1S)-1-cyclopropyl-2,2,2-trifluorethyl]-6-fluor-4-oxo-1,4-dihydro-1,8-naphthyridin-3-carboxamid (Atropisomer 1)

55 mg 7-[3,3-Bis(hydroxymethyl)azetidin-1-yl]-1-(2-chlor-4,6-difluorphenyl)-N-[(1S)-1-cyclopropyl-2,2,2-trifluorethyl]-6-fluor-4-oxo-1,4-dihydro-1,8-naphthyridin-3-carboxamid (Atropisomerengemisch) wurde durch chirale HPLC in die Diastereomere getrennt (präparative HPLC: Säule Daicel® Chiralpak OX-H, 5 μm, 250 x 20 mm; Eluent: 80% n-Heptan/ 20% Ethanol + 0.2% Diethylamin; Fluss 20 ml/min; Temperatur: 23°C, Detektion: 220 nm). Die Produktfraktionen wurden bei 30°C eingedampft.
Atropisomer 1: 22 mg (>99% stereochemische Reinheit)
$R_t$ = 4.16 min [analytische HPLC:Säule Daicel® Chiralpak OX, 1ml/min; 3 μm, 50 x 4.6 mm; Eluent: 90% n-Hexan/ 20% Ethanol + 0.2% Diethylamin; Detektion: 220 nm].
LC-MS (Methode 3): $R_t$ = 1.90 min; MS (ESIpos): m/z = 591 $[M+H]^+$
$^1$H-NMR (400 MHz, DMSO-$d_6$) δ [ppm]: -0.149 (0.82), -0.008 (7.57), 0.008 (6.50), 0.146 (0.85), 0.312 (1.26), 0.322 (2.17), 0.335 (2.11), 0.346 (1.65), 0.358 (0.85), 0.508 (1.45), 0.519 (2.22), 0.531 (1.98), 0.543 (2.14), 0.561 (2.22), 0.571 (1.81), 0.582 (1.59), 0.593 (1.32), 0.606 (0.80), 0.621 (1.15), 0.631 (1.15), 0.642 (1.95), 0.652 (1.73), 0.664 (1.54), 1.099 (0.82), 1.118 (1.51), 1.135 (0.71), 1.182 (1.26), 1.194 (2.14), 1.202 (1.56), 1.214 (2.14), 1.226 (1.21), 1.234 (1.13), 2.327 (1.56), 2.366 (0.91), 2.523 (4.89), 2.670 (1.54), 2.710 (0.91), 2.820 (0.41), 3.465 (15.78), 3.479 (16.00), 4.131 (0.93), 4.348 (1.13), 4.370 (1.92), 4.392 (1.84), 4.411 (0.99), 4.831 (5.19), 4.844 (12.24), 4.857 (5.10), 7.666 (1.29), 7.673 (1.92), 7.690 (2.17), 7.697 (3.49), 7.713 (3.60), 7.720 (3.95), 7.731 (2.66), 7.967 (7.27), 7.996 (7.03), 8.758 (15.86), 10.482 (4.56), 10.506 (4.42).

**Beispiel 71**

7-[3,3-Bis(hydroxymethyl)azetidin-1-yl]-1-(2-chlor-4,6-difluorphenyl)-N-[(1S)-1-cyclopropyl-2,2,2-trifluorethyl]-6-fluor-4-oxo-1,4-dihydro-1,8-naphthyridin-3-carboxamid (Atropisomer 2)

55 mg 7-[3,3-Bis(hydroxymethyl)azetidin-1-yl]-1-(2-chlor-4,6-difluorphenyl)-N-[(1S)-1-cyclopropyl-2,2,2-trifluorethyl]-6-fluor-4-oxo-1,4-dihydro-1,8-naphthyridin-3-carboxamid (Atropisomerengemisch) wurde durch chirale HPLC in die Diastereomere getrennt (präparative HPLC: Säule Daicel® Chiralpak OX-H, 5 μm, 250 x 20 mm; Eluent: 80% n-Heptan/ 20% Ethanol + 0.2% Diethylamin; Fluss 20 ml/min; Temperatur: 23°C, Detektion: 220 nm). Die Produktfraktionen wurden bei 30°C eingedampft.
Atropisomer 2: 22 mg (>98.5% stereochemische Reinheit)
$R_t$ = 6.25 min [analytische HPLC:Säule Daicel® Chiralpak OX, 1ml/min; 3 μm, 50 x 4.6 mm; Eluent: 90% n-Hexan/ 20% Ethanol + 0.2% Diethylamin; Detektion: 220 nm].
LC-MS (Methode 3): $R_t$ = 1.90 min; MS (ESIpos): m/z = 591 $[M+H]^+$
$^1$H-NMR (400 MHz, DMSO-$d_6$) δ [ppm]: -0.149 (0.93), -0.008 (8.98), 0.008 (6.74), 0.146 (0.86), 0.323 (1.38), 0.333 (2.14), 0.346 (2.11), 0.359 (1.59), 0.370 (0.79), 0.513 (1.52), 0.525 (2.14), 0.538 (1.90), 0.552 (1.76), 0.560 (1.66), 0.572 (2.11), 0.582 (1.80), 0.593 (1.66), 0.604 (1.35), 0.616 (0.90), 0.630 (1.00), 0.640 (1.17), 0.651 (1.83), 0.661 (1.73), 0.675 (1.62), 0.696 (0.55), 1.101 (1.42), 1.119 (2.80), 1.137 (1.35), 1.175 (0.90), 1.183 (1.31), 1.196 (2.18), 1.205 (1.56), 1.216 (2.11), 1.228 (1.24), 2.323 (1.42), 2.327 (1.83), 2.366 (1.52), 2.523 (5.46), 2.670 (1.73), 2.710 (1.35), 2.825 (0.59), 2.843 (0.59), 3.465 (15.86), 3.479 (16.00), 4.127 (0.90), 4.333 (1.14), 4.354 (1.90), 4.374 (1.87), 4.395 (1.00), 4.830 (5.36), 4.844 (12.75), 4.857 (5.22), 7.667 (1.24), 7.673 (1.90), 7.690 (2.14), 7.697 (3.46), 7.713 (3.52), 7.720 (3.84), 7.732 (2.63), 7.967 (7.02), 7.995 (6.88), 8.758 (15.14), 10.480 (4.53), 10.503 (4.32).

**Beispiel 72**

1-(2-Chlor-4,6-difluophenyl)-N-[(1S)-1-cyclopropyl-2,2,2-trifluorethyl]-6-fluor-7-{[(2S)-2-hydroxypropyl](methyl)amino}-4-oxo-1,4-dihydro-1,8-naphthyridin-3-carboxamid (Atropisomerengemisch)

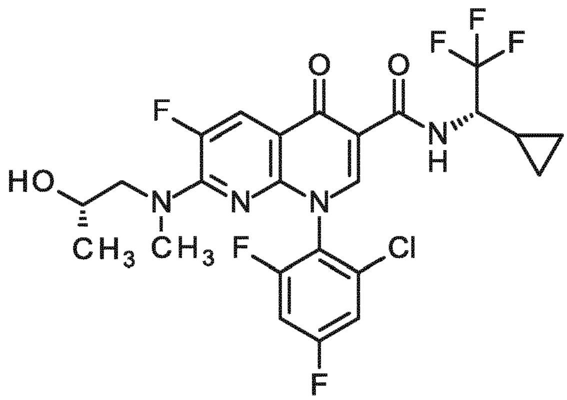

7-Chlor-1-(2-chlor-4,6-difluorphenyl)-N-[(1S)-1-cyclopropyl-2,2,2-trifluorethyl]-6-fluor-4-oxo-1,4-dihydro-1,8-naphthyridin-3-carboxamid (Atropisomerengemisch, Reinheit 57%, 90.0 mg, 176 μmol) wurde in 1.8 ml DMF vorgelegt, mit (2S)-1-(Methylamino)propan-2-ol (31.4 mg, 353 μmol) und N,N-Diisopropylethylamin (110 μl, 620 μmol) versetzt und über Nacht bei Raumtemperatur gerührt. Die Reaktionslösung wurde auf Wasser gegeben, der entstandene Feststoff wurde ca. 30 min verrührt, anschließend abfitlriert, mit Wasser nachgewaschen und im Hochvakuum getrocknet. Der Rückstand wurde mittels Dickschichtchromatographie gereinigt (Laufmittel: Cyclohexan/Ethylacetat = 2/1). Es wurden 31 mg der Zielverbindung (31% d. Th., Reinheit 98%) erhalten.
LC-MS (Methode 3): $R_t$ = 2.20 min; MS (ESIpos): m/z = 563 $[M+H]^+$
$^1$H-NMR (400 MHz, DMSO-$d_6$) δ [ppm]: -0.149 (0.69), -0.008 (7.05), 0.008 (5.36), 0.146 (0.72), 0.314 (1.84), 0.325 (3.49), 0.336 (4.05), 0.348 (3.84), 0.360 (2.56), 0.525 (3.62), 0.545 (3.56), 0.552 (3.49), 0.563 (3.49), 0.573 (4.02), 0.584 (3.59), 0.593 (3.12), 0.607 (2.03), 0.623 (1.53), 0.632 (2.09), 0.642 (2.90), 0.653 (3.59), 0.667 (3.34), 0.676 (2.68), 0.807 (11.54), 0.822 (7.64), 1.165 (0.78), 1.178 (1.59), 1.185 (2.34), 1.198 (4.02), 1.207 (2.99), 1.218 (3.84), 1.230 (2.12), 1.238 (1.43), 1.250 (0.65), 2.073 (0.53), 2.328 (1.72), 2.366 (1.03), 2.524 (4.96), 2.670 (1.81), 2.710 (1.06), 3.011 (1.40), 3.175 (6.49), 3.422 (2.50), 3.447 (2.50), 3.470 (1.72), 3.681 (2.50), 4.339 (1.31), 4.359 (2.68), 4.378 (3.27), 4.397 (2.46),

4.418 (1.09), 4.723 (5.02), 4.730 (6.80), 4.735 (5.99), 4.743 (5.68), 7.690 (1.28), 7.697 (2.00), 7.708 (2.56), 7.713 (2.50), 7.721 (4.05), 7.736 (6.83), 7.743 (5.74), 7.757 (6.11), 7.995 (13.35), 8.029 (13.13), 8.791 (14.07), 8.795 (16.00), 10.460 (7.42), 10.484 (7.14).

**Beispiel 73**

1-(2-Chlor-4,6-difluorphenyl)-7-[(3R,4R)-3,4-dihydroxypyrrolidin-1-yl]-6-fluor-N-(2-methylbutan-2-yl)-4-oxo-1,4-dihydro-1,8-naphthyridin-3-carboxamid (Atropisomerengemisch)

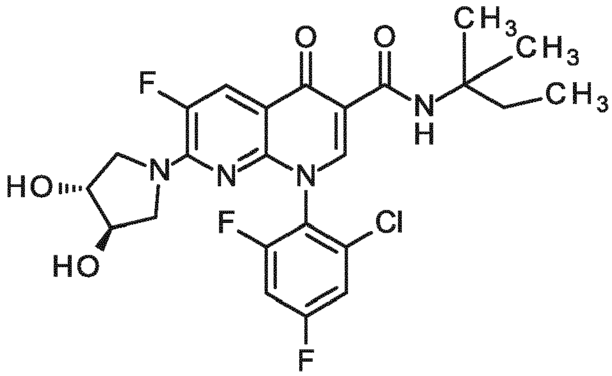

1-(2-Chlor-4,6-difluorphenyl)-7-[(3R,4R)-3,4-dihydroxypyrrolidin-1-yl]-6-fluor-4-oxo-1,4-dihydro-1,8-naphthyridin-3-carbonsäure (80.0 mg, 176 μmol) wurde in 1.2 ml DMF vorgelegt, mit HATU (80.1 mg, 211 μmol) und N,N-Diisopropylethylamin (110 μl, 610 μmol) versetzt und 30 min bei Raumtemperatur gerührt. 2-Methylbutan-2-amin (18.4 mg, 211 μmol) wurde hinzugeben und über Nacht bei Raumtemperatur rühren gelassen. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt und der Rückstand wurde zweimal mit Dichlormethan extrahiert. Die vereinigten organischen Phasen wurden zweimal mit gesättigter, wässriger Natriumhydrogencarbonatlösung gewaschen. Die vereinigten wässrigen Phasen wurden mit Dichlormethan reextrahiert. Die vereinigten organischen Phasen wurden über Natriumsulfat getrocknet, filtriert und im Vakuum eingeengt. Es wurden 58 mg der Zielverbindung (62% d. Th., Reinheit 99%) erhalten.
LC-MS (Methode 3): $R_t$ = 1.82 min; MS (ESIpos): m/z = 525 $[M+H]^+$

**Beispiel 74**

N-tert-Butyl-1-(2-chlor-4,6-difluorphenyl)-7-[(3R,4R)-3,4-dihydroxypyrrolidin-1-yl]-6-fluor-4-oxo-1,4-dihydro-1,8-naphthyridin-3-carboxamid (Atropisomerengemisch)

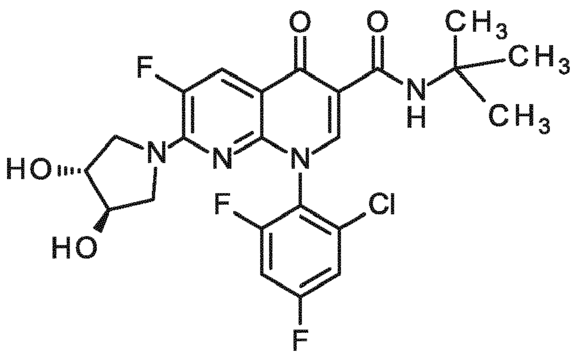

1-(2-Chlor-4,6-difluorphenyl)-7-[(3R,4R)-3,4-dihydroxypyrrolidin-1-yl]-6-fluor-4-oxo-1,4-dihydro-1,8-naphthyridin-3-carbonsäure (100 mg, 219 μmol) wurde in 3.1 ml DMF vorgelegt, mit HATU (100 mg, 263 μmol) und N,N-Diiso-

propylethylamin (130 µl, 770 µmol) versetzt und 30 min bei Raumtemperatur gerührt. 2-Methylpropan-2-amin (19.3 mg, 263 µmol) wurde hinzugeben und über Nacht bei Raumtemperatur rühren gelassen. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt und der Rückstand wurde zweimal mit Dichlormethan extrahiert. Die vereinigten organischen Phasen wurden zweimal mit gesättigter, wässriger Natriumhydrogencarbonatlösung gewaschen. Die vereinigten wässrigen Phasen wurden mit Dichlormethan reextrahiert. Die vereinigten organischen Phasen wurden über Natriumsulfat getrocknet, filtriert und im Vakuum eingeengt. Es wurden 86 mg der Zielverbindung (76% d. Th., Reinheit 99%) erhalten.
LC-MS (Methode 3): $R_t$ = 1.71 min; MS (ESIpos): m/z = 511 $[M+H]^+$

**Beispiel 75**

1-(3,5-Difluorpyridin-2-yl)-7-[(3R,4R)-3,4-dihydroxypyrrolidin-1-yl]-6-fluor-N-(2-methylbutan-2-yl)-4-oxo-1,4-dihydro-1,8-naphthyridin-3-carboxamid

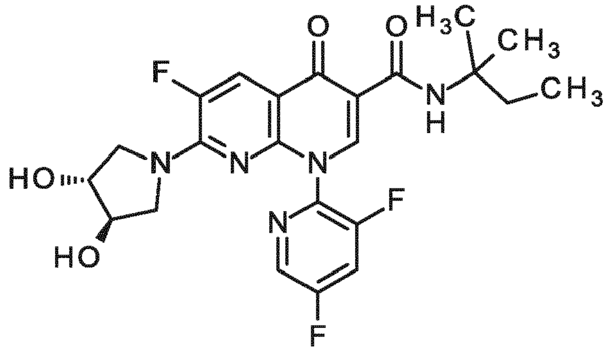

1-(3,5-Difluorpyridin-2-yl)-7-[(3R,4R)-3,4-dihydroxypyrrolidin-1-yl]-6-fluor-4-oxo-1,4-dihydro-1,8-naphthyridin-3-carbonsäure (57.0 mg, 135 µmol) wurde in 1.4 ml DMF vorgelegt, mit HATU (61.6 mg, 162 µmol) und N,N-Diisopropylethylamin (94 µl, 540 µmol) versetzt und 10 min bei Raumtemperatur gerührt. Das Reaktionsgemisch wurde mit 2-Methylbutan-2-amin (24 µl, 200 µmol) versetzt und 2 h bei Raumtemperatur gerührt Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt, der Rückstand wurde mit gesättigter, wässriger Natriumhydrogencarboantlösung basisch gestellt und zweimal mit Dichlormethan extrahiert. Die vereinigten organischen Phasen wurden über Natriumsulfat getrocknet, filtriert und im Vakuum eingeengt. Es wurden 55 mg der Zielverbindung (82% d. Th., Reinheit 98%) erhalten.
LC-MS (Methode 3): $R_t$ = 1.63 min; MS (ESIpos): m/z = 492 $[M+H]^+$

**Beispiel 76**

1-(3,5-Difluorpyridin-2-yl)-7-[(3R,4R)-3,4-dihydroxypyrrolidin-1-yl]-6-fluor-N-(3-methylpentan-3-yl)-4-oxo-1,4-dihydro-1,8-naphthyridin-3-carboxamid

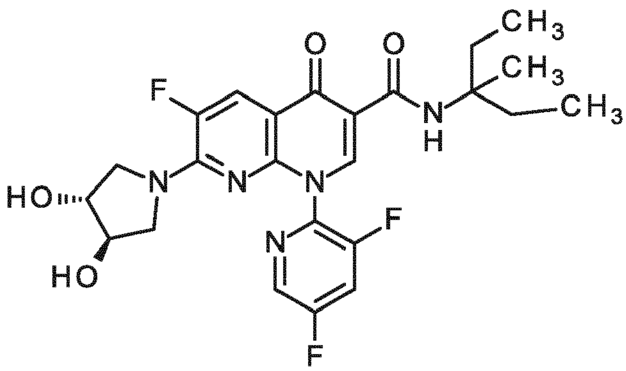

1-(3,5-Difluorpyridin-2-yl)-7-[(3R,4R)-3,4-dihydroxypyrrolidin-1-yl]-6-fluor-4-oxo-1,4-dihydro-1,8-naphthyridin-3-carbonsäure (57.0 mg, 135 µmol) wurde in 1.4 ml DMF vorgelegt, mit HATU (61.6 mg, 162 µmol) und N,N-Diisopropylethylamin (140 µl, 810 µmol) versetzt und 10 min bei Raumtemperatur gerührt. Das Reaktionsgemisch wurde mit 3-Methylpentan-3-amin-Hydrochlorid (27.9 mg, 202 µmol) versetzt und 2 h bei Raumtemperatur gerührt. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt, der Rückstand wurde mit gesättigter, wässriger Natriumhydrogencarboantlösung basisch gestellt und zweimal mit Dichlormethan extrahiert. Die vereinigten organischen Phasen wurden über Natriumsulfat getrocknet, filtriert und im Vakuum eingeengt. Es wurden 51 mg der Zielverbindung (74% d. Th., Reinheit 98%) erhalten.
LC-MS (Methode 3): $R_t$ = 1.74 min; MS (ESIpos): m/z = 506 $[M+H]^+$

## Beispiel 77

7-[(3R,4S)-3,4-Dihydroxypyrrolidin-1-yl]-N-(3-ethylpentan-3-yl)-6-fluor-4-oxo-1-(2,4,6-trifluorphenyl)-1,4-dihydro-1,8-naphthyridin-3-carboxamid

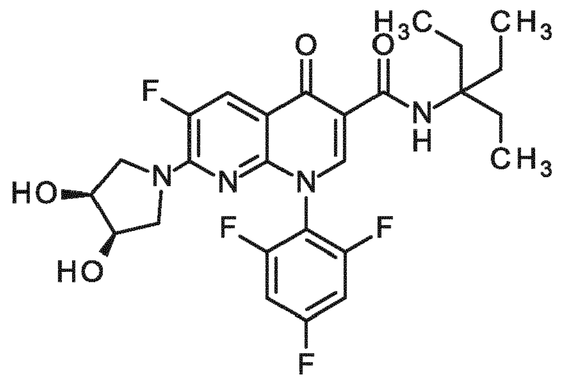

7-[(3R,4S)-3,4-Dihydroxypyrrolidin-1-yl]-6-fluor-4-oxo-1-(2,4,6-trifluorphenyl)-1,4-dihydro-1,8-naphthyridin-3-carbonsäure (75.0 mg, 81 % Reinheit, 138 µmol) wurde in 1.9 ml DMF vorgelegt, mit 3-Ethylpentan-3-amin (19.1 mg, 166 µmol), N,N-Diisopropylethylamin (84 µl, 480 µmol) und HATU (63.1 mg, 166 µmol) versetzt und für 4 h bei Raumtemperatur gerührt. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt, der wässrige Rückstand wurde mit gesättigter, wässriger Natriumhydrogencarbonatlösung basisch gestellt und zweimal mit Dichlormethan extrahiert. Die vereinigten organischen Phasen wurden über Natriumsulfat getrocknet, filtriert und im Vakuum eingeengt. Es wurden 59 mg der Zielverbindung (78% d. Th., Reinheit 98%) erhalten.
LC-MS (Methode 5): $R_t$ = 1.41 min; MS (ESIpos): m/z = 537 $[M+H]^+$
$^1$H-NMR (400 MHz, DMSO-$d_6$) [ppm]: -0.008 (1.80), 0.008 (1.47), 0.768 (6.83), 0.786 (16.00), 0.805 (7.41), 1.699 (1.98), 1.718 (6.06), 1.736 (5.80), 1.755 (1.82), 2.328 (0.52), 2.366 (0.40), 2.670 (0.59), 4.030 (0.92), 4.991 (0.91), 7.545 (1.37), 7.567 (2.66), 7.589 (1.38), 7.979 (2.75), 8.011 (2.73), 8.667 (4.92), 9.612 (2.83).

**Beispiel 78**

7-[(3R,4S)-3,4-Dihydroxypyrrolidin-1-yl]-6-fluor-N-(3-methylpentan-3-yl)-4-oxo-1-(2,4,6-trifluophenyl)-1,4-dihydro-1,8-naphthyridin-3-carboxamid

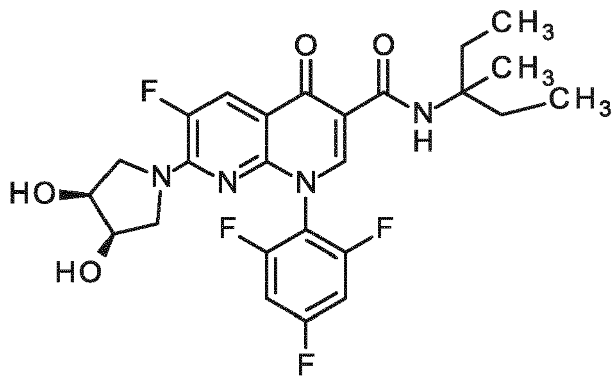

7-[(3R,4S)-3,4-Dihydroxypyrrolidin-1-yl]-6-fluor-4-oxo-1-(2,4,6-trifluorphenyl)-1,4-dihydro-1,8-naphthyridin-3-carbonsäure (75.0 mg, 81 % Reinheit, 138 μmol) wurde in 1.9 ml DMF vorgelegt, mit 3-Methylpentan-3-amin-Hydrochlorid (22.8 mg, 166 μmol), N,N-Diisopropylethylamin (84 μl, 480 μmol) und HATU (63.1 mg, 166 μmol) versetzt und für 4 h bei Raumtemperatur rühren gelassen. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt, der wässrige Rückstand wurde mit gesättigter, wässriger Natriumhydrogencarbonatlösung basisch gestellt und zweimal mit Dichlormethan extrahiert. Die vereinigten organischen Phasen wurden über Natriumsulfat getrocknet, filtriert und im Vakuum eingeengt. Es wurden 63 mg der Zielverbindung (85% d. Th., Reinheit 98%) erhalten.
LC-MS (Methode 5): $R_t$ = 1.35 min; MS (ESIpos): m/z = 523 $[M+H]^+$
$^1$H-NMR (400 MHz, DMSO-$d_6$) δ [ppm]: -0.008 (1.46), 0.008 (1.29), 0.814 (6.64), 0.833 (15.52), 0.851 (7.27), 1.234 (0.50), 1.279 (16.00), 1.613 (0.42), 1.631 (1.48), 1.649 (1.89), 1.665 (2.44), 1.684 (1.96), 1.703 (0.49), 1.769 (0.57), 1.787 (2.03), 1.806 (2.32), 1.822 (1.86), 1.841 (1.35), 2.073 (8.37), 2.328 (0.49), 2.523 (1.67), 2.670 (0.52), 4.031 (1.20), 4.989 (1.19), 7.546 (1.83), 7.568 (3.46), 7.590 (1.84), 7.977 (3.61), 8.008 (3.53), 8.669 (6.19), 9.728 (3.98).

**Beispiel 79**

7-[(3R,4S)-3,4-Dihydroxypyrrolidin-1-yl]-6-fluor-N-(2-methylbutan-2-yl)-4-oxo-1-(2,4,6-trifluorphenyl)-1,4-dihydro-1,8-naphthyridin-3-carboxamid

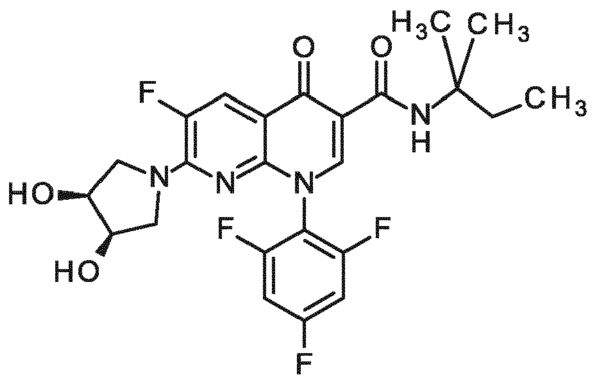

7-[(3R,4S)-3,4-Dihydroxypyrrolidin-1-yl]-6-fluor-4-oxo-1-(2,4,6-trifluorphenyl)-1,4-dihydro-1,8-naphthyridin-3-carbonsäure (75.0 mg, 81 % Reinheit, 138 μmol) wurde in 1.9 ml DMF vorgelegt, mit 2-Methylbutan-2-amin (19 μl, 170 μmol), N,N-Diisopropylethylamin (84 μl, 480 μmol) und HATU (63.1 mg, 166 μmol) versetzt und für 4 h bei Raumtemperatur rühren gelassen. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt, der wässrige Rückstand wurde mit gesättigter, wässriger Natriumhydrogencarbonatlösung basisch gestellt und zweimal mit Dichlormethan extrahiert. Die vereinigten organischen Phasen wurden über Natriumsulfat getrocknet, filtriert und im Vakuum eingeengt. Es wurden 63 mg der Zielverbindung (88% d. Th., Reinheit 98%) erhalten.
LC-MS (Methode 5): $R_t$ = 1.29 min; MS (ESIpos): m/z = 509 $[M+H]^+$
$^1$H-NMR (400 MHz, DMSO-$d_6$) $\delta$ [ppm]: -0.008 (0.76), 0.008 (0.52), 0.848 (1.75), 0.867 (4.17), 0.885 (1.87), 1.341 (16.00), 1.703 (0.53), 1.722 (1.62), 1.740 (1.54), 1.759 (0.45), 2.073 (3.41), 2.518 (0.96), 2.523 (0.79), 4.032 (0.61), 4.989 (0.60), 5.754 (0.54), 7.547 (0.88), 7.569 (1.67), 7.591 (0.89), 7.970 (1.66), 8.001 (1.63), 8.673 (2.83), 9.810 (2.01).

**Beispiel 80**

N-tert-Butyl-7-[(3R,4S)-3,4-dihydroxypyrrolidin-1-yl]-6-fluor-4-oxo-1-(2,4,6-trifluorphenyl)-1,4-dihydro-1,8-naphthyridin-3-carboxamid

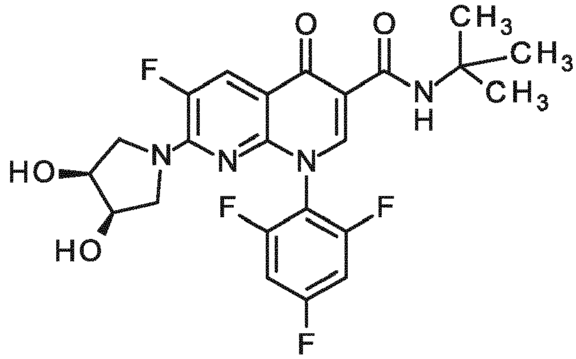

7-[(3R,4S)-3,4-Dihydroxypyrrolidin-1-yl]-6-fluor-4-oxo-1-(2,4,6-trifluorphenyl)-1,4-dihydro-1,8-naphthyridin-3-carbonsäure (75.0 mg, 81 % Reinheit, 138 μmol) wurde in 1.9 ml DMF vorgelegt, mit 2-Methylpropan-2-amin (17 μl, 170 μmol), N,N-Diisopropylethylamin (84 μl, 480 μmol) und HATU (63.1 mg, 166 μmol) versetzt und über das Wochenende bei Raumtemperatur rühren gelassen. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden im Vakuum eingeengt, der Rückstand wurde in Dichlormethan/wenig Methanol gelöst. Die organische Phase wurde zweimal mit gesättigter, wässriger Natriumhydrogencarbonatlösung gewaschen. Die vereinigten wässrigen Phasen wurde einmal mit Dichlormethan reextrahiert. Die vereinigten organischen Phasen wurden über Natriumsulfat getrocknet, filtriert und im Vakuum eingeengt. Es wurden 42 mg der Zielverbindung (60% d. Th., Reinheit 98%) erhalten.
LC-MS (Methode 3): $R_t$ = 1.72 min; MS (ESIpos): m/z = 495 $[M+H]^+$
$^1$H-NMR (400 MHz, DMSO-$d_6$) $\delta$ [ppm]: 0.008 (1.47), 1.388 (16.00), 2.073 (1.33), 4.028 (0.44), 4.989 (0.44), 7.547 (0.61), 7.569 (1.21), 7.591 (0.63), 7.957 (1.13), 7.989 (1.12), 8.679 (2.13), 9.865 (1.33).

**Beispiel 81**

N-[(1S)-1-Cyclopropyl-2,2,2-trifluorethyl]-6-fluor-4-oxo-7-(piperazin-1-yl)-1-(2,4,6-trifluorphenyl)-1,4-dihydro-1,8-naphthyridin-3-carboxamid

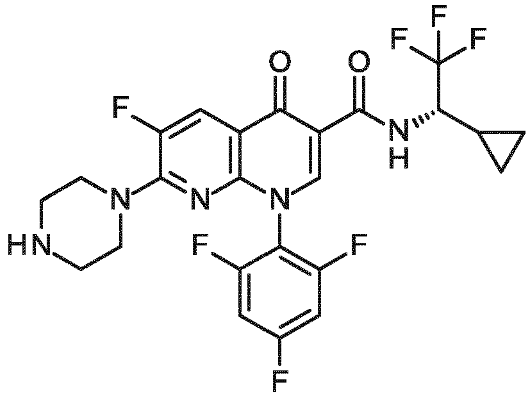

tert-Butyl-4-[6-{[(1S)-1-cyclopropyl-2,2,2-trifluorethyl]carbamoyl}-3-fluor-5-oxo-8-(2,4,6-trifluorphenyl)-5,8-dihydro-1,8-naphthyridin-2-yl]piperazin-1-carboxylat (113 mg, 69 % Reinheit, 121 μmol) wurde in 0.72 ml Dichlormethan vorgelegt, mit Trifluoressigsäure (360 μl, 4.7 mmol) versetzt und 1 h bei Raumtemperatur gerührt. Die Reaktionslösung wurde im Vakuum eingeengt, mit Acetonitril/Wasser/TFA versetzt und mittels präparativer HPLC gereinigt (RP18 Säule, Laufmittel: Methanol/Wasser-Gradient unter Zusatz von 0.1% TFA). Die Produktfraktionen wurden weitestgehend eingedampft. Der Rückstand wurde in Ethylacetat aufgenommen, die wässrige Phase wurde mit gesättigter, wässriger Natriumhydrogencarbonatlösung basisch gestellt. Die wässrige Phase wurde dreimal mit Ethylacetat extrahiert. Die vereinigten organischen Phasen wurden über Natriumsulfat getrocknet, filtriert und im Vakuum eingeengt. Das Rohprodukt wurde mittels Dickschichtchromatographie weiter gereinigt (Laufmittel: Dichlormethan/ 2M Ammoniak in Methanol = 20/1). Es wurden 43 mg der Zielverbindung (65% d. Th., Reinheit 99%) erhalten.
LC-MS (Methode 3): $R_t$ = 1.36 min; MS (ESIpos): m/z = 544 $[M+H]^+$
$^1$H-NMR (400 MHz, DMSO-$d_6$) δ [ppm]: -0.149 (0.64), -0.008 (6.43), 0.008 (5.61), 0.146 (0.68), 0.319 (2.04), 0.329 (3.29), 0.342 (3.21), 0.353 (2.50), 0.365 (1.25), 0.504 (0.82), 0.516 (2.18), 0.528 (3.29), 0.541 (3.07), 0.550 (3.43), 0.568 (3.57), 0.578 (2.79), 0.589 (2.54), 0.599 (2.07), 0.613 (1.29), 0.628 (1.86), 0.638 (1.71), 0.648 (3.14), 0.659 (2.61), 0.665 (2.43), 0.671 (2.39), 0.680 (1.18), 0.693 (0.79), 1.170 (0.64), 1.182 (1.32), 1.190 (1.89), 1.202 (3.18), 1.211 (2.32), 1.223 (3.18), 1.235 (2.07), 1.243 (1.29), 1.256 (0.57), 2.073 (0.96), 2.328 (1.21), 2.367 (1.04), 2.524 (4.82), 2.663 (12.39), 2.675 (16.00), 2.687 (12.50), 2.710 (1.39), 3.440 (12.39), 3.452 (15.11), 3.464 (11.71), 4.333 (0.43), 4.353 (1.64), 4.374 (2.89), 4.395 (2.86), 4.415 (1.54), 5.754 (0.43), 7.555 (5.61), 7.577 (10.82), 7.599 (5.71), 8.060 (9.39), 8.094 (9.29), 8.876 (15.57), 10.387 (6.43), 10.411 (6.29).

## Beispiel 82

N-[(1S)-1-Cyclopropyl-2,2,2-trifluorethyl]-6-fluor-7-[(3S)-3-methylpiperazin-1-yl]-4-oxo-1-(2,4,6-trifluorphenyl)-1,4-dihydro-1,8-naphthyridin-3-carboxamid

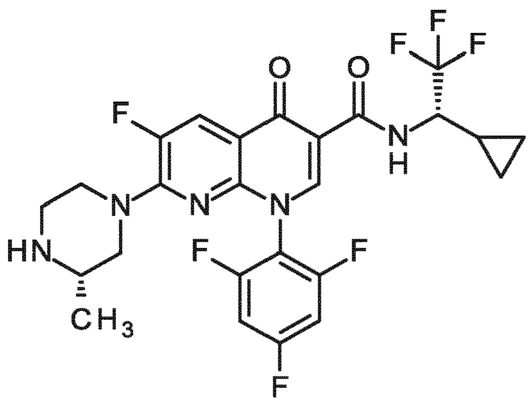

tert-Butyl-(2S)-4-[6-{[(1S)-1-cyclopropyl-2,2,2-trifluorethyl]carbamoyl}-3-fluor-5-oxo-8-(2,4,6-trifluorphenyl)-5,8-dihydro-1,8-naphthyridin-2-yl]-2-methylpiperazin-1-carboxylat (81.5 mg, 90% Reinheit, 112 μmol) wurde in 0.66 ml Dichlormethan vorgelegt, mit Trifluoressigsäure (330 μl, 4.3 mmol) versetzt und 1 h bei Raumtemperatur gerührt. Die

Reaktionslösung wurde mit Dichlormethan verdünnt und dreimal mit gesättigter, wässriger Natriumchloridlösung gewaschen. Die vereinigten wässrigen Phasen wurden mit Dichlormethan reextrahiert. Die vereinigten organischen Phasen wurden über Natriumsulfat getrocknet, filtriert und im Vakuum eingeengt. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend eingedampft. Der Rückstand wurde in Ethylacetat aufgenommen, die wässrige Phase wurde mit gesättigter, wässriger Natriumhydrogencarbonatlösung basisch gestellt. Die wässrige Phase wurde dreimal mit Ethylacetat extrahiert. Die vereinigten organischen Phasen wurden über Natriumsulfat getrocknet, filtriert und im Vakuum eingeengt. Das Rohprodukt wurde mittels Dickschichtchromatographie weiter gereinigt (Laufmittel: Dichlormethan/ 2 N Ammoniaklösung in Methanol = 20/1). Es wurden 26.3 mg der Zielverbindung (40 % d. Th., Reinheit 95%) erhalten.
LC-MS (Methode 3): $R_t$ = 1.40 min; MS (ESIpos): m/z = 558 $[M+H]^+$
$^1$H-NMR (400 MHz, DMSO-$d_6$) $\delta$ [ppm]: -0.149 (0.92), -0.008 (8.28), 0.008 (6.83), 0.146 (0.92), 0.320 (1.78), 0.330 (2.85), 0.342 (2.76), 0.354 (2.17), 0.365 (1.07), 0.516 (1.90), 0.527 (2.85), 0.540 (2.64), 0.549 (2.97), 0.568 (3.09), 0.578 (2.40), 0.588 (2.20), 0.599 (1.81), 0.613 (1.10), 0.628 (1.60), 0.638 (1.54), 0.648 (2.73), 0.659 (2.29), 0.664 (2.11), 0.670 (2.11), 0.680 (1.10), 0.693 (0.80), 0.837 (15.47), 0.852 (16.00), 0.919 (0.53), 1.169 (0.62), 1.182 (1.22), 1.190 (1.75), 1.202 (2.82), 1.210 (2.08), 1.222 (2.82), 1.234 (1.99), 1.242 (1.16), 1.255 (0.53), 2.119 (0.42), 2.302 (2.94), 2.323 (1.22), 2.328 (1.34), 2.366 (0.74), 2.524 (4.36), 2.573 (3.06), 2.605 (1.51), 2.666 (0.95), 2.670 (1.28), 2.675 (0.92), 2.711 (0.74), 2.805 (2.64), 2.834 (2.05), 2.965 (1.40), 2.972 (1.57), 2.998 (2.52), 3.027 (1.57), 3.869 (3.06), 3.896 (3.06), 3.972 (2.43), 4.004 (2.29), 4.355 (1.45), 4.375 (2.52), 4.397 (2.49), 4.417 (1.37), 7.577 (4.93), 7.599 (9.26), 7.622 (4.96), 7.630 (1.75), 8.053 (8.55), 8.088 (8.37), 8.890 (14.58), 8.913 (0.50), 10.390 (5.67), 10.414 (5.34).

**Beispiel 83**

N-[(1S)-1-Cyclopropyl-2,2,2-trifluorethyl]-6-fluor-7-(2-oxa-6-azaspiro[3.3]hept-6-yl)-4-oxo-1-(2,4,6-trifluorphenyl)-1,4-dihydro-1,8-naphthyridin-3-carboxamid

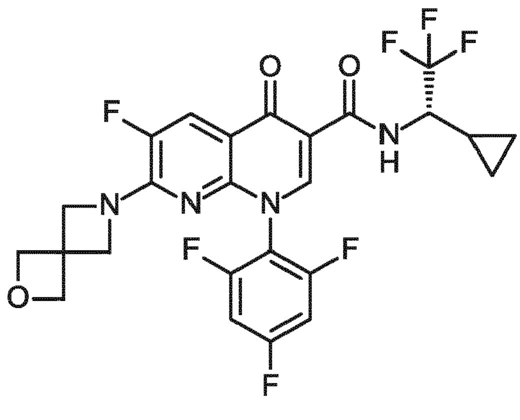

7-Chlor-N-[(1S)-1-cyclopropyl-2,2,2-trifluorethyl]-6-fluor-4-oxo-1-(2,4,6-trifluorphenyl)-1,4-dihydro-1,8-naphthyridin-3-carboxamid (1.20 g, 2.43 mmol) wurde in 23 ml DMF vorgelegt, mit Ethandisäure-2-oxa-6-azaspiro[3.3]heptan (1:1) (644 mg, 3.40 mmol) und N,N-Diisopropylethylamin (2.1 ml, 12 mmol) versetzt und über Nacht bei Raumtemperatur gerührt. Die Reaktionslösung wurde mit Wasser versetzt, der dabei ausgefallene Feststoff wurde abfiltriert und im Hochvakuum getrocknet. Das Rohprodukt wurde mittels Kieselgelchromatographie gereinigt (Laufmittel: 100% Dichlormethan nach Dichlormethan/Methanol = 100/1). Es wurde 1.0 g der Zielverbindung (73% d. Th., Reinheit 99%) erhalten.
LC-MS (Methode 3): $R_t$ = 2.20 min; MS (ESIpos): m/z = 557 $[M+H]^+$

**Beispiel 84**

7-[3,3-Bis(hydroxymethyl)azetidin-1-yl]-N-[(1S)-1-cyclopropyl-2,2,2-trifluorethyl]-6-fluor-4-oxo-1-(2,4,6-trifluorphenyl)-1,4-dihydro-1,8-naphthyridin-3-carboxamid

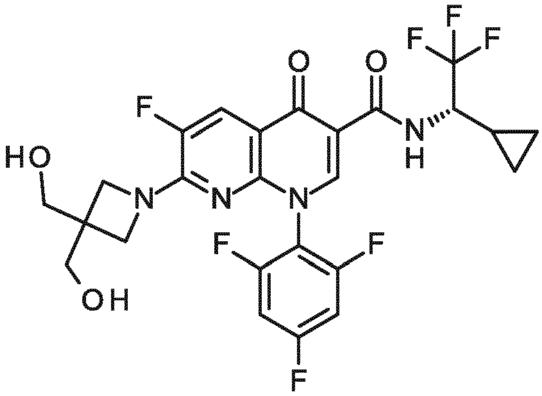

N-[(1S)-1-Cyclopropyl-2,2,2-trifluorethyl]-6-fluor-7-(2-oxa-6-azaspiro[3.3]hept-6-yl)-4-oxo-1-(2,4,6-trifluorphenyl)-1,4-dihydro-1,8-naphthyridin-3-carboxamid (65.0 mg, 117 μmol) wurde in Trifluoressigsäure (730 μl, 9.5 mmol) vorgelegt, mit 730 μl Wasser und 730 μl Acetonitril versetzt und zwei Tage bei Raumtemperatur gerührt. Die Reaktionslösung wurde mittels präparativer HPLC gereinigt (RP18 Säule, Laufmittel: Acetonitril/Wasser-Gradient unter Zusatz von 0.1% TFA). Die Produktfraktionen wurden im Vakuum eingeengt, der Rückstand wurde in Dichlormethan aufgenommen und zweimal mit gesättigter, wässriger Natriumhydrogencarbonatlösung gewaschen. Die vereinigten wässrigen Phasen wurden mit Dichlormethan reextrahiert. Die vereinigten organischen Phasen wurden über Natriumsulfat getrocknet, filtriert und im Vakuum eingeengt. Es wurden 45 mg der Zielverbindung (66% d. Th., Reinheit 99%) erhalten.
LC-MS (Methode 3): $R_t$ = 1.86 min; MS (ESIpos): m/z = 575 $[M+H]^+$
$^1$H-NMR (400 MHz, DMSO-$d_6$) [ppm]: -0.149 (0.42), -0.008 (3.70), 0.008 (3.30), 0.146 (0.41), 0.316 (1.31), 0.326 (2.15), 0.338 (2.07), 0.350 (1.69), 0.362 (0.80), 0.510 (1.45), 0.521 (2.09), 0.534 (1.89), 0.545 (2.18), 0.553 (1.61), 0.564 (2.23), 0.575 (1.72), 0.585 (1.64), 0.595 (1.33), 0.609 (0.83), 0.624 (1.13), 0.634 (1.13), 0.645 (1.97), 0.655 (1.70), 0.668 (1.59), 1.163 (0.46), 1.175 (0.92), 1.183 (1.27), 1.195 (2.15), 1.204 (1.50), 1.215 (2.10), 1.228 (1.24), 1.236 (1.21), 2.074 (10.78), 2.328 (0.75), 2.366 (0.47), 2.670 (0.66), 2.710 (0.41), 3.475 (15.83), 3.488 (16.00), 4.130 (0.94), 4.349 (1.22), 4.369 (1.91), 4.390 (1.84), 4.410 (0.97), 4.835 (5.32), 4.848 (12.45), 4.861 (5.07), 5.754 (4.77), 7.532 (3.99), 7.554 (7.54), 7.576 (3.92), 7.963 (6.93), 7.992 (6.79), 8.808 (12.92), 10.463 (4.45), 10.487 (4.20).

## Beispiel 85

tert-Butyl-4-[({7-[(3R,4R)-3,4-dihydroxypyrrolidin-1-yl]-6-fluor-4-oxo-1-(2,4,6-trifluorphenyl)-1,4-dihydro-1,8-naphthyridin-3-yl}carbonyl)amino]-3,3-difluorpiperidin-1-carboxylat

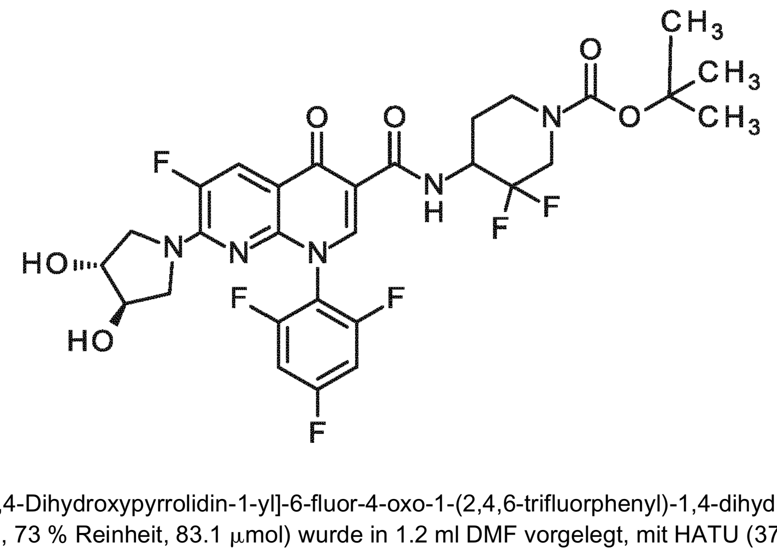

7-[(3R,4R)-3,4-Dihydroxypyrrolidin-1-yl]-6-fluor-4-oxo-1-(2,4,6-trifluorphenyl)-1,4-dihydro-1,8-naphthyridin-3-carbonsäure (50.0 mg, 73 % Reinheit, 83.1 μmol) wurde in 1.2 ml DMF vorgelegt, mit HATU (37.9 mg, 99.7 μmol) und N,N-Diisopropylethylamin (36 μl, 210 μmol) versetzt und 30 min bei Raumtemperatur gerührt. tert-Butyl-4-amino-3,3-difluopiperidin-1-carboxylat (23.6 mg, 99.7 μmol) wurde hinzugeben und das Gemisch wurde 2 h bei Raumtemperatur

rühren gelassen. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt und der Rückstand zweimal mit Dichlormethan extrahiert. Die vereinigten organischen Phasen wurden zweimal mit gesättigter, wässriger Natriumhydrogencarbonatlösung gewaschen. Die vereinigten wässrigen Phasen wurde mit Dichlormethan reextrahiert. Die vereinigten organischen Phasen wurden über Natriumsulfat getrocknet, filtriert und im Vakuum eingeengt. Es wurden 48 mg der Zielverbindung (87% d. Th., Reinheit 99%) erhalten.
LC-MS (Methode 3): $R_t$ = 1.81 min; MS (ESIpos): m/z = 658 $[M+H]^+$
$^1$H-NMR (400 MHz, DMSO-$d_6$) [ppm]: -0.008 (0.48), 1.157 (0.55), 1.175 (1.10), 1.193 (0.55), 1.427 (16.00), 1.988 (2.06), 4.021 (0.59), 4.038 (0.53), 5.192 (0.56), 7.572 (0.73), 7.995 (0.80), 8.026 (0.78), 8.807 (1.58), 10.314 (0.54), 10.337 (0.51).

## Beispiel 86

Methyl-4-[({7-[(3R,4R)-3,4-dihydroxypyrrolidin-1-yl]-6-fluor-4-oxo-1-(2,4,6-trifluorphenyl)-1,4-dihydro-1,8-naphthyridin-3-yl}carbonyl)amino]bicyclo[2.2.1]heptan-1-carboxylat

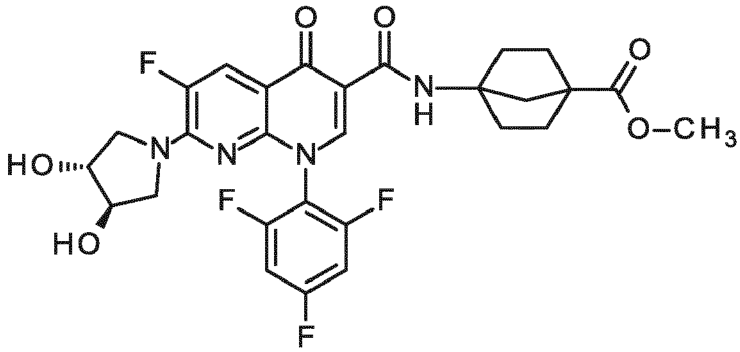

7-[(3R,4R)-3,4-Dihydroxypyrrolidin-1-yl]-6-fluor-4-oxo-1-(2,4,6-trifluorphenyl)-1,4-dihydro-1,8-naphthyridin-3-carbonsäure (50.0 mg, 98 % Reinheit, 112 μmol) wurde in 1.6 ml DMF vorgelegt, mit HATU (50.9 mg, 134 μmol) und N,N-Diisopropylethylamin (49 μl, 280 μmol) versetzt und 30 min bei Raumtemperatur gerührt. Methyl-4-aminobicyclo[2.2.1]heptan-1-carboxylat (22.6 mg, 134 μmol) wurde hinzugeben und 2 h bei Raumtemperatur rühren gelassen. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt, der Rückstand zweimal mit Dichlormethan extrahiert. Die vereinigten organischen Phasen wurden zweimal mit gesättigter, wässriger Natriumhydrogencarbonatlösung gewaschen. Die vereinigten wässrigen Phasen wurde mit Dichlormethan reextrahiert. Die vereinigten organischen Phasen wurden über Natriumsulfat getrocknet, filtriert und im Vakuum eingeengt. Es wurden 52 mg der Zielverbindung (78% d. Th., Reinheit 99%) erhalten.
LC-MS (Methode 3): $R_t$ = 1.68 min; MS (ESIpos): m/z = 591 $[M+H]^+$
$^1$H-NMR (400 MHz, CHLOROFORM-d) δ [ppm]: -0.008 (0.56), 0.008 (0.54), 1.584 (6.18), 1.725 (0.82), 1.751 (1.60), 1.765 (1.06), 1.903 (0.99), 1.917 (1.54), 1.943 (0.96), 2.006 (0.84), 2.083 (0.44), 2.124 (3.16), 2.138 (3.63), 2.155 (4.94), 2.279 (1.00), 3.693 (16.00), 4.255 (2.00), 6.860 (1.01), 6.880 (1.82), 6.899 (1.04), 7.997 (1.62), 8.029 (1.61), 8.522 (3.08), 10.228 (2.05).

## Beispiel 87

7-[(3R,4R)-3,4-Dihydroxypyrrolidin-1-yl]-N-(3-ethylpentan-3-yl)-6-fluor-4-oxo-1-(2,4,6-trifluorphenyl)-1,4-dihydro-1,8-naphthyridin-3-carboxamid

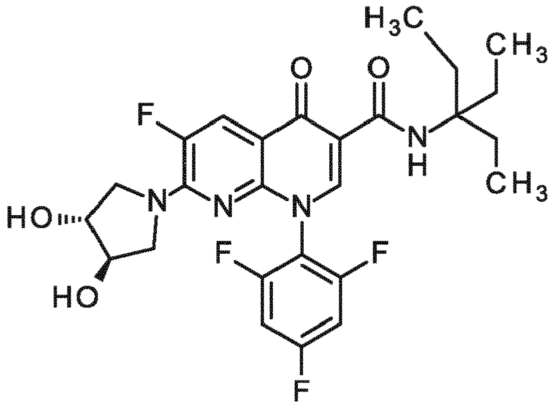

7-[(3R,4R)-3,4-Dihydroxypyrrolidin-1-yl]-6-fluor-4-oxo-1-(2,4,6-trifluorphenyl)-1,4-dihydro-1,8-naphthyridin-3-carbonsäure (50.0 mg, 73 % Reinheit, 83.1 µmol) wurde in 1.2 ml DMF vorgelegt, mit HATU (37.9 mg, 99.7 µmol) und N,N-Diisopropylethylamin (36 µl, 210 µmol) versetzt und 30 min bei Raumtemperatur gerührt. 3-Ethylpentan-3-amin (11.5 mg, 99.7 µmol) wurde hinzugeben und über Nacht bei Raumtemperatur rühren gelassen. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt und der Rückstand zweimal mit Ethylacetat extrahiert. Die vereinigten organischen Phasen wurden zweimal mit gesättigter, wässriger Natriumhydrogencarbonatlösung gewaschen. Die vereinigten wässrigen Phasen wurde mit Ethylacetat reextrahiert. Die vereinigten organischen Phasen wurden über Natriumsulfat getrocknet, filtriert und im Vakuum eingeengt. Es wurden 36 mg der Zielverbindung (80% d. Th., Reinheit 99%) erhalten.
LC-MS (Methode 3): $R_t$ = 1.99 min; MS (ESIpos): m/z = 537 $[M+H]^+$
$^1$H-NMR (400 MHz, DMSO-d6) δ [ppm]: -0.008 (0.97), 0.008 (0.75), 0.770 (6.80), 0.789 (16.00), 0.807 (7.33), 1.702 (1.95), 1.720 (5.99), 1.739 (5.74), 1.757 (1.74), 2.524 (0.69), 3.918 (0.49), 5.191 (1.51), 7.549 (1.11), 7.571 (1.93), 7.592 (1.09), 7.991 (2.75), 8.023 (2.69), 8.671 (4.76), 9.621 (2.85).

## Beispiel 88

7-[(3R,4R)-3,4-Dihydroxypyrrolidin-1-yl]-6-fluor-N-(3-methylpentan-3-yl)-4-oxo-1-(2,4,6-trifluorphenyl)-1,4-dihydro-1,8-naphthyridin-3-carboxamid

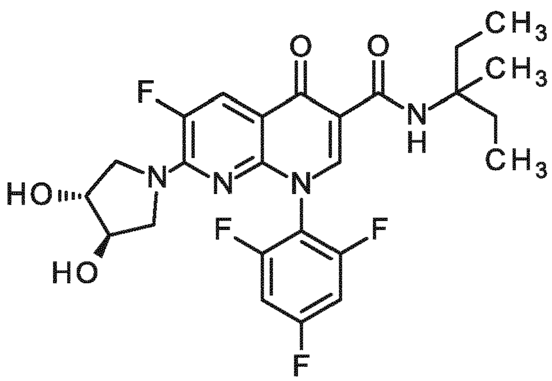

7-[(3R,4R)-3,4-Dihydroxypyrrolidin-1-yl]-6-fluor-4-oxo-1-(2,4,6-trifluorphenyl)-1,4-dihydro-1,8-naphthyridin-3-carbonsäure (40.0 mg, 73 % Reinheit, 66.5 µmol) wurde in 0.93 DMF vorgelegt, mit HATU (30.3 mg, 79.8 µmol) und N,N-Diisopropylethylamin (29 µl, 170 µmol) versetzt und 30 min bei Raumtemperatur gerührt. 3-Methylpentan-3-aminhydrochlorid (11.0 mg, 79.8 µmol) wurde hinzugeben und 2 h bei Raumtemperatur rühren gelassen. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt, der Rückstand zweimal mit Dichlormethan extrahiert. Die vereinigten organischen Phasen wurden zweimal mit gesättigter, wässriger Natriumhydrogencarbonatlösung gewaschen. Die vereinigten wässrigen Phasen wurden mit Dichlormethan reextrahiert. Die vereinigten organischen Phasen wurden über Natriumsulfat getrocknet, filtriert und im Vakuum

eingeengt. Es wurden 29 mg der Zielverbindung (83% d. Th., Reinheit 99%) erhalten.
LC-MS (Methode 3): $R_t$ = 1.88 min; MS (ESIpos): m/z = 523 $[M+H]^+$
$^1$H-NMR (400 MHz, DMSO-d6) δ [ppm]: 0.008 (2.16), 0.817 (6.77), 0.835 (15.54), 0.854 (7.44), 1.282 (16.00), 1.616 (0.41), 1.634 (1.39), 1.652 (1.86), 1.668 (2.36), 1.687 (1.89), 1.705 (0.53), 1.773 (0.62), 1.791 (2.07), 1.809 (2.44), 1.826 (1.91), 1.844 (1.39), 3.908 (0.77), 5.190 (2.41), 7.550 (1.66), 7.572 (3.01), 7.593 (1.62), 7.990 (3.59), 8.022 (3.54), 8.673 (6.67), 9.737 (4.26).

**Beispiel 89**

7-[(3R,4R)-3,4-Dihydroxypyrrolidin-1-yl]-6-fluor-N-(2-methylbutan-2-yl)-4-oxo-1-(2,4,6-trifluorphenyl)-1,4-dihydro-1,8-naphthyridin-3-carboxamid

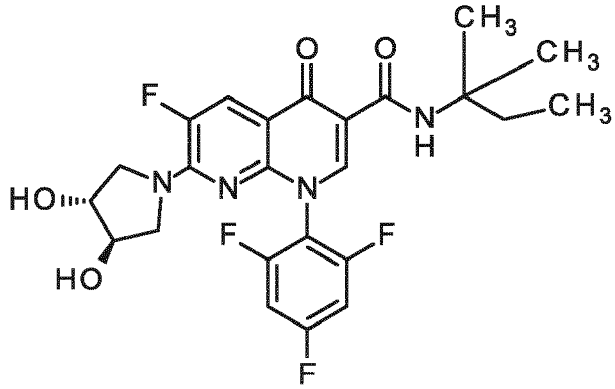

7-[(3R,4R)-3,4-Dihydroxypyrrolidin-1-yl]-6-fluor-4-oxo-1-(2,4,6-trifluorphenyl)-1,4-dihydro-1,8-naphthyridin-3-carbonsäure (50.0 mg, 73 % Reinheit, 83.1 µmol) wurde in 1.2 ml DMF vorgelegt, mit HATU (37.9 mg, 99.7 µmol) und N,N-Diisopropylethylamin (36 µl, 210 µmol) versetzt und 30 min bei Raumtemperatur gerührt. 2-Methylbutan-2-amin (12 µl, 100 µmol) wurde hinzugeben und 2 h bei Raumtemperatur rühren gelassen. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt und der Rückstand zweimal mit Ethylacetat extrahiert. Die vereinigten organischen Phasen wurden zweimal mit gesättigter, wässriger Natriumhydrogencarbonatlösung gewaschen. Die vereinigten wässrigen Phasen wurde mit Ethylacetat reextrahiert. Die vereinigten organischen Phasen wurden über Natriumsulfat getrocknet, filtriert und im Vakuum eingeengt. Es wurden 34 mg der Zielverbindung (80% d. Th., Reinheit 99%) erhalten.
LC-MS (Methode 3): $R_t$ = 1.76 min; MS (ESIpos): m/z = 509 $[M+H]^+$
$^1$H-NMR (400 MHz, DMSO-d6) δ [ppm]: 0.008 (1.67), 0.851 (1.89), 0.869 (4.29), 0.888 (1.90), 1.343 (16.00), 1.706 (0.62), 1.724 (1.70), 1.743 (1.61), 1.761 (0.47), 3.909 (0.41), 5.186 (1.22), 7.551 (0.86), 7.573 (1.45), 7.593 (0.83), 7.982 (1.87), 8.014 (1.83), 8.676 (3.26), 9.818 (2.08).

**Beispiel 90**

N-tert-Butyl-7-[(3R,4R)-3,4-dihydroxypyrrolidin-1-yl]-6-fluor-4-oxo-1-(2,4,6-trifluorphenyl)-1,4-dihydro-1,8-naphthyridin-3-carboxamid

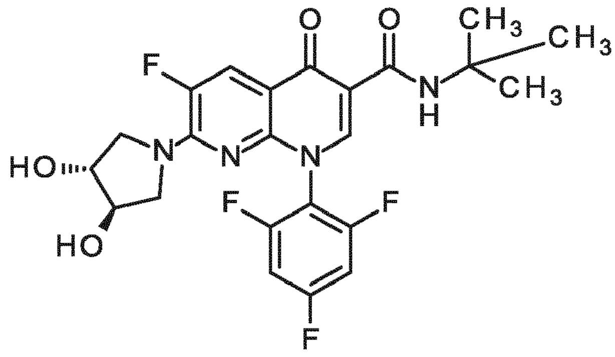

7-[(3R,4R)-3,4-Dihydroxypyrrolidin-1-yl]-6-fluor-4-oxo-1-(2,4,6-trifluorphenyl)-1,4-dihydro-1,8-naphthyridin-3-carbonsäure (60.0 mg, 73 % Reinheit, 99.7 µmol) wurde in 1.4 ml DMF vorgelegt, mit HATU (45.5 mg, 120 µmol) und N,N-Diisopropylethylamin (43 µl, 250 µmol) versetzt und 30 min bei Raumtemperatur gerührt. 2-Methylpropan-2-amin (8.75 mg, 120 µmol) wurde hinzugeben und 2 h bei Raumtemperatur rühren gelassen. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden weitestgehend im Vakuum eingeengt, der Rückstand zweimal mit Dichlormethan extrahiert. Die vereinigten organischen Phasen wurden zweimal mit gesättigter, wässriger Natriumhydrogencarbonatlösung gewaschen. Die vereinigten wässrigen Phasen wurde mit Dichlormethan reextrahiert. Die vereinigten organischen Phasen wurden über Natriumsulfat getrocknet, filtriert und im Vakuum eingeengt. Es wurden 26 mg der Zielverbindung (52% d. Th., Reinheit 99%) erhalten.
LC-MS (Methode 3): $R_t$ = 1.66 min; MS (ESIpos): m/z = 495 $[M+H]^+$
$^1$H-NMR (400 MHz, DMSO-d6) δ [ppm]: 0.008 (1.87), 1.245 (0.76), 1.260 (0.86), 1.275 (0.48), 1.390 (16.00), 5.185 (0.79), 7.551 (0.52), 7.573 (0.92), 7.594 (0.53), 7.970 (1.13), 8.002 (1.11), 8.682 (2.04), 9.872 (1.36).

**Beispiel 91**

7-[(3*R*,4*S*)-3,4-Dihydroxypyrrolidin-1-yl]-6-fluor-4-oxo-*N*-(4,4,4-trifluor-2-methylbutan-2-yl)-1-(2,4,6-trifluophenyl)-1,4-dihydro-1,8-naphthyridin-3-carboxamid

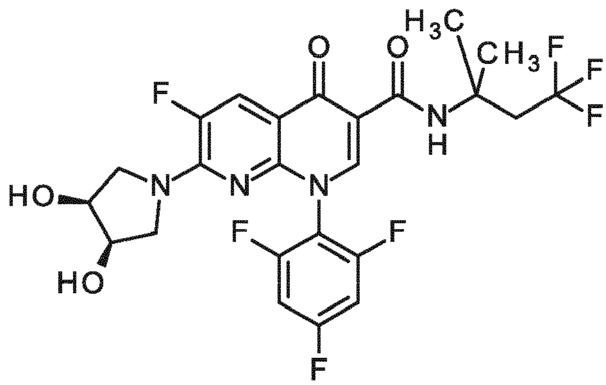

Gemäß AAV1 wurden 80.0 mg (182 µmol) 7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure mit 45.3 mg (255 µmol) 4,4,4-Trifluor-2-methylbutan-2-amin Hydrochlorid in Gegenwart von 83.1 mg (219 µmol) HATU und 95 µl (550 µmol) DIPEA in 730 µl DMF zur Reaktion gebracht. Das Rohprodukt wurde mittels präparativer HPLC (Säule: Acetonitril / Wasser / 0.1% Ameisensäure) gereinigt. Es wurden 88.9 mg (87% d. Th., 100% Reinheit) der Titelverbindung erhalten.
LC-MS (Methode 3): $R_t$ = 1.84 min; MS (ESIpos): m/z = 563 $[M+H]^+$
$^1$H-NMR (400 MHz, DMSO-d6): δ [ppm] = 10.08 (s, 1 H), 8.72 (s, 1 H), 7.98 (d, 1 H), 7.53 - 7.61 (m, 2 H), 4.87 - 5.10 (m, 2 H), 3.83 - 4.11 (m, 3 H), 3.48 - 3.69 (m, 1 H), 3.12 - 3.27 (m, 1 H), 2.87 - 3.09 (m, 3 H), 1.48 (s, 6 H).

**Beispiel 92**

7-[(3*R*,4*S*)-3,4-Dihydroxypyrrolidin-1-yl]-6-fluor-4-oxo-*N*-[3,3,4,4,4-pentafluorbutan-2-yl]-1-(2,4,6-trifluorphenyl)-1,4-dihydro-1,8-naphthyridin-3-carboxamid (Diastereomerengemisch)

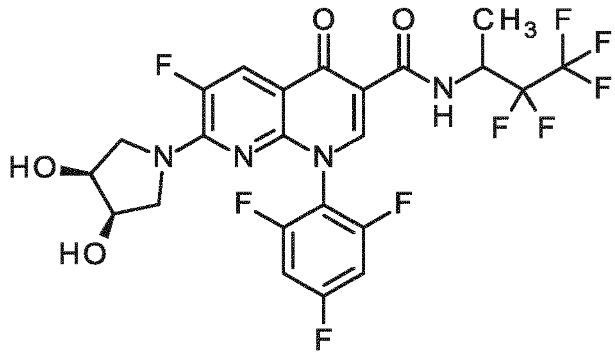

Gemäß AAV1 wurden 150 mg (341 μmol) 7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure mit 95.4 mg (478 μmol) 3,3,4,4,4-Pentafluorbutan-2-amin Hydrochlorid (Racemat) in Gegenwart von 156 mg (410 μmol) HATU und 180 μl (1.00 mmol) DIPEA in 1.4 ml DMF zur Reaktion gebracht. Das Rohprodukt wurde mittels präparativer HPLC (Säule: Acetonitril / Wasser / 0.1% Ameisensäure) gereinigt. Es wurden 149 mg (75% d. Th., 100% Reinheit) der Titelverbindung erhalten.
LC-MS (Methode 3): $R_t$ = 1.87 min; MS (ESIpos): m/z = 585 $[M+H]^+$
$^1$H-NMR (400 MHz, DMSO-d6): δ [ppm] = 10.46 (d, 1 H), 8.84 (s, 1 H), 8.00 (d, 1 H), 7.53 - 7.61 (m, 2 H), 4.89 - 5.12 (m, 3 H), 3.85 - 4.12 (m, 3 H), 3.47 - 3.70 (m, 1 H), 2.91 - 3.28 (m, 2 H), 1.39 (d, 3 H).

146 mg der Titelverbindung (Diastereomerengemisch) wurde durch chirale HPLC in die Diastereomere getrennt (präparative HPLC: Säule Daicel Chiralcel OX-H, 5 μm, 250x30 mm; Eluent: 80% *n*-Heptan, 20% Ethanol; Temperatur: 25°C; Fluss: 40 ml/min; UV-Detektion: 265 nm.)

Man erhielt (in der Reihenfolge der Elution von der Säule) 56.0 mg Diastereomer 1 (99% de) $R_t$ = 6.40 min und 55.8 mg Diastereomer 2 (98% de) $R_t$ = 8.57 min.

[Analytische HPLC: Säule Daicel OX-3, 3 μm, 50x4.6 mm; Eluent: 80% Iso-Hexan, 20% Ethanol; UV-Detektion: 220 nm].

Diastereomer 1 wurde zusätzlich mittels präparativer HPLC (Säule: Acetonitril / Wasser / 0.1% Ameisensäure) gereinigt und 41.0 mg (21% d. Th., 100% Reinheit) der Titelverbindung aus Beispiel 93 erhalten.

Diastereomer 2 wurde zusätzlich mittels präparativer HPLC (Säule: Acetonitril / Wasser / 0.1% Ameisensäure) gereinigt und 42.0 mg (21% d. Th., 100% Reinheit) der Titelverbindung aus Beispiel 94 erhalten.

**Beispiel 93**

7-[(3*R*,4*S*)-3,4-Dihydroxypyrrolidin-1-yl]-6-fluor-4-oxo-*N*-[3,3,4,4,4-pentafluorbutan-2-yl]-1-(2,4,6-trifluorphenyl)-1,4-dihydro-1,8-naphthyridin-3-carboxamid (Diastereomer 1)

LC-MS (Methode 3): $R_t$ = 1.89 min; MS (ESIpos): m/z = 585 $[M+H]^+$

$^1$H-NMR (400 MHz, DMSO-d6): δ [ppm] = 10.46 (d, 1 H), 8.84 (s, 1 H), 8.00 (d, 1 H), 7.54 - 7.61 (m, 2 H), 4.91 - 5.10 (m, 3 H), 3.84 - 4.12 (m, 3 H), 3.43 - 3.67 (m, 1 H), 3.12 - 3.28 (m, 1 H), 2.88 - 3.11 (m, 1 H), 1.39 (d, 3 H).

**Beispiel 94**

7-[(3*R*,4*S*)-3,4-Dihydroxypyrrolidin-1-yl]-6-fluor-4-oxo-*N*-[3,3,4,4,4-pentafluorbutan-2-yl]-1-(2,4,6-trifluorphenyl)-1,4-dihydro-1,8-naphthyridin-3-carboxamid (Diastereomer 2)

LC-MS (Methode 3): $R_t$ = 1.89 min; MS (ESIpos): m/z = 585 $[M+H]^+$

$^1$H-NMR (400 MHz, DMSO-d6): δ [ppm] = 10.46 (d, 1 H), 8.84 (s, 1 H), 8.00 (d, 1 H), 7.54 - 7.61 (m, 2 H), 4.92 - 5.09 (m, 3 H), 3.85 - 4.11 (m, 3 H), 3.42 - 3.68 (m, 1 H), 3.12 - 3.28 (m, 1 H), 2.92 - 3.11 (m, 1 H), 1.39 (d, 3 H).

## Beispiel 95

7-[(3*R*,4*S*)-3,4-Dihydroxypyrrolidin-1-yl]-6-fluor-4-oxo-*N*-[1-(trifluormethoxy)butan-2-yl]-1-(2,4,6-trifluophenyl)-1,4-dihydro-1,8-naphthyridin-3-carboxamid (Diastereomerengemisch)

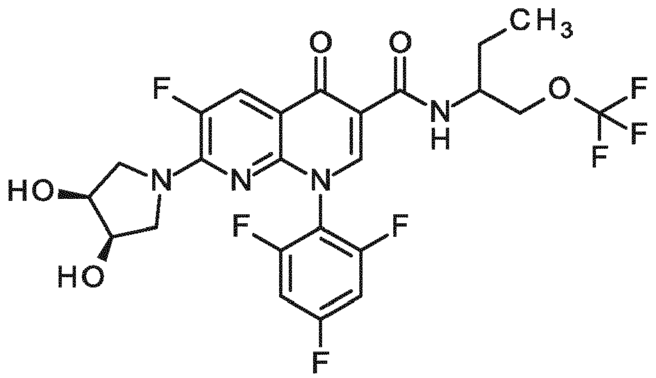

Gemäß AAV1 wurden 120 mg (273 µmol) 7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure mit 74.0 mg (382 µmol) 1-(Trifluor-methoxy)butan-2-amin Hydrochlorid (Racemat) in Gegenwart von 125 mg (328 µmol) HATU und 140 µl (820 µmol) DIPEA in 1.1 ml DMF zur Reaktion gebracht. Das Rohprodukt wurde mittels präparativer HPLC (Säule: Acetonitril / Wasser / 0.1% Ameisensäure) gereinigt. Es wurden 103 mg (65% d. Th., 100% Reinheit) der Titelverbindung erhalten.
LC-MS (Methode 1): $R_t$ = 0.99 min; MS (ESIpos): m/z = 579 $[M+H]^+$
$^1$H-NMR (400 MHz, DMSO-d6): δ [ppm] = 9.99 (br d, 1 H), 8.76 (s, 1 H), 8.00 (d, 1 H), 7.53 - 7.61 (m, 2 H), 4.79 - 5.20 (m, 2 H), 4.11 - 4.23 (m, 3 H), 3.77 - 4.10 (m, 3 H), 3.43 - 3.74 (m, 1 H), 2.85 - 3.26 (m, 2 H), 1.52 - 1.73 (m, 2 H), 0.94 (t, 3 H).

100 mg der Titelverbindung (Diastereomerengemisch) wurde durch chirale HPLC in die Diastereomere getrennt (präparative HPLC: Säule Chiralpak AD-H, 5 µm, 250x30 mm; Eluent: 80% *n*-Heptan, 20% Ethanol; Temperatur: 25°C; Fluss: 40 ml/min; UV-Detektion: 265 nm.)

Man erhielt (in der Reihenfolge der Elution von der Säule) 23.6 mg Diastereomer 1 (99% de) Rt = 10.77 min und 13.5 mg (9% d. Th., 100% Reinheit) Diastereomer 2 aus Beispiel 97 (98% de) Rt = 12.40 min.

[Analytische HPLC: Säule Chiraltek AD-3, 3 µm; Eluent: 80% Iso-Hexan, 20% Ethanol; UV-Detektion: 220 nm].

Diastereomer 1 wurde zusätzlich mittels präparativer HPLC (Säule: Acetonitril / Wasser/ 0.1% Ameisensäure) gereinigt und 4.30 mg (3% d. Th., 100% Reinheit) der Titelverbindung aus Beispiel 96 erhalten.

## Beispiel 96

7-[(3*R*,4*S*)-3,4-Dihydroxypyrrolidin-1-yl]-6-fluor-4-oxo-*N*-[1-(trifluormethoxy)butan-2-yl]-1-(2,4,6-trifluorphenyl)-1,4-dihydro-1,8-naphthyridin-3-carboxamid (Diastereomer 1)

LC-MS (Methode 3): $R_t$ = 1.87 min; MS (ESIpos): m/z = 579 $[M+H]^+$

$^1$H-NMR (500 MHz, DMSO-d6): δ [ppm] = 9.98 (br d, 1 H), 8.76 (s, 1 H), 8.00 (d, 1 H), 7.54 - 7.60 (m, 2 H), 4.91 - 5.07 (m, 2 H), 4.13 - 4.22 (m, 3 H), 3.82 - 4.10 (m, 3 H), 3.44 - 3.66 (m, 1 H), 3.12 - 3.29 (m, 1 H), 2.93 - 3.11 (m, 1 H), 1.63 - 1.72 (m, 1 H), 1.53 - 1.63 (m, 1 H), 0.94 (t, 3 H).

**Beispiel 97**

7-[(3*R*,4*S*)-3,4-Dihydroxypyrrolidin-1-yl]-6-fluor-4-oxo-*N*-[1-(trifluormethoxy)butan-2-yl]-1-(2,4,6-trifluoiphenyl)-1,4-dihydro-1,8-naphthyridin-3-carboxamid (Diastereomer 2)

LC-MS (Methode 3): $R_t$ = 1.87 min; MS (ESIpos): m/z = 579 $[M+H]^+$

$^1$H-NMR (400 MHz, DMSO-d6): δ [ppm] = 9.99 (br d, 1 H), 8.76 (s, 1 H), 8.00 (d, 1 H), 7.53 - 7.61 (m, 2 H), 4.90 - 5.08 (m, 2 H), 4.13 - 4.23 (m, 3 H), 3.79 - 4.10 (m, 3 H), 3.45 - 3.69 (m, 1 H), 3.11 - 3.27 (m, 1 H), 2.86 - 3.11 (m, 1 H), 1.53 - 1.72 (m, 2 H), 0.94 (t, 3 H).

**Beispiel 98**

7-[(3*R*,4*S*)-3,4-Dihydroxypyrrolidin-1-yl]-6-fluor-4-oxo-*N*-[(2*S*)-1,1,1-trifluorbutan-2-yl]-1-(2,4,6-trifluorphenyl)-1,4-dihydro-1,8-naphthyridin-3-carboxamid

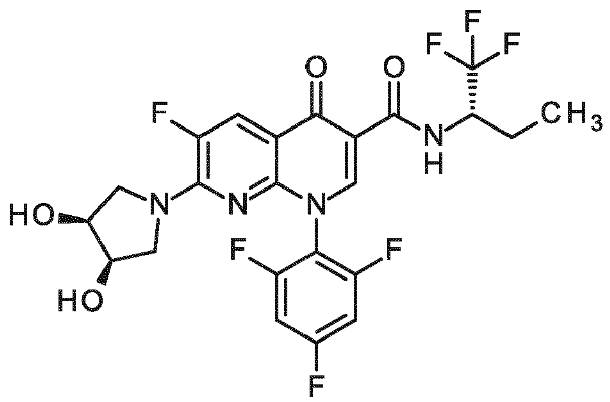

Gemäß AAV1 wurden 50.0 mg (114 μmol) 7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure mit 26.1 mg (159 μmol) (2*S*)-1,1,1-Trifluorbutan-2-amin Hydrochlorid in Gegenwart von 51.9 mg (137 μmol) HATU und 59 μl (340 μmol) DIPEA in 460 μl DMF zur Reaktion gebracht. Das Rohprodukt wurde mittels präparativer HPLC (Säule: Acetonitril / Wasser / 0.1% Ameisensäure) gereinigt. Es wurden 47.2 mg (76% d. Th., 100% Reinheit) der Titelverbindung erhalten.
LC-MS (Methode 1): $R_t$ = 0.98 min; MS (ESIpos): m/z = 549 $[M+H]^+$
$^1$H-NMR (400 MHz, DMSO-d6): δ [ppm] = 10.33 (d, 1 H), 8.84 (s, 1 H), 8.00 (d, 1 H), 7.54 - 7.61 (m, 2 H), 4.84 - 5.23 (m, 2 H), 4.67 - 4.83 (m, 1 H), 3.81 - 4.16 (m, 3 H), 3.42 - 3.70 (m, 2 H), 2.95 - 3.14 (m, 1 H), 1.83 - 1.93 (m, 1 H), 1.58 - 1.70 (m, 1 H), 0.97 (t, 3 H).

**Beispiel 99**

7-[(3*R*,4*S*)-3,4-Dihydroxypyrrolidin-1-yl]-6-fluor-*N*-[(2*R*)-3-methylbutan-2-yl]-4-oxo-1-(2,4,6-trifluorphenyl)-1,4-dihydro-1,8-naphthyridin-3-carboxamid

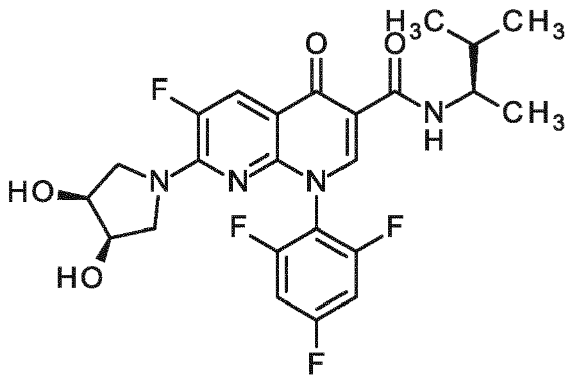

Gemäß AAV1 wurden 50.0 mg (114 µmol) 7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure mit 13.9 mg (159 µmol) (2R)-3-Methylbutan-2-amin in Gegenwart von 51.9 mg (137 µmol) HATU und 59 µl (340 µmol) DIPEA in 460 µl DMF zur Reaktion gebracht. Das Rohprodukt wurde mittels präparativer HPLC (Säule: Acetonitril / Wasser / 0.1% Ameisensäure) gereinigt. Es wurden 21.6 mg (37% d. Th., 100% Reinheit) der Titelverbindung erhalten.
LC-MS (Methode 3): $R_t$ = 1.74 min; MS (ESIpos): m/z = 509 $[M+H]^+$
$^1$H-NMR (400 MHz, DMSO-d6): δ [ppm] = 9.87 (d, 1 H), 8.71 (s, 1 H), 8.00 (d, 1 H), 7.53 - 7.60 (m, 2 H), 4.88 - 5.10 (m, 2 H), 3.80 - 4.16 (m, 4 H), 3.47 - 3.72 (m, 1 H), 3.12 - 3.27 (m, 1 H), 2.88 - 3.11 (m, 1 H), 1.72 - 1.81 (m, 1 H), 1.10 (d, 3 H), 0.93 (d, 3 H), 0.91 (d, 3 H).

**Beispiel 100**

7-[(3*R*,4*S*)-3,4-Dihydroxypyrrolidin-1-yl]-6-fluor-*N*-[(2*S*)-3-methylbutan-2-yl]-4-oxo-1-(2,4,6-trifluorphenyl)-1,4-dihydro-1,8-naphthyridin-3-carboxamid

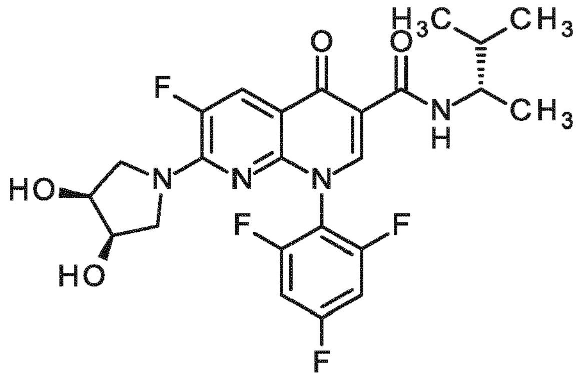

Gemäß AAV1 wurden 50.0 mg (114 µmol) 7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure mit 13.9 mg (159 µmol) (2*S*)-3-Methylbutan-2-amin in Gegenwart von 51.9 mg (137 µmol) HATU und 59 µl (340 µmol) DIPEA in 460 µl DMF zur Reaktion gebracht. Das Rohprodukt wurde mittels präparativer HPLC (Säule: Acetonitril / Wasser / 0.1% Ameisensäure) gereinigt. Es wurden 52.0 mg (90% d. Th., 100% Reinheit) der Titelverbindung erhalten.
LC-MS (Methode 3): $R_t$ = 1.74 min; MS (ESIpos): m/z = 509 $[M+H]^+$
$^1$H-NMR (400 MHz, DMSO-d6): δ [ppm] = 9.87 (d, 1 H), 8.71 (s, 1 H), 7.99 (d, 1 H), 7.53 - 7.60 (m, 2 H), 4.79 - 5.23 (m, 2 H), 3.81 - 4.10 (m, 4 H), 3.44 - 3.71 (m, 1 H), 2.86 - 3.23 (m, 2 H), 1.72 - 1.81 (m, 1 H), 1.10 (d, 3 H), 0.93 (br d, 3 H), 0.91 (br d, 3 H).

**Beispiel 101**

*N*-[(1S)-1-Cyclopropyl-2,2,2-trifluorethyl]-7-[(3*R*,4*S*)-3,4-dihydroxypyrrolidin-1-yl]-6-fluor-4-oxo-1-(2,4,6-trifluorphenyl)-1,4-dihydro-1,8-naphthyridin-3-carboxamid

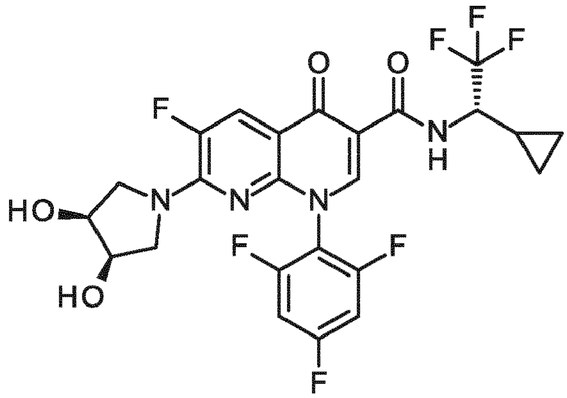

Gemäß AAV1 wurden 50.0 mg (114 µmol) 7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure mit 28.0 mg (159 µmol) (1*S*)-1-Cyclopropyl-2,2,2-trifluorethanamin Hydrochlorid in Gegenwart von 51.9 mg (137 µmol) HATU und 59 µl (340 µmol) DIPEA in 460 µl DMF zur Reaktion gebracht. Das Rohprodukt wurde mittels präparativer HPLC (Säule: Acetonitril / Wasser / 0.1% Ameisensäure) gereinigt. Es wurden 36.9 mg (58% d. Th., 100% Reinheit) der Titelverbindung erhalten.
LC-MS (Methode 3): $R_t$ = 1.85 min; MS (ESIpos): m/z = 561 $[M+H]^+$
$^1$H-NMR (400 MHz, DMSO-d6): δ [ppm] = 10.47 (d, 1 H), 8.83 (s, 1 H), 8.01 (d, 1 H), 7.53 - 7.61 (m, 2 H), 4.91 - 5.09 (m, 2 H), 4.33 - 4.43 (m, 1 H), 3.86 - 4.14 (m, 3 H), 3.39 - 3.67 (m, 1 H), 3.13 - 3.27 (m, 1 H), 2.92 - 3.12 (m, 1 H), 1.16 - 1.25 (m, 1 H), 0.50 - 0.69 (m, 3 H), 0.29 - 0.37 (m, 1 H).

**Beispiel 102**

7-[(3*R*,4*S*)-3,4-Diliydroxypyrrolidin-1-yl]-6-fluor-*N*-[(2*S*)-1-methoxy-3-methylbutan-2-yl]-4-oxo-1-(2,4,6-trifluorphenyl)-1,4-dihydro-1,8-naphthyridin-3-carboxamid

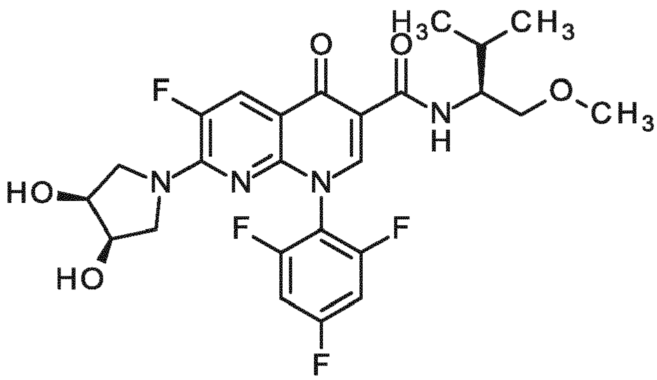

Gemäß AAV1 wurden 30.0 mg (68.3 µmol) 7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure mit 11.2 mg (95.6 µmol) (2*S*)-1-Methoxy-3-methylbutan-2-amin in Gegenwart von 31.2 mg (81.9 µmol) HATU und 36 µl (200 µmol) DIPEA in 270 µl DMF zur Reaktion gebracht. Das Rohprodukt wurde mittels präparativer HPLC (Säule: Acetonitril / Wasser / 0.1% Ameisensäure) gereinigt. Es wurden 32.1 mg (87% d. Th., 100% Reinheit) der Titelverbindung erhalten.
LC-MS (Methode 3): $R_t$ = 1.64 min; MS (ESIpos): m/z = 539 $[M+H]^+$
$^1$H-NMR (400 MHz, DMSO-d6): δ [ppm] = 9.93 (d, 1 H), 8.72 (s, 1 H), 8.00 (d, 1 H), 7.53 - 7.61 (m, 2 H), 4.82 - 5.15 (m,

2 H), 3.81 - 4.14 (m, 4 H), 3.50 - 3.73 (m, 1 H), 3.34 - 3.49 (m, 3 H), 3.13 - 3.24 (m, 1 H), 2.88 - 3.10 (m, 1 H), 1.87 - 1.97 (m, 1 H), 0.92 (d, 6 H).

**Beispiel 103**

7-[(3*R*,4*S*)-3,4-Dihydroxypyrrolidin-1-yl]-*N*-(2,4-dimethylpentan-3-yl)-6-fluor-4-oxo-1-(2,4,6-trifluorphenyl)-1,4-dihydro-1,8-naphthyridin-3-carboxamid

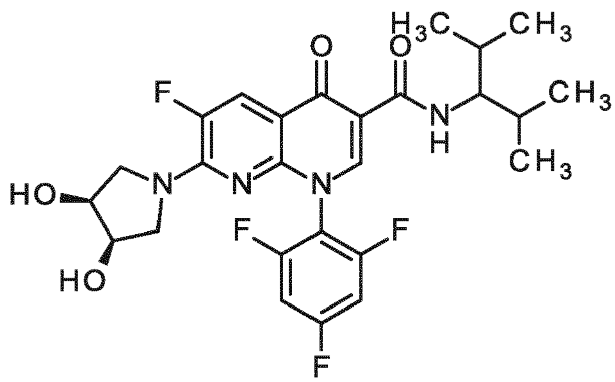

Gemäß AAV1 wurden 30.0 mg (68.3 μmol) 7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure mit 11.0 mg (95.6 μmol) 2,4-Dimethylpentan-3-amin in Gegenwart von 31.2 mg (81.9 μmol) HATU und 36 μl (200 μmol) DIPEA in 270 μl DMF zur Reaktion gebracht. Das Rohprodukt wurde mittels präparativer HPLC (Säule: Acetonitril / Wasser / 0.1% Ameisensäure) gereinigt. Es wurden 29.1 mg (79% d. Th., 100% Reinheit) der Titelverbindung erhalten.
LC-MS (Methode 3): $R_t$ = 1.94 min; MS (ESIpos): m/z = 537 $[M+H]^+$
$^1$H-NMR (400 MHz, DMSO-d6): δ [ppm] = 9.76 (d, 1 H), 8.72 (s, 1 H), 8.02 (d, 1 H), 7.53 - 7.60 (m, 2 H), 4.91 - 5.07 (m, 2 H), 3.80 - 4.15 (m, 3 H), 3.48 - 3.74 (m, 2 H), 2.89 - 3.28 (m, 2 H), 1.80 - 1.90 (m, 2 H), 0.88 (dd, 12 H).

**Beispiel 104**

7-[(3*R*,4*S*)-3,4-Dihydroxypyrrolidin-1-yl]-6-fluor-*N*-[2-methylpentan-3-yl]-4-oxo-1-(2,4,6-trifluorphenyl)-1,4-dihydro-1,8-naphthyridin-3-carboxamid (Diastereomerengemisch)

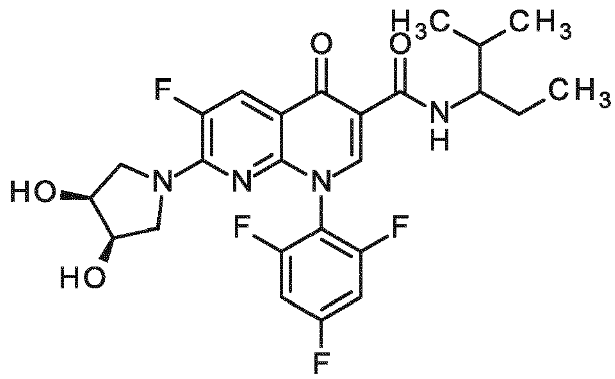

Gemäß AAV1 wurden 100 mg (228 μmol) 7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure mit 32.2 mg (319 μmol) 2-Methylpentan-3-amin in Gegenwart von 104 mg (273 μmol) HATU und 120 μl (680 μmol) DIPEA in 920 μl DMF zur Reaktion gebracht. Das Rohprodukt wurde mittels präparativer HPLC (Säule: Acetonitril / Wasser / 0.1% Ameisensäure) gereinigt. Es wurden 68.5 mg (58% d. Th., 100% Reinheit) der Titelverbindung erhalten.
LC-MS (Methode 3): $R_t$ = 1.85 min; MS (ESIpos): m/z = 523 $[M+H]^+$

$^1H$-NMR (400 MHz, DMSO-d6): δ [ppm] = 9.77 (d, 1 H), 8.71 (s, 1 H), 8.00 (d, 1 H), 7.53 - 7.61 (m, 2 H), 4.90 - 5.09 (m, 2 H), 3.86 - 4.16 (m, 3 H), 3.72 - 3.85 (m, 1 H), 3.41 - 3.69 (m, 1 H), 3.13 - 3.28 (m, 1 H), 2.90 - 3.12 (m, 1 H), 1.77 - 1.87 (m, 1 H), 1.51 - 1.62 (m, 1 H), 1.35 - 1.47 (m, 1 H), 0.84 - 0.92 (m, 9 H).

65.0 mg der Titelverbindung (Diastereomerengemisch) wurde durch chirale HPLC in die Diastereomere getrennt (präparative HPLC: Säule Daicel Chiralcel OX-H, 5 μm, 250x20 mm; Eluent: 80% *n*-Heptan, 20% Ethanol; Temperatur: 23°C; Fluss: 20 ml/min; UV-Detektion: 220 nm.)

Man erhielt (in der Reihenfolge der Elution von der Säule) 26.1 mg (22% d. Th., 100% Reinheit) Diastereomer 1 aus Beispiel 105 (99% de) Rt = 11.82 min und 32.0 mg (27% d. Th., 100% Reinheit) Diastereomer 2 aus Beispiel 106 (99% de) Rt = 15.94 min.

[Analytische HPLC: Säule Chiraltek OX-3, 3 μm; Eluent: 80% *n*-Heptan, 20% Ethanol; UV-Detektion: 220nm].

**Beispiel 105**

7-[(3*R*,4*S*)-3,4-Dihydroxypyrrolidin-1-yl]-6-fluor-*N*-[2-methylpentan-3-yl]-4-oxo-1-(2,4,6-trifluorphenyl)-1,4-dihydro-1,8-naphthyridin-3-carboxamid (Diastereomer 1)

LC-MS (Methode 3): $R_t$ = 1.89 min; MS (ESIpos): m/z = 523 $[M+H]^+$

$^1H$-NMR (400 MHz, DMSO-d6): δ [ppm] = 9.77 (d, 1 H), 8.71 (s, 1 H), 8.00 (d, 1 H), 7.53 - 7.60 (m, 2 H), 4.91 - 5.07 (m, 2 H), 3.85 - 4.15 (m, 3 H), 3.76 - 3.83 (m, 1 H), 3.43 - 3.64 (m, 1 H), 3.11 - 3.28 (m,

1 H), 2.92 - 3.10 (m, 1 H), 1.77 - 1.86 (m, 1 H), 1.51 - 1.61 (m, 1 H), 1.36 - 1.47 (m, 1 H), 0.84 - 0.92 (m, 9 H).

**Beispiel 106**

7-[(3*R*,4*S*)-3,4-Dihydroxypyrrolidin-1-yl]-6-fluor-*N*-[2-methylpentan-3-yl]-4-oxo-1-(2,4,6-trifluorphenyl)-1,4-dihydro-1,8-naphthyridin-3-carboxamid (Diastereomer 2)

LC-MS (Methode 3): $R_t$ = 1.89 min; MS (ESIpos): m/z = 523 $[M+H]^+$

$^1H$-NMR (400 MHz, DMSO-d6): δ [ppm] = 9.77 (d, 1 H), 8.71 (s, 1 H), 8.00 (d, 1 H), 7.48 - 7.66 (m, 2 H), 4.88 - 5.11 (m, 2 H), 3.86 - 4.15 (m, 3 H), 3.76 - 3.83 (m, 1 H), 3.44 - 3.69 (m, 1 H), 3.13 - 3.29 (m, 1 H), 2.87 - 3.11 (m, 1 H), 1.76 - 1.86 (m, 1 H), 1.51 - 1.62 (m, 1 H), 1.29 - 1.47 (m, 1 H), 0.84 - 0.93 (m, 9 H).

**Beispiel 107**

6-Fluor-7-[(3*S*)-3-hydroxypyrrolidin-1-yl]-4-oxo-*N*-(4,4,4-trifluor-2-methylbutan-2-yl)-1-(2,4,6-trifluorphenyl)-1,4-dihydro-1,8-naphthyridin-3-carboxamid

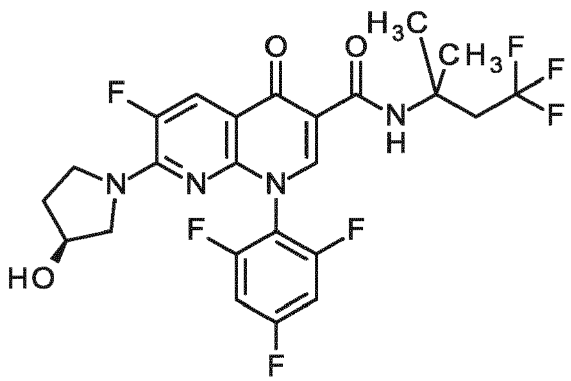

Gemäß AAV1 wurden 50.0 mg (118 µmol) 6-Fluor-7-[(3*S*)-3-hydroxypyrrolidin-1-yl]-4-oxo-1-(2,4,6-trifluorphenyl)-1,4-dihydro-1,8-naphthyridin-3-carbonsäure mit 23.1 mg (130 µmol) 4,4,4-Trifluor-2-methylbutan-2-amin Hydrochlorid in Gegenwart von 53.9 mg (142 µmol) HATU und 82 µl (470 µmol) DIPEA in 750 µl DMF zur Reaktion gebracht. Das Rohprodukt wurde mittels präparativer HPLC (Säule: Acetonitril / Wasser / 0.1% Ameisensäure) gereinigt. Es wurden 51.0 mg (79% d. Th., 100% Reinheit) der Titelverbindung erhalten.
LC-MS (Methode 3): $R_t$ = 2.06 min; MS (ESIpos): m/z = 547 $[M+H]^+$
$^1$H-NMR (400 MHz, DMSO-d6): δ [ppm] = 10.09 (s, 1 H), 8.72 (s, 1 H), 7.98 (d, 1 H), 7.56 (t, 2 H), 4.95 - 5.04 (m, 1 H), 4.18 - 4.37 (m, 1 H), 3.34 - 4.01 (m, 3 H), 3.06 - 3.27 (m, 1 H), 2.95 (q, 2 H), 1.72 - 1.98 (m, 2 H), 1.48 (s, 6 H).

## Beispiel 108

*N*-[(1*S*)-1-Cyclopropyl-2,2,2-trifluorethyl]-6-fluor-7-[(3*S*)-3-hydroxypyrrolidin-1-yl]-4-oxo-1-(2,4,6-trifluorphenyl)-1,4-dihydro-1,8-naphthyridin-3-carboxamid

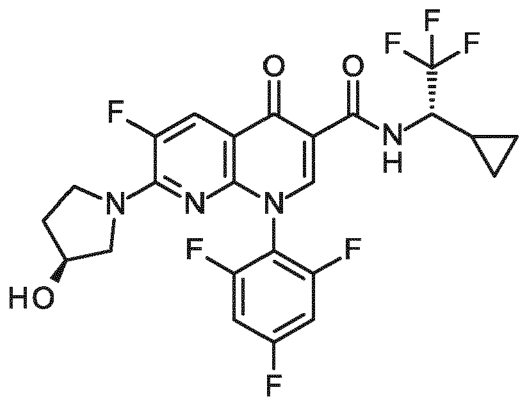

Gemäß AAV1 wurden 50.0 mg (118 µmol) 6-Fluor-7-[(3*S*)-3-hydroxypyrrolidin-1-yl]-4-oxo-1-(2,4,6-trifluorphenyl)-1,4-dihydro-1,8-naphthyridin-3-carbonsäure mit 22.8 mg (130 µmol) (1*S*)-1-Cyclopropyl-2,2,2-trifluorethanamin Hydrochlorid in Gegenwart von 53.9 mg (142 µmol) HATU und 82 µl (470 µmol) DIPEA in 750 µl DMF zur Reaktion gebracht. Das Rohprodukt wurde mittels präparativer HPLC (Säule: Acetonitril / Wasser / 0.1% Ameisensäure) gereinigt. Es wurden 50.3 mg (78% d. Th., 100% Reinheit) der Titelverbindung erhalten.
LC-MS (Methode 3): $R_t$ = 2.07 min; MS (ESIpos): m/z = 545 $[M+H]^+$
$^1$H-NMR (400 MHz, DMSO-d6): δ [ppm] = 10.48 (d, 1 H), 8.83 (s, 1 H), 8.01 (d, 1 H), 7.53 - 7.60 (m, 2 H), 4.97 - 5.04 (m, 1 H), 4.21 - 4.43 (m, 2 H), 3.34 - 4.03 (m, 3 H), 3.01 - 3.29 (m, 1 H), 1.74 - 1.98 (m, 2 H), 1.16 - 1.25 (m, 1 H), 0.50 - 0.69 (m, 3 H), 0.30 - 0.37 (m, 1 H).

## Beispiel 109

6-Fluor-7-[(3*S*)-3-hydroxypyrrolidin-1-yl]-4-oxo-*N*-[1-(trifluormethoxy)butan-2-yl]-1-(2,4,6-trifluorphenyl)-1,4-dihydro-1,8-naphthyridin-3-carboxamid (Diastereomerengemisch)

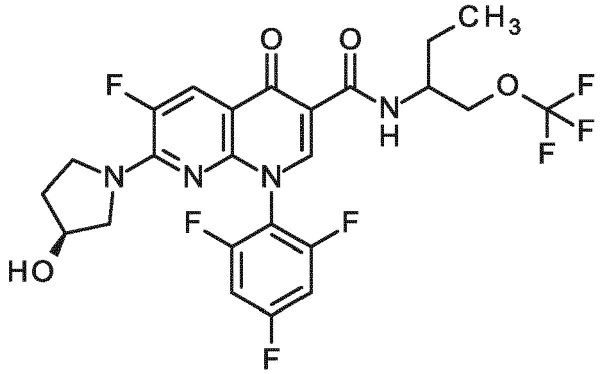

Gemäß AAV1 wurden 100 mg (236 μmol) 6-Fluor-7-[(3*S*)-3-hydroxypyrrolidin-1-yl]-4-oxo-1-(2,4,6-trifluorphenyl)-1,4-dihydro-1,8-naphthyridin-3-carbonsäure mit 50.3 mg (260 μmol) 1-(Trifluor-methoxy)butan-2-amin Hydrochlorid (Racemat) in Gegenwart von 108 mg (283 μmol) HATU und 160 μl (940 μmol) DIPEA in 1.5 ml DMF zur Reaktion gebracht. Das Rohprodukt wurde mittels präparativer HPLC (Säule: Acetonitril / Wasser / 0.1% Ameisensäure) gereinigt. Es wurden 89.3 mg (67% d. Th., 100% Reinheit) der Titelverbindung erhalten.
LC-MS (Methode 3): $R_t$ = 2.08 min; MS (ESIpos): m/z = 563 $[M+H]^+$
$^1$H-NMR (400 MHz, DMSO-d6): δ [ppm] = 10.00 (br d, 1 H), 8.75 (s, 1 H), 8.00 (d, 1 H), 7.56 (br t, 2 H), 4.95 - 5.04 (m, 1 H), 4.24 - 4.35 (m, 1 H), 4.12 - 4.24 (m, 3 H), 3.33 - 4.07 (m, 3 H), 3.02 - 3.29 (m, 1 H), 1.74 - 2.00 (m, 2 H), 1.55 - 1.73 (m, 2 H), 0.94 (t, 3 H).

88.0 mg der Titelverbindung (Diastereomerengemisch) wurde durch chirale HPLC in die Diastereomere getrennt (präparative HPLC: Säule Daicel Chiralpak IE, 5 μm, 250x20 mm; Eluent: 85% *n*-Heptan, 15% Ethanol + 0.2% DEA; Temperatur: 23°C; Fluss: 20 ml/min; UV-Detektion: 220 nm.)

Man erhielt (in der Reihenfolge der Elution von der Säule) 22.6 mg (17% d. Th., 95% Reinheit) Diastereomer 1 aus Beispiel 110 (99% de) Rt = 11.90 min und 24.7 mg (19% d. Th., 95% Reinheit) Diastereomer 2 aus Beispiel 111 (93% de) Rt = 13.32 min.

[Analytische HPLC: Säule Daicel Chiralpak IE-3, 3 μm, 50x4.6 mm; Eluent: 90% *n*-Heptan, 10% Ethanol + 0.2% DEA; Fluss: 1.0 ml/min; UV-Detektion: 220nm].

## Beispiel 110

6-Fluor-7-[(3*S*)-3-hydroxypyrrolidin-1-yl]-4-oxo-*N*-[1-(trifluormethoxy)butan-2-yl]-1-(2,4,6-trifluorphenyl)-1,4-dihydro-1,8-naphthyridin-3-carboxamid (Diastereomer 1)

LC-MS (Methode 3): $R_t$ = 2.08 min; MS (ESIpos): m/z = 563 $[M+H]^+$

$^1$H-NMR (500 MHz, DMSO-d6): δ [ppm] = 10.00 (d, 1 H), 8.75 (s, 1 H), 8.00 (d, 1 H), 7.56 (br t, 2 H), 4.96 - 5.03 (m, 1 H), 4.23 - 4.36 (m, 1 H), 4.13 - 4.22 (m, 3 H), 3.36 - 4.04 (m, 2 H), 2.96 - 3.29 (m, 1 H), 1.74 - 2.00 (m, 2 H), 1.54 - 1.73 (m, 2 H), 0.94 (t, 3 H).

## Beispiel 111

6-Fluor-7-[(3*S*)-3-hydroxypyrrolidin-1-yl]-4-oxo-*N*-[1-(trifluormethoxy)butan-2-yl]-1-(2,4,6-trifluorphenyl)-1,4-dihydro-1,8-naphthyridin-3-carboxamid (Diastereomer 2)

LC-MS (Methode 3): $R_t$ = 2.08 min; MS (ESIpos): m/z = 563 $[M+H]^+$

$^1$H-NMR (500 MHz, DMSO-d6): δ [ppm] = 10.00 (d, 1 H), 8.75 (s, 1 H), 8.00 (d, 1 H), 7.53 - 7.59 (m, 2 H), 4.96 - 5.03 (m, 1 H), 4.23 - 4.35 (m, 1 H), 4.13 - 4.22 (m, 3 H), 3.33 - 4.01 (m, 3 H), 3.05 - 3.29 (m, 1 H), 1.73 - 1.99 (m, 2 H), 1.54 - 1.72 (m, 2 H), 0.94 (t, 3 H).

**Beispiel 112**

6-Fluor-7-[(3*S*)-3-hydroxypyrrolidin-1-yl]-4-oxo-*N*-[3,3,4,4,4-pentafluorbutan-2-yl]-1-(2,4,6-trifluorphenyl)-1,4-dihydro-1,8-naphthyridin-3-carboxamid (Diastereomerengemisch)

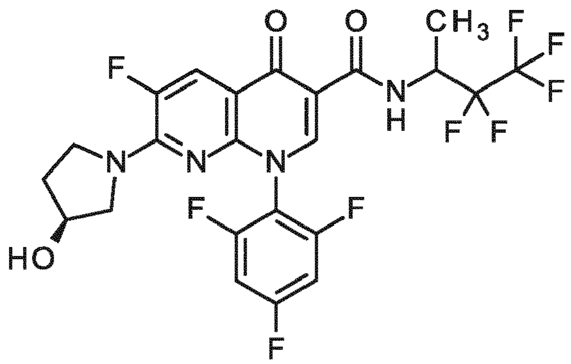

Gemäß AAV1 wurden 100 mg (236 μmol) 6-Fluor-7-[(3*S*)-3-hydroxypyrrolidin-1-yl]-4-oxo-1-(2,4,6-trifluorphenyl)-1,4-dihydro-1,8-naphthyridin-3-carbonsäure mit 51.9 mg (260 μmol) 3,3,4,4,4-Pentafluorbutan-2-amin Hydrochlorid (Racemat) in Gegenwart von 108 mg (283 μmol) HATU und 160 μl (940 μmol) DIPEA in 1.5 ml DMF zur Reaktion gebracht. Das Rohprodukt wurde mittels präparativer HPLC (Säule: Acetonitril / Wasser / 0.1% Ameisensäure) gereinigt. Es wurden 107 mg (80% d. Th., 100% Reinheit) der Titelverbindung erhalten.
LC-MS (Methode 3): $R_t$ = 2.10 min; MS (ESIpos): m/z = 569 $[M+H]^+$
$^1$H-NMR (400 MHz, DMSO-d6): δ [ppm] = 10.47 (d, 1 H), 8.84 (s, 1 H), 8.00 (d, 1 H), 7.56 (t, 2 H), 4.95 - 5.08 (m, 2 H), 4.19 - 4.37 (m, 1 H), 3.34 - 4.06 (m, 3 H), 3.01 - 3.28 (m, 1 H), 1.73 - 1.98 (m, 2 H), 1.39 (d, 3 H).
105 mg der Titelverbindung (Diastereomerengemisch) wurde durch chirale SFC in die Diastereomere getrennt (präparative SFC: Säule Chiralpak AD, 250x20 mm; Eluent: 80% Kohlendioxid, 20% Iso-Propanol; Temperatur: 40°C; Fluss: 60 ml/min; UV-Detektion: 210 nm.)
Man erhielt (in der Reihenfolge der Elution von der Säule) 39.2 mg (29% d. Th., 100% Reinheit) Diastereomer 1 aus Beispiel 113 (99% de) Rt = 2.07 min und 32.8 mg (25% d. Th., 100% Reinheit) Diastereomer 2 aus Beispiel 114 (99% de) Rt = 2.59 min.
[Analytische SFC: Säule AD; Eluent: 80% Kohlendioxid, 20% Iso-Propanol; Fluss: 3.0 ml/min; UV-Detektion: 210nm].

**Beispiel 113**

6-Fluor-7-[(3*S*)-3-hydroxypyrrolidin-1-yl]-4-oxo-*N*-[3,3,4,4,4-pentafluorbutan-2-yl]-1-(2,4,6-trifluorphenyl)-1,4-dihydro-1,8-naphthyridin-3-carboxamid (Diastereomer 1)

LC-MS (Methode 3): $R_t$ = 2.11 min; MS (ESIpos): m/z = 569 $[M+H]^+$

$^1$H-NMR (400 MHz, DMSO-d6): δ [ppm] = 10.47 (d, 1 H), 8.84 (s, 1 H), 8.00 (d, 1 H), 7.57 (br t, 2 H), 4.95 - 5.08 (m, 2 H), 4.21 - 4.37 (m, 1 H), 3.36 - 4.05 (m, 3 H), 3.01 - 3.27 (m, 1 H), 1.72 - 1.98 (m, 2 H), 1.39 (d, 3 H).

**Beispiel 114**

6-Fluor-7-[(3*S*)-3-hydroxypyrrolidin-1-yl]-4-oxo-*N*-[3,3,4,4,4-pentafluorbutan-2-yl]-1-(2,4,6-trifluorphenyl)-1,4-dihydro-1,8-naphthyridin-3-carboxamid (Diastereomer 2)

LC-MS (Methode 3): $R_t$ = 2.11 min; MS (ESIpos): m/z = 569 $[M+H]^+$

$^1$H-NMR (400 MHz, DMSO-d6): δ [ppm] = 10.47 (d, 1 H), 8.84 (s, 1 H), 8.00 (d, 1 H), 7.53 - 7.60 (m, 2 H), 4.96 -

5.07 (m, 2 H), 4.26 - 4.34 (m, 1 H), 3.34 - 3.98 (m, 3 H), 3.00 - 3.26 (m, 1 H), 1.70 - 2.01 (m, 2 H), 1.39 (d, 3 H).

**Beispiel 115**

6-Fluor-7-[(3*S*)-3-hydroxypyrrolidin-1-yl]-4-oxo-*N*-[(2*S*)-1,1,1-trifluorbutan-2-yl]-1-(2,4,6-trifluorphenyl)-1,4-dihydro-1,8-naphthyridin-3-carboxamid

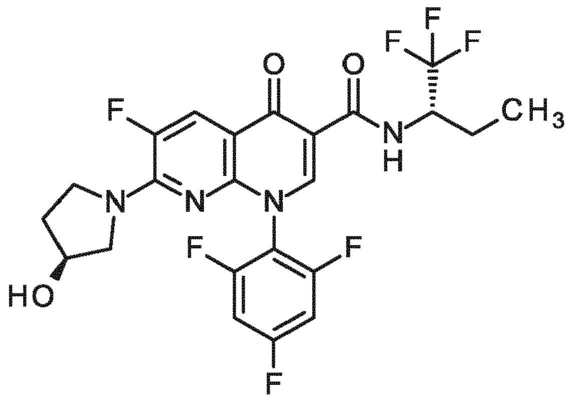

Gemäß AAV1 wurden 50.0 mg (118 µmol) 6-Fluor-7-[(3*S*)-3-hydroxypyrrolidin-1-yl]-4-oxo-1-(2,4,6-trifluorphenyl)-1,4-dihydro-1,8-naphthyridin-3-carbonsäure mit 21.3 mg (130 µmol) (2*S*)-1,1,1-Trifluorbutan-2-amin Hydrochlorid in Gegenwart von 53.9 mg (142 µmol) HATU und 82 µl (470 µmol) DIPEA in 750 µl DMF zur Reaktion gebracht. Das Rohprodukt wurde mittels präparativer HPLC (Säule: Acetonitril / Wasser / 0.1% Ameisensäure) gereinigt. Es wurden 46.8 mg (74% d. Th., 100% Reinheit) der Titelverbindung erhalten.
LC-MS (Methode 3): $R_t$ = 2.05 min; MS (ESIpos): m/z = 533 $[M+H]^+$
$^1$H-NMR (400 MHz, DMSO-d6): δ [ppm] = 10.34 (d, 1 H), 8.83 (s, 1 H), 8.00 (d, 1 H), 7.53 - 7.61 (m, 2 H), 4.96 - 5.05 (m, 1 H), 4.68 - 4.79 (m, 1 H), 4.19 - 4.39 (m, 1 H), 3.33 - 4.04 (m, 3 H), 3.02 - 3.28 (m, 1 H), 1.72 - 1.97 (m, 3 H), 1.58 - 1.70 (m, 1 H), 0.97 (t, 3 H).

**Beispiel 116**

6-Fluor-7-[(3*S*)-3-hydroxypyrrolidin-1-yl]-*N*-[2-methylpentan-3-yl]-4-oxo-1-(2,4,6-trifluorphenyl)-1,4-dihydro-1,8-naphthyridin-3-carboxamid (Diastereomerengemisch)

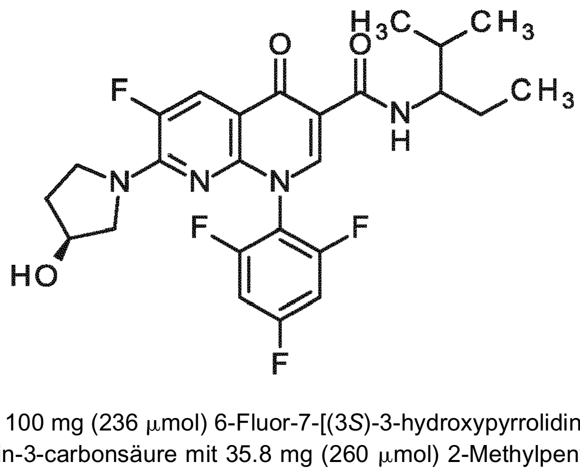

Gemäß AAV1 wurden 100 mg (236 µmol) 6-Fluor-7-[(3*S*)-3-hydroxypyrrolidin-1-yl]-4-oxo-1-(2,4,6-trifluorphenyl)-1,4-dihydro-1,8-naphthyridin-3-carbonsäure mit 35.8 mg (260 µmol) 2-Methylpentan-3-amin Hydrochlorid (Racemat) in Gegenwart von 108 mg (283 µmol) HATU und 160 µl (940 µmol) DIPEA in 1.5 ml DMF zur Reaktion gebracht. Das Rohprodukt wurde mittels präparativer HPLC (Säule: Acetonitril / Wasser / 0.1% Ameisensäure) gereinigt. Es wurden 90.1 mg (75% d. Th., 100% Reinheit) der Titelverbindung erhalten.

LC-MS (Methode 3): $R_t$ = 2.10 min; MS (ESIpos): m/z = 507 $[M+H]^+$
$^1$H-NMR (400 MHz, DMSO-d6): δ [ppm] = 9.78 (d, 1 H), 8.71 (s, 1 H), 8.00 (d, 1 H), 7.56 (br t, 2 H), 4.96 - 5.03 (m, 1 H), 4.20 - 4.38 (m, 1 H), 3.35 - 4.05 (m, 4 H), 3.01 - 3.30 (m, 1 H), 1.73 - 1.98 (m, 3 H), 1.51 - 1.62 (m, 1 H), 1.36 - 1.47 (m, 1 H), 0.84 - 0.92 (m, 9 H).

99 mg der Titelverbindung (Diastereomerengemisch) wurde durch chirale HPLC in die Diastereomere getrennt (präparative HPLC: Säule Daicel Chiralpak AY-H, 5 μm, 250x20 mm; Eluent: 70% n-Heptan, 30% Ethanol + 0.2% DEA; Temperatur: 60°C; Fluss: 15 ml/min; UV-Detektion: 260 nm.)

Man erhielt (in der Reihenfolge der Elution von der Säule) 21.0 mg (17% d. Th., 100% Reinheit) Diastereomer 1 aus Beispiel 117 (97% de) Rt = 4.45 min und 23.0 mg (19% d. Th., 100% Reinheit) Diastereomer 2 aus Beispiel 118 (76% de) Rt = 7.56 min.

[Analytische SFC: Säule Daicel Chiralpak AY-H, 5 μm, 250x4.6 mm; Eluent: 70% Iso-Hexan, 30% Ethanol + 0.2% DEA; Temperatur: 60°C; Fluss: 1.0 ml/min; UV-Detektion: 260nm].

**Beispiel 117**

6-Fluor-7-[(3*S*)-3-hydroxypyrrolidin-1-yl]-N-[2-methylpentan-3-yl]-4-oxo-1-(2,4,6-trifluorphenyl)-1,4-dihydro-1,8-naphthyridin-3-carboxamid (Diastereomer 1)

LC-MS (Methode 3): $R_t$ = 2.10 min; MS (ESIpos): m/z = 507 $[M+H]^+$

$^1$H-NMR (400 MHz, DMSO-d6): δ [ppm] = 9.78 (d, 1 H), 8.71 (s, 1 H), 8.00 (d, 1 H), 7.53 - 7.59 (m, 2 H), 4.98 - 5.01 (m, 1 H), 4.26 - 4.32 (m, 1 H), 3.36 - 4.10 (m, 4 H), 2.99 - 3.27 (m, 1 H), 1.76 - 1.94 (m, 3 H), 1.52 - 1.60 (m, 1 H), 1.37 - 1.45 (m, 1 H), 0.84 - 0.92 (m, 9 H).

**Beispiel 118**

6-Fluor-7-[(3*S*)-3-hydroxypyrrolidin-1-yl]-*N*-[2-methylpentan-3-yl]-4-oxo-1-(2,4,6-trifluorphenyl)-1,4-dihydro-1,8-naphthyridin-3-carboxamid (Diastereomer 2)

LC-MS (Methode 3): $R_t$ = 2.11 min; MS (ESIpos): m/z = 507 $[M+H]^+$

$^1$H-NMR (400 MHz, DMSO-d6): δ [ppm] = 9.78 (d, 1 H), 8.71 (s, 1 H), 8.00 (d, 1 H), 7.53 - 7.60 (m, 2 H), 4.96 - 5.02 (m, 1 H), 4.24 - 4.34 (m, 1 H), 3.33 - 4.08 (m, 3 H), 3.07 - 3.29 (m, 1 H), 1.75 - 1.96 (m, 3 H), 1.51 - 1.63 (m, 1 H), 1.36 - 1.47 (m, 1 H), 0.83 - 0.92 (m, 9 H).

**Beispiel 119**

6-Fluor-7-[(3*S*)-3-hydroxypyrrolidin-1-yl]-*N*-[(2*S*)-3-methylbutan-2-yl]-4-oxo-1-(2,4,6-trifluorphenyl)-1,4-dihydro-1,8-naphthyridin-3-carboxamid

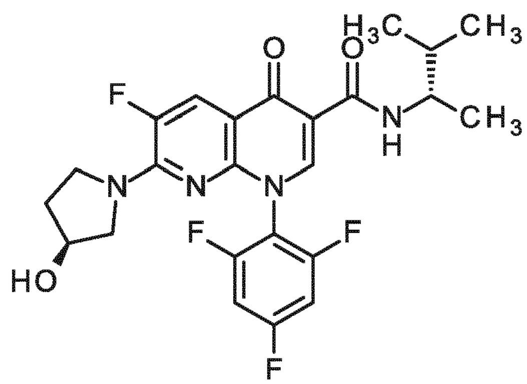

Gemäß AAV1 wurden 50.0 mg (118 μmol) 6-Fluor-7-[(3*S*)-3-hydroxypyrrolidin-1-yl]-4-oxo-1-(2,4,6-trifluophenyl)-1,4-dihydro-1,8-naphthyridin-3-carbonsäure mit 11.3 mg (130 μmol) (2*S*)-3-Methylbutan-2-amin in Gegenwart von 53.9 mg (142 μmol) HATU und 62 μl (350 μmol) DIPEA in 750 μl DMF zur Reaktion gebracht. Das Rohprodukt wurde mittels präparativer HPLC (Säule: Acetonitril / Wasser / 0.1% Ameisensäure) gereinigt. Es wurden 45.2 mg (78% d. Th., 100% Reinheit) der Titelverbindung erhalten.
LC-MS (Methode 3): $R_t$ = 1.99 min; MS (ESIpos): m/z = 493 $[M+H]^+$
$^1H$-NMR (400 MHz, DMSO-d6): δ [ppm] = 9.88 (d, 1 H), 8.70 (s, 1 H), 7.99 (d, 1 H), 7.56 (br t, 2 H), 4.95 - 5.03 (m, 1 H), 4.19 - 4.37 (m, 1 H), 3.33 - 4.10 (m, 4 H), 3.01 - 3.26 (m, 1 H), 1.70 - 1.96 (m, 3 H), 1.10 (d, 3 H), 0.88 - 0.95 (m, 6 H).

## Beispiel 120

6-Fluor-7-[(3*S*)-3-hydroxypyrrolidin-1-yl]-*N*-[(2*R*)-3-methylbutan-2-yl]-4-oxo-1-(2,4,6-trifluorphenyl)-1,4-dihydro-1,8-naphthyridin-3-carboxamid

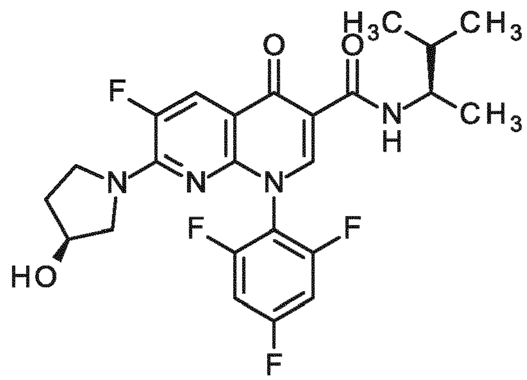

Gemäß AAV1 wurden 50.0 mg (118 μmol) 6-Fluor-7-[(3*S*)-3-hydroxypyrrolidin-1-yl]-4-oxo-1-(2,4,6-trifluorphenyl)-1,4-dihydro-1,8-naphthyridin-3-carbonsäure mit 11.3 mg (130 μmol) (2R)-3-Methylbutan-2-amin in Gegenwart von 53.9 mg (142 μmol) HATU und 62 μl (350 μmol) DIPEA in 750 μl DMF zur Reaktion gebracht. Das Rohprodukt wurde mittels präparativer HPLC (Säule: Acetonitril / Wasser / 0.1% Ameisensäure) gereinigt. Es wurden 45.8 mg (79% d. Th., 100% Reinheit) der Titelverbindung erhalten.
LC-MS (Methode 3): $R_t$ = 1.99 min; MS (ESIpos): m/z = 493 $[M+H]^+$
$^1H$-NMR (400 MHz, DMSO-d6): δ [ppm] = 9.88 (d, 1 H), 8.70 (s, 1 H), 7.99 (d, 1 H), 7.56 (br t, 2 H), 4.96 - 5.03 (m, 1 H), 4.21 - 4.37 (m, 1 H), 3.36 - 4.11 (m, 4 H), 3.02 - 3.28 (m, 1 H), 1.71 - 1.97 (m, 3 H), 1.10 (d, 3 H), 0.88 - 0.96 (m, 6 H).

## Beispiel 121

6-Fluor-7-[(3*S*)-3-hydroxypyrrolidin-1-yl]-*N*-[(2*R*)-1-methoxy-3-methylbutan-2-yl]-4-oxo-1-(2,4,6-trifluorphenyl)-1,4-dihydro-1,8-naphthyridin-3-carboxamid

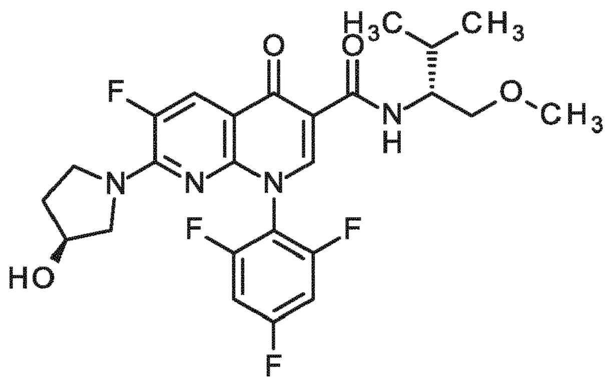

Gemäß AAV1 wurden 50.0 mg (118 μmol) 6-Fluor-7-[(3*S*)-3-hydroxypyrrolidin-1-yl]-4-oxo-1-(2,4,6-trifluorphenyl)-1,4-dihydro-1,8-naphthyridin-3-carbonsäure mit 20.0 mg (130 μmol) (2*R*)-1-Methoxy-3-methylbutan-2-amin Hydrochlorid in Gegenwart von 53.9 mg (142 μmol) HATU und 62 μl (350 μmol) DIPEA in 750 μl DMF zur Reaktion gebracht. Das Rohprodukt wurde mittels präparativer HPLC (Säule: Acetonitril / Wasser / 0.1% Ameisensäure) gereinigt. Es wurden 45.5 mg (74% d. Th., 100% Reinheit) der Titelverbindung erhalten.
LC-MS (Methode 3): $R_t$ = 1.88 min; MS (ESIpos): m/z = 523 $[M+H]^+$
$^1$H-NMR (400 MHz, DMSO-d6): δ [ppm] = 9.94 (d, 1 H), 8.72 (s, 1 H), 8.00 (d, 1 H), 7.52 - 7.60 (m, 2 H), 4.94 - 5.05 (m, 1 H), 4.29 (br s, 1 H), 3.96 - 4.03 (m, 1 H), 3.50 - 3.94 (m, 2 H), 3.34 - 3.49 (m, 3 H), 3.27 (s, 3 H), 2.90 - 3.24 (m, 1 H), 1.74 - 1.99 (m, 3 H), 0.92 (d, 6 H).

## Beispiel 122

*N*-(2,4-Dimethylpentan-3-yl)-6-fluor-7-[(3*S*)-3-hydroxypyrrolidin-1-yl]-4-oxo-1-(2,4,6-trifluorphenyl)-1,4-dihydro-1,8-naphthyridin-3-carboxamid

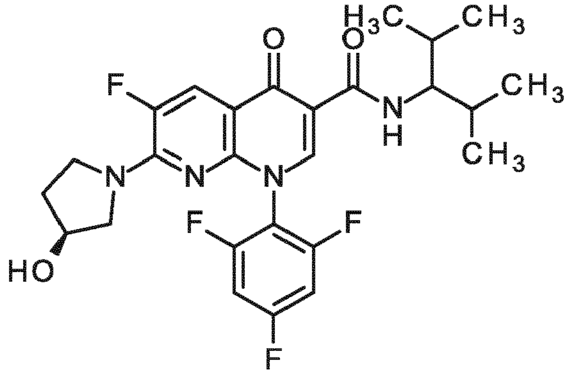

Gemäß AAV1 wurden 50.0 mg (118 μmol) 6-Fluor-7-[(3*S*)-3-hydroxypyrrolidin-1-yl]-4-oxo-1-(2,4,6-trifluorphenyl)-1,4-dihydro-1,8-naphthyridin-3-carbonsäure mit 15.0 mg (130 μmol) 2,4-Dimethyl-pentan-3-amin in Gegenwart von 53.9 mg (142 μmol) HATU und 62 μl (350 μmol) DIPEA in 750 μl DMF zur Reaktion gebracht. Das Rohprodukt wurde mittels präparativer HPLC (Säule: Acetonitril / Wasser / 0.1 % Ameisensäure) gereinigt. Es wurden 50.7 mg (82% d. Th., 100% Reinheit) der Titelverbindung erhalten.
LC-MS (Methode 3): $R_t$ = 2.19 min; MS (ESIpos): m/z = 521 $[M+H]^+$
$^1$H-NMR (400 MHz, DMSO-d6): δ [ppm] = 9.77 (d, 1 H), 8.72 (s, 1 H), 8.01 (d, 1 H), 7.56 (br t, 2 H), 4.96 - 5.03 (m, 1 H), 4.21 - 4.36 (m, 1 H), 3.37 - 3.98 (m, 4 H), 3.01 - 3.27 (m, 1 H), 1.74 - 1.96 (m, 4 H), 0.88 (dd, 12 H).

## Beispiel 123

7-[(3*R*,4*S*)-3,4-Dihydroxypyrrolidin-1-yl]-6-fluor-4-oxo-*N*-[1,1,1-trifluor-3-methylbutan-2-yl]-1-(2,4,6-trifluorphenyl)-1,4-dihydro-1,8-naphthyridin-3-carboxamid (Diastereomerengemisch)

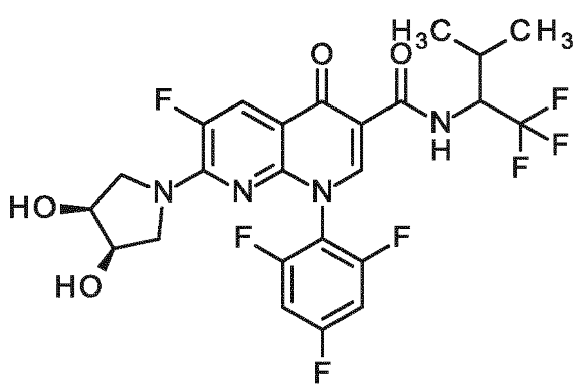

Gemäß AAV1 wurden 100 mg (228 μmol) 7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure mit 36.4 mg (250 μmol, 97% Reinheit) 1,1,1-Trifluor-3-methylbutan-2-amin (Racemat) in Gegenwart von 104 mg (273 μmol) HATU und 160 μl (910 μmol) DIPEA in 2.0 ml DMF zur Reaktion gebracht. Das Rohprodukt wurde mittels präparativer HPLC (Säule: Acetonitril / Wasser / 0.1% Ameisensäure) gereinigt. Es wurden 52.0 mg (41% d. Th., 100% Reinheit) der Titelverbindung erhalten.
LC-MS (Methode 3): $R_t$ = 1.94 min; MS (ESIpos): m/z = 563 $[M+H]^+$
$^1$H-NMR (400 MHz, DMSO-d6): δ [ppm] = 10.53 (d, 1 H), 8.84 (s, 1 H), 8.04 (d, 1 H), 7.58 (br t, 2 H), 4.92 - 5.08 (m, 2 H), 4.71 - 4.81 (m, 1 H), 3.86 - 4.12 (m, 3 H), 3.47 - 3.68 (m, 1 H), 2.88 - 3.25 (m, 2 H), 2.18 - 2.30 (m, 1 H), 1.02 (d, 3 H), 0.96 (d, 3 H).

**Beispiel 124**

7-[(3*R*,4*S*)-3,4-Dihydroxypyrrolidin-1-yl]-6-fluor-4-oxo-*N*-(1,1,1-trifluor-2-methylpropan-2-yl)-1-(2,4,6-trifluorphenyl)-1,4-dihydro-1,8-naphthyridin-3-carboxamid

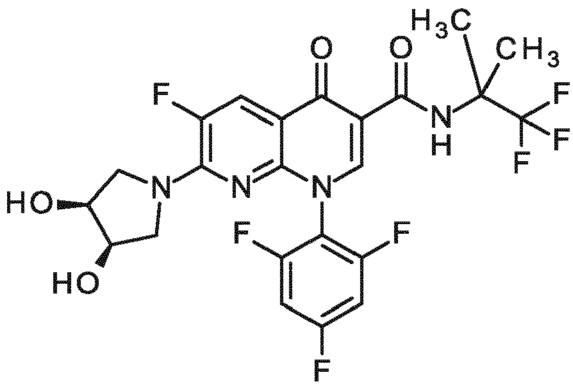

Gemäß AAV1 wurden 50.0 mg (114 μmol) 7-[(3*R*,4*S*)-3,4-Dihydroxypyrrolidin-1-yl]-6-fluor-4-oxo-1-(2,4,6-trifluorphenyl)-1,4-dihydro-1,8-naphthyridin-3-carbonsäure mit 15.9 mg (125 μmol) 1,1,1-Trifluor-2-methylpropan-2-amin in Gegenwart von 51.9 mg (137 μmol) HATU und 59 μl (340 μmol) DIPEA in 1.0 ml DMF zur Reaktion gebracht. Das Rohprodukt wurde mittels präparativer HPLC (Säule: Acetonitril / Wasser / 0.1% Ameisensäure) gereinigt. Es wurden 45.0 mg (72% d. Th., 100% Reinheit) der Titelverbindung erhalten.
LC-MS (Methode 3): $R_t$ = 1.87 min; MS (ESIpos): m/z = 549 $[M+H]^+$
$^1$H-NMR (400 MHz, DMSO-d6): δ [ppm] = 10.55 (s, 1 H), 8.77 (s, 1 H), 8.01 (d, 1 H), 7.54 - 7.61 (m, 2 H), 4.89 - 5.10 (m, 2 H), 3.79 - 4.14 (m, 3 H), 3.44 - 3.67 (m, 1 H), 3.12 - 3.28 (m, 1 H), 2.87 - 3.12 (m, 1 H), 1.63 (s, 6 H).

**Beispiel 125**

6-Fluor-7-[(3*S*)-3-hydroxypyrrolidin-1-yl]-4-oxo-*N*-(1,1,1-trifluor-2-methylpropan-2-yl)-1-(2,4,6-trifluorphenyl)-1,4-dihydro-1,8-naphthyridin-3-carboxamid

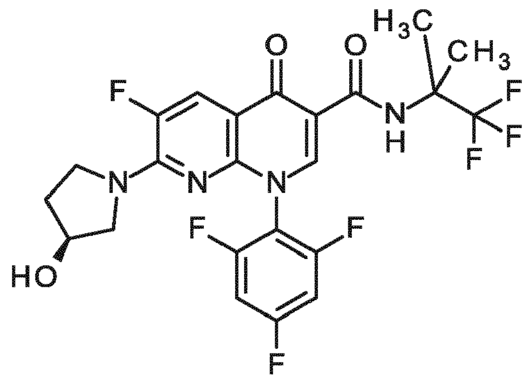

Gemäß AAV1 wurden 20.0 mg (47.2 µmol) 6-Fluor-7-[(3*S*)-3-hydroxypyrrolidin-1-yl]-4-oxo-1-(2,4,6-trifluorphenyl)-1,4-dihydro-1,8-naphthyridin-3-carbonsäure mit 6.60 mg (52.0 µmol) 1,1,1-Trifluor-2-methylpropan-2-amin in Gegenwart von 21.6 mg (56.7 µmol) HATU und 25 µl (140 µmol) DIPEA in 420 µl DMF zur Reaktion gebracht. Das Rohprodukt wurde mittels präparativer HPLC (Säule: Acetonitril / Wasser/ 0.1 % Ameisensäure) gereinigt. Es wurden 18.0 mg (72% d. Th., 100% Reinheit) der Titelverbindung erhalten.
LC-MS (Methode 1): $R_t$ = 1.10 min; MS (ESIpos): m/z = 533 $[M+H]^+$
$^1$H-NMR (400 MHz, DMSO-d6): δ [ppm] = 10.56 (s, 1 H), 8.77 (s, 1 H), 8.01 (d, 1 H), 7.53 - 7.61 (m, 2 H), 4.96 - 5.03 (m, 1 H), 4.20 - 4.35 (m, 1 H), 3.37 - 4.07 (m, 3 H), 2.98 - 3.26 (m, 1 H), 1.74 - 2.00 (m, 2 H), 1.63 (s, 6 H).

**Beispiel 126**

6-Fluor-7-[(3*S*)-3-hydroxypyrrolidin-1-yl]-4-oxo-*N*-[1,1,1-trifluor-3-methylbutan-2-yl]-1-(2,4,6-trifluorphenyl)-1,4-dihydro-1,8-naphthyridin-3-carboxamid (Diastereomerengemisch)

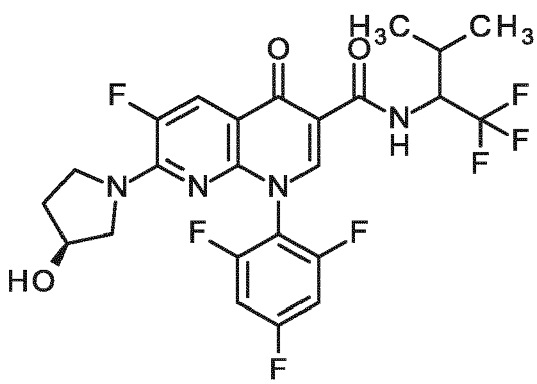

Gemäß AAV1 wurden 65.0 mg (154 µmol) 6-Fluor-7-[(3*S*)-3-hydroxypyrrolidin-1-yl]-4-oxo-1-(2,4,6-trifluorphenyl)-1,4-dihydro-1,8-naphthyridin-3-carbonsäure mit 24.6 mg (169 µmol, 97% Reinheit) 1,1,1-Trifluor-3-methylbutan-2-amin (Racemat) in Gegenwart von 70.1 mg (184 µmol) HATU und 80 µl (460 µmol) DIPEA in 1.3 ml DMF zur Reaktion gebracht. Das Rohprodukt wurde mittels präparativer HPLC (Säule: Acetonitril / Wasser / 0.1 % Ameisensäure) gereinigt. Es wurden 54.0 mg (64% d. Th., 99% Reinheit) der Titelverbindung erhalten.
LC-MS (Methode 3): $R_t$ = 2.16 min; MS (ESIpos): m/z = 547 $[M+H]^+$
$^1$H-NMR (400 MHz, DMSO-d6): δ [ppm] = 10.54 (d, 1 H), 8.84 (s, 1 H), 8.04 (d, 1 H), 7.54 - 7.60 (m, 2 H), 4.97 - 5.04 (m, 1 H), 4.71 - 4.82 (m, 1 H), 4.24 - 4.36 (m, 1 H), 3.33 - 4.10 (m, 3 H), 2.97 - 3.27 (m, 1 H), 2.20 - 2.28 (m, 1 H), 1.71 - 2.00 (m, 2 H), 1.03 (d, 3 H), 0.96 (d, 3 H).

**Beispiel 127**

1-(3,5-Difluorpyridin-2-yl)-6-fluor-7-[(3*S*)-3-hydroxypyrrolidin-1-yl]-4-oxo-*N*-[3,3,4,4,4-pentafluorbutan-2-yl]-1,4-dihydro-1,8-naphthyridin-3-carboxamid (Diastereomerengemisch)

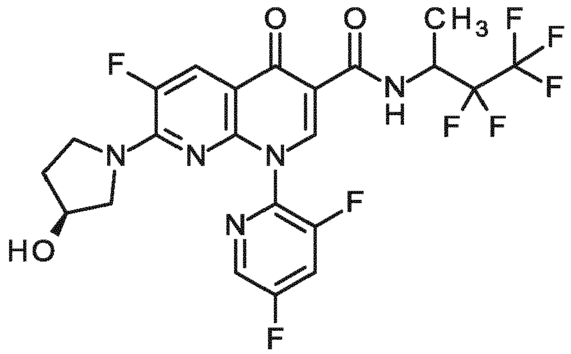

Gemäß AAV1 wurden 100 mg (246 µmol) 1-(3,5-Difluorpyridin-2-yl)-6-fluor-7-[(3*S*)-3-hydroxypyrrolidin-1-yl]-4-oxo-1,4-dihydro-1,8-naphthyridin-3-carbonsäure mit 54.0 mg (271 µmol) 3,3,4,4,4-Pentafluorbutan-2-amin Hydrochlorid (Racemat) in Gegenwart von 112 mg (295 µmol) HATU und 170 µl (980 µmol) DIPEA in 2.2 ml DMF zur Reaktion gebracht. Das Rohprodukt wurde mittels präparativer HPLC (Säule: Acetonitril / Wasser / 0.1% Ameisensäure) gereinigt. Es wurden 100 mg (74% d. Th., 100% Reinheit) der Titelverbindung erhalten.
LC-MS (Methode 3): $R_t$ = 1.98 min; MS (ESIpos): m/z = 552 $[M+H]^+$
$^1$H-NMR (400 MHz, DMSO-d6): δ [ppm] = 10.46 (d, 1 H), 8.83 (d, 1 H), 8.61 (d, 1 H), 8.30 - 8.37 (m, 1 H), 8.00 (d, 1 H), 4.93 - 5.09 (m, 2 H), 4.20 - 4.39 (m, 1 H), 3.35 - 4.06 (m, 3 H), 3.01 - 3.28 (m, 1 H), 1.73 - 1.98 (m, 2 H), 1.39 (br d, 3 H).

98.0 mg der Titelverbindung (Diastereomerengemisch) wurde durch chirale HPLC in die Diastereomere getrennt (präparative HPLC: Säule Daicel Chiralpak IE, 5 µm, 250x20 mm; Eluent: 70% *n*-Heptan, 30% Ethanol + 0.2% DEA; Temperatur: 35°C; Fluss: 15 ml/min; UV-Detektion: 265 nm.)

Man erhielt (in der Reihenfolge der Elution von der Säule) 46.0 mg Diastereomer 1 (99% de) $R_t$ = 8.64 min und 47.0 mg Diastereomer 2 (99% de) $R_t$= 12.08 min.

[Analytische HPLC: Säule Daicel Chiralpak IE, 5 µm, 250x4.6 mm; Eluent: 70% *n*-Heptan, 30% Ethanol + 0.2% DEA; Temperatur: 35°C; Fluss: 1.0 ml/min; UV-Detektion: 265 nm].

Diastereomer 1 wurde zusätzlich mittels präparativer HPLC (Säule: Acetonitril / Wasser / 0.1% Ameisensäure) gereinigt und 40.0 mg (30% d. Th., 100% Reinheit) der Titelverbindung aus Beispiel 128 erhalten.

Diastereomer 2 wurde zusätzlich mittels präparativer HPLC (Säule: Acetonitril / Wasser / 0.1% Ameisensäure) gereinigt und 42.0 mg (31% d. Th., 100% Reinheit) der Titelverbindung aus Beispiel 129 erhalten.

## Beispiel 128

1-(3,5-Difluorpyridin-2-yl)-6-fluor-7-[(3*S*)-3-hydroxypyrrolidin-1-yl]-4-oxo-*N*-[3,3,4,4,4-pentafluorbutan-2-yl]-1,4-dihydro-1,8-naphthyridin-3-carboxamid (Diastereomer 1)

LC-MS (Methode 3): $R_t$ = 1.97 min; MS (ESIpos): m/z = 552 $[M+H]^+$

$^1$H-NMR (400 MHz, DMSO-d6): δ [ppm] = 10.46 (d, 1 H), 8.83 (d, 1 H), 8.60 - 8.63 (m, 1 H), 8.31 - 8.37 (m, 1 H), 8.00 (d, 1 H), 4.95 - 5.08 (m, 2 H), 4.22 - 4.36 (m, 1 H), 3.36 - 4.04 (m, 3 H), 2.95 - 3.27 (m, 1 H), 1.73 - 1.96 (m, 2 H), 1.39 (br d, 3 H).

## Beispiel 129

1-(,3,5-Difluorpyridin-2-yl)-6-fluor-7-[(3*S*)-3-hydroxypyrrolidin-1-yl]-4-oxo-*N*-[3,3,4,4,4-pentafluorbutan-2-yl]-1,4-dihydro-1,8-naphthyridin-3-carboxamid (Diastereomer 2)

LC-MS (Methode 3): $R_t$ = 1.97 min; MS (ESIpos): m/z = 552 $[M+H]^+$

$^1$H-NMR (400 MHz, DMSO-d6): δ [ppm] = 10.46 (br d, 1 H), 8.83 (d, 1 H), 8.61 (d, 1 H), 8.30 - 8.38 (m, 1 H), 8.00 (d, 1 H), 4.93 - 5.10 (m, 2 H), 4.22 - 4.37 (m, 1 H), 3.36 - 4.07 (m, 3 H), 2.96 - 3.29 (m, 1 H), 1.73 - 1.98 (m, 2 H), 1.39 (br d, 3 H).

**Beispiel 130**

*N*-(2,6-Dichlorphenyl)-7-[(3*R*,4*R*)-3,4-dihydroxypyrrolidin-1-yl]-6-fluor-4-oxo-1-(2,4,6-trifluorphenyl)-1,4-dihydro-1,8-naphthyridin-3-carboxamid

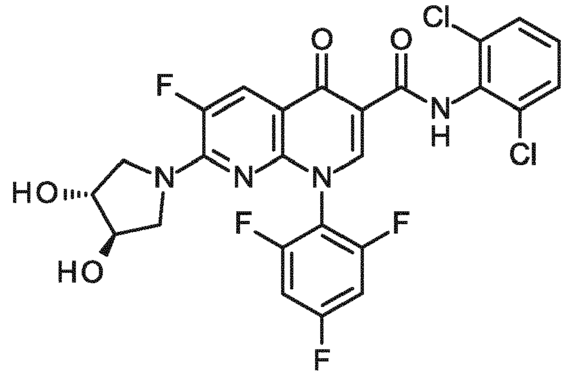

Gemäß AAV3 wurden 255 mg (494 μmol) 7-Chlor-*N*-(2,6-dichlorphenyl)-6-fluor-4-oxo-1-(2,4,6-trifluorphenyl)-1,4-dihydro-1,8-naphthyridin-3-carboxamid mit 75.8 mg (543 μmol) (3R,4R)-Pyrrolidin-3,4-diol Hydrochlorid und 300 μl (1.70 mmol) *N*,*N*-Diisopropylethylamin in 5 ml Dimethylformamid zur Reaktion gebracht. Das Rohprodukt wurde mit etwas Acetonitril verdünnt und mittels präparativer HPLC (Säule: Acetonitril / Wasser / 0.1% Ameisensäure) gereinigt. Es wurden 216 mg (75% d. Th., 100% Reinheit) der Titelverbindung erhalten.
LC-MS (Methode 3): $R_t$ = 1.82 min; MS (ESIpos): m/z = 583 $[M+H]^+$
$^1$H-NMR (400 MHz, DMSO-d6) δ [ppm]: 0.947 (0.79), 1.257 (3.15), 2.328 (0.67), 2.366 (0.53), 2.671 (0.73), 2.710 (0.55), 2.731 (4.61), 2.890 (5.57), 3.054 (1.06), 3.705 (0.95), 3.912 (1.83), 4.029 (1.36), 5.216 (3.69), 7.360 (2.39), 7.380 (4.91), 7.400 (3.33), 7.562 (3.25), 7.581 (16.00), 7.601 (11.10), 7.952 (0.78), 8.062 (4.69), 8.093 (4.59), 8.929 (8.31), 11.845 (8.05).

**Beispiel 131**

*N*-[1-(2-Chlorphenyl)-2,2,2-trifluorethyl]-7-[(3*R*,4*R*)-3,4-dihydroxypyrrolidin-1-yl]-6-fluor-4-oxo-1-(2,4,6-trifluorphenyl)-1,4-dihydro-1,8-naphthyridin-3-carboxamid (Diastereomerengemisch)

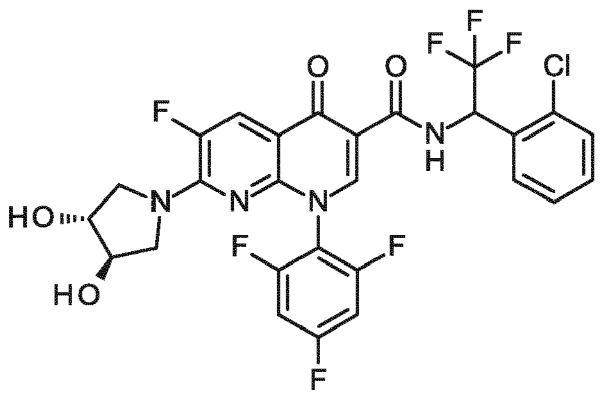

Gemäß AAV1 wurden 100 mg (228 μmol) 7-[(3*R*,4*R*)-3,4-Dihydroxypyrrolidin-1-yl]-6-fluor-4-oxo-1-(2,4,6-trifluorphenyl)-1,4-dihydro-1,8-naphthyridin-3-carbonsäure mit 52.5 mg (250 μmol) 1-(2-Chlorphenyl)-2,2,2-trifluorethanamin (Racemat) in Gegenwart von 104 mg (273 μmol) HATU und 120 μl (680 μmol) DIPEA in 1.4 ml DMF zur Reaktion gebracht. Das Rohprodukt wurde mittels präparativer HPLC (Säule: Acetonitril / Wasser / 0.1% Ameisensäure) gereinigt. Es wurden 104 mg (71% d. Th., 98% Reinheit) der Titelverbindung erhalten.
LC-MS (Methode 1): $R_t$ = 1.09 min; MS (ESIpos): m/z = 631 $[M+H]^+$

$^1$H-NMR (400 MHz, DMSO-d6) δ [ppm]: -0.149 (0.55), -0.008 (5.54), 0.146 (0.64), 2.074 (0.71), 2.329 (1.11), 2.367 (0.95), 2.671 (1.20), 2.711 (0.93), 3.064 (1.11), 3.696 (1.09), 3.897 (2.04), 4.021 (1.60), 5.203 (4.37), 6.404 (0.75), 6.423 (2.53), 6.445 (3.43), 6.465 (2.39), 7.484 (1.62), 7.499 (4.37), 7.503 (4.83), 7.517 (4.54), 7.522 (4.79), 7.533 (3.68), 7.551 (7.45), 7.566 (7.05), 7.589 (4.65), 7.607 (12.96), 7.627 (7.80), 8.050 (8.75), 8.082 (8.62), 8.861 (16.00), 11.447 (5.70), 11.470 (5.39).

**Beispiel 132**

*N*-(,2,6-Dichlorbenzyl)-7-[(3*R*,4*R*)-3,4-dihydroxypyrrolidin-1-yl]-6-fluor-4-oxo-1-(2,4,6-trifluorphenyl)-1,4-dihydro-1,8-naphthyridin-3-carboxamid

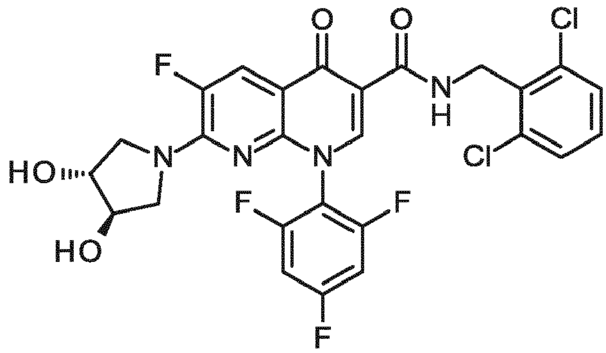

Gemäß AAV1 wurden 100 mg (228 μmol) 7-[(3*R*,4*R*)-3,4-Dihydroxypyrrolidin-1-yl]-6-fluor-4-oxo-1-(2,4,6-trifluorphenyl)-1,4-dihydro-1,8-naphthyridin-3-carbonsäure mit 44.1 mg (250 μmol) 1-(2,6-Dichlorphenyl)methanamin in Gegenwart von 104 mg (273 μmol) HATU und 120 μl (680 μmol) DIPEA in 1.4 ml DMF zur Reaktion gebracht. Das Rohprodukt wurde mittels präparativer HPLC (Säule: Acetonitril / Wasser / 0.1% Ameisensäure) gereinigt. Es wurden 121 mg (89% d. Th., 100% Reinheit) der Titelverbindung erhalten.
LC-MS (Methode 1): $R_t$ = 1.00 min; MS (ESIpos): m/z = 597 $[M+H]^+$
$^1$H-NMR (400 MHz, DMSO-d6) δ [ppm]: -0.149 (0.53), -0.008 (5.53), 0.008 (3.79), 0.146 (0.53), 2.367 (0.81), 2.519 (3.30), 2.524 (2.91), 2.711 (0.74), 3.046 (0.63), 3.671 (0.60), 3.903 (1.44), 4.809 (10.37), 4.823 (10.16), 5.181 (4.47), 7.379 (3.42), 7.398 (5.05), 7.401 (5.23), 7.420 (5.84), 7.525 (16.00), 7.545 (12.28), 7.569 (5.33), 7.591 (3.09), 7.953 (7.16), 7.985 (6.98), 8.782 (12.02), 10.219 (2.47), 10.232 (4.91), 10.245 (2.14).

**Beispiel 133**

6-Chlor-*N*-(2,6-dichlorphenyl)-1-(2,4-difluorphenyl)-4-oxo-1,4-dihydro-1,8-naphthyridin-3-carboxamid

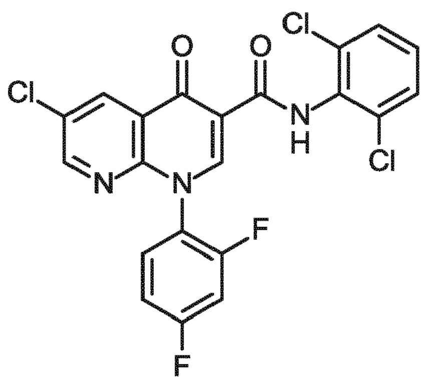

Zu einer Lösung von 158 mg (446 μmol) 6-Chlor-1-(2,4-difluorphenyl)-4-oxo-1,4-dihydro-1,8-naphthyridin-3-carbonylchlorid in 1.0 ml DMF wurde eine Lösung von 79.4 mg (490 μmol) 2,6-Dichloranilin in 1.0 ml DMF hinzugegeben

und anschließend wurden 19.6 mg (490 μmol) Natriumhydrid (60% in Mineralöl) hinzugegeben. Es wurde 2 h bei RT nachgerührt. Die Reaktion wurde durch Zugabe von Wasser, Acetonitril und Ameisensäure beendet und das Rohprodukt mittels präparativer HPLC (Säule: Acetonitril / Wasser / 0.1% Ameisensäure) gereinigt. Es wurden 36.0 mg (16% d. Th., 93% Reinheit) der Titelverbindung erhalten.
LC-MS (Methode 1): $R_t$ = 1.24 min; MS (ESIpos): m/z = 480 $[M+H]^+$
$^1$H-NMR (400 MHz, DMSO-d6): δ [ppm] = 11.41 (s, 1 H), 9.02 (s, 1 H), 8.92 (d, 1 H), 8.81 (d, 1 H), 7.85 - 7.94 (m, 1 H), 7.57 - 7.67 (m, 3 H), 7.32 - 7.44 (m, 2 H).

**Beispiel 134**

6-Chlor-*N*-[1-(2-chlorphenyl)-2,2,2-trifluorethyl]-1-(2,4-difluorphenyl)-4-oxo-1,4-dihydro-1,8-naphthyridin-3-carboxamid (Racemat)

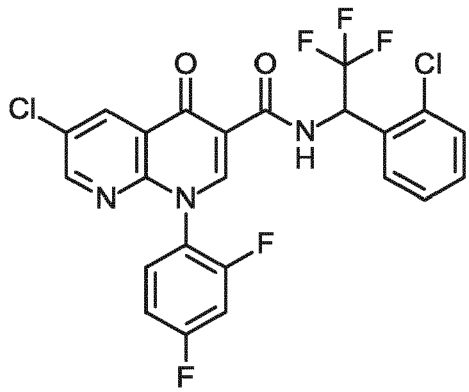

Gemäß AAV1 wurden 150 mg (446 μmol) 6-Chlor-1-(2,4-difluorphenyl)-4-oxo-1,4-dihydro-1,8-naphthyridin-3-carbonsäure mit 140 mg (668 μmol) 1-(2-Chlorphenyl)-2,2,2-trifluorethanamin (Racemat) in Gegenwart von 203 mg (535 μmol) HATU und 230 μl (1.30 mmol) DIPEA in 1.5 ml DMF zur Reaktion gebracht. Das Rohprodukt wurde mittels präparativer HPLC (Säule: Acetonitril / Wasser/ 0.1% Ameisensäure) gereinigt. Es wurden 197 mg (83% d. Th., 99% Reinheit) der Titelverbindung erhalten.
LC-MS (Methode 1): $R_t$ = 1.37 min; MS (ESIpos): m/z = 528 $[M+H]^+$
$^1$H-NMR (400 MHz, DMSO-d6): δ [ppm] = 11.03 (d, 1 H), 8.96 (s, 1 H), 8.90 (d, 1 H), 8.80 (d, 1 H), 7.74 - 7.91 (m, 1 H), 7.48 - 7.67 (m, 5 H), 7.31 - 7.41 (m, 1 H), 6.43 - 6.53 (m, 1 H).

**Beispiel 135**

6-Chlor-1-(2,4-difluorphenyl)-4-oxo-*N*-[1-(trifluormethoxy)propan-2-yl]-1,4-dihydro-1,8-naphthyridin-3-carboxamid (Racemat)

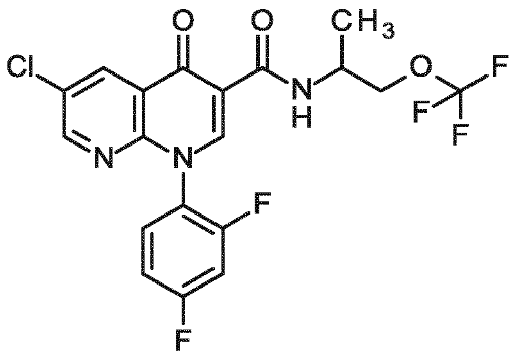

Gemäß AAV1 wurden 150 mg (446 μmol) 6-Chlor-1-(2,4-difluorphenyl)-4-oxo-1,4-dihydro-1,8-naphthyridin-3-carbonsäure mit 120 mg (668 μmol) 1-(Trifluormethoxy)propan-2-amin Hydrochlorid (Racemat) in Gegenwart von 203 mg (535 μmol) HATU und 310 μl (1.80 mmol) DIPEA in 1.5 ml DMF zur Reaktion gebracht. Das Rohprodukt wurde mittels präparativer HPLC (Säule: Acetonitril / Wasser / 0.1% Ameisensäure) gereinigt. Es wurden 159 mg (77% d. Th., 100% Reinheit) der Titelverbindung erhalten.
LC-MS (Methode 1): $R_t$ = 1.21 min; MS (ESIpos): m/z = 462 $[M+H]^+$
$^1$H-NMR (400 MHz, DMSO-d6): δ [ppm] = 9.69 (d, 1 H), 8.86 - 8.89 (m, 2 H), 8.73 (d, 1 H), 7.81 - 7.89 (m, 1 H), 7.62 (ddd, 1 H), 7.33 - 7.39 (m, 1 H), 4.33 - 4.42 (m, 1 H), 4.16 - 4.23 (m, 2 H), 1.27 (d, 3 H).

## B. BEWERTUNG DER PHARMAKOLOGISCHEN WIRKSAMKEIT

Die pharmakologische Aktivität der erfindungsgemäßen Verbindungen kann durch *in vitro*- und *in vivo*-Untersuchungen, wie sie dem Fachmann bekannt sind, nachgewiesen werden. Die nachfolgenden Anwendungsbeispiele beschreiben die biologische Wirkung der erfindungsgemäßen Verbindungen, ohne die Erfindung auf diese Beispiele zu beschränken.

**Abkürzungen und Akronyme:**

| | |
|---|---|
| $B_{Max}$ | Anzahl spezifischer Bindungstellen des Radioliganden |
| CAFTY | calcium free tyrode |
| CHO | chinese hamster ovary |
| CRE | cAMP-responsive element |
| DMEM | Dulbecco's modified eagle medium |
| DMSO | Dimethylsulfoxid |
| FCS | fötales Kälberserum |
| FRET | fluorescence resonance energy transfer |
| GIRK1/4 | G-protein-coupled inward rectifier potassium channel, member 1/4 |
| HEPES | Hydroxyethylpiperazin-Ethansulfonsäure |
| HTRF | homogeneous time resolved fluorescence |
| $K_d$ | Gleichgewichtsdissoziationskonstante |
| $K_i$ | Gleichgewichtsinhibitor-Konstante |
| $k_{off}$ | Dissoziationsrate |
| $k_{on}$ | Assoziationsrate |
| nM | nano-molar |
| MEM | minimum essential medium |
| μL | Mikro-Liter |
| μM | mikro-molar |
| mL | milli-Liter |
| mM | milli-molar |
| mtClytin | mitochondriales Clytin |
| min | Minuten |
| NMS | N-Me-Scopolamin |
| PAM | positiv allosterischer Modulator |
| PEI | Polyethylenimin |
| Pen/Strep | Penicillin/Streptomycin |
| sec | Sekunden |

## B-1. Funktioneller M2-GIRK1/4-Aktivierungstest

Sowohl die Aktivierung des M2-Rezeptors durch orthosterische Agonisten allein als auch die allosterische Verstärkung der Orthoster-induzierten Aktivierung durch positiv allosterische Modulatoren (PAMs) kann mittels eines zellbasierten funktionellen GIRK1/4-Aktivitätstest bestimmt werden. Die Bindung von orthosterischen Agonisten (endogener Ligand: Acetylcholin) an den M2-Rezeptor führt zu einer Rezeptoraktivierung bzw. Konformationsänderung des Rezeptors im Sinne einer Gleichgewichtsverschiebung zugunsten der aktiven Rezeptorkonformation. Die Bindung der orthosterischen Agonisten an den M2-Rezeptor und damit dessen Aktivierung kann durch positive allosterische Modulatoren, welche nicht an die orthosterische Bindungsstelle der Agonisten, sondern an eine separate allosterische Bindungsstelle binden, verstärkt werden.

Die Agonisten-induzierte Konformationsänderung des M2-Rezeptors hat eine Gai-Proteinaktivierung zur Folge. Die Aktivierung der $G\alpha$-Untereinheit wiederum führt zu einer Dissoziation und damit Freisetzung der $G\beta\gamma$-Untereinheiten von der $G\alpha$-Untereinheit und der Aktivierung separater nachgeschalteter Signaltransduktionskaskaden. Der freigesetzte heterodimere $G\beta\gamma$-Komplex bindet an den GIRK1/4 Kalium-Kanal und induziert eine ligandengesteuerte Kanalaktivierung bzw. -öffnung (Reuveny et al., Nature, July 1994, 370, 143-146). Unter physiologischen Bedingungen kommt es dann zu einem selektiven Ausstrom von Kalium aus der Zelle entlang des elektrochemischen Gradienten. Der Export positiver Ladung führt zu einer Erniedrigung des Transmembranpotentials und damit zu einer Hyperpolarisation der Zelle. Das Ausmaß der Hyperpolarisation kann daher als Maß für die Aktivierung des M2-Rezeptors angesehen werden.

Als Testzelle dient eine rekombinante CHO-DUKX-Zelllinie, welche mit cDNA kodierend für den humanen M2-Rezeptor sowie mit cDNA kodierend für beide GIRK1/4-Untereinheiten stabil transfiziert wurde (CHO-DUKX-M2-GIRK). Die Bestimmung des Transmembranpotentials bzw. der relativen Änderungen des Transmembranpotentials in Abhängigkeit von Substanzzugabe bzw. M2-Aktivierung erfolgt mittels eines spannungssensitiven Farbstoffes (FLIPR Membrane Potential Assay Kit Blue, Molecular Devices # R8034) und der Messung der Zellfluoreszenz unter Verwendung eines proprietären Fluoreszenz-Imaging-Messgerätes.

### B-1.1. Bestimmung der allosterischen Potenz der Testsubstanzen ($EC_{50}$-Wert)

Die Testsubstanzen werden in Dimethylsulfoxid (DMSO) mit einer Konzentration von 10 mM gelöst und für eine 10 Punkt-Dosis-Wirkungsanalyse in Schritten von 1:3.16 seriell in DMSO verdünnt. Entsprechend der gewünschten Testkonzentrationen werden die Substanzen in Beladungspuffer (Zusammensetzung: 0,6 ml FLIPR Membrane Potential Assay Kit Blue (10 mg/ml), 0,6 ml Brilliant Black (10 mg/ml), 2 mM $CaCl_2$ und 2 mM KCl ad 50 ml Natrium-Gluconat-Tyrode (PAA, #T21-155)) vorverdünnt.

Die in MEM alpha-Medium (supplementiert mit 10% FCS, 2% Glutamax, 1 mg/ml Geniticin) kultivierten Reporter-Zellen wurden mit 2000 Zellen (Messung nach 48 h) bzw. 4000 Zellen (Messung nach 24 h) in 30 µl pro 384 well in µCLEAR/schwarz Greiner-Zellkulturplatten (#781092) ausgesät und 24 h bzw. 48 h bei 37°C inkubiert. Das Aussaatmedium bestand aus MEM alpha-Medium (supplementiert mit 5% FCS, 2% Glutamax, kein Geniticin).

Für die jeweilige Messung wurde das Medium entfernt und die Zellen für mindestens 6 min bei Raumtemperatur mit dem spannungssensitiven Farbstoff beladen (30 µl Beladungspuffer) pro 384 Well). Anschließend erfolgte in einer ersten Messung die Bestimmung der Fluoreszenz für das Ruhe-Transmembranpotential für einen Zeitraum von 5 sec. Hiernach erfolgte die Zugabe von je 10 µl der in Beladungspuffer verdünnten Testsubstanzen, gefolgt von einer zweiten Messung zur Bestimmung des Transmembranpotentials für einen Zeitraum von 50 sec. Schließlich wurden die Zellen mit 10 µl Agonistenlösung (Acetylcholin gelöst in Beladungspuffer) versetzt. Acetylcholin wurde mit der dem $EC_{20}$-Wert entsprechenden Konzentration eingesetzt, die in einem Vortest bestimmt wurde. Die M2-vermittelte GIRK1/4-Aktivierung bzw. Hyperpolarisation wurde dann in einer dritten Messung über einen Zeitraum von 60 sec verfolgt. Der $EC_{50}$-Wert (Maß der allosterischen Potenz der Testverbindung) und die Effizienz (Maß der Verstärkung des Acetylcholin-Effektes bei einer $EC_{20}$-Acetylcholin-Konzentration) wurden mit Hilfe einer 4-Parameter-Logistik-Funktion (Hill-Funktion) ermittelt.

### B-1.2. Bestimmung der positiven Kooperativität ($\alpha$-Faktor)

Die Testsubstanzen wurden in DMSO mit einer Konzentration von 10 mM gelöst und für eine 10 Punkt-Dosis-Wirkungsanalyse in Schritten von 1:3.16 seriell in DMSO verdünnt. Entsprechend der gewünschten Testkonzentrationen wurden die Substanzen in Beladungspuffer) (s.o.) vorverdünnt.

Die in MEM alpha-Medium (supplementiert mit 10% FCS, 2% Glutamax, Img/ml Geniticin) kultivierten Reporter-Zellen werden mit 2000 Zellen (Messung nach 48 h) bzw. 4000 Zellen (Messung nach 24 h) in 30 µl pro 384 well in µCLEAR/schwarz Greiner-Zellkulturplatten (#781092) ausgesät und 24 h bzw. 48 h bei 37°C inkubiert. Das Aussaatmedium bestand aus MEM alpha-Medium (supplementiert mit 5% FCS, 2% Glutamax, kein Geniticin).

Für die jeweilige Messung wurde das Medium entfernt und die Zellen für mindestens 6 min bei Raumtemperatur mit dem spannungssensitiven Farbstoff beladen (30 µl Beladungspuffer pro 384 well). Anschließend erfolgt in einer

ersten Messung die Bestimmung des Ruhe-Transembranpotentials für einen Zeitraum von 5 sec in 1 sec-Inkrementen. Hiernach erfolgt die Zugabe von je 10 µl der in Beladungspuffer verdünnten Testsubstanzen, gefolgt von einer zweiten Messung zur Bestimmung des Transmembranpotentials für einen Zeitraum von 50 sec in 1 sec-Inkrementen.

Schließlich werden die Zellen mit 10 µl Agonistenlösung (Acetylcholin gelöst in Beladungspuffer) versetzt. Im Gegensatz zur $EC_{50}$-Bestimmung der Testsubstanzen (siehe B-1.1) wird dabei aber nicht nur eine Acetylcholin-Konzentration eingesetzt, sondern jede Konzentration der Testsubstanz wird mit einer Acetylcholin-8 Punkt-Dosis-Wirkungskurve kombiniert. Für die Acetylcholin-Verdünnungsreihe wird der Agonist beginnend mit einer maximalen Endkonzentration von 3 µM in Schritten von 1:3.16 seriell in Beladungspuffer entsprechend der gewünschten Endkonzentrationen vorverdünnt. Die M2-vermittelte GIRK1/4-Aktivierung bzw. Hyperpolarisation wird dann in einer dritten Messung über einen Zeitraum von 60 sec in 1 sec-Inkrementen verfolgt. Die Verschiebung der Acetylcholin-Dosis-Wirkungskurve in Anwesenheit zunehmender Konzentrationen der Testsubstanz wird mittels GraphPad PRISM (Allosteric $EC_{50}$ shift) analysiert und quantifiziert. Der ermittelte α-Faktor ist dabei ein Maß für die Stärke und Richtung des allosterischen Effektes. α-Werte > 1 spiegeln eine Erniedrigung des $EC_{50}$-Wertes bzw. eine Erhöhung der Potenz des Agonisten (Acetylcholin) in Anwesenheit von Alloster wider und bedeuten damit eine positive Kooperativität zwischen Orthoster (Acetylcholin) und Alloster (Testsubstanz). Eine positive Kooperativität ist das Kennzeichen eines positiven allosterischen Modulators. Umgekehrt sind α -Werte < 1 bezeichnend für eine negative Kooperativität zwischen Orthoster und Alloster und kennzeichnen damit negative allosterische Modulatoren. α-Werte = 1 bedeuten keine Kooperativität zwischen Orthoster und Alloster, d.h. die Bindungsaffinitäten von Orthoster und Alloster an den Rezeptor beeinflussen sich nicht wechselseitig. Je größer der Betrag des α-Wertes ist, umso größer ist das Ausmaß der Kooperativität zwischen Orthoster und Alloster.

In der folgenden Tabelle 2 sind für individuelle Ausführungsbeispiele die so ermittelten $EC_{50}$- und Effizienz-Werte sowie die α-Werte aus diesem Assay aufgeführt (zum Teil als Mittelwerte aus mehreren unabhängigen Einzelbestimmungen):

**Tabelle 1**

| Bsp.Nr. | Rezeptor-aktivität $EC_{50}$ [µmol/L] | Effizienz [%] | Kooperativität (alpha-Faktor) |
|---|---|---|---|
| 1 | 0.021 | 92 | 35 |
| 2 | 0.0355 | 96 | |
| 3 | 0.038 | 97 | |
| 4 | 0.038 | 89 | |
| 5 | 0.069 | 99 | |
| 6 | 0.00617 | 94 | 58 |
| 7 | 0.00564 | 93 | 57 |
| 8 | 0.0043 | 96 | |
| 9 | 0.00199 | 91 | 42 |
| 10 | 0.00527 | 99 | 70 |
| 11 | 0.0058 | 100 | 60 |
| 12 | 0.02 | 90 | 40 |
| 13 | 0.0062 | 94 | 49 |
| 14 | 0.0055 | 100 | 49 |
| 15 | 0.00915 | 96 | 41 |
| 16 | 0.00845 | 99 | 42 |
| 17 | 0.0795 | 83 | |
| 18 | 0.0205 | 99 | 43 |
| 19 | 0.016 | 98 | |
| 20 | 0.013 | 92 | |
| 21 | 0.003 | 100 | |

(fortgesetzt)

| Bsp.Nr. | Rezeptor-aktivität $EC_{50}$ [$\mu$mol/L] | Effizienz [%] | Kooperativität (alpha-Faktor) |
|---|---|---|---|
| 22 | 0.01 | 95 | 42 |
| 23 | 0.0055 | 100 | 41 |
| 24 | 0.00135 | 100 | 57 |
| 25 | 0.00405 | 95 | 45 |
| 26 | 0.00403 | 100 | |
| 27 | 0.00258 | 92 | |
| 28 | 0.00315 | 100 | |
| 29 | 0.0025 | 100 | |
| 30 | 0.0026 | 100 | |
| 31 | 0.00415 | 100 | 37 |
| 32 | 0.0043 | 100 | 54 |
| 33 | 0.00175 | 100 | 50 |
| 34 | 0.0012 | 100 | 53 |
| 35 | 0.0029 | 100 | |
| 36 | 0.005 | 92 | |
| 37 | 0.006 | 100 | 38 |
| 38 | 0.0101 | 100 | |
| 39 | 0.0205 | 100 | |
| 40 | 0.0023 | 98 | |
| 41 | 0.0033 | 100 | |
| 42 | 0.004 | 100 | |
| 43 | 0.0075 | 100 | |
| 44 | 0.012 | 100 | |
| 45 | 0.00847 | 100 | 62 |
| 46 | 0.051 | 100 | |
| 47 | 0.048 | 81 | |
| 48 | 0.0018 | 78 | |
| 49 | 0.068 | 74 | |
| 50 | 0.0025 | 60 | |
| 51 | 0.036 | 81 | |
| 52 | 0.0013 | 83 | |
| 53 | 0.016 | 100 | 30 |
| 54 | 0.025 | 100 | 30 |
| 55 | 0.025 | 96 | |
| 56 | 0.035 | 100 | |
| 57 | 0.0785 | 89 | |
| 58 | 0.104 | 90 | |
| 59 | 0.0915 | 97 | |

(fortgesetzt)

| Bsp.Nr. | Rezeptor-aktivität $EC_{50}$ [$\mu$mol/L] | Effizienz [%] | Kooperativität (alpha-Faktor) |
|---|---|---|---|
| 60 | 0.0036 | 100 | |
| 61 | 0.0022 | 94 | |
| 62 | 0.0041 | 88 | |
| 63 | 0.0039 | 92 | |
| 64 | 0.012 | 96 | |
| 65 | 0.03 | 89 | 14 |
| 66 | 0.035 | 92 | |
| 67 | 0.01 | 95 | |
| 68 | 0.032 | 92 | |
| 69 | 0.0059 | 100 | |
| 70 | 0.1 | 86 | |
| 71 | 0.0042 | 94 | 26 |
| 72 | 0.011 | 89 | |
| 73 | 0.0027 | 94 | |
| 74 | 0.0039 | 95 | |
| 75 | 0.087 | 100 | |
| 76 | 0.029 | 100 | |
| 77 | 0.0016 | 100 | |
| 78 | 0.0028 | 100 | |
| 79 | 0.0093 | 99 | |
| 80 | 0.024 | 100 | |
| 81 | 0.15 | 87 | |
| 82 | 0.14 | 65 | |
| 83 | 0.044 | 94 | |
| 84 | 0.00835 | 88 | 34 |
| 85 | 0.033 | 100 | |
| 86 | 0.014 | 95 | |
| 87 | 0.0014 | 100 | |
| 88 | 0.0022 | 100 | |
| 89 | 0.00475 | 100 | 44 |
| 90 | 0.0185 | 95 | 30 |
| 91 | 0.0065 | 100 | |
| 92 | 0.0066 | 100 | |
| 93 | 0.012 | 100 | |
| 94 | 0.0047 | 100 | 34 |
| 95 | 0.0155 | 100 | |
| 96 | 0.016 | 96 | |
| 97 | 0.013 | 97 | |

(fortgesetzt)

| Bsp.Nr. | Rezeptor-aktivität $EC_{50}$ [$\mu$mol/L] | Effizienz [%] | Kooperativität (alpha-Faktor) |
|---|---|---|---|
| 98 | 0.0143 | 98 | 50 |
| 99 | 0.0355 | 96 | |
| 100 | 0.0315 | 100 | |
| 101 | 0.0135 | 100 | |
| 102 | 0.295 | 92 | |
| 103 | 0.0081 | 100 | |
| 104 | 0.013 | 97 | |
| 105 | 0.0075 | 97 | |
| 106 | 0.0072 | 100 | |
| 107 | 0.00355 | 92 | |
| 108 | 0.0054 | 96 | 47 |
| 109 | 0.0077 | 100 | |
| 110 | 0.019 | 100 | |
| 111 | 0.0072 | 99 | |
| 112 | 0.00425 | 100 | 33 |
| 113 | 0.002 | 100 | |
| 114 | 0.007 | 94 | |
| 115 | 0.00665 | 98 | 53 |
| 116 | 0.0035 | 97 | |
| 117 | 0.0069 | 99 | |
| 118 | 0.0028 | 100 | |
| 119 | 0.0087 | 100 | |
| 120 | 0.0105 | 96 | |
| 121 | 0.125 | 100 | |
| 122 | 0.00355 | 100 | |
| 123 | 0.0031 | 100 | |
| 124 | 0.018 | 100 | |
| 125 | 0.012 | 97 | |
| 126 | 0.0039 | 97 | |
| 127 | 0.013 | 92 | |
| 128 | 0.0098 | 90 | |
| 129 | 0.066 | 95 | |
| 130 | 0.0023 | 96 | |
| 131 | 0.0046 | 100 | |
| 132 | 0.01 | 99 | |
| 133 | 1.6 | 81 | |
| 134 | 1.83 | 55 | |
| 135 | 2.61 | 75 | |

## B-2. Funktioneller Ca2+-Freisetzungstest mittels M2-Gα16-Reporterzellen

Eine potentiell agonistische wie auch die positiv allosterische Wirkung der Testsubstanzen auf den M2-Rezeptor kann anhand eines funktionellen $Ca^{2+}$-Freisetzungstests bestimmt werden. Die Aktivierung des M2-Rezeptors durch Bindung orthosterischer Agonisten (Acetylcholin) oder anderer Substanzen mit einem agonistischen Effekt führt zu einer Konformationsänderung des Rezeptors, welche im endogenen Zustand eine Gai-Protein-Aktivierung zur Folge hat. Durch Kopplung des M2-Rezeptors an das exogen exprimierte promiskuitive Gaq-Protein Gα16 kommt es jedoch zu einer Gα16-Proteinaktivierung nach Aktivierung des M2-Rezeptors, welche - über eine nachgeschaltete Signaltransduktionskaskade - eine intrazelluläre $Ca^{2+}$-Freisetzung bedingt. Das Ausmaß der intrazellulären $Ca^{2+}$-Mobilisierung kann daher in diesem Fall als Maß für die Aktivierung des M2-Rezeptors angesehen werden.

Als Testzelle dient eine rekombinante CHO-Zelllinie, welche mit cDNA kodierend für den humanen M2-Rezeptor und das Gα16-Protein sowie mit cDNA kodierend für das mitochondrial exprimierte Photoprotein Clytin (mtClytin) stabil transfiziert wurde (CHO mtClytin Gα16 M2). Die Bestimmung der intrazellulären $Ca^{2+}$-Freisetzung in Abhängigkeit von Substanzzugabe bzw. M2-Aktivierung erfolgt mittels eines $Ca^{2+}$-sensitiven Farbstoffes (Fluo-8) und der Messung der Zellfluoreszenz unter Verwendung eines FLIPR$^{TETRA}$-Instrumentes (Molecular Devices).

### B-2.1. Agonismus-Assay

Die Testsubstanzen werden in DMSO mit einer Konzentration von 10 mM gelöst und für eine 10 Punkt-Dosis-Wirkungsanalyse in Schritten von 1:3.16 seriell in DMSO verdünnt. Entsprechend der gewünschten Testkonzentrationen werden die Substanzen in Fluo-8-Puffer (Zusammensetzung pro 100 ml: 500 μl Probenecid, 2 ml Brilliant Black (20 mg/ml), 440 μl Fluo-8, 2 mM $CaCl_2$ ad 100 ml CAFTY-Tyrode (130 mM NaCl, 5 mM KCl, 20 mM HEPES, 1 mM $MgCl_2$, 5 mM $NaHCO_3$, pH 7.4)) vorverdünnt.

Die in MEM alpha-Medium (supplementiert mit 10% FCS, 2% Glutamax) kultivierten Reporter-Zellen wurden mit 3000 Zellen in 30 μl-Aussaatmedium pro 384 Well in μCLEAR/schwarz Greiner-Zellkulturplatten (#781092) ausgesät und 24 h bei 37°C inkubiert. Das Aussaatmedium besteht aus MEM alpha-Medium (supplementiert mit 5% FCS, 2% Glutamax). Für die jeweilige Messung wird das Medium entfernt und die Zellen nach Zugabe von je 20 μl Fluo-8-Puffer pro 384 Well für 1h 37°C im Brutschrank inkubiert. Nach Zugabe von je 10 μl pro 384 Well der vorverdünnten Testsubstanzen erfolgt die Messung der Zellfluoreszenz für einen Zeitraum von 5 min in 1 sec-Inkrementen. Das relative Maß der maximalen Aktivierung des M2-Rezeptors durch die jeweiligen Testsubstanzen wird durch Normierung des Testsignals auf das der $E_{Max}$-Konzentration von Acetylcholin (3μM) entsprechende Signal berechnet.

### B-2.2. Bestimmung des positiv allosterischen Modulator-Effektes

Um die positive Kooperativität der Testsubstanzen in Bezug auf die Acetylcholin-vermittelte M2-Rezeptoraktivierung bestimmen zu können, wird anschließend Referenzagonist (Acetylcholin) für eine vollständige Dosis-Wirkungsanalyse hinzugefügt. Hierzu wird Acetylcholin beginnend mit einer maximalen finalen Konzentration von 1 μM in Schritten von 1:3.16 seriell in Fluo-8-Puffer verdünnt. Nach Zugabe von je 10 μl Agonistenlösung pro 384 Well erfolgt wiederum die Messung der Zellfluoreszenz für einen Zeitraum von 5 min in 1 sec-Inkrementen. Dabei wird dieselbe Assay-Platte verwendet wie unmittelbar zuvor für den M2-Agonismus-Assay. Die Verschiebung der Acetylcholin-Dosis-Wirkungskurve in Anwesenheit zunehmender Konzentrationen der Testsubstanz wird mittels GraphPad PRISM (Allosteric $EC_{50}$ shift) analysiert und quantifiziert (s.o.).

## B-3. Selektivitätstest gegenüber humanen muskarinischen Acetylcholin-Rezeptoren

Eine potentiell agonistische Wirkung wie auch positiv allosterische Wirkung der Testsubstanzen auf anderen humanen muskarinischen Acetylcholin-Rezeptoren kann in einem in funktionellen $Ca^{2+}$-Freisetzungstests bestimmt werden (Eurofins; GPCRProfiler® Services in agonistic and allosteric mode for Mx Receptors; cat#: HTS600GPCR).

Als Testzellen dienen mit dem jeweiligen Rezeptor transfizierten Chem-1- oder Chem-4-Zelllinien (ChemiScreen™ M1 Calcium-Optimized FLIPR Cell Lines, Eurofins; M1: HTS044C; ChemiScreen™ Calcium-Optimized Stable Cell Line Human Recombinant M2 Muscarininc Acetylcholine Receptor, Eurofins; M2: HTS115C; ChemiScreen™ Human Recombinant M3 Muscarinic Acetylcholine Receptor Calcium-Optimized Stable Cell Line, Eurofins; M3: HTS116C; ChemiScreen™ Human Recombinant M4 Muscarinic Acetylcholine Receptor Calcium-Optimized Stable Cell Line, Eurofins; M4: HTS117C; ChemiScreen™ M5 Calcium-Optimized FLIPR Cell Lines, Eurofins; M5: HTS075C). Der Substanztest wird mit einem FLIPR$^{TETRA}$-Instrument (Molecular Devices) durchgeführt.

B-3.1. Agonismus-Assay

Um einen potentiellen agonistischen Effekt der Testsubstanzen zu ermitteln, werden die jeweiligen Testsubstanzen mit einer finalen Testkonzentration von 10 μM oder 1 μM zugefügt. Die $Ca^{2+}$-Freisetzung bzw. Zellfluoreszenz wird über einen Zeitraum von 180 sec gemessen. Als Positivkontrolle zur Normierung des Substanzeffektes auf die Rezeptoraktivierung wird eine dem $E_{Max}$-Wert entsprechende Konzentration von Acetylcholin eingesetzt.

Nach Beendigung des Agonismus-Assay wird die Assay-Platte bei 25°C für 7 min inkubiert. Nach der Inkubationsperiode wird der positiv allosterische Modulator-Assay initialisiert.

**B-3.2. Allosterischer Modulator-Assay**

Um eine positiv oder negativ allosterische Wirkung der Testsubstanzen auf andere humane muskarinische Acetylcholinrezeptoren sowie den M2-Rezeptor selbst zu untersuchen, wird jede Substanzkonzentration mit einer Acetylcholin-8 Punkt-Dosis-Wirkungskurve kombiniert. Nach Zugabe von Agonistenlösung erfolgt wiederum die Messung der Zellfluoreszenz für einen Zeitraum von 180 sec. Die Verschiebung der Acetylcholin-Dosis-Wirkungskurve (maximale Verschiebung des $EC_{50}$-Wertes von Acetylcholin) wird mittels GraphPad PRISM (Sigmoidal dose-response (variable slope) - $EC_{50}$) analysiert und quantifiziert. Abschließend werden Quotienten der allosterischen Verschiebung für den M2-Rezeptor und M4-Rezeptor gebildet, die ihrerseits als Maß für die jeweilige Selektivität fungieren.

**B-4. In vitro M2 PAM Gi-Assay**

Für die Charakterisierung von Testsubstanzen auf positiv allosterische Modulation des humanen M2 Rezeptors wird die Carbachol induzierte Hemmung des cAMP Anstieg durch Forskolin in rekombinant M2 Rezeptor-exprimierenden CHO Zellen gemessen, die zusätzlich ein Luciferase Gen unter der Kontrolle eine cAMP responsiven Elementes (CRE) exprimieren: 3000 Zellen in 25 μl Vollmedium (DMEM F12 PAN Medium, 10 % FCS, 1.35 mM Na-Pyruvat, 20 mM Hepes, 4mM Glutamax, 2 % Natrium Bicarbonat, 1 % Pen/Strep, 1 % 100x non-essential amino acids) werden je Loch einer 384 Multititerplatte (Greiner, TC Platte, schwarz mit klarem Boden) ausgesät und bei 37°C, 5 % $CO_2$ für 24 Stunden inkubiert. Vor der Messung wird das Medium durch 30 μl Messmedium (Optimem) ersetzt und bei 37°C, 5 % $CO_2$ für 10 Minuten inkubiert. Die Testsubstanz wird in DMSO in verschiedenen Konzentrationen (Startkonzentration 10 mM, Verdünnungsfaktor 3.16) als Dosiswirkungskurve vorbereitet und 1:50 mit calciumfreier Tyrode, 2 mM $CaCl_2$, 0.01% BSA vomerdünnt. 10 μl der vorverdünnten Substanzlösung werden zu den Zellen gegeben und bei 37°C, 5 % $CO_2$ für 10 Minuten inkubiert. Der M2 Rezeptor wird durch Zugabe von 10 μl Carbachol in verschiedenen Konzentrationen in calciumfreier Tyrode, 2 mM $CaCl_2$ aktiviert und bei 37°C, 5 % $CO_2$ für 5 Minuten inkubiert. Die Adenylylcyclase wird durch Zugabe von 10 μl 1 μM (finale Konzentration) Forskolin in calciumfreier Tyrode, 2 mM $CaCl_2$ aktiviert und bei 37°C, 5 % $CO_2$ für 5 Stunden inkubiert. Nach Entfernen des Zellüberstandes und Zugabe von 20 μl Luci/Triton Puffer (1:1) wurde die Lumineszenz in einem Luminometer für 60 Sekunden bestimmt.

Calciumfreie Tyrode: 130 mM NaCl, 5 mM KCl, 20 mM HEPES, 1 mM $MgCl_2$, 4.8 mM $NaHCO_3$, pH 7.4

Luci/Triton Puffer (1:1): Luci-Puffer (20mM Tricine, pH 7.8, 2,67 mM Magnesiumsulfat, 0.1 mM EDTA, 4 mM DTT, 270 μM Coenzym A, 470 μM D-Luciferin, 530 μM ATP) 1:1 mit Triton-Puffer (25 mM Tris wässr. Salzsäure, pH 7.8, 25 mM $Na_2HPO_4$, 2mM Dithiothreitol, 3 % Triton X-100, 10 % Glycerin) gemischt.

Der $EC_{50}$-Wert wurde mit Hilfe einer 4-Parameter-Logistik-Funktion (Hill-Funktion) ermittelt.

**B-5. Kompetitiver FRET-Bindungstest für humane M2- und M4-Rezeptoren**

Die direkte Bindung der Testsubstanzen an den M2-Rezeptor sowie die Verstärkung der Bindung (Erhöhung der Affinität) des natürlichen Agonisten Acetylcholin an den M2-Rezeptor in Anwesenheit der Testsubstanzen (positiv allosterischer Effekt) wird mittels eines FRET-basierten Bindungsassays (HTRF Tag-lite® Bindungsassay, Cisbio) bestimmt. Zur Kontrolle der Selektivität wird die Bindung der Testsubstanzen an den strukturverwandten M4-Rezeptor analog untersucht. Der HTRF Tag-lite® Assay ist ein homogener Bindungsassay und beruht auf der kompetitiven Bindung eines fluoreszenten Liganden (Sonde) und der unmarkierten Testsubstanz an den Rezeptor, welcher auflebenden Zellen exprimiert ist. Der Rezeptor ist seinerseits mit einem fluoreszenten Donor-Farbstoff (Terbium-Kryptat) derivatisiert, so dass nach Anregung des Donor-Farbstoffes ein FRET-Signal zwischen Rezeptor und Sonde (Akzeptor) entsteht, wenn die Sonde an den Rezeptor gebunden ist. Als Akzeptor-Sonde wurde ein Telenzepin-Derivat eingesetzt, welches mit einem HTRF Fluoreszenz-Farbstoff konjugiert ist (red ligand; L0040RED). Die Sonde bindet daher in der konservierten orthosterischen Bindungsstelle sowohl des M2- als auch des M4-Rezeptors. Die allosterische Bindungsstelle des M2-Rezeptors wurde röntgenkristallographisch charakterisiert und wird direkt oberhalb der orthosterischen Bindungstasche postuliert (Kruse et al., Nature, 2013, 504, 101-106). Sowohl die Bindung von unmarkierten orthosterischen Agonisten (Acetylcholin) an die orthosterische Bindungsstelle als auch die Bindung von allosterischen Modulatoren (Testsubstanzen) an die allosterische Bindungsstelle führt daher zu einer konzentrationsabhängigen kompetitiven Verdrängung der Sonde und damit einer Abnahme des FRET-basierten Fluoreszenzsignals.

Alle Bindungstests werden auf weißen 384 Mikrotiterplatten (small-volume) in einem Gesamtvolumen von 20 µl durchgeführt. Die HTRF-Messungen werden mit einem PHERAstar-Instrument (BMG Labtech) vorgenommen. Für den muskarinischen M2- oder M4-Rezeptor-Bindungstest werden SNAPed-M2-exprimierende Zellen (C1TT1M2) oder SNAPed-M4-exprimierende Zellen (C1TT1M4) verwendet, welche mit einem Donor-Fluorophor (Lumi4Tb; CELLCUST) markiert wurden. Die Zellen werden mit der Akzeptor-Sonde in Tag-lite Bindungspuffer (LABMED) in Anwesenheit von Testsubstanz bzw. Acetylcholin inkubiert. Anschließend wird das Fluoreszenz-Signal bei Wellenlängen von 665 nm und 620 nm gemessen und der HTRF-Quotient (Signal bei 665 nm / Signal bei 620 nm) bestimmt. Das relative spezifische Signal wird durch Subtraktion des HTRF-Quotienten der Negativkontrolle (nur Tag-lite Puffer ohne Sonde) ermittelt.

**B-5.1. Bindung der Testsubstanzen**

Um die Bindung der Testsubstanzen an den M2- oder M4-Rezeptor in Abwesenheit von orthosterischem Agonisten zu bestimmen, wird eine Dosis-Wirkungsanalyse der Testsubstanzen im kompetitiven Format des M2-Tag-lite®- oder M4-Tag-lite®-Bindungsassays vorgenommen. Die Testsubstanzen werden in DMSO mit einer Konzentration von 10 mM gelöst und für eine Dosis-Wirkungsanalyse in Schritten von 1:3.16 seriell in DMSO verdünnt. Die maximale Testkonzentration entspricht 10 µM. Die molare Konzentration der Testsubstanz, die eine halbmaximale Reduktion des HTRF-Signals in Bezug auf das maximale und verbleibende HTRF-Signal bei höchster Substanzkonzentration bewirkte ($EC_{50}$-Wert der Bindung), wird mittels GraphPad PRISM (Sigmoidal dose response) ermittelt. Zugleich wird die Stärke des Kompetitionseffektes bestimmt, indem die maximale Abnahme des spezifischen HTRF-Signals bei höchster Substanzkonzentration berechnet wird (% max. Kompetition).

**B-5.2. Bindung der Testsubstanzen im allosterischen Modus**

Um die allosterische Modulation des M2-Rezeptors durch die Testverbindungen zu untersuchen, wird zum einen eine Dosis-Wirkungsanalyse der Testsubstanzen im kompetitiven Format des M2-Tag-lite®-oder M4-Tag-lite®-Bindungsassays in Anwesenheit einer dem $EC_{20}$-Wert entsprechenden Konzentration von Acetylcholin vorgenommen, welche in einer separaten 11 Punkt-Acetylcholin-Dosis-Wirkungsanalyse bestimmt wird (3 µM). Die Testsubstanzen werden in DMSO mit einer Konzentration von 10 mM gelöst und für eine 10 Punkt-Dosis-Wirkungsanalyse in Schritten von 1:3.16 seriell in DMSO verdünnt. Die maximale Testkonzentration entspricht 10 µM. Die molare Konzentration der Testsubstanz, die eine halbmaximale Reduktion des HTRF-Signals in Bezug auf das maximale und verbleibende HTRF-Signal bei höchster Substanzkonzentration in Anwesenheit einer dem EC20-Wert entsprechenden Acetylcholin-Konzentration bewirkt ($EC_{50}$-Wert der Bindung ), wird mittels GraphPad PRISM (Sigmoidal dose response) ermittelt. Zugleich wird die Stärke des Kompetitionseffektes bestimmt, indem die maximale Abnahme des spezifischen HTRF-Signals bei höchster Substanzkonzentration berechnet wird (% max. Kompetition).

Um die Verstärkung der Bindung von Acetylcholin an den M2- oder M4-Rezeptor zu untersuchen, wurde zusätzlich zum anderen eine 11 Pmikt-Dosis-Wirkungsanalyse von Acetylcholin im kompetitiven Format des M2-Tag-lite®- oder M4-Tag-lite®-Bindungsassays in Abwesenheit oder in Anwesenheit von 1 µM oder 10 µM Testsubstanzvorgenommen. Die Verschiebung der Acetylcholin-Dosis-Wirkungskurve (maximale Verschiebung des $EC_{50}$-Wertes von Acetylcholin) wurde mittels GraphPad PRISM (Sigmoidal dose-response) analysiert und quantifiziert.

**B-6. Radioligand-Bindungstests für humane M2-Rezeptoren**

Der allosterische Wirkmechanismus der Testsubstanzen kann mittels verschiedener Radioligand-Bindungstests näher untersucht und quantifiziert werden. Die Bindung des Allosters an die allosterische Bindungsstelle des M2-Rezeptors hat im Falle einer positiven Kooperativität eine Erhöhung der Bindungaffinität des orthosterischen Liganden für den M2-Rezeptor zur Folge. Die Erhöhung der Bindungsaffinität des orthosterischen Liganden durch den Alloster im ternären Komplex bestehend aus Orthoster, Alloster und M2-Rezeptor wiederum ist auf eine Modulation der Bindungskinetiken des Orthosters zurückzuführen. Dabei kann der Alloster die Assoziations- und/oder Dissoziationsgeschwindigkeit des Orthosters am M2-Rezeptor verändern. Eine Erniedrigung der Dissoziationsgeschwindigkeit spiegelt dabei eine Stabilisierung des ternären Komplexes wieder und geht daher auch mit einer Emiedrigung der Dissoziationskonstante des orthosterischen Liganden unter Gleichgewichtsbedingungen einher (Lazareno, Determination of Allosteric Interactions Using Radioligand-Binding Techniques in Methods in Molecular Biology, vol. 259, Receptor Signal Transduction Protocols, 2nd ed.; Kostenis and Mohr, Trends Pharmacol. Sci. 1996, 17(8), 280-283).

### B-6.1. $^3$H-Oxotremorin-M Radioligand-Bindungstest unter Gleichgewichtsbedingungen

Um den Einfluss der Testsubstanzen auf die Bindungsaffinität von orthosterischen Agonisten für den M2-Rezeptor zu überprüfen und zu quantifizieren, kann ein Radioligand-Bindungstest unter Gleichgewichtsbedingungen durchgeführt werden. Dabei wird die Bindung des radioaktiv markierten M2-Rezeptor-Agonisten $^3$H-Oxotremorin-M an den M2-Rezeptor für unterschiedliche $^3$H-Oxotremorin-M-Konzentrationen im Bindungsgleichgewicht gemessen (Croy et al., Mo/. Pharmacol. 2014, 86, 106-115). Basierend auf der spezifisch gebundenen Menge von radioaktivem Agonisten an den M2-Rezeptor in Abhängigkeit von der Agonisten-Konzentration (graphisch dargestellt als sog. Langmuir-Isotherme) kann dann zum einen die Gleichgewichtsdissoziationskonstante $K_d$ des Agonisten als quantitatives Maß seiner Bindungsaffinität für den M2-Rezeptor sowie zum anderen die Konzentration bzw. Anzahl spezifischer Bindungstellen des Radioliganden (Agonisten) $B_{max}$ in Abwesenheit oder Anwesenheit unterschiedlicher Konzentrationen der Testsubstanzen (positiven allosterischen Modulatoren) berechnet werden (Hulme and Trevethick, Brit. J. Pharmacol. 2010, 161, 1219-1237).

Der Radioligandenbindungsassay für den M2 Rezeptor (Euroscreen, FAST-0261B) wird mittels $^3$H-markiertem Oxotremorin-M (NET671) als Agonisten durchgeführt. Die Agonistenbindung an den M2-Rezeptor wird auf 96 Mikrotiterplatten (Master Block, Greiner, 786201) in Bindungspuffer (Natrium-/Kalium-Phosphat-Pufer, pH 7,4) in Triplikaten vorgenommen. Hierzu werden pro Ansatz M2-Membranextrakte (20 μg Protein / 96 Well) mit verschiedenen Konzentrationen von radioaktiv markiertem Agonisten (0,2 - 100 nM) allein oder in Anwesenheit von 1 μM oder 10 μM Testsubstanz oder Bindungspuffer allein in einem Gesamtvolumen von 0,1 mL für 60 min bei 37°C inkubiert. Die nichtspezifische Bindung von $^3$H-markiertem Oxotremorin-M an die Membran wird durch Co-Inkubation mit N-Me-Scopolamin (NMS), einem orthosterischen Antagonisten des M2-Rezeptors, in 200-fachem Überschuss bestimmt. Anschließend werden die Proben zum Stoppen der Bindungsreaktion über GF/C Filter (Perkin Elmer, 6005174), welche zuvor mit 0,5 %-iger Polyethylenimin- (PEI-) Lösung benetzt wurden, für 2 h bei Raumtemperatur filtriert. Die Filter werden sechsmal mit je 0,5 mL eiskaltem Waschpuffer (10 mM Natrium-/Kalium-Phosphat-Puffer, pH 7.4) gewaschen und anschließend je 50 μL Microscint 20 Szintillationslösung (Packard) pro Ansatz zugegeben. Die Proben werden dann für 15 min auf einem Orbitalschüttler inkubiert, bevor die Messung der Radioaktivität mittels eines TopCount™-Gerätes (1 min / Well) vorgenommen wird.

Die Testsubstanzen werden in DMSO mit einer Konzentration von 10 mM gelöst und entsprechend der finalen Testkonzentration weiter in DMSO verdünnt, um eine 100-fache Verdünnung der eingesetzten DMSO-Lösung in Bindungspuffer zu erhalten.

Der $K_d$-Wert sowie der $B_{max}$-Wert von $^3$H-Oxotremorin-M für den M2-Rezeptor werden mit Hilfe eines "one-site" spezifischen Bindungsmodells bestimmt (Croy et al., Mol. Pharmacol. 2014, 86, 106-115).

### B-6.2. $^3$H-NMS Radioligand-Verdrängungstest unter Gleichgewichtsbedingungen

Um den Einfluss der Testsubstanzen auf die Bindungsaffinität von orthosterischen Agonisten für den M2-Rezeptor weitergehend zu überprüfen und zu quantifizieren, wird zusätzlich ein kompetitiver Radioligand-Bindungstest unter Gleichgewichtsbedingungen durchgeführt. Hierbei wird die Bindung des antagonistischen Radioliganden $^3$H-N-Me-Scopolamin ($^3$H-NMS) an den M2-Rezeptor in Abwesenheit oder Anwesenheit unterschiedlicher Konzentrationen des nicht radioaktiv markierten Agonisten Oxotremorin-M bestimmt (Croy et al., Mol. Pharmacol. 2014, 86, 106-115; Schober et al., Mol. Pharmacol. 2014, 86, 116-123). Die radioaktiv markierte Sonde (Antagonist) und der unmarkierte Agonist kompetieren um die Bindung an die orthosterische Bindungsstelle des M2-Rezeptors. Die Fähigkeit zur Verdrängung der radioaktiv markierten Sonde dient daher als Maß für die Bindungsaffinität des Agonisten für den Rezeptor und kann gemäß der Cheng-Prusoff-Gleichung als Gleichgewichtsinhibitor-Konstante ($K_i$-Wert) quantifiziert werden (Cheng and Prusoff, Biochem. Pharmacol. 1973, 22(23), 3099-3108). Um den allosterischen Effekt der Testsubstanzen weitergehend zu untersuchen, wird der Einfluss der Testsubstanzen auf den $K_i$-Wert von Oxotremorin-M bestimmt.

Der Antagonisten-Inhibitionsbindungsassay für den M2-Rezeptor (Euroscreen, FAST-0261B) wird auf 96 Mikrotiterplatten (Master Block, Greiner, 786201) in Bindungspuffer (50 mM Tris-Puffer pH 7.4, 1 mM EDTA, 10 μg/ml Saponin) mit $^3$H-NMS als M2 Rezeptor-Antagonisten durchgeführt. Zur Einstellung des Bindungsgleichewichts werden pro Ansatz M2-Membranextrakte (20 μg Protein / 96 Well) mit einer definierten Konzentration von radioaktiv markiertem Antagonisten (0,5 nM) allein oder in Anwesenheit von unterschiedlichen Konzentrationen von unmarkierten Agonisten (Oxotremorin-M; 0,001nM bis 1 mM) mit oder ohne 1 μM oder 10 μM Testsubstanz oder Bindungspuffer allein in einem Gesamtvolumen von 0,1 mL für 2 h bei 25°C inkubiert. Die nichtspezifische Bindung von $^3$H-markiertem NMS an die Membran wird durch Co-Inkubation mit nicht radioaktiv markiertem Acetylcholin in 200-fachem Überschuss bestimmt. Anschließend werden die Proben zum Stoppen der Bindungsreaktion über GF/C Filter (Perkin Elmer, 6005174), welche zuvor mit 0,5 %-iger PEI-Lösung benetzt wurden, für 2 h bei Raumtemperatur filtriert. Die Filter werden sechsmal mit je 0,5 mL eiskaltem Waschpuffer (10 mM Natrium-/Kalium-Phosphat-Puffer, pH 7.4) gewaschen und anschließend je 50 μL Microscint 20 Szintillationslösung (Packard) pro Ansatz zugegeben. Die Proben wurden dann für 15 min auf einem

Orbitalschüttler inkubiert, bevor die Messung der Radioaktivität mittels eines TopCount™-Gerätes (1 min / Well) vorgenommen wird.

Die Testsubstanzen werden in DMSO mit einer Konzentration von 10 mM gelöst und entsprechend der finalen Testkonzentration weiter in DMSO verdünnt, um eine 100-fache Verdünnung der eingesetzten DMSO-Lösung in Bindungspuffer zu erhalten.

Die Ki-Werte in An- oder Abwesenheit von Testsubstanz werden mit Hilfe der Cheng-Prusoff-Gleichung quantifiziert (Cheng and Prusoff, Biochem. Pharmacol. 1973, 22(23), 3099-3108). Dabei werden die $IC_{50}$-Werte der Substanzen gemäß einer vier Parameter enthaltenden logistischen Gleichung ermittelt und der $K_d$-Wert von NMS in einem Radioligand-Bindungstest unter Gleichgewichtsbedingungen ermittelt (Schober et al., Mol. Pharmacol. 2014, 86, 116-123).

### B-6.3. $^3$H-Oxotremorin-M Dissoziationskinetiktest

Mithilfe eines kinetischen Radioligand-Bindungstests kann die Kinetik der Dissoziation des radioaktiv markierten Agonisten $^3$H-Oxotremorin-M für den M2-Rezeptor in An- oder Abwesenheit von Testsubstanz untersucht werden. Hierdurch kann der Einfluss der allosterischen Aktivität der Testsubstanzen auf die Dissoziationskonstante ($k_{off}$-Rate) des M2-Agonisten bestimmt und so der Mechanismus der Allosterie der Testsubstanzen weitergehend charakterisiert werden (Lazareno, Determination of Allosteric Interactions Using Radioligand-Binding Techniques in Methods in Molecular Biology, vol. 259*,* Receptor Signal Transduction Protocols, 2nd ed.; Schrage et al., Biochem. Pharmacol., 2014, 90, 307-319.).

Der Radioliganden Dissoziationsbindungsassay für den M2 Rezeptor (Euroscreen, FAST-0261B) wird mit $^3$H-markiertem Oxotremorin-M (NET671) als Agonisten durchgeführt. Die Bindungsreaktion wird in Bindungspuffer (Natrium-/Kalium-Phosphat-Pufer, pH 7,4) auf 96 Mikrotiterplatten (Master Block, Greiner, 786201) vorgenommen. Hierzu werden pro Ansatz M2-Membranextrakte (20 μg Protein / 96 Well) mit einer definierten Konzentration von radioaktiv markiertem Agonisten (9,65 nM) allein oder in Anwesenheit von 1 μM oder 10 μM Testsubstanz oder Bindungspuffer allein für 60 min bei 37°C vorinkubiert. Anschließend wird NMS in 200-fachem Überschuss zu unterschiedlichen Zeitpunkten hinzugefügt (ein Zeitpunkt pro Ansatz) und die Ansätze in einem Gesamtvolumen von 0,1 mL bei 37°C inkubiert. Anschließend werden die Proben zum Stoppen der Bindungsreaktion über GF/C Filter (Perkin Elmer, 6005174), welche zuvor mit 0,5 %-iger PEI-Lösung benetzt wurden, für 2 h bei Raumtemperatur filtriert. Die Filter werden sechsmal mit je 0,5 mL eiskaltem Waschpuffer (10 mM Natrium-/Kalium-Phosphat-Puffer, pH 7.4) gewaschen und anschließend je 50 μL Microscint 20 Szintillationslösung (Packard) pro Ansatz zugegeben. Die Proben werden dann für 15 min auf einem Orbitalschüttler inkubiert, bevor die Messung der Radioaktivität mittels eines TopCountTM-Gerätes (1 min / Well) vorgenommen wird.

Die Testsubstanzen werden in DMSO mit einer Konzentration von 10 mM gelöst und entsprechend der finalen Testkonzentration weiter in DMSO verdünnt, um eine 100-fache Verdünnung der eingesetzten DMSO-Lösung in Bindungspuffer zu erhalten.

Der $k_{off}$-Wert wurde mit Hilfe eines "one phase" exponentiellen Zerfallsmodells der Dissoziation bestimmt (Hulme and Trevethick, Brit. J. Pharmacol. 2010, 161, 1219-1237; Kostenis and Mohr, Trends Pharmacol. Sci. 1996, 17(8), 280-283).

### B-6.4. $^3$H-M2-PAM Bindungstest

Die Bindungsaffinität der Testsubstanzen für den human M2 Rezeptor kann direkt bestimmt werden mithilfe einer radioaktiv markierten Testsubstanz als Sonde. Hierzu wurpositiv de eine allosterische Testsubstanz mittels Tritiierung radioaktiv markiert ($^3$H-M2-PAM).

Mittels eines Radioligandenbindungstests unter Gleichgewichtsbedingungen können zum einen die Gleichgewichtsdissoziationskonstante $K_d$ der positiv allosterischen Testsubstanz ($^3$H-M2-PAM) als quantitatives Maß seiner Bindungsaffinität für den M2-Rezeptor sowie zum anderen die Anzahl spezifischer Bindungstellen des Radioliganden $B_{max}$ in Abwesenheit oder Anwesenheit eines orthosterischen Agonisten (Acetlycholin) bestimmt werden (Hulme and Trevethick, Brit. J. Pharmacol. 2010, 161, 1219-1237; Schober et al., Mol. Pharmacol. 2014, 86, 116-123). Für den $^3$H-M2-PAM-Gleichgewichtsbindungs-Test werden M2 Rezeptor-Zellmembranpräparationen (CHO-S / hM2, 200 μg) in Inkubationspuffer (10 mM Tris/HCl pH 7,4, 2 mM MgCl2, 120 mM NaCl, Protease Inhibitoren, 0,3 % BSA) zusammen mit jeweils verschiedenen Konzentrationen des allosterischen Radioliganden $^3$H-M2-PAM (0,5 - 4000 nM) in Abwesenheit oder Anwesenheit von Acetylcholin (100 μM) für 1 h bei 4°C inkubiert. Die unspezifische Bindung wird bestimmt durch die Zugabe eines Überschusses von nicht-radioaktiv markiertem allosterischen Liganden (M2-PAM) (10 μM). Zum Stoppen der Bindungsreaktion werden die Proben über ein Brandel Filter-System filtriert und mit Stop-Buffer gewaschen (50 mM Tris/HCl pH 7,4, 500 mM NaCl, 0,3 % BSA). Die Filter wurden zuvor mit 0,3 %-iger PEI-Lösung benetzt. Der Kd- und Bmax-Wert des allosterischen Radioliganden werden basierend auf einem "one-site" spezifischen Bindungsmodell ermittelt (GraphPad Prism).

Mittels eines kompetitiven $^3$H-M2-PAM -Bindungstests kann die Affinität unmarkierter allosterischer Testsubstanzen für die Bindungstelle des Radioliganden $^3$H-M2-PAM an den M2 Rezeptor ermittelt werden. (Croy et al., Mol. Pharmacol. 2014, 86, 106-115; Schober et al., Mol. Pharmacol. 2014, 86, 116-123). Die radioaktiv markierte Sonde $^3$H-M2-PAM) und die unmarkierte allosterische Testsubstanz kompetieren um die Bindung an die allosterische Bindungsstelle des M2-Rezeptors. Die Fähigkeit zur Verdrängung der radioaktiv markierten Sonde dient daher als Maß für die allosterische Bindungsaffinität der Testsubstanzen für den Rezeptor und kann gemäß der Cheng-Prusoff-Gleichung als Gleichgewichtsinhibitor-Konstante ($K_i$-Wert) quantifiziert werden (Cheng and Prusoff, Biochem. Pharmacol. 1973, 22(23), 3099-3108). Die Verdrängung der radioaktiv markierten allosterischen Sonde wird dabei in An- oder Abwesenheit von orthosterischem Agonisten (Acetylcholin) bestimmt. Der $^3$H-M2-PAM Kompetitionsbindungs-Test wird analog zum oben beschriebenen $^3$H-M2-PAM Bindungstest unter Gleichgewichtsbedingungen durchgeführt. Dabei werden die M2 Rezeptor enthaltenen Membranpräparationen mit 1 nM $^3$H-M2-PAM und verschiedenen Konzentrationen von unmarkierter Testsubstanz in Ab- oder Anwesenheit von Acetylcholin (100 $\mu$M) inkubiert. Die $K_i$-Werte in An- oder Abwesenheit von Acetylcholin werden mit Hilfe der Cheng-Prusoff-Gleichung ermittelt (Cheng and Prusoff, Biochem. Pharmacol. 1973, 22(23), 3099-3108).

## B-7. Effekte der Testsubstanzen auf Acetylcholin vermittelte GIRK1/4-Kanalströme in primären atrialen Kardiomyozyten der Ratte

Die Substanztestung erfolgt gemäß eines in der Literatur beschriebenen Patch-Clamp Protokolls zur elektrophysiologischen Messung Acetylcholin-induzierter GIRK1/4-Membranströme in nativen atrialen Myozyten der Ratte (Cheng and Prusoff, Biochem. Pharmacol. 1973, 22(23), 3099-3108 siehe z.B. Beckmann and Rinne et al.,, Cell. Physiol. Biochem. 2008, 21, 259-268).

Zunächst wird eine Acetylcholin Dosis-Wirkungskurve in Abwesenheit von Testsubstanz (DMSO-Kontrolle) für die GIRK1/4-Aktivität bestimmt, indem Testlösungen mit ansteigender Acetylcholin-Konzentration perfundiert und die resultierenden Membranströme gemessen werden. Die Messung der Membranströme bzw. Änderung der Membranströme für eine gegebene ACh-Konzentration erfolgt dabei für ca. 10 bis 20 Sekunden. Nach Applikation der maximalen ACh-Konzentration innerhalb einer DWK-Reihe erfolgt die Perfusion einer Lösung mit Atropin (10 $\mu$M) gefolgt von einem Auswaschen der Substanzlösungen, um die M2-Selektivität und Reversibilität der M2-Aktivierung sicherzustellen. Die Änderungen der Membranströme werden entsprechend aufgenommen. Dabei wird für jede Acetylcholin-Konzentration der jeweils gemessene Membranstrom auf den maximalen Acetylcholininduzierten Membranstrom normiert (I/IMax). Eine Acetylcholin Dosis-Wirkungskurve umfasst dabei fünf verschiedene Konzentrationen (1nM, 10 nM, 100 nM, 1 $\mu$M, 10 $\mu$M). Der $EC_{50}$-Wert wird mit Hilfe einer 4-Parameter-Logistik-Funktion (Hill-Funktion) ermittelt.

Um den allosterischen Effekt der Testsubstanzen auf den M2 Rezeptor zu ermitteln, wird die Acetylcholin Dosis-Wirkungskurve für den GIRK1/4-Membranstrom in Anwesenheit einer konstanten Konzentration der jeweiligen Testsubstanz (z.B. 1 $\mu$M) bestimmt. Hierzu wird nach Präinkubation der Zelle mit der Testsubstanz für ca. 20 Sekunden und Messung der Membranströme eine Testlösung, welche dieselbe Substanzkonzentration und eine definierte ACh-Konzentrationen enthielt, für ca. 10 bis 20 Sekunden perfundiert und die Membranströme gemessen. Nach Applikation der maximalen Acetylcholin-Konzentration innerhalb einer Messreihe erfolgt wiederum die Perfusion einer Lösung mit Atropin (10 $\mu$M), um die M2-Selektivität des Substanzeffektes zu kontrollieren. Der $EC_{50}$-Wert in Anwesenheit von Testsubstanz wird analog mit Hilfe einer 4-Parameter-Logistik-Funktion (Hill-Funktion) ermittelt (s.o.).

Die Verschiebung der Acetylcholin Dosis-Wirkungskurve wird anhand der Veränderung des $EC_{50}$-Wertes für Acetylcholin in Ab- oder Anwesenheit der Testsubstanz ermittelt und quantifiziert.

## B-8. Effekte der Testsubstanzen auf das isolierte perfundiert Rattenherz

Männliche Wistar-Ratten (Stamm: HsdCpb:WU) mit einem Körpergewicht von 200-250g werden mit Narcoren (100 mg/kg) narkotisiert. Nach Eröffnen des Thorax wird das Herz frei präpariert, ausgeschnitten und durch Kanulieren der Aorta an eine Langendorff-Apparatur angeschlossen. Das Herz wird retrograd mit einer Krebs-Henseleit-Pufferlösung (begast mit 95% $O_2$ und 5% $CO_2$, pH 7,4, 35°C) mit folgender Zusammensetzung in mmol/l: NaCl 118; KCl 3; $NaHCO_3$ 22; $KH_2PO_4$ 1,2; Magnesiumsulfat 1,2; $CaCl_2$ 1,8; Glucose 10; Na-Pyruvat 2 mit 9 ml/min flußkonstant perfundiert. Für die Erfassung der Kontraktionskraft des Herzens wird durch eine Öffnung im linken Herzohr an einem PE-Schlauch befestigter und mit Wasser gefüllter Ballon aus dünner Kunststofffolie in den linken Ventrikel eingeführt. Der Ballon wird mit einem Druckaufnehmer verbunden. Der enddiastolische Druck wird auf 5-10 mmHg über das Ballonvolumen eingestellt. Die Daten werden von einem Brückenverstärker verstärkt und von einem Computer mit der LabChart Software (ADInstruments) registriert.

Um die allosterische Wirkung der Testsubstanzen zu untersuchen, werden die Herzen mit Zusatz von 300 nmol/l der Testsubstanz perfundiert. Nach 15 Min wird Carbachol kumulativ in ansteigenden Konzentrationen der Perfusionslösung hinzugefügt. Daraus resultierende Senkung der Herzfrequenz wird als Dosis-Wirkungs-Kurve mit Effekten auf Herzen verglichen, die mit Lösungsmittel statt Testsubstanz behandelt wurden. Die Verschiebung der Carbachol-Dosis-Wirkungskurve wird mittels GraphPad PRISM (sigmoidal dose-response) analysiert und quantifiziert.

**B-9. Effekte der Testsubstanzen auf die Herzfrequenz in narkotisierten Ratten**

Männliche Ratten des Stamm (WI) WU Br des Züchters Charles River werden initial mit einer 4-5%-igen Isofluran-Inhalation ca. 3 min narkotisiert. Danach erfolgt eine Erhaltungsnarkose mit einer 1,5 %-igen Isofluran-Inhalation. Dafür werden die narkotisierten Tiere dorsal auf einer beheizten Operationsplatte fixiert. Über visuelle Kontrolle und Zwischenzehenreflex wird die Narkosetiefe überprüft.

Für die Applikation der Testsubstanz wird ein i.v. Zugang an der Vena jugularis angelegt. Im Folgenden wird ein Hautschnitt in Längsrichtung von kaudal nach kranial durchgeführt und sowohl die Halsmuskulatur als auch die Speicheldrüsen durchtrennt. Die rechte Arteria carotis communis wird dargestellt und sowohl proximal als auch distal werden Blutsperren angelegt. Mit Mikroinstrumentarium wird ein TIP- Katheter (1.2F) in das Gefäß eingebracht um den arteriellen Druck und die Herzfrequenz zu messen.

Zunächst werden beide Parameter für 10 min im Basalzustand ohne Substanzgabe erfasst. Die zu untersuchenden Substanzen werden in geeigneten Lösungsmittelgemischen gelöst und anschliessend in verschiedenen Dosierungen jeweils einer Gruppe von Tieren über die Vena jugalaris über eine Infusionspumpe über 5 Min verabreicht. Eine Lösungsmittel-behandelte Gruppe wird als Kontrolle unter den gleichen Versuchsbedingungen eingesetzt. Die Erfassung des arteriellen Blutdruckes sowie der Herzfrequenz unter Substanzgabe erfolgt für 20 min. Die Daten werden mit dem PowerLab-System (ADinstruments) registriert und mit dem Programm LabChart (ADinstruments) ausgewertet.

**B-10. Radiotelemetrische Messung von Blutdruck und Herzfrequenz an wachen Ratten**

Für die im Folgenden beschriebenen Messungen an wachen Ratten wird ein im Handel erhältliches Telemetriesystem der Firma Data Sciences International DSI, USA, eingesetzt. Das System besteht aus 3 Hauptkomponenten: (1) Implantierbare Sender (PhysioTel® Telemetrietransmitter), (2) Empfänger (PhysioTel® Receiver), die über einen Multiplexer (DSI Data Exchange Matrix) mit einem (3) Datenakquisitionscomputer verbunden sind. Die Telemetrieanlage ermöglicht eine kontinuierliche Erfassung von Blutdruck, Herzfrequenz und Körperbewegung an wachen Tieren in ihrem gewohnten Lebensraum.

Die Untersuchungen werden an ausgewachsenen weiblichen Ratten (Wistar Unilever/WU oder Spontaneous Hypertensive Rat/SHR) mit einem Körpergewicht von > 200 g durchgeführt. Die Versuchstiere werden nach Senderimplantation einzeln in Makroion®-Käfigen Typ III gehalten. Sie haben freien Zugang zu Standardfutter und Wasser. Der Tag/Nacht-Rhythmus im Versuchslabor wird durch Wechsel der Raumbeleuchtung eingestellt.

Senderimplantation:

Die eingesetzten Telemetriesender (z.B. PA-C40, HD-S10, DSI) werden den Versuchstieren mindestens 14 Tage vor dem ersten Versuchseinsatz unter aseptischen Bedingungen chirurgisch implantiert. Zur Implantation werden die nüchternen Tiere mit Isofluran narkotisiert (IsoFlo®, Abbott, Einleitung 5%, Erhaltung 2%) und an der Bauchseite weiträumig rasiert und desinfiziert. Nach Eröffnung des Bauchraumes entlang der Linea alba wird der flüssigkeitsgefüllte Messkatheter des Systems oberhalb der Bifurkation nach cranial in die Aorta descendens eingesetzt und mit Gewebekleber (Vetbond™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum (Ursocyclin® 10%, 60 mg/kg s.c., 0.06 ml/100 g Körpergewicht, Serumwerk Bernburg AG, Deutschland) sowie ein Analgetikum (Rimadyl®, 4 mg/kg s.c., Pfizer, Deutschland) verabreicht.

Substanzen und Lösungen:

Wenn nicht anders beschrieben, werden die zu untersuchenden Substanzen jeweils einer Gruppe von Tieren (M= 6) oral verabreicht. Entsprechend einem Applikationsvolumen von 2 ml/kg Körpergewicht werden die Testsubstanzen in geeigneten Lösungsmittelgemischen gelöst. Eine Lösungsmittel-behandelte Gruppe von Tieren wird als Kontrolle eingesetzt.

Versuchsablauf:

Die Telemetrie-Messeinrichtung ist für 24 Tiere konfiguriert. Den in der Anlage lebenden instrumentierten Ratten ist jeweils eine eigene Empfangsantenne zugeordnet (RPC-1 Receiver, DSI). Die implantierten Sender sind über einen

eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI oder Ponemah, DSI) online erfasst und entsprechend aufgearbeitet werden. Im Standardablauf werden über je 10 Sekunden Dauer gemessen: (1) systolischer Blutdruck (SBP), (2) diastolischer Blutdruck (DBP), (3) arterieller Mitteldruck (MAP), (4) Herzfrequenz (HR) und (5) Aktivität (ACT). Diese Parameter werden im Anschluss an die Applikation über 24 Stunden gemessen. Die Messwerterfassung wird rechnergesteuert in 5 Minuten-Abständen wiederholt. Die als Absolutwert erhobenen Quelldaten werden im Diagramm mit dem aktuell gemessenen Barometerdruck (Ambient Pressure Reference Monitor, APR-1, DSI) korrigiert.

Auswertung:

Nach Versuchsende werden die erhobenen Einzeldaten mit der Analysis-Software (Dataquest™ A.R.T. 4.1 Analysis oder Ponemah, DSI) sortiert. Als Leerwert wird der Zeitpunkt 2 Stunden vor Substanz-Applikation angenommen. Die Daten werden über eine voreinstellbare Zeit durch Mittelwertbestimmung geglättet (30 Minuten-Mittelwert).

**B-11. Effekte der Testsubstanzen auf die Herzfrequenz in anästhesierten Hunden**

Männliche oder weibliche Mischlingshunde (Mongrels, Marshall BioResources, USA) mit einem Gewicht zwischen 20 und 30 Kg werden mit Pentobarbital (30 mg/kg iv, Narcoren®, Merial, Deutschland) annarkotisiert. Pancuroniumchlorid (Pancuronium-Actavis®, Actavis, Deutschland, 1 mg/Tier iv) dient dabei zusätzlich als Muskelrelaxans. Die Hunde werden intubiert und mit einem Sauerstoff-Raumluft-Gemisch (40/60%) beatmet (etwa 5-6L/min). Die Beatmung erfolgt mit einem Beatmungsgerät der Firma GE Healthcare (Avance), welches gleichzeitig als Narkoseüberwachung dient (CO2-Analysator). Die Narkose wird aufrechterhalten durch eine ständige Infusion von Pentobarbital (50µg/kg/min), als Analgetikum dient Fentanyl (10µg/kg/h). Eine Alternative zu Pentobarbital besteht in der Verwendung von Isofluran (1-2Vol%).

Der Hund wird folgendermaßen instrumentiert:

- Blasenkatheter zur Blasenentlastung bzw. zur Messung des Urinflusses
- EKG-Ableitungen an den Extremitäten (zur EKG Messung)
- Einführen eines NaCl-gefüllten Fluidmedic-PE-300-Schlauches in die A. femoralis. Dieser ist verbunden mit einem Drucksensor (Braun Melsungen, Melsungen, Deutschland) zur Messung des systemischen Blutdrucks
- Einführen eines NaCl-gefüllten Venenkatheter (Vygon, Deutschland) in die V.femoralis zur Infusion von Prüfsubstanzen oder zur Blutentnahme.
- Einführen eines Millar Tip Katheters (Typ 350 PC, Millar Instruments, Houston, USA) über den linken Vorhof oder über eine in der A. carotis eingebundene Schleuse zur Messung der Herz-Hämodynamik
- Einführen eines Swan-Ganz-Katheters (CCOmbo 7.5F, Edwards, Irvine, USA) über die V. jugularis in die A. pulmonalis zur Messung von Herzzeitvolumen, Sauerstoffsättigung, Pulmonalartiendrücken und zentralvenösem Druck.
- Anbringen einer Ultraschall-Flussmess-Sonde (Transsonic Systems, Ithaka, USA) an der Aorta descendens zur Messung des Aortenflusses
- Anbringen einer Ultraschall-Flussmess-Sonde (Transsonic Systems, Ithaka, USA) an der linken A. coronaria zur Messung des Koronarflusses.
- Platzieren einer Braunüle in den Venae cephalicae zur Infusion von Pentobarbital, der Flüssigkeitssubstitution und zur Blutentnahme (Bestimmung der Substanz-Plasmaspiegeln oder sonstiger klinischer Blutwerte)
- Platzieren einer Braunüle in der Venae saphenae zur Infusion von Fentanyl und zur Substanzapplikation

Die Primärsignale werden eventuell verstärkt (Gould Verstärker, Gould Instrument Systems, Valley View, USA) bzw. Edwards-Vigilance- Monitor (Edwards, Irvine, USA) und anschließend zur Auswertung in das Ponemah-System (DataSciences Inc, Minneapolis, USA) eingespeist. Die Signale werden kontinuierlich über den gesamten Versuchszeitraum aufgezeichnet, von dieser Software digital weiterarbeitet und über 30 s gemittelt.

**B-12. Effekte der Testsubstanzen auf die Herzfrequenz und Herzfreguenzvariabilität in gesunden, wachen Hunden**

Zur Charakterisierung von Prüfsubstanzen hinsichtlich ihrer Wirkung auf die Herzfrequenz, Herzfrequenzvariabilität (HRV) und Blutdruck werden telemetrische Messungen in gesunden, männlichen Beagle-Hunden durchgeführt. Unter Isofluran-Narkose wird den Tieren zunächst ein Telemetriesender (Modell L21, Firma Data Sciences International, USA) implantiert. Dabei werden nach linksseitiger Thorakotomie Drucksensoren in der Aorta und im linken Ventrikel platziert. Zur Registrierung eines Elektrokardiogramms (EKG) werden weiterhin Elektroden auf dem Herzen platziert. Die Tiere werden anschließend zur Wundheilung unter antibiotischer (Clindamycin, Zoetis, Deutschland) und analgetischer (Fentanyl, Janssen, Deutschland) Nachsorge zurück in den Stall verbracht. Mittels im Tierstall installierter Antennen werden die Blutdruck- und EKG-Signale an einen Datenakquisitionscomputer weitergeleitet und durch eine Analysesoftware (Ponemah, Data Sciences International, USA) ausgewertet. Die Telemetrie-Anlage ermöglicht eine kontinuierliche Erfassung von Blutdrücken und EKG-Signalen in wachen Tieren. Technische Details können der Dokumentation der Herstellerfirma (Data Sciences International, USA) entnommen werden.

Die zu untersuchenden Substanzen werden den gesunden Hunden mittels einer Gelatine-Kapsel in geeigneten Lösungsmittelgemischen oral verabreicht. Eine Vehikel-behandelte Gruppe von Tieren wird als Kontrolle eingesetzt. Die telemetrische Messung wird vor Substanzgabe gestartet und über einen Zeitraum von mehreren Stunden aufgezeichnet. Die graphische Darstellung der zeitlichen Verläufe der durch Mittelwertbestimmung geglätteten Daten erfolgt mit Hilfe der GraphPadPrism Software (GraphPad, USA). Zur Analyse der HRV werden die EKG-Daten einer frequenzbezogenen Herzfrequenzvariabilitäts-Analyse ("frequency-domain") unterzogen. Dazu werden die R-R-Intervalle der aufgezeichneten EKG's verwendet. Daten außerhalb des zuvor definierten Bereiches von 0,2s - 1,5s werden von der Analyse ausgeschlossen. Die ausgeschlossenen Daten werden durch Werte die durch lineare Interpolation gewonnen wurden ersetzt. Diese Daten werden durch Spline-Intepolation in gleichabständige Unterstützungspunkte konvertiert. Zur Analyse der Herzfrequenzvariabilität werden die Daten weiterhin in 30 s Schritten zu Paketen von 300s Länge unterteilt. Für jedes Datenpaket wird eine Fourier-Transformation kalkuliert. Daraus wird weiterhin die Leistung in drei Frequenzbändern (vlf=0.0033 - 0.04 1/s; lf=0.04 - 0.15 1/s; hf=0.15 - 0.5 1/s) kalkuliert. Zur Charakterisierung der Prüfsubstanz wird die Gesamtleistung (Summe aller drei Frequenzbänder) zur HRV- Analyse herangezogen.

## Patentansprüche

1. Verbindung der Formel (I)

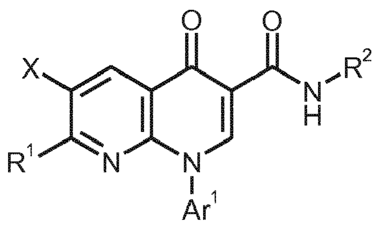

(I),

in welcher

X für Halogen steht,
$R^1$ für Wasserstoff steht,
oder
für $-NR^4R^5$ steht,
worin

$R^4$ Wasserstoff, Methyl, $(C_2-C_4)$-Alkyl oder $(C_3-C_6)$-Cycloalkyl bedeutet, wobei $(C_2-C_4)$-Alkyl mit Hydroxy oder bis zu dreifach mit Fluor substituiert sein kann und
$R^5$ $(C_1-C_6)$-Alkyl, $(C_3-C_6)$-Cycloalkyl, 3- bis 6-gliedriges gesättigtes Heterocyclyl oder $(C_1-C_4)$-Alkylsulfonyl bedeutet, wobei $(C_1-C_6)$-Alkyl, $(C_3-C_6)$-Cycloalkyl und 3- bis 6-gliedriges gesättigtes Heterocyclyl, bis zu dreifach, gleich oder verschieden, mit Methyl, Difluormethyl, Trifluormethyl, Hydroxy, Hydroxycarbonyl, Oxo, Methoxy, Difluormethoxy, Trifluormethoxy, Cyano und weiterhin bis zu vierfach mit Fluor, substituiert

sein können, oder
$R^4$ und $R^5$ bilden zusammen mit dem Stickstoffatom, an das sie gebunden sind, einen gesättigten oder partiell ungesättigten, 3- bis 6-gliedrigen monocyclischen oder 6- bis 10gliedrigen bicyclischen Heterocyclus, der ein oder zwei weitere, gleiche oder verschiedene Heteroatome aus der Reihe N, O, S, SO und/oder $SO_2$ als Ringglieder enthalten kann,
wobei der 3- bis 6-gliedrige monocyclische und der 6- bis 10-gliedrige bicyclische Heterocyclus jeweils mit 1 bis 5 Substituenten unabhängig voneinander ausgewählt aus der Gruppe $(C_1\text{-}C_4)$-Alkyl, Difluormethyl, Trifluormethyl, Hydroxy, Hydroxycarbonyl, Oxo, $(C_1\text{-}C_3)$-Alkoxy, Difluormethoxy, Trifluormethoxy, Cyano, $(C_1\text{-}C_3)$-Alkoxycarbonyl, Aminocarbonyl, Mono-$(C_1\text{-}C_3)$-alkylaminocarbonyloxy,-NHC(=O)$R^{13A}$, -$CH_2$NHC(=O)$R^{13B}$, -OC(=O)$R^{14}$, und weiterhin bis zu vierfach mit Fluor, substituiert sein können,
worin $(C_1\text{-}C_4)$-Alkyl ein- oder zweifach, gleich oder verschieden, mit Hydroxy, $(C_1\text{-}C_3)$-Alkoxy, und bis zu vierfach mit Fluor, substituiert sein kann,
$R^{13A}$ und $R^{13B}$ unabhängig voneinander $(C_1\text{-}C_3)$-Alkyl oder Cyclopropyl darstellen,
und worin
$R^{14}$ $(C_1\text{-}C_4)$-Alkyl darstellt,

$R^2$ für eine Gruppe der Formel

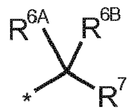

oder *-$L^1$-$Ar^2$ steht, worin

* die Verknüpfungsstelle mit dem N-Atom der Amid-Gruppierung markiert,
$R^{6A}$ Wasserstoff oder $(C_1\text{-}C_4)$-Alkyl bedeutet,
$R^{6B}$ Wasserstoff, $(C_1\text{-}C_4)$-Alkyl, Cyclopropyl, Monofluormethyl, Difluormethyl, Trifluormethyl, Methoxymethyl oder Trifluormethoxymethyl bedeutet,
$R^7$ $(C_1\text{-}C_6)$-Alkyl oder bis zu vierfach mit Fluor substituiertes $(C_3\text{-}C_5)$-Cycloalkyl bedeutet,
wobei $(C_1\text{-}C_6)$-Alkyl mit Amino, Hydroxy, $(C_1\text{-}C_6)$-Alkoxy und bis zu fünffach mit Fluor substituiert sein kann,
worin $(C_1\text{-}C_6)$-Alkoxy bis zu fünffach mit Fluor substituiert sein kann
$L^1$ eine Bindung oder eine Gruppe der Formel $-C(R^{8A}R^{8B})-(C(R^{9A}R^{9B}))_m-$ bedeutet, worin

m 0 oder 1 darstellt,
$R^{8A}$ Wasserstoff oder Methyl darstellt,
$R^{8B}$ Wasserstoff, Methyl, Trifluormethyl, Pentafluorethyl oder Trifluormethoxymethyl darstellt,
$R^{9A}$ und $R^{9B}$ unabhängig voneinander Wasserstoff oder Methyl darstellen,

$Ar^2$ Phenyl bedeutet,
wobei Phenyl ein- bis dreifach, gleich oder verschieden, mit Fluor, Chlor, $(C_1\text{-}C_3)$-Alkyl, Difluomethoxymethyl, Trifluormethoxymethyl und/oder Trifluormethyl substituiert sein kann,

oder
für einen 5- bis 10-gliedrigen monocyclischen, bicyclischen oder tricyclischen Carbocyclus oder Heterocyclus, der ein oder zwei weitere, gleiche oder verschiedene Heteroatome aus der Reihe N und/oder O als Ringglieder enthalten kann, steht,
wobei der 5- bis 10-gliedrige monocyclische, bicyclische oder tricyclische Carbocyclus oder Heterocyclus bis zu dreifach, gleich oder verschieden, mit $(C_1\text{-}C_3)$-Alkyl, Trifluormethyl, $(C_1\text{-}C_4)$-Alkoxycarbonyl und weiterhin bis zu vierfach mit Fluor substituiert sein können,
$Ar^1$ für eine Gruppe der Formel

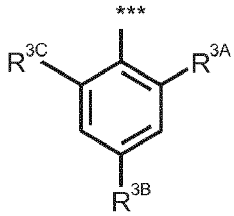

steht, worin

*** die Verknüpfungsstelle mit dem N-Atom markiert,
$R^{3A}$ für Fluor, Chlor, Trifluormethyl oder Methyl steht,
$R^{3B}$ für Wasserstoff oder Fluor steht
und $R^{3C}$ für Wasserstoff, Fluor, Chlor oder Methyl steht,

oder
für einen über ein Ring-Kohlenstoffatom angebundenen Pyridinring steht,
wobei der Pyridinring ein oder zweifach mit Fluor, Chlor, Cyano, Methyl oder Trifluormethyl substituiert sein kann,

sowie ihre *N*-Oxide, Salze, Solvate, Salze der *N*-Oxide und Solvate der *N*-Oxide und Salze.

**2.** Verbindung der Formel (I) nach Anspruch 1,
in welcher

X für Fluor oder Chlor steht,
$R^1$ für Wasserstoff steht,
oder
für $NR^4R^5$ steht, worin

$R^4$ Wasserstoff, Methyl oder Ethyl bedeutet, und
$R^5$ bis zu vierfach mit Fluor substituiertes ($C_1$-$C_3$)-Alkyl bedeutet, wobei ($C_1$-$C_3$)-Alkyl mit Hydroxy substituiert sein kann,

oder
$R^4$ und $R^5$ zusammen mit dem Stickstoffatom, an das sie gebunden sind, einen gesättigten 4-bis 6-gliedrigen monocyclischen oder 6- bis 9-gliedrigen bicyclischen Heterocyclus bilden, der ein oder zwei weitere, gleiche oder verschiedene Heteroatome aus der Reihe N und/oder O als Ringglieder enthalten kann,

wobei der 4- bis 6-gliedrige monocyclische und der 6- bis 9-gliedrige bicyclische Heterocyclus jeweils mit 1 bis 4 Substituenten unabhängig voneinander ausgewählt aus der Gruppe ($C_1$-$C_4$)-Alkyl, Difluormethyl, Trifluormethyl, Hydroxy, Oxo, ($C_1$-$C_3$)-Alkoxy, Difluormethoxy, Trifluormethoxy, ($C_1$-$C_3$)-Alkoxycarbonyl, ($C_1$-$C_3$)-Alkylaminocarbonyloxy, -OC(=O)$R^{14}$, und weiterhin bis zu vierfach mit Fluor, substituiert sein können,

worin ($C_1$-$C_4$)-Alkyl ein- oder zweifach, gleich oder verschieden, mit Hydroxy, ($C_1$-$C_3$)-Alkoxy, und bis zu vierfach mit Fluor, substituiert sein kann, und worin $R^{14}$ ($C_1$-$C_4$)-Alkyl darstellt,

$R^2$ für eine Gruppe der Formel

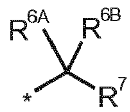

oder *-$L^1$-$Ar^2$ steht, worin

* die Verknüpfungsstelle mit dem N-Atom der Amid-Gruppierung markiert,
$R^{6A}$ Wasserstoff oder ($C_1$-$C_4$)-Alkyl bedeutet,

$R^{6B}$ Methyl, Ethyl, Isopropyl, Cyclopropyl, Monofluormethyl, Difluormethyl, oder Trifluormethyl, bedeutet, und
$R^7$ bis zu fünffach mit Fluor substituiertes ($C_1$-$C_4$)-Alkyl, bis zu vierfach mit Fluor substituiertes ($C_3$-$C_5$)-Cycloalkyl, Methoxymethyl oder Trifluormethoxymethyl bedeutet,
$L^1$ eine Bindung oder eine Gruppe der Formel -$CR^{8A}R^{8B}$- bedeutet,
worin
$R^{8A}$ Wasserstoff darstellt,
$R^{8B}$ Wasserstoff, Methyl, Trifluormethyl, Pentafluorethyl oder Trifluormethoxymethyl darstellt,
$Ar^2$ Phenyl bedeutet,
wobei Phenyl ein- bis dreifach, gleich oder verschieden, mit Fluor oder Chlor, substituiert sein kann,
oder
für einen 5- bis 7-gliedrigen bicyclischen Carbocyclus oder 5- oder 6-gliedrigen monocyclischen Heterocyclus, der ein Stickstoffatom als Ringglied enthält, steht,
wobei der 5- bis 7-gliedrige bicyclische Carbocyclus oder der 5- oder 6-gliedrige monocyclische Heterocyclus jeweils mit ($C_1$-$C_4$)-Alkoxycarbonyl und weiterhin bis zu vierfach mit Fluor substituiert sein können,
$Ar^1$ für eine Gruppe der Formel

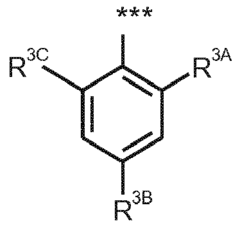

steht, worin

*** die Verknüpfungsstelle mit dem N-Atom markiert,
$R^{3A}$ für Fluor, Chlor, Trifluormethyl oder Methyl steht,
$R^{3B}$ für Wasserstoff oder Fluor steht
und $R^{3C}$ für Wasserstoff, Fluor, Chlor oder Methyl steht,

oder
für einen über ein Ring-Kohlenstoffatom angebundenen Pyridinring steht,
wobei der Pyridinring ein oder zweifach mit Fluor, Chlor oder Cyano substituiert sein kann, sowie ihre Salze, Solvate und Solvate der Salze.

**3.** Verbindung der Formel (I) nach Anspruch 1 oder 2, in welcher

X für Fluor oder Chlor steht,
$R^1$ für $NR^4R^5$ steht, worin
$R^4$ Methyl oder Ethyl bedeutet, und
$R^5$ Methyl, 2-Hydroxyethyl oder 2-Hydroxypropyl bedeutet,
oder
für einen über ein Stickstoff-Atom gebundenen Heterocyclus der Formel

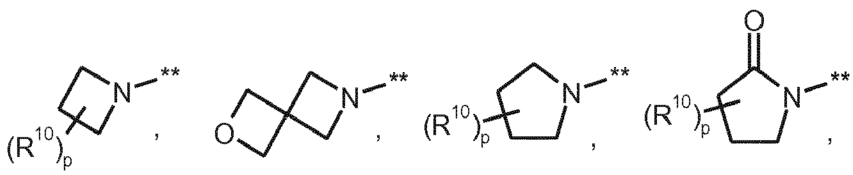

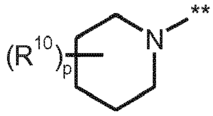 oder 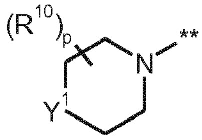

steht, worin

** die Verknüpfungsstelle mit dem Rest des Moleküls markiert,
$R^{10}$ Fluor, Methyl, Hydroxy, Hydroxymethyl, Methoxycarbonyl oder Acetyloxy darstellt,
p die Zahl 0, 1, oder 2 darstellt,
wobei im Fall, dass die Substituenten $R^{10}$ mehrfach auftreten, deren Bedeutungen jeweils gleich oder verschieden sein können,
$Y^1$ -NH-, -N($CH_3$)- oder -O- darstellt,

$R^2$ für eine Gruppe der Formel

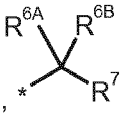 , 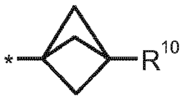 , 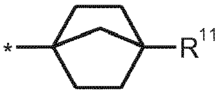 ,

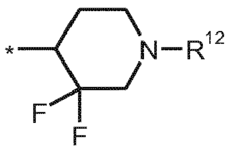 oder $*-L^1-Ar^2$

steht, worin

* die Verknüpfungsstelle mit dem N-Atom der Amid-Gruppierung markiert,
$R^{6A}$ Wasserstoff, Methyl oder Ethyl bedeutet,
$R^{6B}$ Methyl, Ethyl, Trifluormethyl, Isopropyl oder Cyclopropyl, bedeutet, und
$R^7$ Methyl, Ethyl, Difluormethyl, Trifluormethyl, 2,2,2-Trifluorethyl, Pentafluorethyl, Isopropyl, iso-Butyl, Methoxymethyl, Trifluormethoxymethyl oder Cyclopropyl bedeutet,
$R^{10}$ Wasserstoff bedeutet,
$R^{11}$ Methoxycarbonyl bedeutet,
$R^{12}$ Wasserstoff oder tert-Butoxycarbonyl bedeutet,
$L^1$ eine Bindung oder eine Gruppe der Formel $-CR^{8A}R^{8B}-$ bedeutet,
worin
$R^{8A}$ Wasserstoff darstellt,
$R^{8B}$ Wasserstoff, Methyl oder Trifluormethyl darstellt,
$Ar^2$ Phenyl bedeutet,
wobei Phenyl ein- bis zweifach, gleich oder verschieden, mit Fluor oder Chlor, substituiert sein kann,
$Ar^1$ für eine Gruppe der Formel

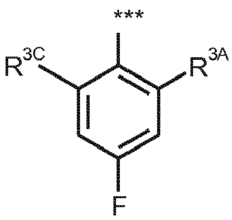 , 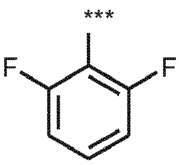 oder 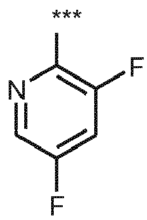

steht, worin

*** die Verknüpfungsstelle mit dem N-Atom markiert,
$R^{3A}$ für Fluor oder Chlor steht,

und $R^{3C}$ für Wasserstoff oder Fluor steht,

sowie ihre Salze, Solvate und Solvate der Salze.

**4.** Verbindung der Formel (I) nach einem der Ansprüche 1 bis 3, in welcher

X für Fluor steht,
$R^1$ für einen über ein Stickstoff-Atom gebundenen Heterocyclus der Formel

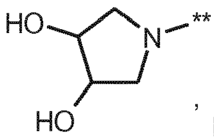 , 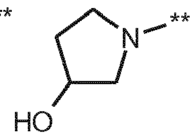 oder 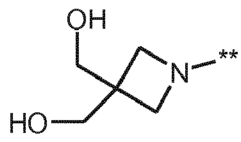

steht, worin

** die Verknüpfungsstelle mit dem Rest des Moleküls markiert,

$R^2$ für eine Gruppe der Formel

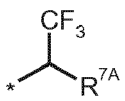 , 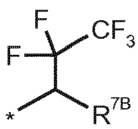 , 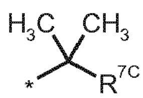 ,

steht, worin

* die Verknüpfungsstelle mit dem N-Atom der Amid-Gruppierung markiert,
$R^{7A}$ Trifluormethyl, Ethyl oder Cyclopropyl bedeutet,
$R^{7B}$ Methyl oder Ethyl bedeutet,
$R^{7C}$ Trifluormethyl oder Pentafluorethyl bedeutet,

$Ar^1$ für eine Gruppe der Formel

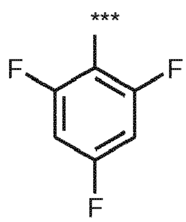 oder 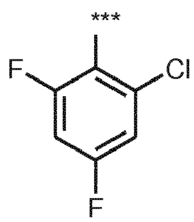

steht, worin

*** die Verknüpfungsstelle mit dem N-Atom markiert,

sowie ihre Salze, Solvate und Solvate der Salze.

**5.** Verfahren zur Herstellung von Verbindungen der Formel (I) wie in einem der Ansprüche 1 bis 4 definiert, **dadurch gekennzeichnet, dass** man

[A] eine Verbindung der Formel (II)

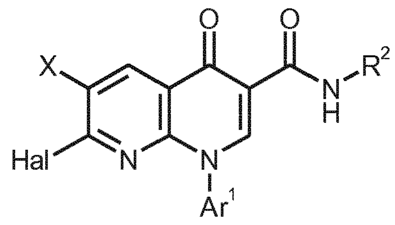

(II),

in welcher X, $R^2$ und $Ar^1$ die in den Ansprüchen 1 bis 4 für Verbindungen der Formel (I) angegebenen Bedeutungen haben,
und

Hal für Fluor, Chlor, Brom oder Iod, bevorzugt für Chlor, steht,

mit einer Verbindung der Formel (III)

$R^1$-H (III),

in welcher $R^1$ die in den Ansprüchen 1 bis 4 für Verbindungen der Formel (I) an gegebene Bedeutung hat, und wobei $R^1$nicht Wasserstoff bedeutet,
zum Carbonsäureamid der Formel (I-A)

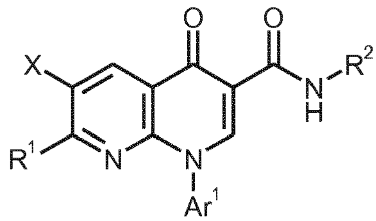

(I-A),

in welcher X, $R^1$, $R^2$ und $Ar^1$ die in den Ansprüchen 1 bis 4 für Verbindungen der Formel (I) angegebenen Bedeutungen haben, und wobei $R^1$nicht Wasserstoff bedeutet, umsetzt, oder
[B] eine Verbindung der Formel (IV)

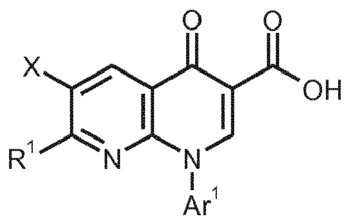

(IV),

in welcher X, $R^1$ und $Ar^1$ die in den Ansprüchen 1 bis 4 für Verbindungen der Formel (I) an gegebenen Bedeutungen haben,
mit einer Verbindung der Formel (V)

$R^2$-$NH_2$ (V),

in welcher $R^2$ die in den Ansprüchen 1 bis 4 für Verbindungen der Formel (I) angegebene Bedeutung hat,
zum Carbonsäureamid der Formel (I)

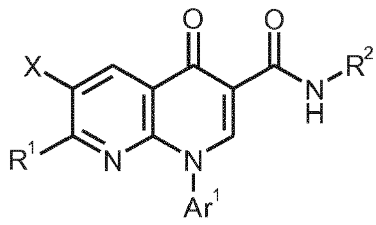

(I),

in welcher $R^1$, $R^2$ und $Ar^1$ die in den Ansprüchen 1 bis 4 für Verbindungen der Formel (I) an gegebenen Bedeutungen haben,
umsetzt,
und gegebenenfalls die so erhaltenen Verbindungen der Formel (I) in ihre Enantiomere und/oder Diastereomere trennt und/oder mit den entsprechenden (i) Lösungsmitteln und/oder (*ii*) Basen oder Säuren in ihre Solvate, Salze und/oder Solvate der Salze überführt.

**6.** Verbindung der Formel (II)

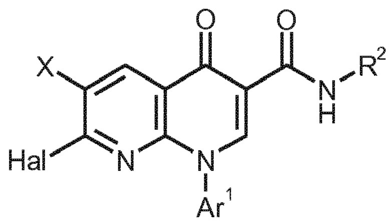

(II),

in welcher X, $R^2$ und $Ar^1$ die in den Ansprüchen 1 bis 4 für Verbindungen der Formel (I) angegebenen Bedeutungen haben
und

Hal für Fluor, Chlor, Brom oder Iod, bevorzugt für Chlor, steht.

**7.** Verbindung der Formel (IV)

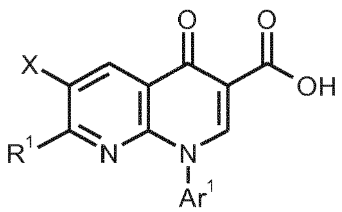

(IV),

in welcher X, $R^1$ und $Ar^1$ die in den Ansprüchen 1 bis 4 für Verbindungen der Formel (I) angegebenen Bedeutungen haben.

**8.** Verwendung einer Verbindung der Formel (II)

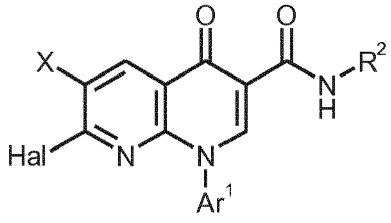

(II),

in welcher X, $R^2$ und $Ar^1$ die in den Ansprüchen 1 bis 4 für Verbindungen der Formel (I) angegebenen Bedeutungen

haben
und

Hal für Fluor, Chlor, Brom oder Iod, bevorzugt für Chlor, steht.

oder
einer Verbindung der Formel (IV)

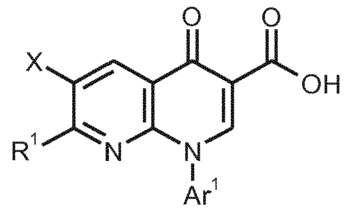

(IV),

in welcher X, $R^1$ und $Ar^1$ die in den Ansprüchen 1 bis 4 für Verbindungen der Formel (I) angegebenen Bedeutungen haben, zur Herstellung einer Verbindung der Formel (I) gemäß einem der Ansprüche 1 bis 4.

**9.** Verbindung, wie in einem der Ansprüche 1 bis 4 definiert, zur Verwendung bei der Behandlung und/oder Prophylaxe von Krankheiten.

**10.** Verbindung der Formel (I), wie in einem der Ansprüche 1 bis 4 definiert, zur Verwendung in einen Verfahren zur Behandlung und/oder Prophylaxe von Herzinsuffizienz, koronarer Herzerkrankung, atrialen und ventrikulären Arrhythmien, Niereninsuffizienz und Nephropathien.

**11.** Arzneimittel enthaltend eine Verbindung, wie in einem der Ansprüche 1 bis 4 definiert, in Kombination mit einem oder mehreren weiteren Wirkstoffen ausgewählt aus der Gruppe bestehend aus den Blutdruck senkende Wirkstoffe, antiarrhythmische Wirkstoffe, Vasopressin-Rezeptor-Antagonisten, PDE 5-Inhibitoren, Thrombozytenaggregationshemmer, sGC-Aktivatoren und sGC-Stimulatoren.

**12.** Arzneimittel enthaltend eine Verbindung, wie in einem der Ansprüche 1 bis 4 definiert, in Kombination mit einem inerten, nichttoxischen, pharmazeutisch geeigneten Hilfsstoff.

**13.** Arzneimittel nach Anspruch 11 oder 12 zur Verwendung bei der Behandlung und/oder Prophylaxe von Herzinsuffizienz, koronarer Herzerkrankung, atrialen und ventrikulären Arrhythmien, Niereninsuffizienz und Nephropathien.

Europäisches Patentamt
European Patent Office
Office européen des brevets

# EUROPÄISCHER RECHERCHENBERICHT

Nummer der Anmeldung
EP 16 18 8728

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 2005/026165 A1 (WARNER LAMBERT CO [US]; ELLSWORTH EDMUND LEE [US]; SCIOTTI RICHARD JOH) 24. März 2005 (2005-03-24)<br>* die auf Seite 206 letztgennante Verbindung *<br>----- | 7 | INV.<br>C07D471/04<br>C07D491/107<br>A61K31/4375<br>A61P9/00<br>A61P13/00 |
| X | WO 2010/093341 A1 (JANSSEN PHARMACEUTICA NV [BE]; MACIELAG MARK J [US]; WEIDNER-WELLS MIC)<br>19. August 2010 (2010-08-19)<br>* Seite 65 - Seite 66; Verbindungen 106, 107 *<br>----- | 7 | |
| X | BOUZARD D ET AL: "FLUORONAPHTHYRIDINES AS ANTIBACTERIAL AGENTS. 4. ÖSYNTHESIS AND STRUCTURE-ACTIVITY RELATIONSHIP OF 5-SUBSTITUTED-6-FLUORO-7-(CYCLOALKZLAMINO-)-1,4-DIHYDRO-4-OXO-1,8-NAPHTHYRIDINE-3- CARBOXYLIC ACIDS",<br>JOURNAL OF MEDICINAL CHEMISTRY, AMERICAN CHEMICAL SOCIETY, US,<br>Bd. 35, Nr. 3,<br>7. Februar 1992 (1992-02-07), Seiten 518-525, XP000563690,<br>ISSN: 0022-2623, DOI: 10.1021/JM00081A013<br>* Tabelle I; Verbindungen 26, 28 *<br>----- | 7 | RECHERCHIERTE SACHGEBIETE (IPC)<br>C07D<br>A61K<br>A61P |
| X | EP 0 350 733 A2 (BAYER AG [DE])<br>17. Januar 1990 (1990-01-17)<br>* die erste Verbindung auf Seite 41, die erste Verbindung auf Seite 44 und die letzten drei Verbindungen auf Seite 48 *<br>-----<br>-/-- | 7 | |

Der vorliegende Recherchenbericht wurde für alle Patentansprüche erstellt

1

| Recherchenort | Abschlußdatum der Recherche | Prüfer |
|---|---|---|
| München | 20. Oktober 2016 | Moriggi, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 16 18 8728

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CURT S. COOPER ET AL: "Preparation and in vitro and in vivo evaluation of quinolones with selective activity against Gram-positive organisms", JOURNAL OF MEDICINAL CHEMISTRY, Bd. 35, Nr. 8, 1. April 1992 (1992-04-01), Seiten 1392-1398, XP055312420, US ISSN: 0022-2623, DOI: 10.1021/jm00086a007 * Tabelle I; Verbindung 25 * ----- | 7 | |
| X | DANIEL T. W. CHU ET AL: "Syntheses and antibacterial activity of novel 6-fluoro-7-( gem -disubstituted piperazin-1-yl)-quinolines", CANADIAN JOURNAL OF CHEMISTRY, Bd. 70, Nr. 5, 1. Mai 1992 (1992-05-01), Seiten 1328-1337, XP055312423, CA ISSN: 0008-4042, DOI: 10.1139/v92-171 * Verbindungen 12d, 12g, 12j * ----- | 7 | RECHERCHIERTE SACHGEBIETE (IPC) |
| X | G KLOPMAN ET AL: "N-1-tert-butyl-substituted quinolones: in vitro anti-Mycobacterium avium activities and structure-activity relationship studies", ANTIMICROBIAL AGENTS AND CHEMOTHERAPY, Bd. 40, Nr. 11, 1. November 1996 (1996-11-01), Seiten 2637-2643, XP055312425, US ISSN: 0066-4804 * Seite 2639; Verbindungen 52, 54 * ----- -/-- | 7 | |

Der vorliegende Recherchenbericht wurde für alle Patentansprüche erstellt

1

| Recherchenort | Abschlußdatum der Recherche | Prüfer |
|---|---|---|
| München | 20. Oktober 2016 | Moriggi, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 16 18 8728

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 5 496 947 A (YOON SUNG-JUNE [KR] ET AL) 5. März 1996 (1996-03-05) * Spalte 13 - Spalte 14; Beispiele 7-10, 12-15 * | 7 | |
| A | WO 2005/056552 A1 (VERTEX PHARMA [US]; GROOTENHUIS PETER D J [US]; GARCIA-GUZMAN BLANCO M) 23. Juni 2005 (2005-06-23) * Seiten 11 und 12; Seite 18, Absatz 068 - Seite 20, Absatz 75; Ansprüche 1, 24; Verbindungen 1-27 * | 1-13 | |

RECHERCHIERTE SACHGEBIETE (IPC)

Der vorliegende Recherchenbericht wurde für alle Patentansprüche erstellt

1

| Recherchenort | Abschlußdatum der Recherche | Prüfer |
|---|---|---|
| München | 20. Oktober 2016 | Moriggi, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Seite 3 von 3

## ANHANG ZUM EUROPÄISCHEN RECHERCHENBERICHT ÜBER DIE EUROPÄISCHE PATENTANMELDUNG NR.

EP 16 18 8728

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-10-2016

| Im Recherchenbericht angeführtes Patentdokument | Datum der Veröffentlichung | Mitglied(er) der Patentfamilie | Datum der Veröffentlichung |
|---|---|---|---|
| WO 2005026165 A1 | 24-03-2005 | US 2005107423 A1 | 19-05-2005 |
| | | WO 2005026165 A1 | 24-03-2005 |
| WO 2010093341 A1 | 19-08-2010 | KEINE | |
| EP 0350733 A2 | 17-01-1990 | AT 135354 T | 15-03-1996 |
| | | AT 292127 T | 15-04-2005 |
| | | AU 616277 B2 | 24-10-1991 |
| | | AU 658667 B2 | 27-04-1995 |
| | | AU 668287 B2 | 26-04-1996 |
| | | AU 671386 B2 | 22-08-1996 |
| | | AU 1028392 A | 27-02-1992 |
| | | CA 1340114 C | 03-11-1998 |
| | | CN 1039589 A | 14-02-1990 |
| | | CN 1097759 A | 25-01-1995 |
| | | CN 1143080 A | 19-02-1997 |
| | | CY 2111 B1 | 26-04-2002 |
| | | DE 3906365 A1 | 18-01-1990 |
| | | DE 19975060 I2 | 14-10-2004 |
| | | DE 58909622 D1 | 25-09-1997 |
| | | DE 58909894 D1 | 04-05-2005 |
| | | DK 350089 A | 16-01-1990 |
| | | EP 0350733 A2 | 17-01-1990 |
| | | EP 0757990 A1 | 12-02-1997 |
| | | ES 2109219 T3 | 16-01-1998 |
| | | ES 2240984 T3 | 16-10-2005 |
| | | FI 893403 A | 16-01-1990 |
| | | GR 3024841 T3 | 30-01-1998 |
| | | HK 1000938 A1 | 08-05-1998 |
| | | HU 208130 B | 30-08-1993 |
| | | HU 211472 A9 | 28-11-1995 |
| | | HU 213099 B | 28-02-1997 |
| | | IE 970856 A1 | 23-02-2000 |
| | | IL 90940 A | 12-04-1994 |
| | | JP 2771853 B2 | 02-07-1998 |
| | | JP 3001848 B2 | 24-01-2000 |
| | | JP H0269474 A | 08-03-1990 |
| | | JP H10182600 A | 07-07-1998 |
| | | LU 90645 I2 | 27-12-2000 |
| | | NL 300111 I1 | 01-04-2003 |
| | | NO 892715 A | 16-01-1990 |
| | | NZ 229914 A | 26-03-1992 |
| | | PT 91165 A | 08-02-1990 |
| | | TW 270119 B | 11-02-1996 |
| | | US 4990517 A | 05-02-1991 |
| | | US 5059597 A | 22-10-1991 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR.

EP 16 18 8728

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-10-2016

| Im Recherchenbericht angeführtes Patentdokument | Datum der Veröffentlichung | Mitglied(er) der Patentfamilie | Datum der Veröffentlichung |
|---|---|---|---|
| | | US 5416096 A | 16-05-1995 |
| | | US 5607942 A | 04-03-1997 |
| US 5496947 A | 05-03-1996 | AU 679961 B2 | 17-07-1997 |
| | | AU 5866694 A | 14-03-1995 |
| | | BR 9407283 A | 01-10-1996 |
| | | CA 2168764 A1 | 23-02-1995 |
| | | CN 1128995 A | 14-08-1996 |
| | | EP 0713487 A1 | 29-05-1996 |
| | | FI 960634 A | 12-02-1996 |
| | | HU 216803 B | 30-08-1999 |
| | | JP 2758722 B2 | 28-05-1998 |
| | | JP H09500143 A | 07-01-1997 |
| | | US 5496947 A | 05-03-1996 |
| | | WO 9505373 A1 | 23-02-1995 |
| WO 2005056552 A1 | 23-06-2005 | AU 2004297241 A1 | 23-06-2005 |
| | | CA 2548009 A1 | 23-06-2005 |
| | | CN 1914204 A | 14-02-2007 |
| | | EP 1709045 A1 | 11-10-2006 |
| | | JP 2007513955 A | 31-05-2007 |
| | | KR 20060123452 A | 01-12-2006 |
| | | MX PA06006532 A | 23-08-2006 |
| | | US 2005171141 A1 | 04-08-2005 |
| | | US 2009042928 A1 | 12-02-2009 |
| | | WO 2005056552 A1 | 23-06-2005 |
| | | ZA 200604721 B | 26-03-2008 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0945435 B1
- WO 2002085886 A2
- WO 2003050107 A1
- WO 2005026145 A2
- WO 2005026165 A1
- WO 2005049602 A1
- EP 1650192 A1
- WO 2005009971 A1
- JP 2005012561 B
- WO 2015189560 A1
- WO 2016081464 A1
- US 3966781 A, J. G. Atkinson
- WO 2012112363 A, K. Kassahun
- EP 0607825 A1
- WO 2010105770 A
- WO 2011104322 A
- WO 2016071212 A
- WO 0006568 A
- WO 0006569 A
- WO 0242301 A
- WO 03095451 A
- WO 2011147809 A
- WO 2012004258 A
- WO 2012028647 A
- WO 2012059549 A
- WO 0119355 A
- WO 0119776 A
- WO 0119778 A
- WO 0119780 A
- WO 02070462 A
- WO 02070510 A
- US 4840954 A


### In der Beschreibung aufgeführte Nicht-Patentliteratur


- **SCHRAGE et al.** *Biochem. Pharmacol.,* 2014, vol. 90 (3), 307-319
- **NEUBIG et al.** *Pharmacol Rev.,* 2003, vol. 55, 597-606
- **KRUSE et al.** *Mol Pharmacol.,* 2013, vol. 84 (4), 528-540
- **GREGORY et al.** *Current Neuropharmacol.,* 2007, vol. 5 (3), 157-167
- **CLARK ; MITCHELSON.** *Br. J. Pharmac.,* 1976, vol. 58, 323-331
- **CONN et al.** *Nat. Rev. Drug Disc.,* 2009, vol. 8, 41-54
- **CONN et al.** *Nat. Rev. Drug. Disc.,* 2014, vol. 13, 692-708
- **CHRISTOPOULOS.** *Mol. Pharmacol.,* 2014, vol. 86, 463-478
- **WANG et al.** *J. Pharmacol. Exp. Therap.,* 2009, vol. 331, 340-348
- **KRUSE et al.** *Nature,* 2013, vol. 504, 101-106
- **CROY et al.** *Molecular Pharmacology,* Juli 2014, vol. 86 (1), 106-115
- **SCHOBER et al.** *Molecular Pharmacology,* Juli 2014, vol. 86 (1), 116-123
- **HE et al.** *, Br. J. Pharmacol.,* 2014
- **RANPURIA et al.** *Nephrol Dial Transplant.,* 2008, vol. 23 (2), 444-4499
- **VINIK et al.** *Diabet Med.,* 2011, vol. 28 (6), 643-651
- **SYKORA et al.** *Stroke,* 2009, vol. 40 (12), 678-682
- **HALARIS et al.** *Mod Trends Pharmacopsychiatri.,* 2013, vol. 28, 144-161
- **RASH ; AGUIRRE-CAMACHO.** *Atten Defic Hyperact Disord.,* 2012, vol. 4 (4), 167-177
- **DE FERRARI.** *J. Cardiovasc. Transl. Res.,* 2014, vol. 7 (3), 310-320
- **DE FERRARI GM et al.** *Eur. Heart J.,* 2011, vol. 32, 847-855
- **PREMCHAND RK et al.** *J. Card. Fail.,* 2014, vol. 20 (11), 808-816
- **ZANNAD et al.** *Eur. Heart J.,* 2015, vol. 36 (7), 425-433
- **GOLD et al.** *J Am Coll Cardiol.,* 29. Marz 2016, vol. 735-1097 (16), 32404-4
- **CHEN et al.** *Circ. Res.,* 2014, vol. 114 (9), 1500-1515
- **MAISEL ; STEVENSON.** *Am. J. Cardiol.,* 2003, vol. 91, 2D-8D
- **LEVALTER T.** *Fortbildungsprogramm Pharmazie,* 2011, vol. 5, 106-127
- **LEONG-SIT ; TANG.** *Curr. Opin. Cardiol.,* 2015
- **SCAMMELLS et al.** *ACS Chem. Neurosci.,* 2013, vol. 4 (7), 1026-1048
- **MISTRY et al.** *J. Med. Chem.,* 2013, vol. 56, 5151-5172

- Isotopic Compositions of the Elements 1997. *Pure Appl. Chem.,* 1998, vol. 70 (1), 217-235
- **H. J. LEIS et al.** *Curr. Org. Chem.,* 1998, vol. 2, 131
- **ESAKI et al.** *Tetrahedron,* 2006, vol. 62, 10954
- **ESAKI et al.** *Chem. Eur. J.,* 2007, vol. 13, 4052
- **J. R. MORANDI et al.** *J. Org. Chem.,* 1969, vol. 34 (6), 1889
- **N. H. KHAN.** *J. Am. Chem. Soc.,* 1952, vol. 74 (12), 3018
- **S. CHANDRASEKHAR et al.** *Tetrahedron,* 2011, vol. 52, 3865
- **HANZLIK et al.** *J. Org. Chem.,* 1990, vol. 55, 3992-3997
- **R. P. HANZLIK et al.** *Biochem. Biophys. Res. Commun.,* 1989, vol. 160, 844
- **P. J. REIDER et al.** *J. Org. Chem.,* 1987, vol. 52, 3326-3334
- **M. JARMAN et al.** *Carcinogenesis,* 1993, vol. 16 (4), 683-688
- **J. ATZRODT et al.** *Angew. Chem., Int. Ed.,* 2007, vol. 46, 7744
- **K. MATOISHI et al.** *J. Chem. Soc, Chem. Commun.,* 2000, 1519-1520
- **A. STREITWIESER et al.** *J. Am. Chem. Soc.,* 1963, vol. 85, 2759
- **C. L. PERRIN et al.** *J. Am. Chem. Soc.,* 2007, vol. 129, 4490
- **C. L. PERRIN et al.** *J. Am. Chem. Soc.,* 2003, vol. 125, 15008
- **C. L. PERRIN.** *Advances in Physical Organic Chemistry,* vol. 44, 144
- **C. L. PERRIN et al.** *J. Am. Chem. Soc.,* 2005, vol. 127, 9641
- **B. TESTA et al.** *Int. J. Pharm.,* 1984, vol. 19 (3), 271
- **D. J. KUSHNER et al.** *Can. J. Physiol. Pharmacol.,* 1999, vol. 77, 79
- **A. E. MUTLIB et al.** *Toxicol. Appl. Pharmacol.,* 2000, vol. 169, 102
- **A. M. SHARMA et al.** *Chem. Res.Toxicol.,* 2013, vol. 26, 410
- **UETRECHT et al.** *Chemical Research in Toxicology,* 2008, vol. 21 (9), 1862
- **A. J. MORALES et al.** *Abstract 285, The 15th North American Meeting of the International Society of Xenobiotics,* 12. Oktober 2008
- **C. J. WENTHUR et al.** *J. Med. Chem.,* 2013, vol. 56, 5208
- **F. SCHNEIDER et al.** *Arzneim. Forsch. Drug. Res.,* 2006, vol. 56, 295
- **F. MALTAIS et al.** *J. Med. Chem.,* 2009, vol. 52, 7993
- *Research Disclosure Database Number 605005,* 01. August 2014, http://www.researchdisclosure.com/searching-disclosures
- **REUVENY et al.** *Nature,* 1994, vol. 370, 143-146
- **LAZARENO.** Determination of Allosteric Interactions Using Radioligand-Binding Techniques. *Methods in Molecular Biology,* vol. 259
- **KOSTENIS ; MOHR.** Receptor Signal Transduction Protocols. *Trends Pharmacol. Sci.,* 1996, vol. 17 (8), 280-283
- **CROY et al.** *Mol. Pharmacol.,* 2014, vol. 86, 106-115
- **HULME ; TREVETHICK.** *Brit. J. Pharmacol.,* 2010, vol. 161, 1219-1237
- **CROY et al.** *Mol. Pharmacol.,* 2014, vol. 86, 106-115
- **SCHOBER et al.** *Mol. Pharmacol.,* 2014, vol. 86, 116-123
- **CHENG ; PRUSOFF.** *Biochem. Pharmacol.,* 1973, vol. 22 (23), 3099-3108
- **SCHRAGE et al.** Receptor Signal Transduction Protocols. *Biochem. Pharmacol.,* 2014, vol. 90, 307-319
- **KOSTENIS ; MOHR.** *Trends Pharmacol. Sci.,* 1996, vol. 17 (8), 280-283
- **BECKMANN ; RINNE et al.** *Cell. Physiol. Biochem.,* 2008, vol. 21, 259-268